(19) Europäisches Patentamt
European Patent Office
Office européen des brevets

(11) EP 3 722 301 B1

(12) EUROPEAN PATENT SPECIFICATION

(45) Date of publication and mention of the grant of the patent:
**01.11.2023 Bulletin 2023/44**

(21) Application number: **20168158.2**

(22) Date of filing: **06.04.2020**

(51) International Patent Classification (IPC):
***C07F 15/00*** (2006.01) ***C09K 11/07*** (2006.01)
***C09K 11/06*** (2006.01) ***H10K 85/30*** (2023.01)

(52) Cooperative Patent Classification (CPC):
**C07F 15/0033; C09K 11/06; H10K 85/342;**
C09K 2211/185

(54) **ORGANOMETALLIC COMPOUND, ORGANIC LIGHT-EMITTING DEVICE INCLUDING THE ORGANOMETALLIC COMPOUND, DIAGNOSTIC COMPOSITION INCLUDING THE ORGANOMETALLIC COMPOUND**

ORGANOMETALLISCHE VERBINDUNG, ORGANISCHE LICHTEMITTIERENDE VORRICHTUNG MIT DER ORGANOMETALLISCHEN VERBINDUNG, DIAGNOSTISCHE ZUSAMMENSETZUNG MIT DER ORGANOMETALLISCHEN VERBINDUNG

COMPOSÉ ORGANOMÉTALLIQUE, DISPOSITIF ÉLECTROLUMINESCENT ORGANIQUE LE COMPRENANT, COMPOSITION DE DIAGNOSTIC COMPRENANT LE COMPOSÉ ORGANOMÉTALLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.04.2019 KR 20190042745**

(43) Date of publication of application:
**14.10.2020 Bulletin 2020/42**

(73) Proprietor: **Samsung Electronics Co., Ltd.**
**Gyeonggi-do 16677 (KR)**

(72) Inventors:
- **LEE, Kum Hee**
  **16678 Gyeonggi-do (KR)**
- **KWAK, Yoonhyun**
  **16678 Gyeonggi-do (KR)**
- **ARATANI, Sukekazu**
  **16678 Gyeonggi-do (KR)**
- **LEE, Jiyoun**
  **16678 Gyeonggi-do (KR)**
- **CHO, Yuri**
  **16678 Gyeonggi-do (KR)**
- **CHOI, Jongwon**
  **16678 Gyeonggi-do (KR)**
- **CHOI, Whail**
  **16678 Gyeonggi-do (KR)**
- **HWANG, Kyuyoung**
  **16678 Gyeonggi-do (KR)**

(74) Representative: **Elkington and Fife LLP**
**Prospect House**
**8 Pembroke Road**
**Sevenoaks, Kent TN13 1XR (GB)**

(56) References cited:
**EP-A1- 3 560 940** **EP-A1- 3 637 489**
**US-A1- 2016 164 007**

- **LI TIAN-YI ET AL: "Rational design of phosphorescent iridium(III) complexes for emission color tunability and their applications in OLEDs", COORDINATION CHEMISTRY REVIEWS, ELSEVIER SCIENCE, AMSTERDAM, NL, vol. 374, 10 July 2018 (2018-07-10), pages 55-92, XP085443666, ISSN: 0010-8545, DOI: 10.1016/J.CCR.2018.06.014**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### FIELD OF THE INVENTION

One or more embodiments relate to an organometallic compound, an organic light-emitting device including the organometallic compound, and a diagnostic composition including the organometallic compound.

### BACKGROUND OF THE INVENTION

Organic light-emitting devices are self-emission devices, which have excellent characteristics in terms of a viewing angle, response time, brightness, driving voltage, and response speed, and produce full-color images.

In an example, an organic light-emitting device includes an anode, a cathode, and an organic layer between the anode and the cathode, wherein the organic layer includes an emission layer. A hole transport region may be between the anode and the emission layer, and an electron transport region may be between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. These excitons transit from an excited state to a ground state, thereby generating light.

Luminescent compounds may be used to monitor, sense, or detect a variety of biological materials including cells and proteins. An example of the luminescent compounds is a phosphorescent luminescent compound.

EP 3 637 489 discloses an organometallic compound including a transition metal and at least one organic ligand.

US 2016/164007 discloses organic metal compounds and organic light-emitting devices employing the same.

EP 3 560 940 discloses an organometallic compound, an organic light-emitting device including the organometallic compound, and a diagnostic composition including the organometallic compound.

T. Li et.al, in "Rational design of phosphorescent iridium(III) complexes for emission color tunability and their applications in OLEDs", Coordination Chemistry Reviews, Volume 374, 1 November 2018, Pages 55-92, provides a review on recent developments in emissive iridium(III) phosphorescent complexes and their applications in organic light-emitting diodes (OLEDs).

### SUMMARY OF THE INVENTION

Aspects of the present disclosure provide a novel organometallic compound, an organic light-emitting device including the novel organometallic compound, and a diagnostic composition including the novel organometallic compound.

Additional aspects will be set forth in part in the description which follows and, in part, will be apparent from the description, or may be learned by practice of the presented embodiments.

An aspect of the present invention provides an organometallic compound in accordance with claim 1.

Another aspect of the present invention provides an organic light-emitting device including: a first electrode; a second electrode; and an organic layer disposed between the first electrode and the second electrode and including an emission layer, wherein the organic layer includes at least one organometallic compound represented by Formula 1.

The organometallic compound included in the emission layer of the organic layer may act as a dopant.

Another aspect of the present invention provides a diagnostic composition including at least one organometallic compound represented by Formula 1.

### BRIEF DESCRIPTION OF THE DRAWING

These and/or other aspects will become apparent and more readily appreciated from the following description of the embodiments, taken in conjunction with FIGURE which is a schematic cross-sectional view of an organic light-emitting device according to an embodiment.

### DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not

modify the individual elements of the list.

It will be understood that when an element is referred to as being "on" another element, it can be directly on the other element or intervening elements may be present therebetween. In contrast, when an element is referred to as being "directly on" another element, there are no intervening elements present.

It will be understood that, although the terms "first," "second," "third" etc. may be used herein to describe various elements, components, regions, layers and/or sections, these elements, components, regions, layers and/or sections should not be limited by these terms. These terms are only used to distinguish one element, component, region, layer, or section from another element, component, region, layer, or section. Thus, "a first element," "component," "region," "layer," or "section" discussed below could be termed a second element, component, region, layer, or section without departing from the teachings herein.

The terminology used herein is for the purpose of describing particular embodiments only and is not intended to be limiting. As used herein, the singular forms "a," "an," and "the" are intended to include the plural forms, including "at least one," unless the content clearly indicates otherwise. "At least one" is not to be construed as limiting "a" or "an." "Or" means "and/or." As used herein, the term "and/or" includes any and all combinations of one or more of the associated listed items. It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom" and "upper" or "top," may be used herein to describe one element's relationship to another element as illustrated in the Figures. It will be understood that relative terms are intended to encompass different orientations of the device in addition to the orientation depicted in the Figures. For example, if the device in one of the figures is turned over, elements described as being on the "lower" side of other elements would then be oriented on "upper" sides of the other elements. The exemplary term "lower," can therefore, encompasses both an orientation of "lower" and "upper," depending on the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10%, or 5% of the stated value.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

An aspect of the present invention provides an organometallic compound represented by Formula 1 below:

Formula 1 $$M(L_1)_{n1}(L_2)_{n2}.$$

$L_1$ in Formula 1 is a ligand represented by Formula 2, and n1 in Formula 1 indicates the number of $L_1$ in Formula 1 and is 1, 2, or 3. When n1 is 2 or more, two or more $L_1$(s) are identical to or different from each other:

Formula 2

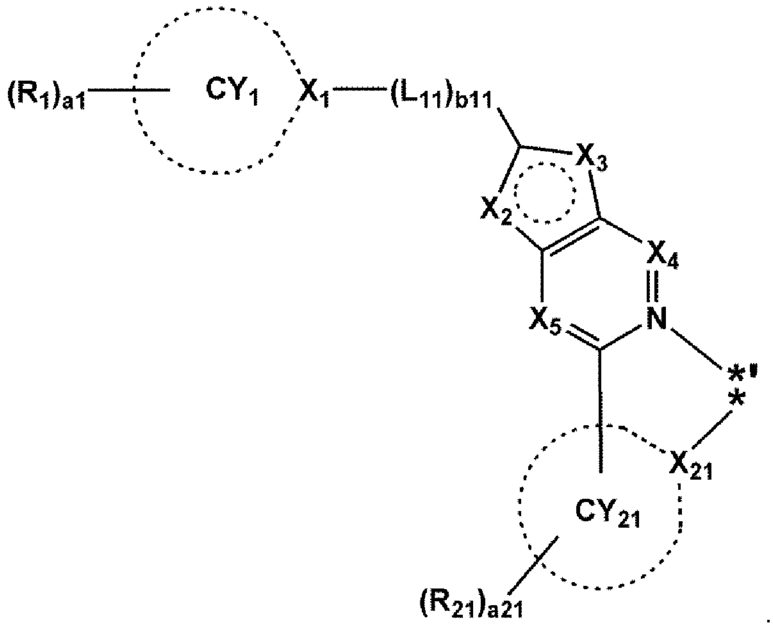

Formula 2 is the same as described below.

For example, n1 may be 1 or 2.

$L_2$ in Formula 1 is a bidentate ligand represented by Formula 3:

Formula 3

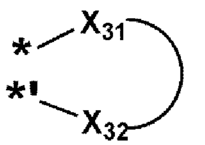

wherein, in Formula 3,

$X_{31}$ and $X_{32}$ are each O;
$X_{31}$ is O and $X_{32}$ is N; or
$X_{31}$ is N and $X_{32}$ is C,

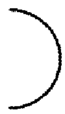

indicates any atomic group linking $X_{31}$ and $X_{32}$ to each other, and * and *' each indicate a binding site to M in Formula 1. n2 in Formula 1 indicates the number of $L_2$ and is 1, 2, 3, or 4. When n2 is 2 or more, two or more $L_2$(s) are identical to or different from each other. $L_2$ is the same as described below.

For example, n2 in Formula 1 may be 1 or 2.

In Formula 1, $L_1$ and $L_2$ are different from each other.

M is Ir or Os, and the sum of n1 and n2 is 3 or 4; or M is Pt, and the sum of n1 and n2 is 2.

In Formula 2, $X_1$ is C, N, Si, or P, and $X_{21}$ is C or N.

For example, in Formula 2, $X_{21}$ may be C.

In Formula 2, ring $CY_1$ and ring $CY_{21}$ are each independently a $C_5$-$C_{30}$ carbocyclic group or a $C_2$-$C_{30}$ heterocyclic group.

For example, ring $CY_1$ and ring $CY_{21}$ may each independently be i) a first ring, ii) a second ring, iii) a condensed ring in which at least two first rings are condensed with each other, iv) a condensed ring in which at least two second

rings are condensed with each other, or v) a condensed ring in which at least one first ring and at least one second ring are condensed with each other.

The first ring may be a cyclopentane group, a cyclopentadiene group, a furan group, a thiophene group, a pyrrole group, a silole group, an indene group, a benzofuran group, a benzothiophene group, an indole group, a benzosilole group, an oxazole group, an isoxazole group, an oxadiazole group, an isoxadiazole group, an oxatriazole group, an isoxatriazole group, a thiazole group, an isothiazole group, a thiadiazole group, an isothiadiazole group, a thiatriazole group, an isothiatriazole group, a pyrazole group, an imidazole group, a triazole group, a tetrazole group, an azasilole group, a diazasilole group, or a triazasilole group.

The second ring may be an adamantane group, a norbornane group, a norbornene group, a cyclohexane group, a cyclohexene group, a benzene group, a pyridine group, a pyrimidine group, a pyrazine group, a pyridazine group, or a triazine group.

In one embodiment, ring $CY_1$ and ring $CY_{21}$ may each independently be a cyclopentene group, a cyclohexene group, a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group, but embodiments of the present disclosure are not limited thereto.

In one or more embodiments, ring $CY_1$ may be a cyclopentane group, a cyclohexane group, a cycloheptane group, a cyclooctane group, an adamantane group, a norbornane group, a norbornene group, a cyclopentene group, a cyclohexene group, a cycloheptene group, a benzene group, a naphthalene group, a fluorene group, a phenanthrene group, an anthracene group, a fluoranthene group, a triphenylene group, a pyrene group, a chrysene group, a pyrrole group, a thiophene group, a furan group, an imidazole group, a pyrazole group, a thiazole group, an isothiazole group, an oxazole group, an isoxazole group, a pyridine group, a pyrazine group, a pyrimidine group, a pyridazine group, an isoindole group, an indole group, an indazole group, a purine group, a quinoline group, an isoquinoline group, a benzoquinoline group, a quinoxaline group, a quinazoline group, a cinnoline group, a carbazole group, a phenanthroline group, a benzimidazole group, a benzofuran group, a benzothiophene group, an isobenzothiazole group, a benzoxazole group, an isobenzoxazole group, a triazole group a tetrazole group, an oxadiazole group, a triazine group, a dibenzofuran group, a dibenzothiophene group, a dibenzosilole group, a benzocarbazole group, a dibenzocarbazole group, an imidazopyridine group, or an imidazopyrimidine group, but embodiments of the present disclosure are not limited thereto.

In Formula 2, $X_2$ and $X_3$ are each independently O, S, Se, or $C(R_2)$, wherein $X_2$ or $X_3$ is O, S, or Se.

For example, in Formula 2, i) $X_2$ may be O, S, or Se, and $X_3$ may be $C(R_2)$; or ii) $X_2$ may be $C(R_2)$, and $X_3$ may be O, S, or Se.

In Formula 2, $X_4$ is N or $C(R_4)$, and $X_5$ is N or $C(R_5)$.

For example, $X_4$ may be $C(R_4)$, and $X_5$ may be $C(R_5)$.

In Formula 2, $R_1$, $R_2$, $R_4$, $R_5$, and $R_{21}$ are each independently hydrogen, deuterium, -F, -Cl, -Br, -I, -SFs, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_2$-$C_{10}$ heterocycloalkyl group, a substituted or unsubstituted $C_3$-$C_{10}$ cycloalkenyl group, a substituted or unsubstituted $C_2$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted monovalent non-aromatic condensed polycyclic group, a substituted or unsubstituted monovalent non-aromatic condensed heteropolycyclic group, $-N(Q_1)(Q_2)$, $-Si(Q_3)(Q_4)(Q_5)$, $-B(Q_6)(Q_7)$, $-P(=O)(Q_8)(Q_9)$ or $-P(Q_8)(Q_9)$, wherein $Q_1$ to $Q_9$ are each the same as described

above.

For example, $R_1$, $R_2$, $R_4$, $R_5$, $R_{21}$, and $R_{10a}$ may each independently be:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SFs, a $C_1$-$C_{20}$ alkyl group, or a $C_1$-$C_{20}$ alkoxy group;

a $C_1$-$C_{20}$ alkyl group or a $C_1$-$C_{20}$ alkoxy group, each substituted with deuterium, - F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a cyclopentyl group, a cyclohexyl group, a cycloheptyl group, a cycloctyl group, an adamantyl group, a norbornenyl group, a cyclopentenyl group, a cyclohexenyl group, a cycloheptenyl group, a bicyclo[1 .1.1]pentyl group, a bicyclo[2.1.1]hexyl group, a bicyclo[2.2.1]heptyl group, a bicyclo[2.2.2]octyl group, a phenyl group, a biphenyl group, a terphenyl group, a naphthyl group, a pyridinyl group, a pyrimidinyl group, or any combination thereof;

a cyclopentyl group, a cyclohexyl group, a cycloheptyl group, a cycloctyl group, an adamantyl group, a norbornenyl group, a cyclopentenyl group, a cyclohexenyl group, a cycloheptenyl group, a cyclooctenyl group, a bicyclo[1.1.1]pentyl group, a bicyclo[2.1 .1]hexyl group, a bicyclo[2.2.1]heptyl group, a bicyclo[2.2.2]octyl group, a phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a cyclopentyl group, a cyclohexyl group, a cycloheptyl group, a cycloctyl group, an adamantyl group, a norbornenyl group, a cyclopentenyl group, a cyclohexenyl group, a cycloheptenyl group, a cyclooctenyl group, a bicyclo[1 .1.1]pentyl group, a bicyclo[2.1.1]hexyl group, a bicyclo[2.2.1]heptyl group, a bicyclo[2.2.2]octyl group, a phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, $-Si(Q_{33})(Q_{34})(Q_{35})$, or any combination thereof; or

$-N(Q_1)(Q_2)$, $-Si(Q_3)(Q_4)(Q_5)$, $-B(Q_6)(Q_7)$, $-P(=O)(Q_8)(Q_9)$, or $-P(Q_8)(Q_9)$, and

$Q_1$ to $Q_9$ and $Q_{33}$ to $Q_{35}$ are each independently:

$-CH_3$, $-CD_3$, $-CD_2H$, $-CDH_2$, $-CH_2CH_3$, $-CH_2CD_3$, $-CH_2CD_2H$, $-CH_2CDH_2$, $- CHDCH_3$, $-CHDCD_2H$, $-CHDCDH_2$, $-CHDCD_3$, $-CD_2CD_3$, $-CD_2CD_2H$, or $-CD_2CDH_2$; or

an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group, each unsubstituted or substituted with deuterium, a $C_1$-$C_{10}$ alkyl group, a phenyl group, or any combination thereof,

but embodiments of the present disclosure are not limited thereto.

In Formula 2, a1 and a21 each indicate the number of $R_1$ and the number of $R_{21}$, respectively, and are each independently an integer from 0 to 20 (for example, an integer from 0 to 10 or an integer from 0 to 5). When a1 is 2 or more, two or more $R_1$(s) may be identical to or different from each other, and when a21 is 2 or more, two or more $R_{21}$(s) may be identical to or different from each other.

In Formula 2, ring $CY_1$ and $R_2$ are not linked to each other, and $R_1$ and $R_2$ are not linked to each other.

In one embodiment, a group represented by

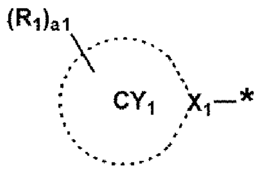

in Formula 2 may be a $C_2$-$C_{10}$ cycloalkenyl group, a $C_2$-$C_{10}$ heterocycloalkenyl group, a $C_6$-$C_{30}$ aryl group, a $C_2$-$C_{30}$ heteroaryl group, a monovalent non-aromatic condensed polycyclic group, or a monovalent non-aromatic condensed heteropolycyclic group, each unsubstituted or substituted with $R_1$(s) in the number of a1.

In Formula 2, $R_1$ and $R_2$ may each independently be:

hydrogen, deuterium, -F, -Cl, -Br, -I, a cyano group, or -SFs; or

a $C_1$-$C_{60}$ alkyl group, a $C_1$-$C_{60}$ alkoxy group, a $C_3$-$C_{10}$ cycloalkyl group, a $C_2$-$C_{10}$ heterocycloalkyl group, a $C_3$-$C_{10}$ cycloalkenyl group, or a $C_2$-$C_{10}$ heterocycloalkenyl group, each unsubstituted or substituted with deuterium, -F, -Cl, -Br, -I, $-CD_3$, $-CD_2H$, - $CDH_2$, $-CF_3$, $-CF_2H$, $-CFH_2$, a cyano group, a $C_1$-$C_{20}$ alkyl group, a cyclopentyl group, a cyclohexyl group, a cycloheptyl group, a cycloctyl group, an adamantyl group, a norbornyl group, a norbornenyl group, a cyclopentenyl group, a cyclohexenyl group, a cycloheptenyl group, a phenyl group, a biphenyl group, a naphthyl group, a pyridinyl group, a pyrimidinyl group, or any combination thereof, and

a1 may be an integer from 0 to 10.

Detailed descriptions of a1, $R_1$, a $C_1$-$C_{60}$ alkyl group, a $C_1$-$C_{60}$ alkoxy group, a $C_3$-$C_{10}$ cycloalkyl group, a $C_2$-$C_{10}$ heterocycloalkyl group, a $C_3$-$C_{10}$ cycloalkenyl group, a $C_2$-$C_{10}$ heterocycloalkenyl group, a $C_6$-$C_{30}$ aryl group, a $C_2$-$C_{30}$ heteroaryl group, a monovalent non-aromatic condensed polycyclic group, and a monovalent non-aromatic condensed heteropolycyclic group are the same as described above.

In one or more embodiments, a group represented by

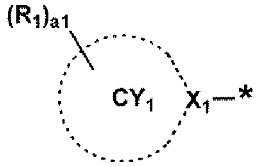

in Formula 2 may be a phenyl group, a biphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group, each unsubstituted or substituted with $R_1$(s) in the number of a1.

In Formula 2, $R_1$ and $R_2$ may each independently be:

hydrogen, deuterium, -F, -Cl, -Br, -I, a cyano group, or -SFs; or

a methyl group, an ethyl group, a propyl group, an n-butyl group, an isobutyl group, a sec-butyl group, a tert-butyl group, an n-pentyl group, an isopentyl group, a sec-pentyl group, a tert-pentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonanyl group (an n-nonyl group), an isononanyl group (an isononyl group), a sec-nonanyl group (a sec-nonyl group), a tert-nonanyl group (a tert-nonyl group), an n-decanyl group, an isodecanyl group, a sec-decanyl group, a tert-decanyl group, a $C_1$-$C_{10}$ alkoxy, a cyclopentyl group, a cyclohexyl group, a cycloheptyl group, a cycloctyl group, an adamantyl group, a norbornanyl group, a norbornenyl group, a cyclopentenyl group, a cyclohexenyl group or a cycloheptenyl group, each unsubstituted or substituted with deuterium, -F, -Cl, -Br, -I, $-CD_3$, $-CD_2H$, $-CDH_2$, $-CF_3$, $-CF_2H$, $-CFH_2$, a cyano group, a $C_1$-$C_{20}$ alkyl group, a cyclopentyl group, a cyclohexyl group, a cycloheptyl group, a cycloctyl group, an adamantyl group, a norbornyl group, a norbornenyl group, a cyclopentenyl group, a cyclohexenyl group, a cycloheptenyl group, a phenyl group, a biphenyl group, a naphthyl group, a pyridinyl group, a pyrimidinyl group, or any combination thereof, and

a1 may be an integer from 0 to 5, but embodiments of the present disclosure are not limited thereto.

In one or more embodiments, a group represented by

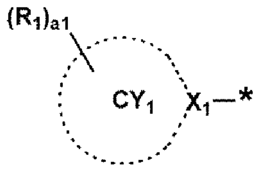

in Formula 2 may be a group represented by one of Formulae 10-13(1) to 10-13(18) and 10-13:

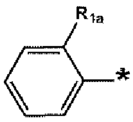
10-13(1)

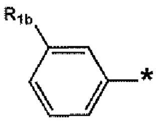
10-13(2)

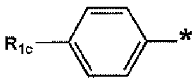
10-13(3)

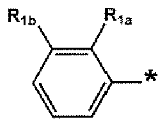
10-13(4)

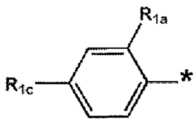
10-13(5)

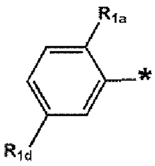
10-13(6)

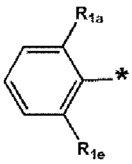
10-13(7)

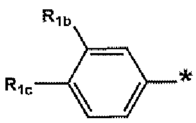
10-13(8)

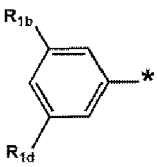
10-13(9)

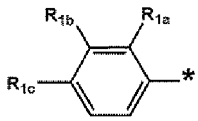
10-13(10)

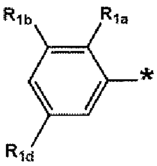
10-13(11)

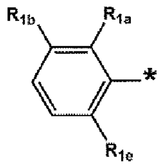
10-13(12)

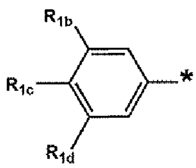
10-13(13)

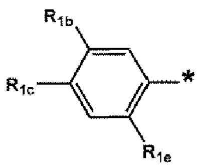
10-13(14)

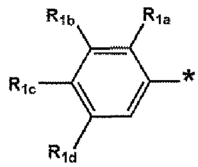
10-13(15)

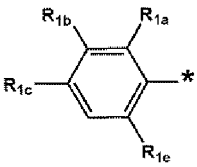
10-13(16)

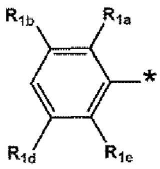
10-13(17)

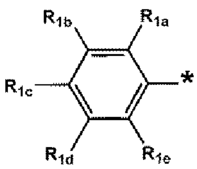
10-13(18)

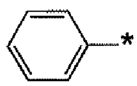
10-13 .

In Formulae 10-13(1) to 10-13(18) and 10-13, $R_{1a}$ to $R_{1e}$ are each independently the same as defined in connection with $R_1$, wherein $R_{1a}$ to $R_{1e}$ are not each hydrogen, and * indicates a binding site to a neighboring atom (e.g., carbon atom).

For example, a group represented by

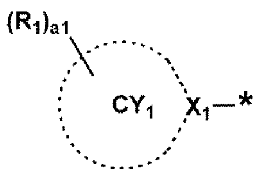

in Formula 2 may be a group represented by one of Formulae 10-13 to 10-240, wherein $R_1$, $R_2$, $R_4$, $R_5$, $R_{21}$, and $R_{10a}$ may each independently be hydrogen, deuterium, -F, a cyano group, a nitro group, -SFs, $-CH_3$, $-CD_3$, $-CD_2H$, $-CDH_2$, $-CF_3$, $-CF_2H$, $-CFH_2$, a group represented by Formulae one of 9-1 to 9-19, a group represented by one of Formulae 10-1 to 10-240, or $-Si(Q_3)(Q_4)(Q_5)$ (wherein $Q_3$ to $Q_5$ are the same as described above), but embodiments of the present disclosure are not limited thereto:

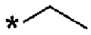
9-1
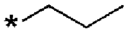
9-2
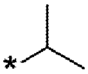
9-3
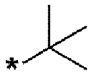
9-4
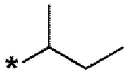
9-5
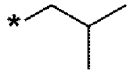
9-6
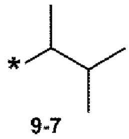
9-7
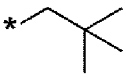
9-8
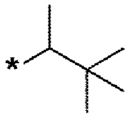
9-9
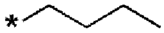
9-10
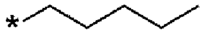
9-11
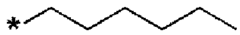
9-12
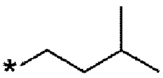
9-13
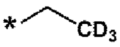
9-14
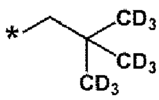
9-15
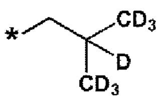
9-16
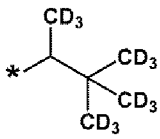
9-17
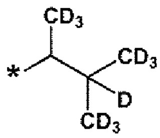
9-18
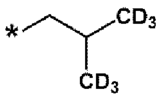
9-19
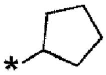
10-1
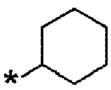
10-2
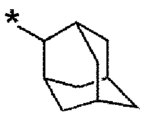
10-3
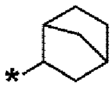
10-4
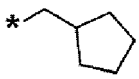
10-5
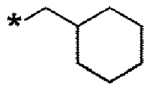
10-6
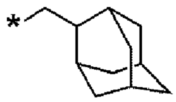
10-7
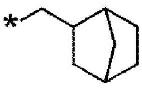
10-8
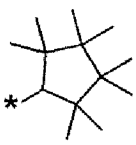
10-9
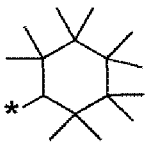
10-10
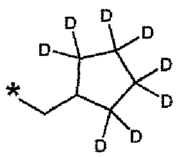
10-11
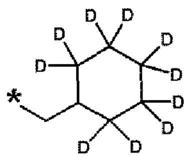
10-12
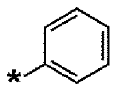
10-13
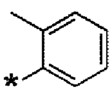
10-14
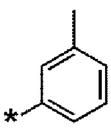
10-15
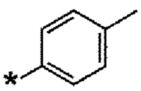
10-16
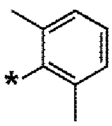
10-17
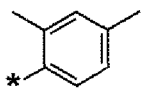
10-18
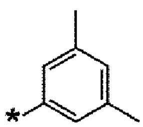
10-19
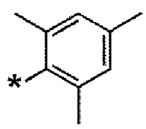
10-20

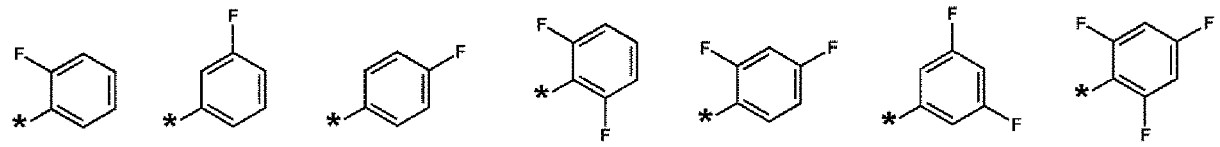

10-21 10-22 10-23 10-24 10-25 10-26 10-27

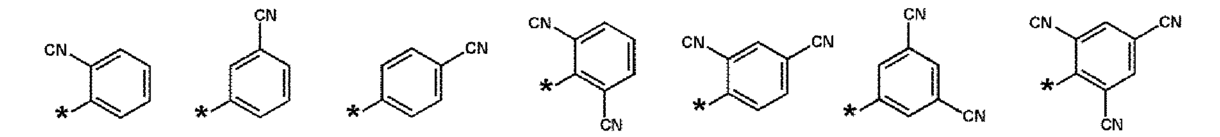

10-28 10-29 10-30 10-31 10-32 10-33 10-34

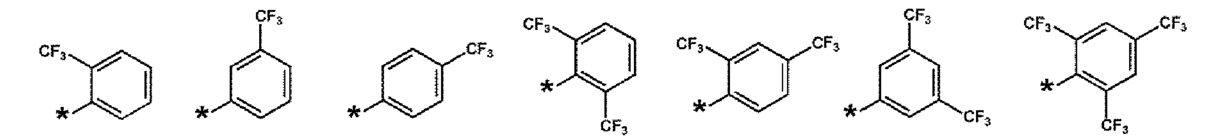

10-35 10-36 10-37 10-38 10-39 10-40 10-41

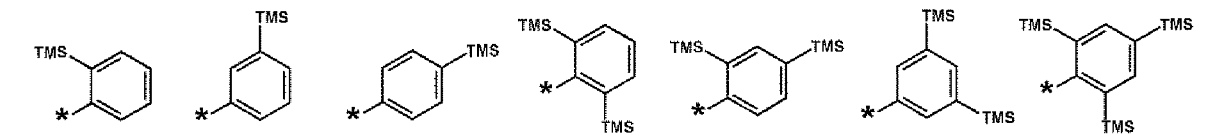

10-42 10-43 10-44 10-45 10-46 10-47 10-48

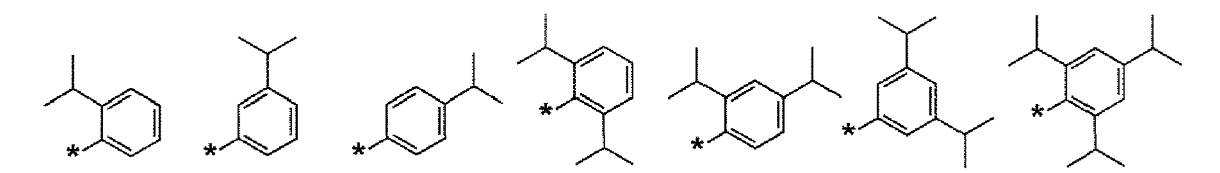

10-49 10-50 10-51 10-52 10-53 10-54 10-55

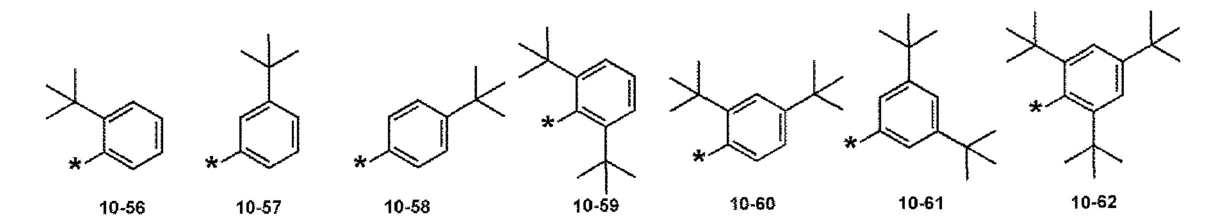

10-56 10-57 10-58 10-59 10-60 10-61 10-62

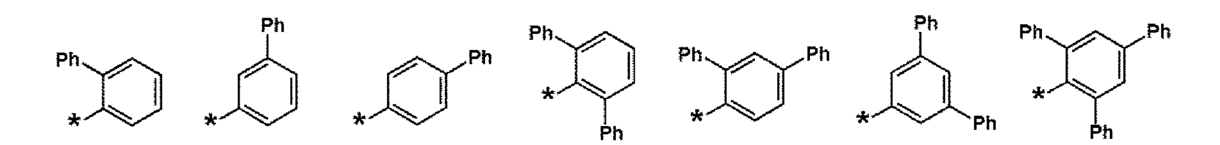

10-63 10-64 10-65 10-66 10-67 10-68 10-69

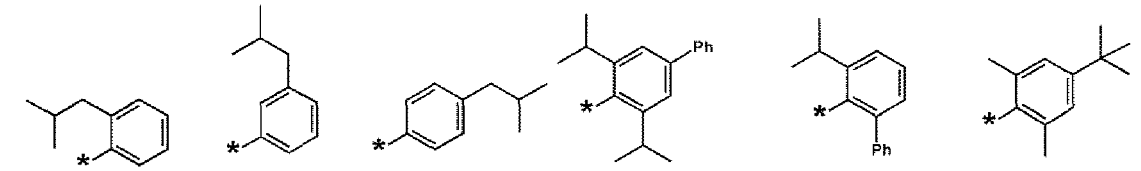

10-70 10-71 10-72 10-73 10-74 10-75

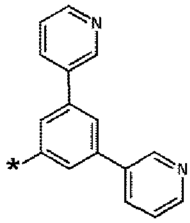
10-76

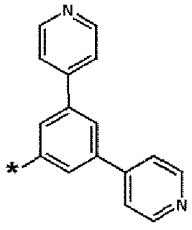
10-77

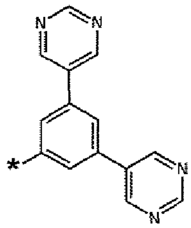
10-78

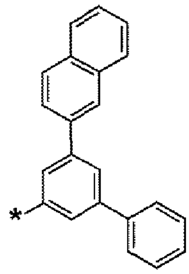
10-79

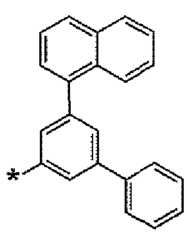
10-80

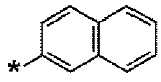
10-81

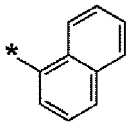
10-82

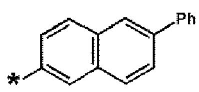
10-83

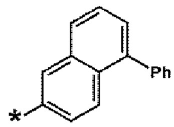
10-84

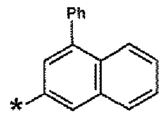
10-85

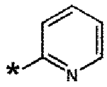
10-86

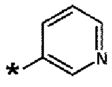
10-87

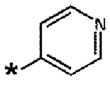
10-88

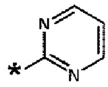
10-89

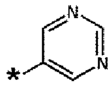
10-90

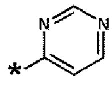
10-91

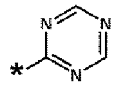
10-92

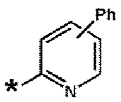
10-93

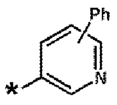
10-94

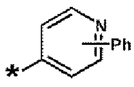
10-95

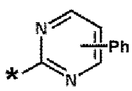
10-96

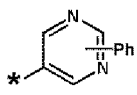
10-97

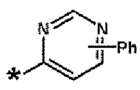
10-98

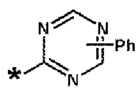
10-99

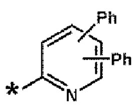
10-100

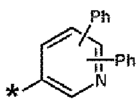
10-101

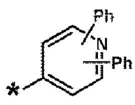
10-102

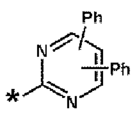
10-103

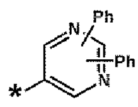
10-104

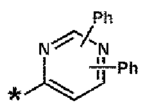
10-105

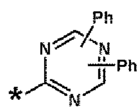
10-106

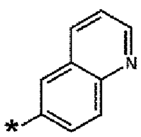
10-107

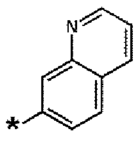
10-108

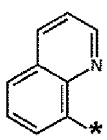
10-109

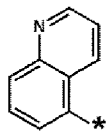
10-110

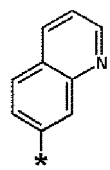
10-111

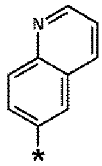
10-112

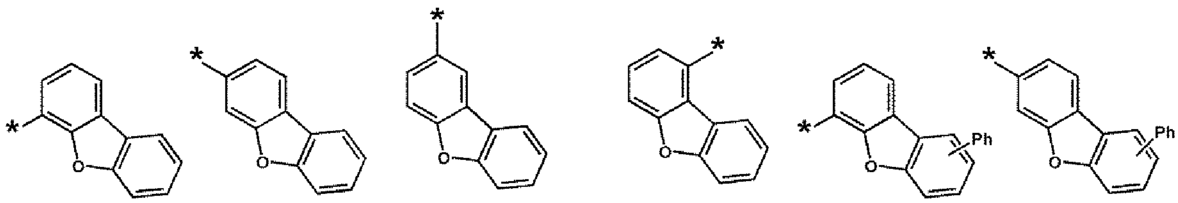
10-113

10-114

10-115

10-116

10-117

10-118

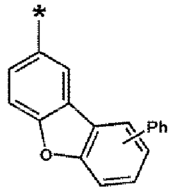
10-119

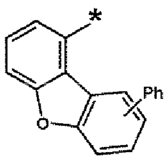
10-120

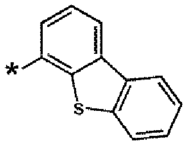
10-121

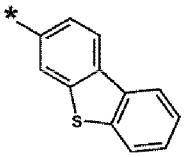
10-122

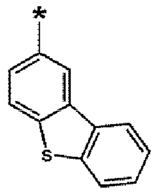
10-123

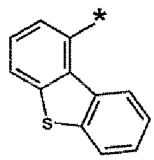
10-124

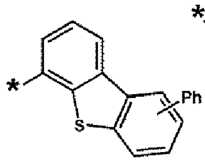
10-125

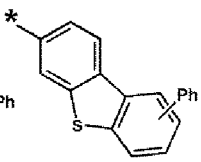
10-126

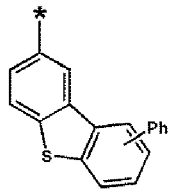
10-127

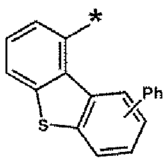
10-128

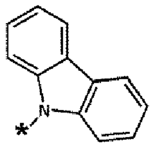
10-129

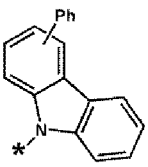
10-130

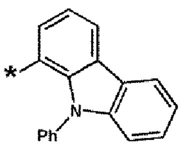
10-131

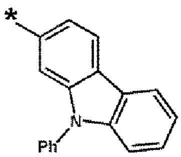
10-132

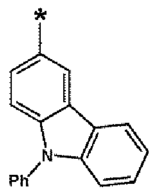
10-133

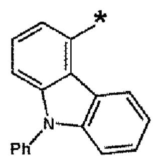
10-134

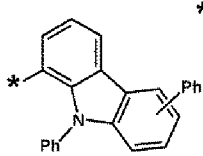
10-135

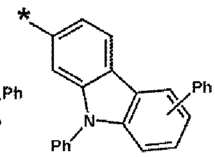
10-136

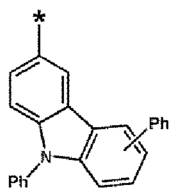
10-137

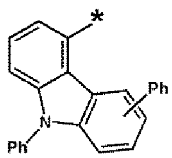
10-138

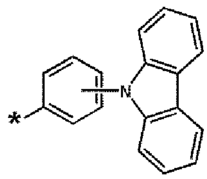
10-139

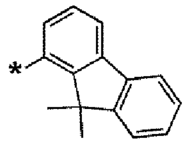
10-140

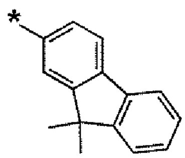
10-141

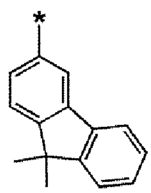
10-142

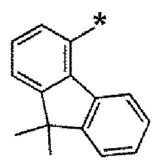
10-143

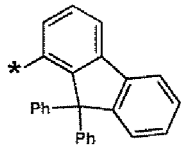
10-144

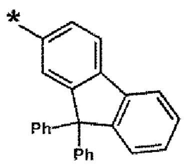
10-145

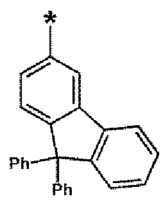
10-146

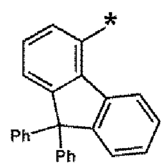
10-147

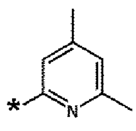
10-148

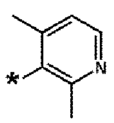
10-149

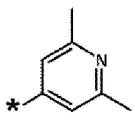
10-150

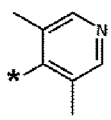
10-151

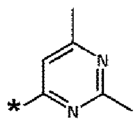
10-152

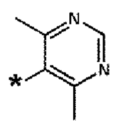
10-153

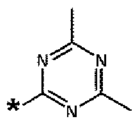
10-154

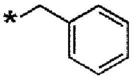 10-155
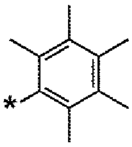 10-156
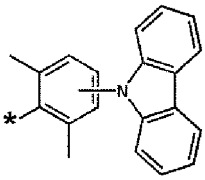 10-157
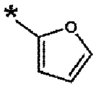 10-158
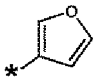 10-159
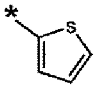 10-160
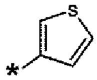 10-161

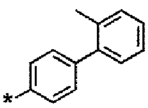 10-162
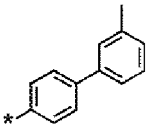 10-163
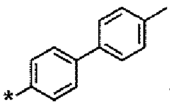 10-164
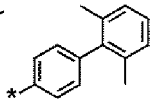 10-165
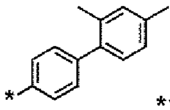 10-166
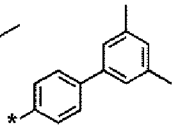 10-167
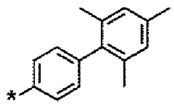 10-168

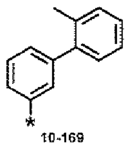 10-169
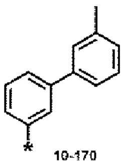 10-170
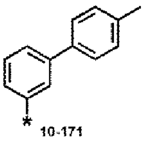 10-171
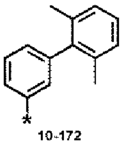 10-172
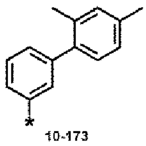 10-173
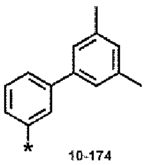 10-174
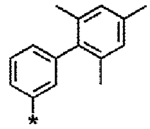 10-175

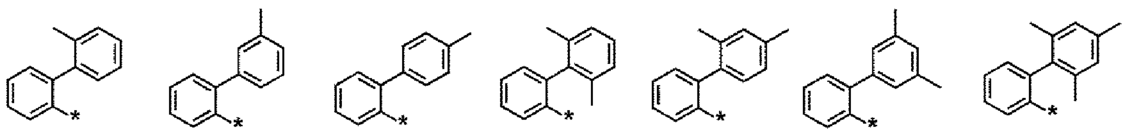

10-176 10-177 10-178 10-179 10-180 10-181 10-182

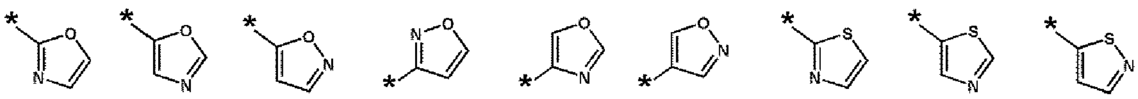

10-183 10-184 10-185 10-186 10-187 10-188 10-189 10-190 10-191

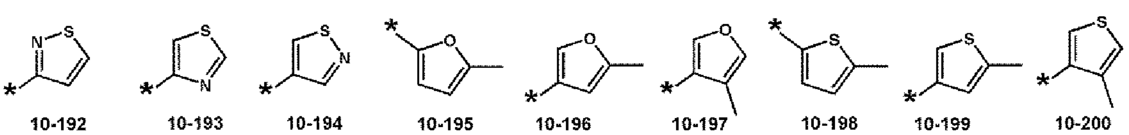

10-192 10-193 10-194 10-195 10-196 10-197 10-198 10-199 10-200

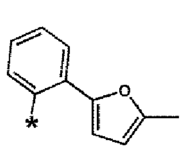 10-201
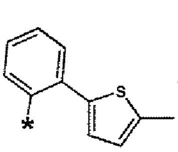 10-202
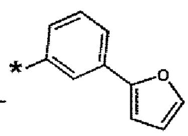 10-203
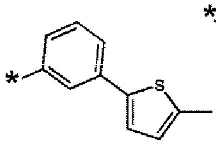 10-204
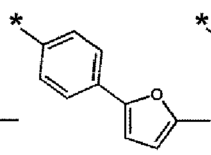 10-205
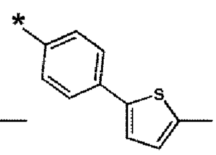 10-206

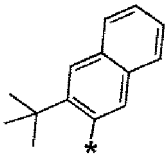 10-207
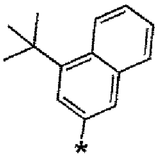 10-208
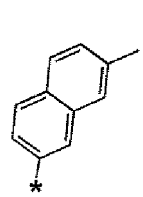 10-209
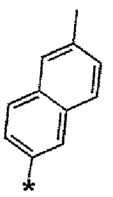 10-210
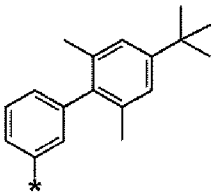 10-211
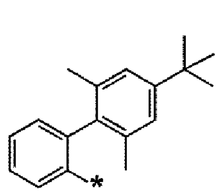 10-212

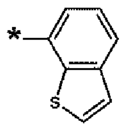
10-213
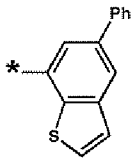
10-214
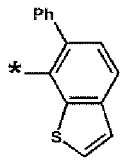
10-215
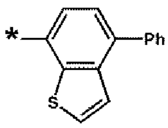
10-216
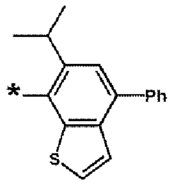
10-217
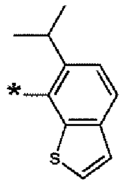
10-218
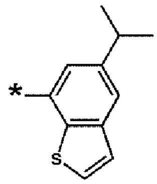
10-219

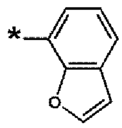
10-220
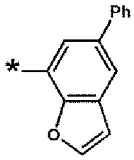
10-221
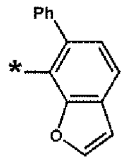
10-222
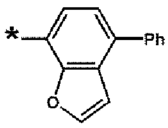
10-223
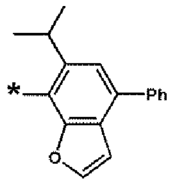
10-224
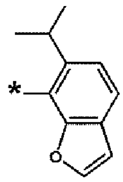
10-225
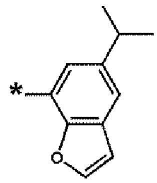
10-226

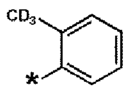
10-227
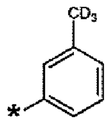
10-228
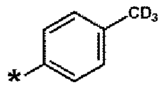
10-229
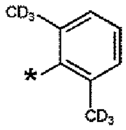
10-230
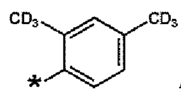
10-231
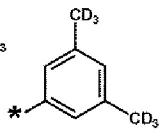
10-232
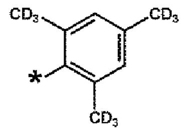
10-233

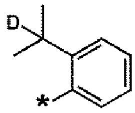
10-234
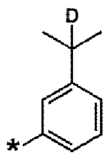
10-235
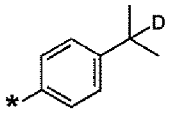
10-236
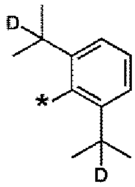
10-237
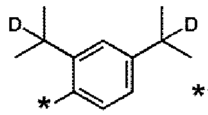
10-238
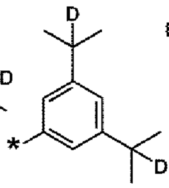
10-239
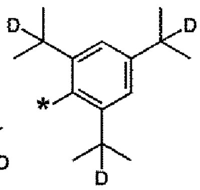
10-240
.

In Formulae 9-1 to 9-19 and 10-1 to 10-240, * indicates a binding site to a neighboring atom, Ph indicates a phenyl group, and TMS indicates a trimethylsilyl group.

In Formula 2, $L_{11}$ may be a $C_5$-$C_{30}$ carbocyclic group that is unsubstituted or substituted with at least one $R_{10a}$ or a $C_2$-$C_{30}$ heterocyclic group that is unsubstituted or substituted with at least one $R_{10a}$, wherein $R_{10a}$ is the same as described above.

For example, $L_{11}$ may be a benzene group, a naphthalene group, an anthracene group, a phenanthrene group, a triphenylene group, a pyrene group, a chrysene group, a cyclopentadiene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, or a benzothiadiazole group, each unsubstituted or substituted with at least one $R_{10a}$, but embodiments of the present disclosure are not limited thereto.

In Formula 2, b11 indicates the number of $L_{11}$, and may be an integer from 0 to 10, wherein, when b11 is 0, a

group represented by *-$(L_{11})_{b11}$-*' may be a single bond, and when b11 is 2 or more, two or more $L_{11}$(s) may be identical to or different from each other. For example, b11 may be 0, 1, 2, or 3, but embodiments of the present disclosure are not limited thereto.

In Formula 2, two of a plurality of neighboring $R_{21}$(s) may optionally be linked to form a $C_5$-$C_{30}$ carbocyclic group that is unsubstituted or substituted with at least one $R_{10a}$ or a $C_2$-$C_{30}$ heterocyclic group that is unsubstituted or substituted with at least one $R_{10a}$ (for example, a benzene group, a cyclopentane group, a cyclopentadiene group, a furan group, a thiophene group, a pyrrole group, a silole group, an indene group, a benzofuran group, a benzothiophene group, an indole group, or a benzosilole group, each unsubstituted or substituted with at least one $R_{10a}$, wherein $R_{10a}$ is the same as defined in connection with $R_{21}$). Detailed descriptions of a $C_5$-$C_{30}$ carbocyclic group and a $C_2$-$C_{30}$ heterocyclic group are the same as described above.

In Formula 2, * and *' each indicate a binding site to M in Formula 1.

In one embodiment, a group represented by

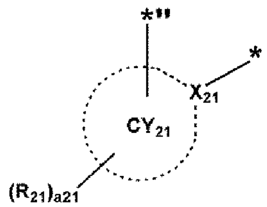

in Formula 2 may be a group represented by one of Formulae CY21-1 to CY21-25:

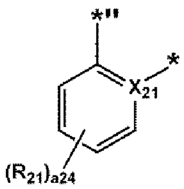

CY21-1

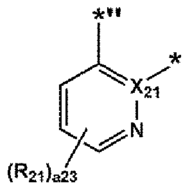

CY21-2

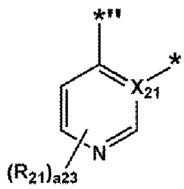

CY21-3

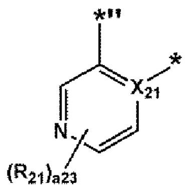

CY21-4

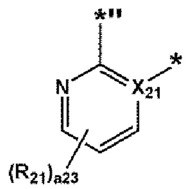

CY21-5

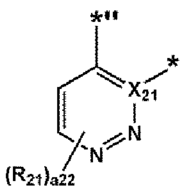

CY21-6

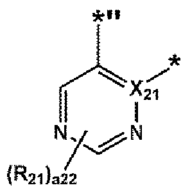

CY21-7

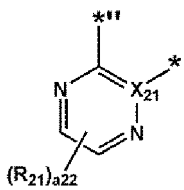

CY21-8

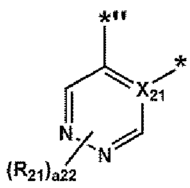

CY21-9

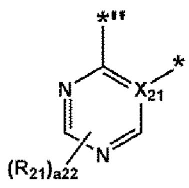

CY21-10

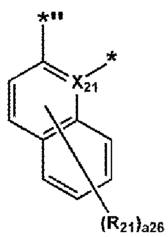

CY21-11

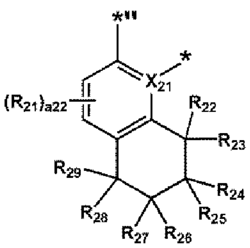

CY21-12

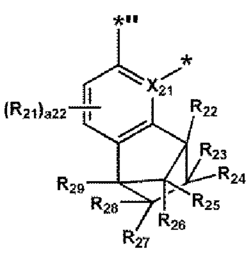

CY21-13

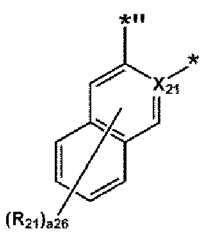

CY21-14

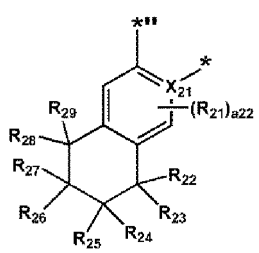

CY21-15

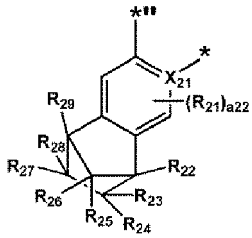
CY21-16

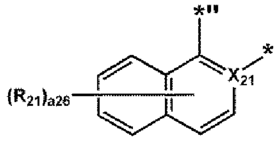
CY21-17

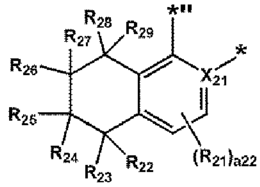
CY21-18

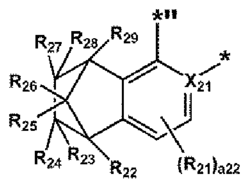
CY21-19

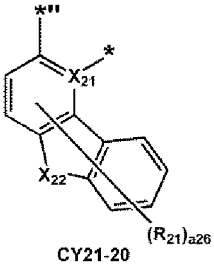
CY21-20

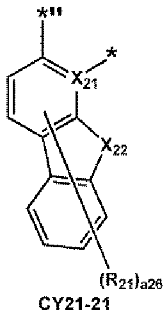
CY21-21

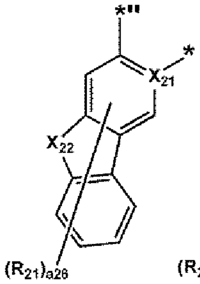
CY21-22

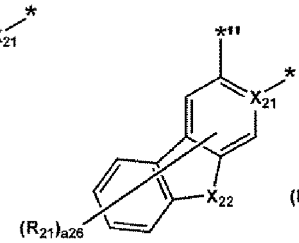
CY21-23

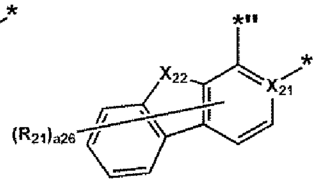
CY21-24

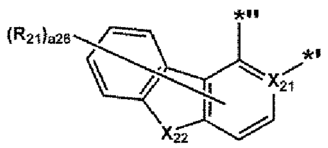
CY21-25

In Formulae CY21-1 to CY21-25,

$X_{21}$ and $R_{21}$ may each independently be the same as described herein,
$X_{22}$ may be $C(R_{22})(R_{23})$, $N(R_{22})$, O, S, or $Si(R_{22})(R_{23})$,
$R_{22}$ to $R_{29}$ may each independently be the same as defined in connection with $R_{21}$,
a26 may be an integer from 0 to 6,
a24 may be an integer from 0 to 4,
a23 may be an integer from 0 to 3,
a22 may be an integer from 0 to 2,
*" indicates a binding site to a carbon atom of a neighboring 6-membered ring in Formula 2, and
* indicates a binding site to M in Formula 1.

In one embodiment, a group represented by

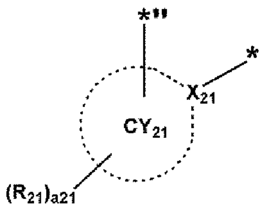

in Formula 2 may be a group represented by one of Formulae CY21(1) to CY21(56) or a group represented by Formulae one of CY21-20 to CY21-25:

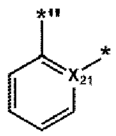
CY21(1)
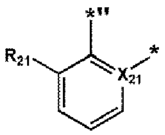
CY21(2)
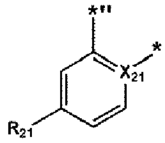
CY21(3)
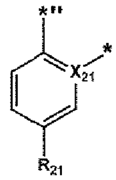
CY21(4)
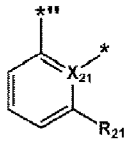
CY21(5)
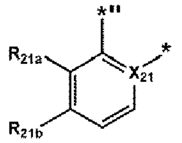
CY21(6)

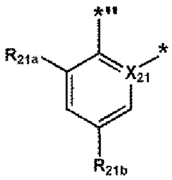
CY21(7)
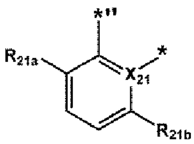
CY21(8)
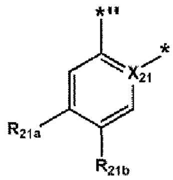
CY21(9)
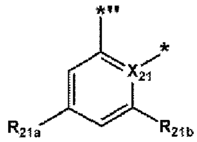
CY21(10)
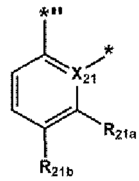
CY21(11)
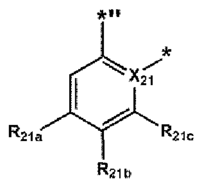
CY21(12)

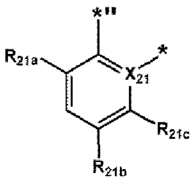
CY21(13)
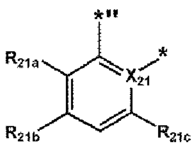
CY21(14)
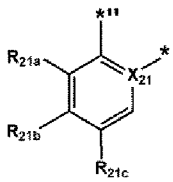
CY21(15)
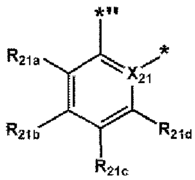
CY21(16)
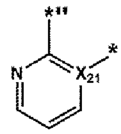
CY21(17)
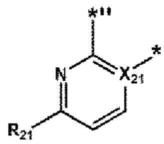
CY21(18)

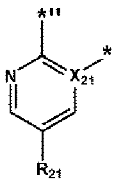
CY21(19)
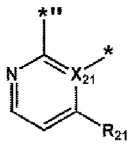
CY21(20)
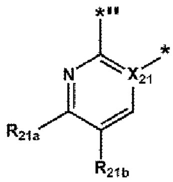
CY21(21)
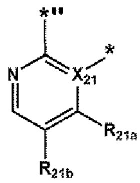
CY21(22)
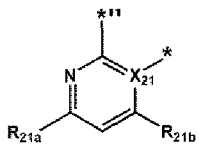
CY21(23)
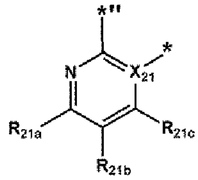
CY21(24)

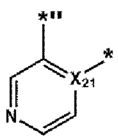
CY21(25)
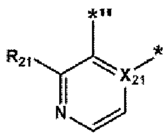
CY21(26)
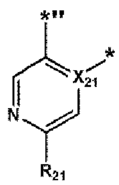
CY21(27)
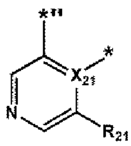
CY21(28)
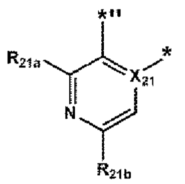
CY21(29)
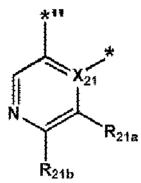
CY21(30)

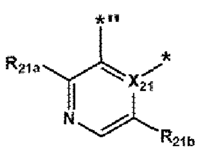
CY21(31)
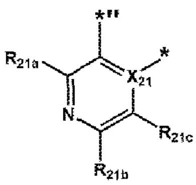
CY21(32)
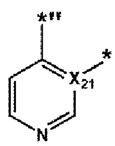
CY21(33)
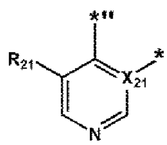
CY21(34)
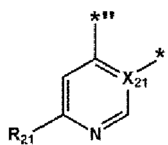
CY21(35)
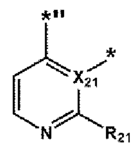
CY21(36)

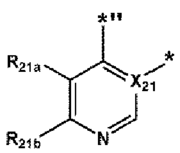
CY21(37)
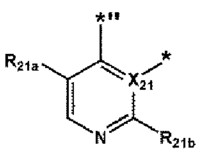
CY21(38)
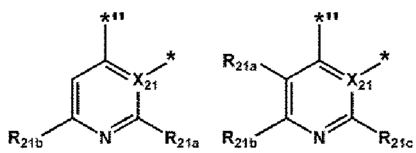
CY21(39)
CY21(40)
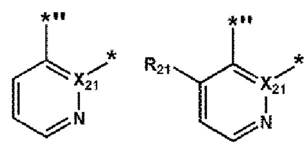
CY21(41)
CY21(42)

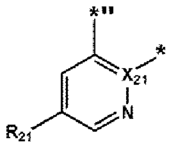
CY21(43)

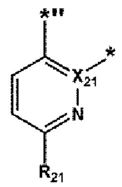
CY21(44)

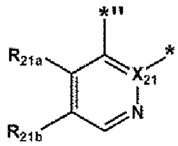
CY21(45)

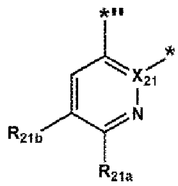
CY21(46)

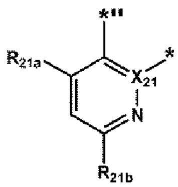
CY21(47)

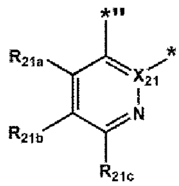
CY21(48)

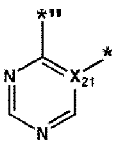
CY21(49)

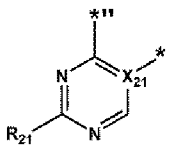
CY21(50)

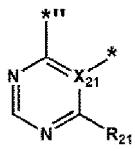
CY21(51)

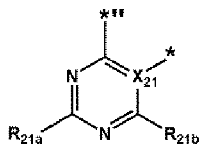
CY21(52)

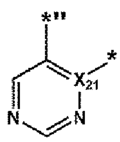
CY21(53)

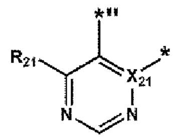
CY21(54)

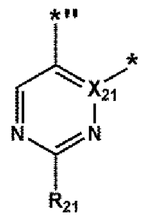
CY21(55)

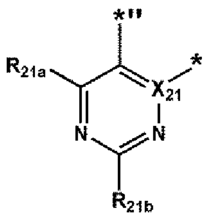
CY21(56)

.

In Formulae $CY_{21}$ (1) to $CY_{21}$ (56),

$X_{21}$ and $R_{21}$ may each independently be the same as described herein,
$R_{21a}$ to $R_{21d}$ may each independently be the same as defined in connection with $R_{21}$, and $R_{21}$ and $R_{21a}$ to $R_{21d}$ are not each hydrogen,
*" indicates a binding site to a carbon atom of a neighboring 6-membered ring in Formula 2, and
* indicates a binding site to M in Formula 1.

In one embodiment, a group represented by

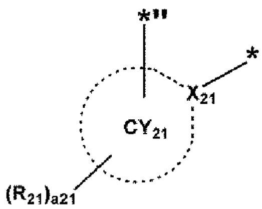

in Formula 2 may be a group represented by Formulae CY21(1), CY21(3), or CY21(10) or a group represented by one of Formulae CY21-20 to CY21-25, but embodiments of the present disclosure are not limited thereto. For example, in Formula $CY_{21}$ (10), $R_{21a}$ and $R_{21b}$ may be identical to or different from each other. In one embodiment, in Formula $CY_{21}$ (10), $R_{21a}$ and $R_{21b}$ may be different from each other, and the number of carbon atoms included in $R_{21a}$ may be larger than the number of carbon atoms included in $R_{21b}$.

In one embodiment, in Formula 1, $L_1$ may be a ligand represented by Formula 2A or 2B, but embodiments of the present disclosure are not limited thereto:

Formula 2A

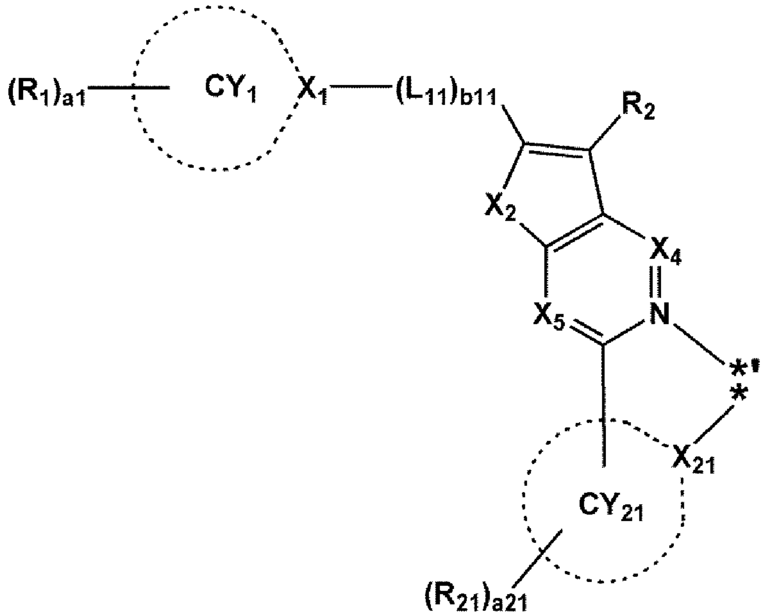

Formula 2B

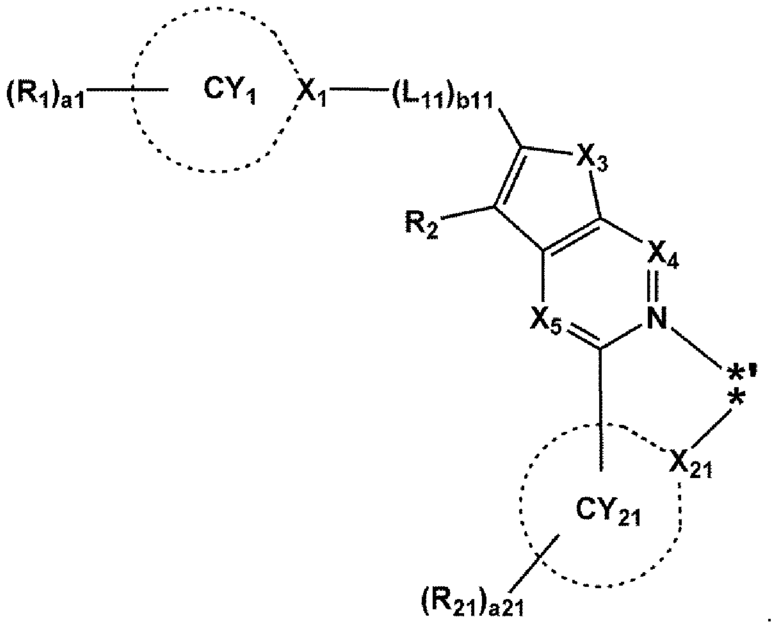

In Formulae 2A and 2B, $X_1$, $X_{21}$, ring $CY_1$, ring $CY_{21}$, $X_4$, $X_5$, $R_1$, $R_2$, $R_{21}$, a1, a21, $L_{11}$, b11, *, and *' may each independently be the same as described herein, wherein $X_2$ and $X_3$ may each independently be O, S, or Se.

In Formula 1, $L_2$ may be a bidentate ligand linked to M of Formula 1 via O, S, N, C, P, Si, or As.

In one embodiment, in Formula 3, i) $X_{31}$ and $X_{32}$ may each be O; ii) $X_{31}$ may be O, and $X_{32}$ may be N, or iii) $X_{31}$ may be N, and $X_{32}$ may be C, but embodiments of the present disclosure are not limited thereto.

In one or more embodiments, in Formula 1, $L_2$ may be oxalate, acetylacetonate, picolinic acid, or glycinate, but embodiments of the present disclosure are not limited thereto.

In one or more embodiments, in Formula 1, $L_2$ may be a group represented by one of Formulae 3A to 3D:

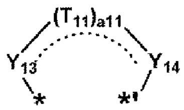
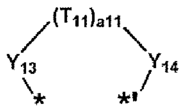
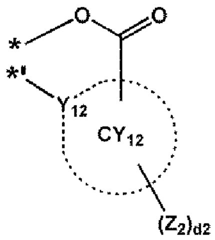
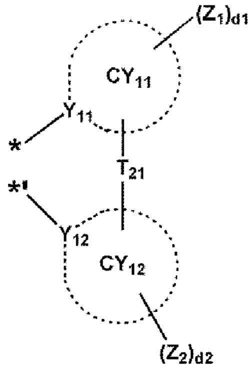

3A 3B 3C 3D .

In Formulae 3A to 3D,

$Y_{13}$ may be O, N, or $N(Z_1)$,
$Y_{14}$ may be O, N, or $N(Z_3)$,
$T_{11}$ may be a single bond, a double bond, $*-C(Z_{11})(Z_{12})-*'$, $*-C(Z_{11})=C(Z_{12})-*'$, $*=C(Z_{11})-*'$, $*-C(Z_{11})=*'$, $*=C(Z_{11})-C(Z_{12})=C(Z_{13})-*'$, $*-C(Z_{11})=C(Z_{12})-C(Z_{13})=*'$, $*-N(Z_{11})-*'$, or a $C_5$-$C_{30}$ carbocyclic group that is unsubstituted or substituted with at least one $Z_{11}$,
a11 may be an integer from 1 to 10,

$Y_{11}$ and $Y_{12}$ may each independently be C or N,
$T_{21}$ may be a single bond, a double bond, O, S, $C(Z_{11})(Z_{12})$, $Si(Z_{11})(Z_{12})$, or $N(Z_{11})$,
ring $CY_{11}$ and ring $CY_{12}$ may each independently be a $C_5$-$C_{30}$ carbocyclic group or a $C_2$-$C_{30}$ heterocyclic group,
$Z_1$ to $Z_3$ and $Z_{11}$ to $Z_{13}$ may each independently be the same as defined in connection with $R_{21}$,
d1 and d2 may each independently be an integer from 0 to 10, and
* and *' each indicate a binding site to M in Formula 1.

In Formulae 3A to 3D, the $C_5$-$C_{30}$ carbocyclic group and the $C_2$-$C_{30}$ heterocyclic group may each independently be the same as defined in connection with ring $CY_{21}$.

For example, a moiety represented by

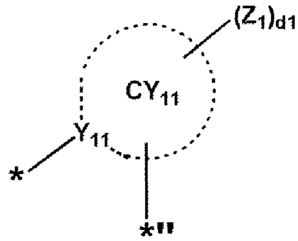

in Formula 3D may be a group represented by Formulae one of CY11-1 to CY11-34, and/or a moiety represented by

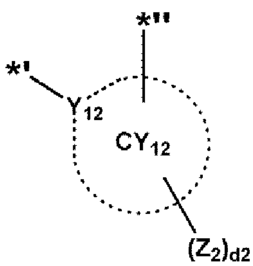

in Formulae 3C and 3D may be a group represented by Formulae one of CY12-1 to CY12-34:

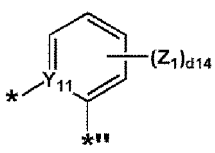
CY11-1

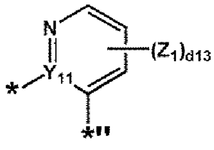
CY11-2

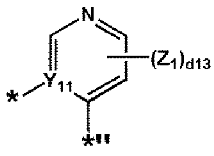
CY11-3

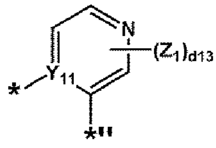
CY11-4

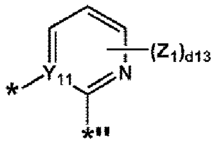
CY11-5

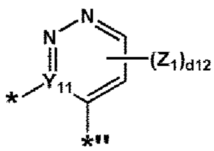
CY11-6

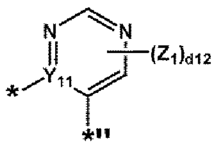
CY11-7

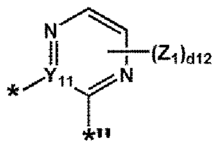
CY11-8

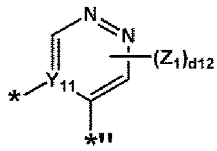
CY11-9

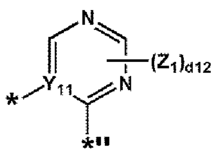
CY11-10

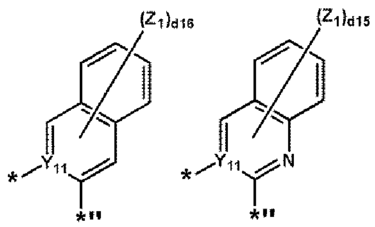
CY11-11

CY11-12

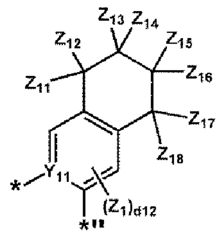
CY11-13

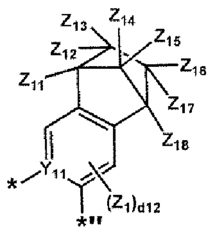
CY11-14

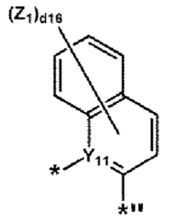
CY11-15

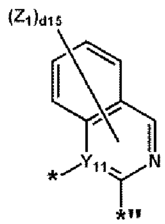
CY11-16

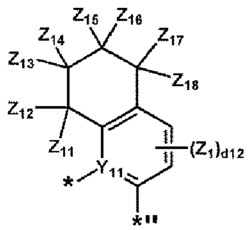
CY11-17

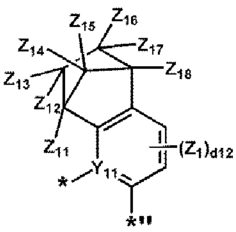
CY11-18

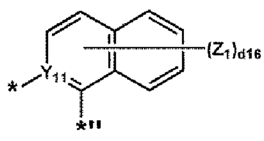
CY11-19

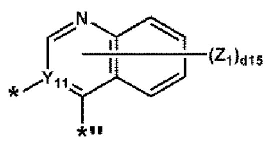
CY11-20

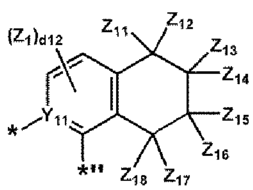
CY11-21

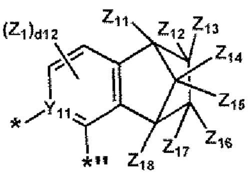
CY11-22

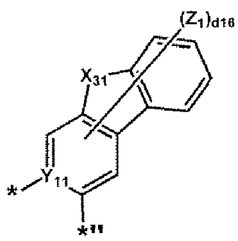
CY11-23

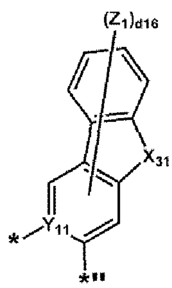
CY11-24

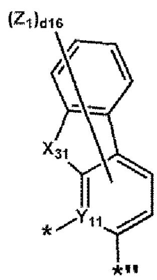
CY11-25

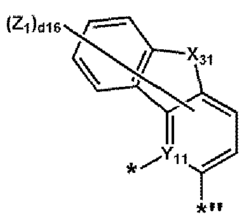
CY11-26

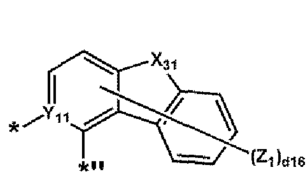
CY11-27

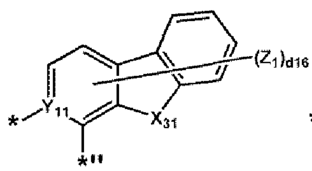
CY11-28

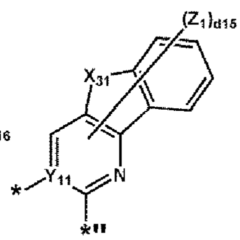
CY11-29

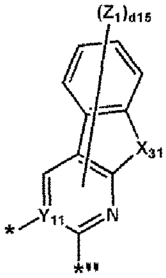
CY11-30

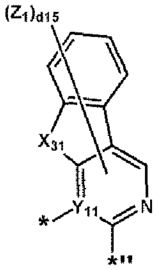
CY11-31

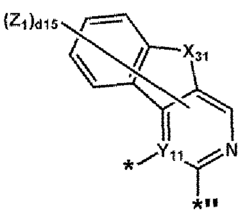
CY11-32

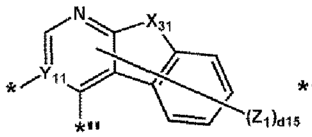
CY11-33

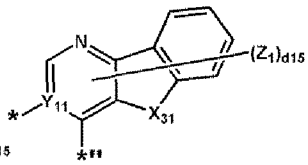
CY11-34

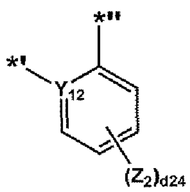
CY12-1

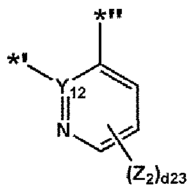
CY12-2

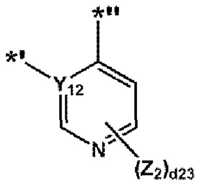
CY12-3

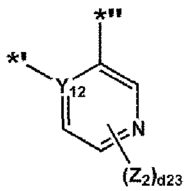
CY12-4

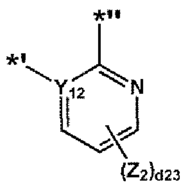
CY12-5

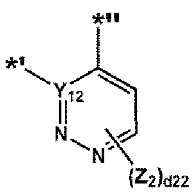
CY12-6

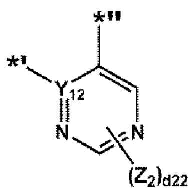
CY12-7

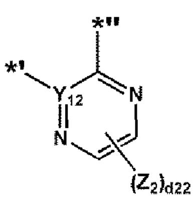
CY12-8

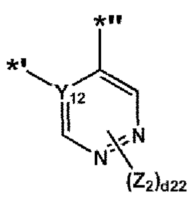
CY12-9

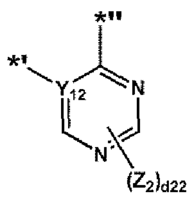
CY12-10

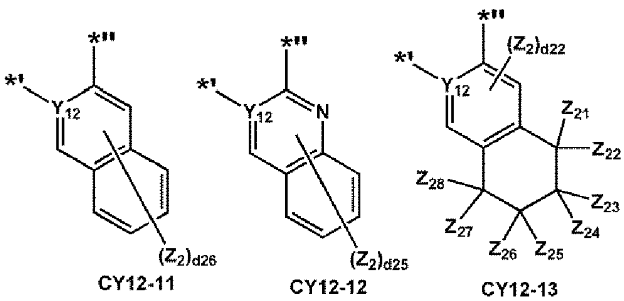
CY12-11 CY12-12 CY12-13

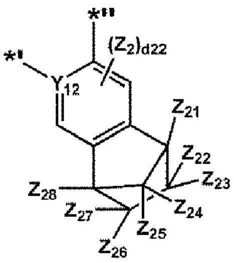
CY12-14

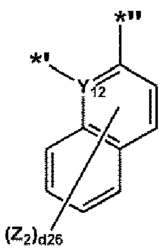
CY12-15

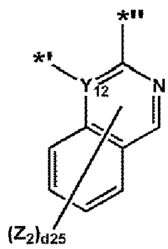
CY12-16

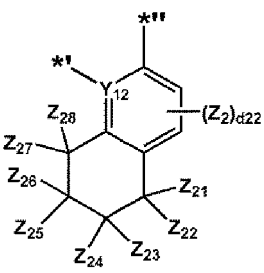
CY12-17

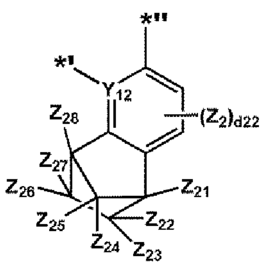
CY12-18

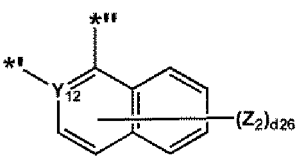
CY12-19

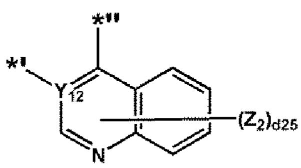
CY12-20

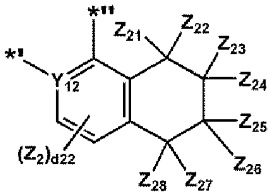

CY12-21

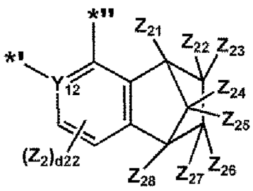

CY12-22

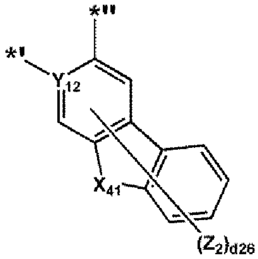

CY12-23

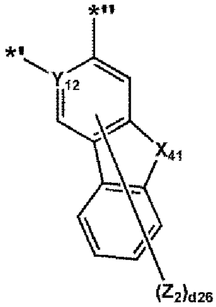

CY12-24

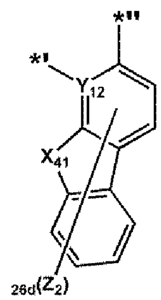

CY12-25

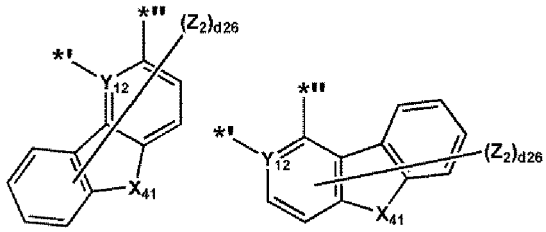

CY12-26 CY12-27

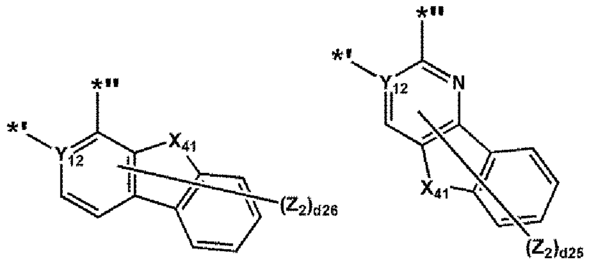

CY12-28 CY12-29

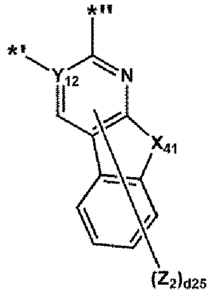

CY12-30

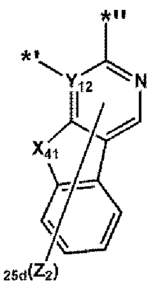

CY12-31

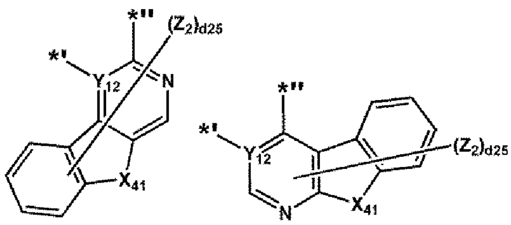

CY12-32 CY12-33

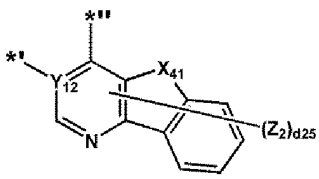

CY12-34

In Formulae CY11-1 to CY11-34 and CY12-1 to CY12-34,

$X_{31}$ may be O, S, $N(Z_{11})$, $C(Z_{11})(Z_{12})$, or $Si(Z_{11})(Z_{12})$,
$X_{41}$ may be O, S, $N(Z_{21})$, $C(Z_{21})(Z_{22})$, or $Si(Z_{21})(Z_{22})$,
$Y_{11}$, $Y_{12}$, $Z_1$, and $Z_2$ may each independently be the same as described herein,
$Z_{11}$ to $Z_{18}$ and $Z_{21}$ to $Z_{28}$ may each independently be the same as defined in connection with $R_{21}$,
d12 and d22 may each independently be an integer from 0 to 2,
d13 and d23 may each independently be an integer from 0 to 3,
d14 and d24 may each independently be an integer from 0 to 4,
d15 and d25 may each independently be an integer from 0 to 5,
d16 and d26 may each independently be an integer from 0 to 6, and
in Formulae CY11-1 to CY11-34 and CY12-1 to CY12-34, * and *' each indicate a binding site to M in Formula 1, and *" indicates a binding site to a neighboring atom in Formula 3C or $T_{21}$ in Formula 3D.

In one embodiment, in Formula 1, $L_2$ may be a group represented by one of Formulae 3-1(1) to 3-1(66), 3-1(301) and 3-1(308), but embodiments of the present disclosure are not limited thereto:

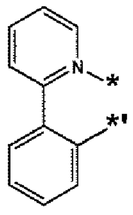
3-1(1)

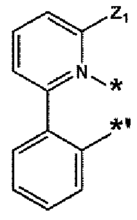
3-1(2)

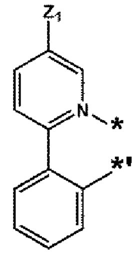
3-1(3)

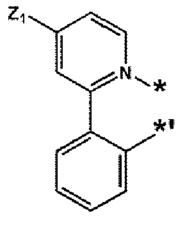
3-1(4)

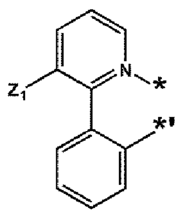
3-1(5)

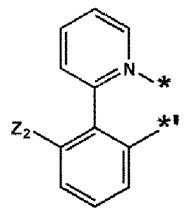
3-1(6)

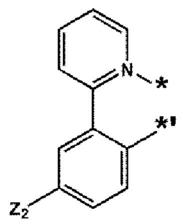
3-1(7)

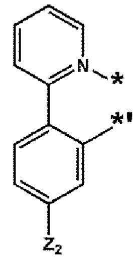
3-1(8)

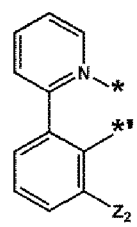
3-1(9)

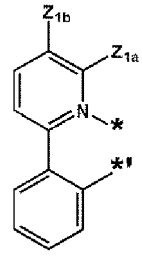
3-1(10)

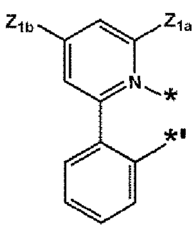
3-1(11)

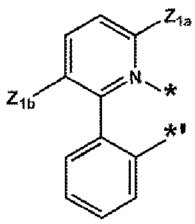
3-1(12)

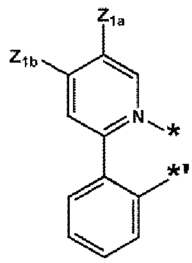
3-1(13)

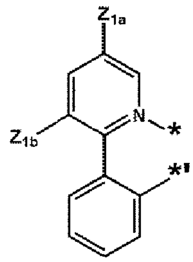
3-1(14)

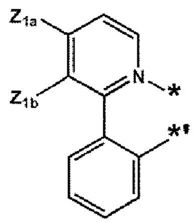
3-1(15)

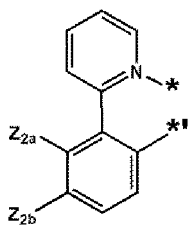
3-1(16)

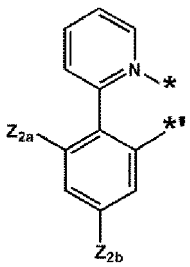
3-1(17)

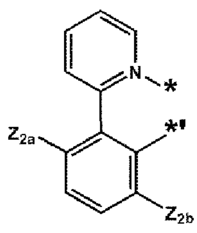
3-1(18)

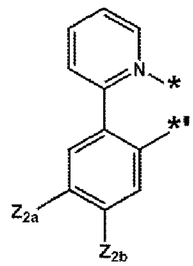
3-1(19)

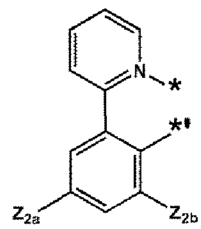
3-1(20)

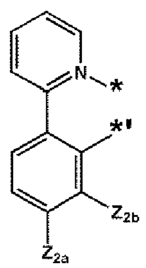
3-1(21)

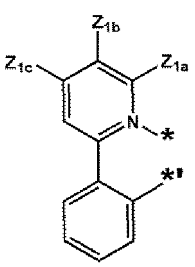
3-1(22)

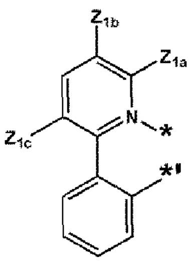
3-1(23)

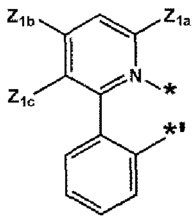
3-1(24)

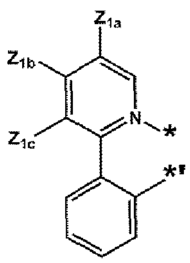
3-1(25)

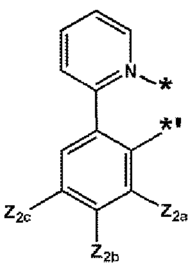
3-1(26)

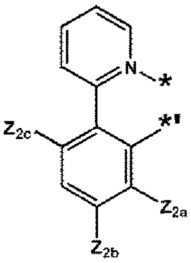
3-1(27)

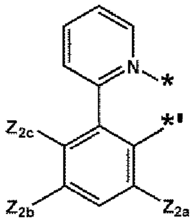
3-1(28)

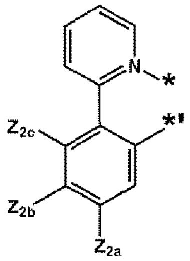
3-1(29)

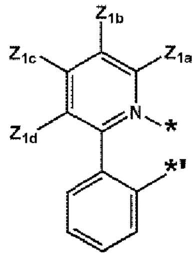
3-1(30)

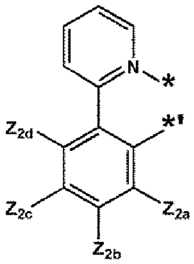
3-1(31)

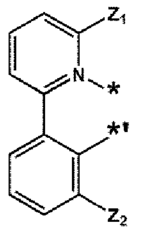
3-1(32)

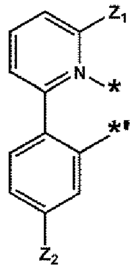
3-1(33)

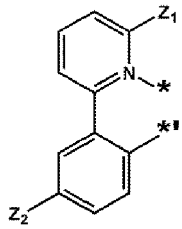
3-1(34)

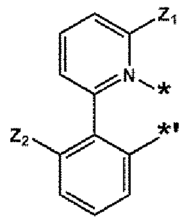
3-1(35)

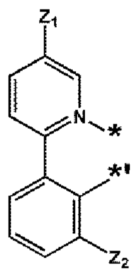
3-1(36)

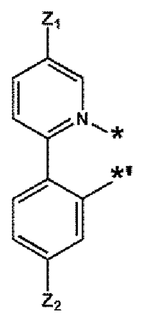
3-1(37)

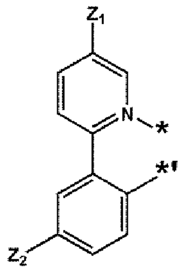
3-1(38)

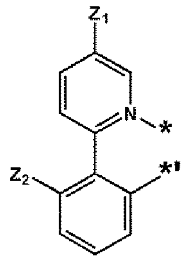
3-1(39)

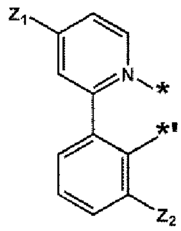
3-1(40)

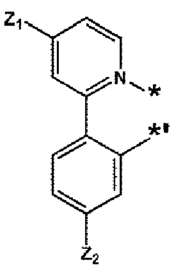
3-1(41)

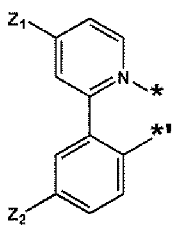
3-1(42)

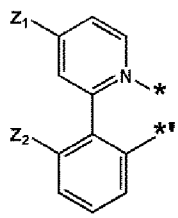
3-1(43)

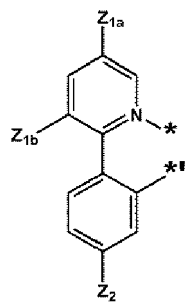
3-1(44)

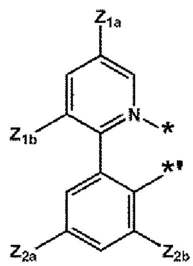
3-1(45)

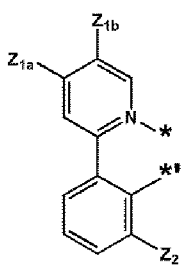
3-1(46)

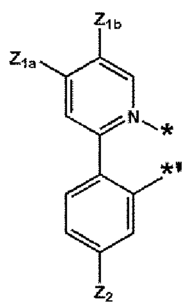
3-1(47)

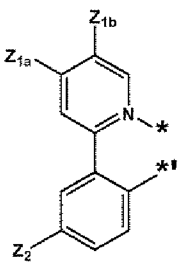
3-1(48)

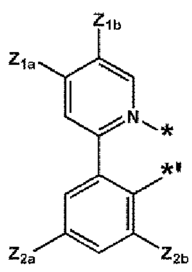
3-1(49)

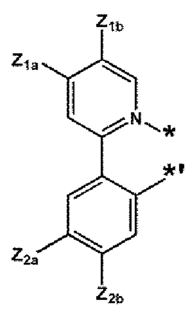
3-1(50)

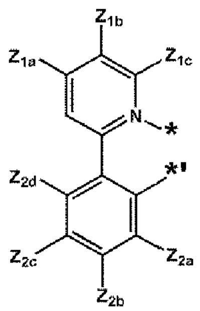
3-1(51)

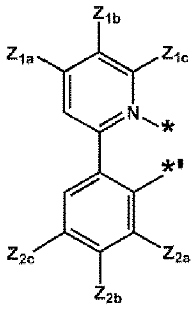
3-1(52)

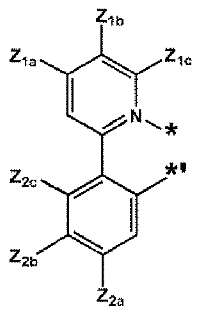
3-1(53)

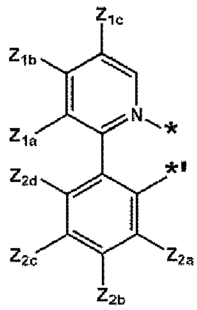
3-1(54)

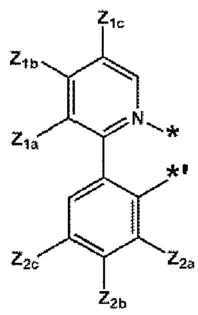
3-1(55)

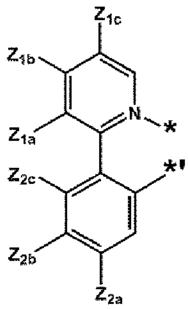
3-1(56)

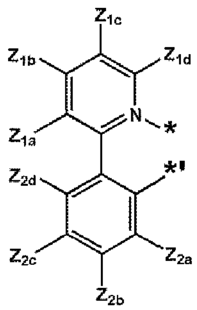
3-1(57)

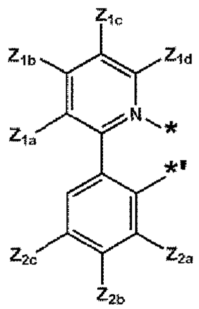
3-1(58)

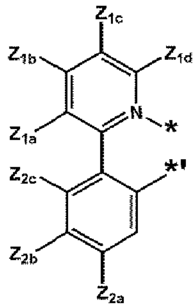
3-1(59)

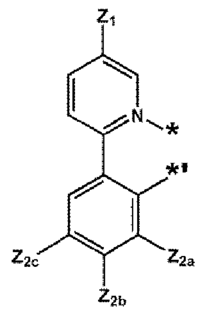
3-1(60)

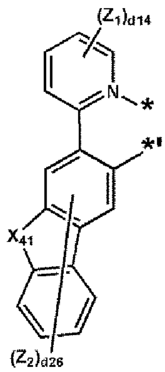
3-1(61)

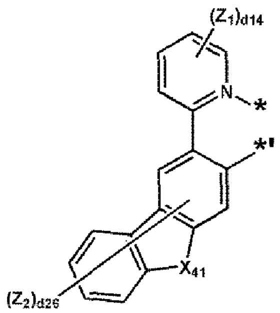
3-1(62)

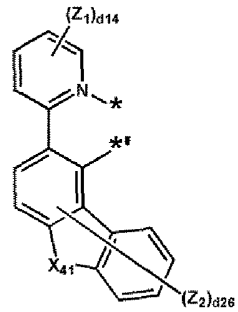
3-1(63)

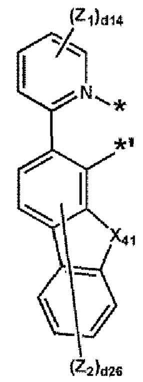
3-1(64)

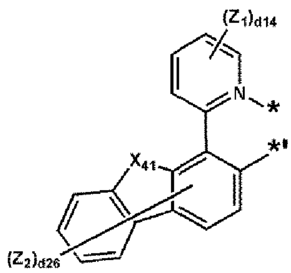
3-1(65)

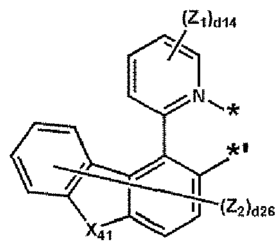
3-1(66)

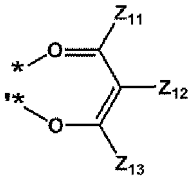

3-1(301)

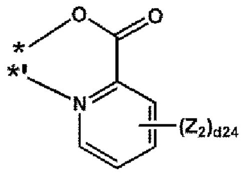

3-1(308)

In Formulae 3-1(1) to 3-1(66), and 3-1(301) and 3-1(308),

$X_{41}$ may be O, S, $N(Z_{21})$, $C(Z_{21})(Z_{22})$, or $Si(Z_{21})(Z_{22})$,
$Z_1$, $Z_2$, $Z_{1a}$, $Z_{1b}$, $Z_{1c}$, $Z_{1d}$, $Z_{2a}$, $Z_{2b}$, $Z_{2c}$, $Z_{2d}$, $Z_{11}$ to $Z_{13}$, $Z_{21}$ and $Z_{22}$ may each independently be the same as defined in connection with $R_{21}$,
d14 may be an integer from 0 to 4,
d26 may be an integer from 0 to 6, and
* and *' each indicate a binding site to M in Formula 1.

For example, $Z_{11}$ and $Z_{13}$ in Formula 3-1(301) may each independently be a methyl group.
In one embodiment, $Z_{11}$, $Z_{13}$, or a combination thereof in Formula 3-1(301) may each independently be a substituted or unsubstituted $C_2$-$C_{30}$ alkyl group or a substituted or unsubstituted $C_3$-$C_{10}$ cycloalkyl group, but embodiments of the present disclosure are not limited thereto.
In one embodiment, the organometallic compound represented by Formula 1 may emit red light or green light.
The terms "an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, and an azadibenzothiophene 5,5-dioxide group" as used herein each refer to a heterocyclic group having the same backbone as "an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, and a dibenzothiophene 5,5-dioxide group" in which at least one carbon atom constituting the cyclic groups is replaced with N.
In one or more embodiments, the organometallic compound represented by Formula 1 may be one of Compounds 1 to 15, 17-218, 220-328, 330-438, 440-453, 455-466, 468 and 469, but embodiments of the present disclosure are not limited thereto:

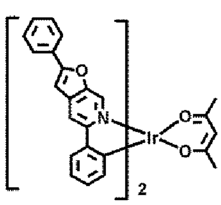

1

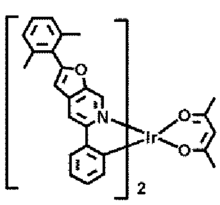

2

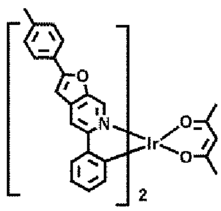

3

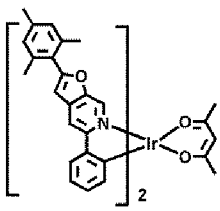

4

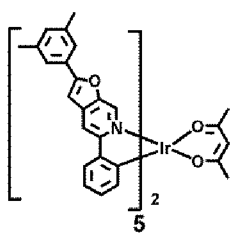

5

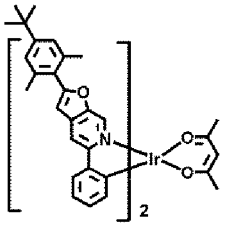
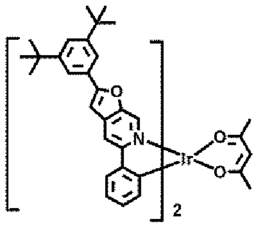
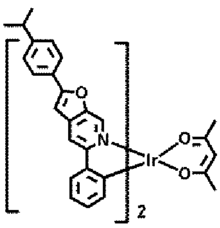
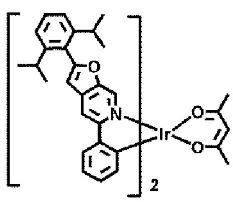
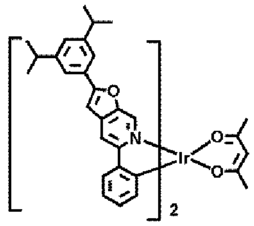

6 7 8 9 10

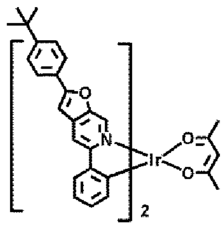
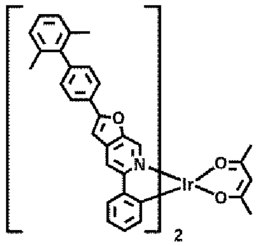
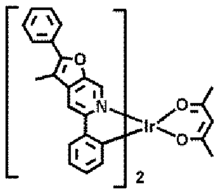
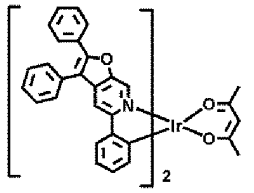
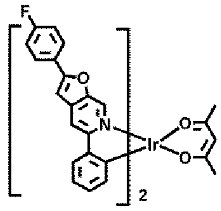

11 12 13 14 15

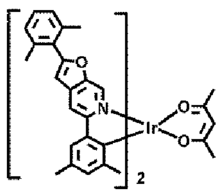
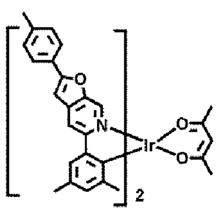
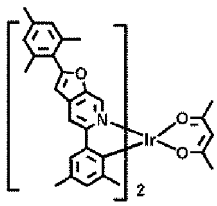
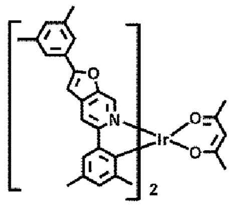

17 18 19 20

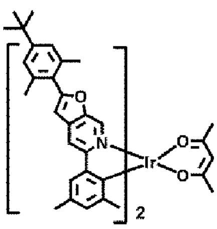
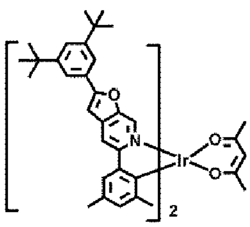
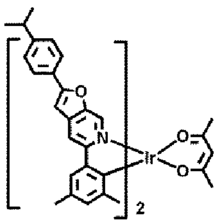
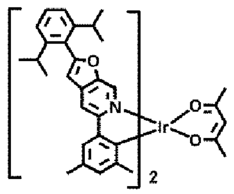
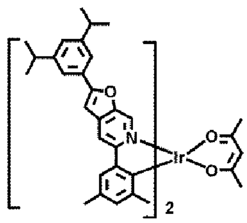

21 22 23 24 25

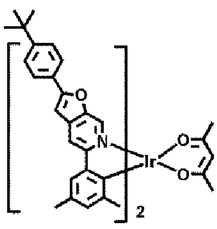
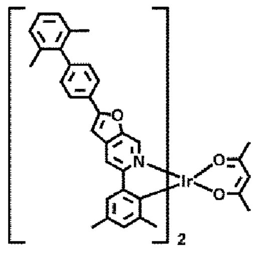
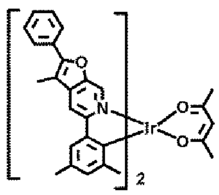
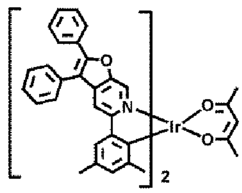
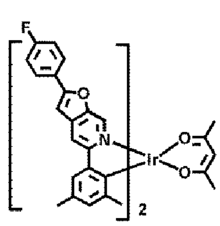

26 27 28 29 30

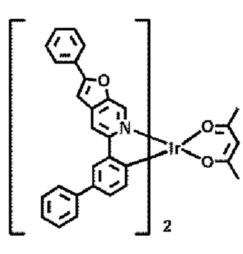

31

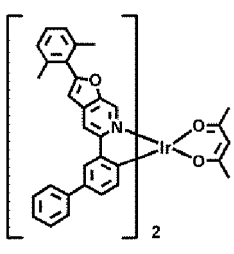

32

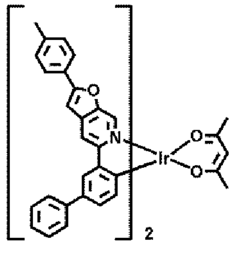

33

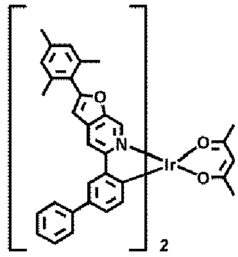

34

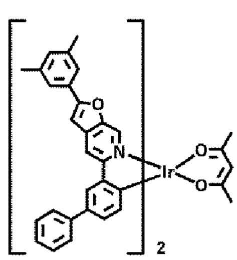

35

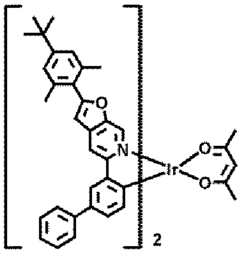

36

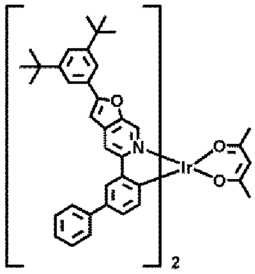

37

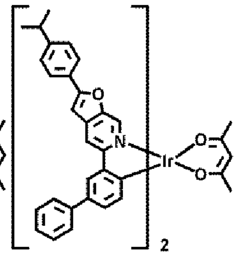

38

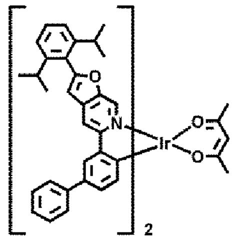

39

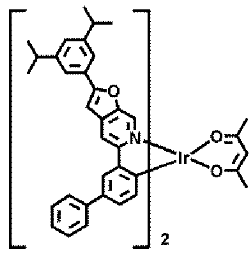

40

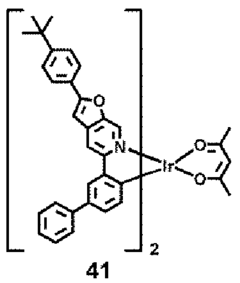

41

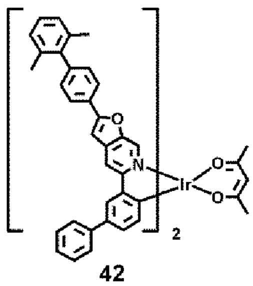

42

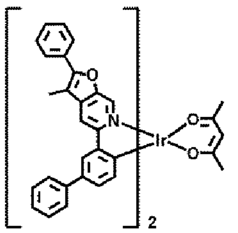

43

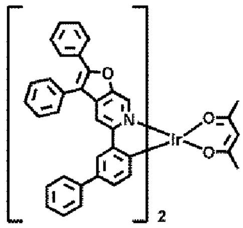

44

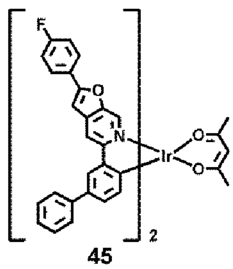

45

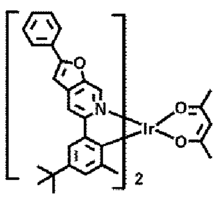

46

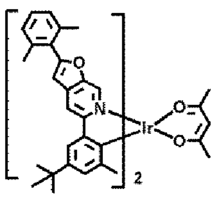

47

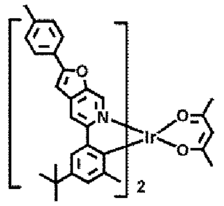

48

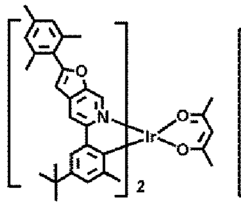

49

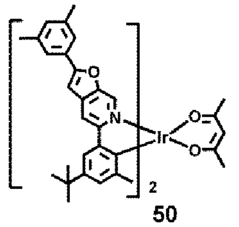

50

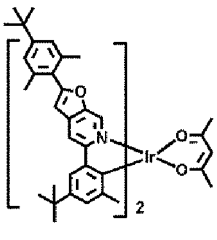

51

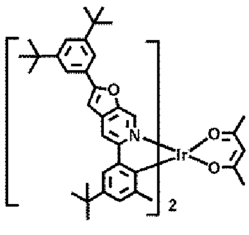

52

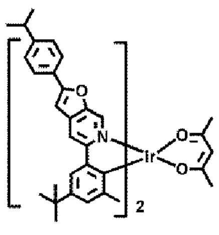

53

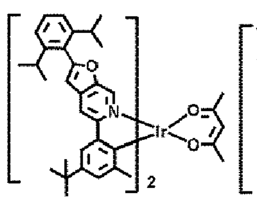

54

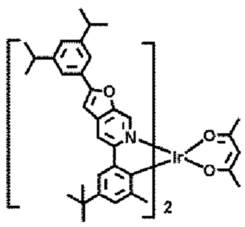

55

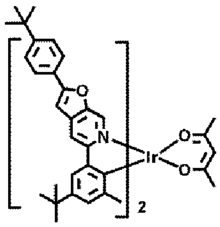
56
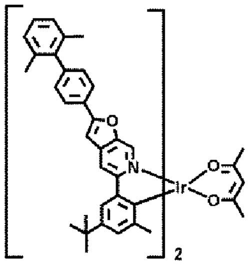
57
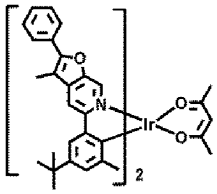
58
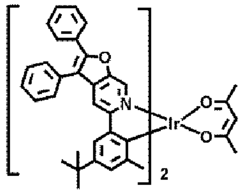
59
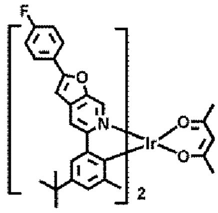
60

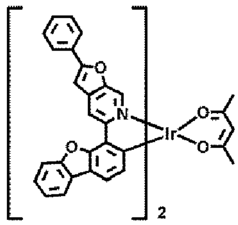
61
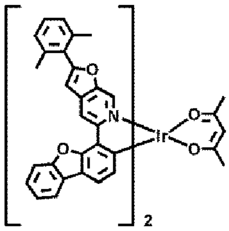
62
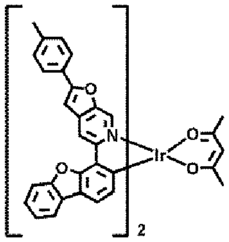
63
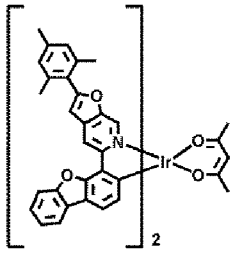
64
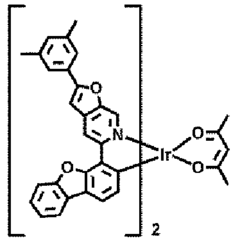
65

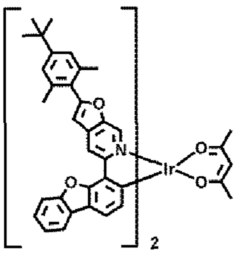
66
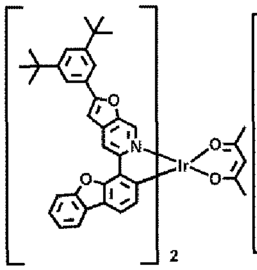
67
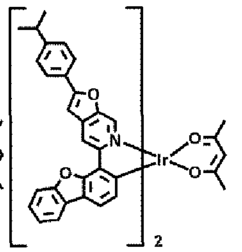
68
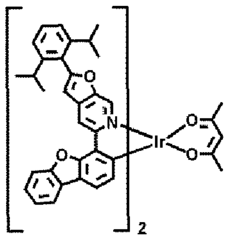
69
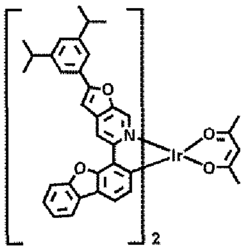
70

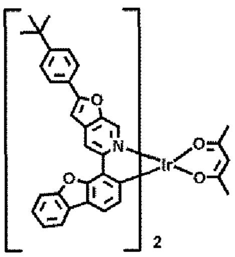
71
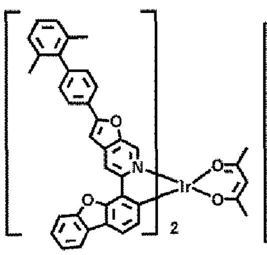
72
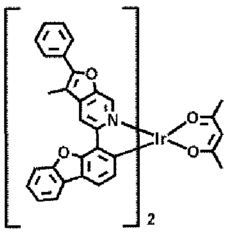
73
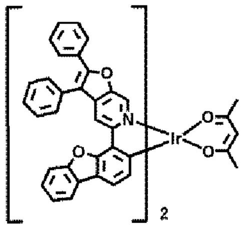
74
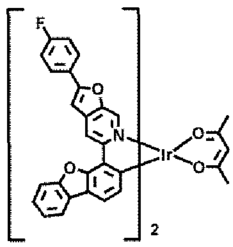
75

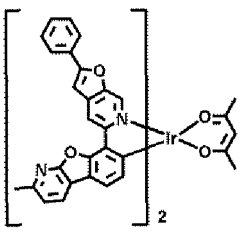
76
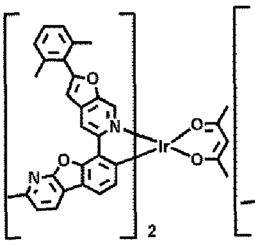
77
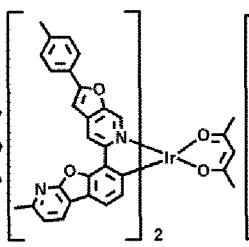
78
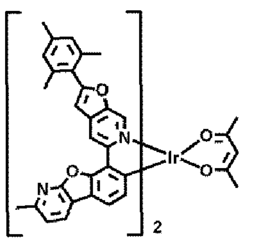
79
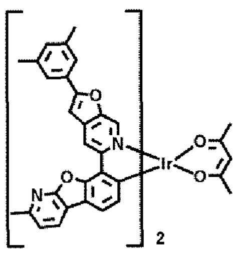
80

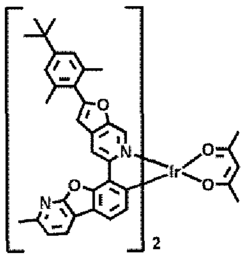
81

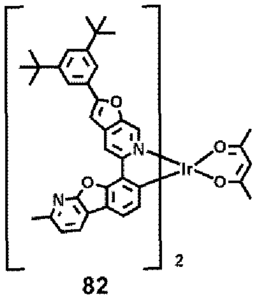
82

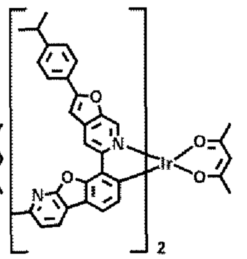
83

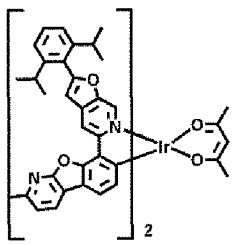
84

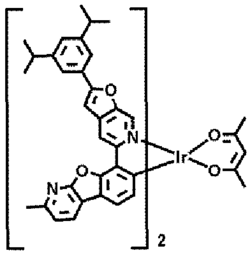
85

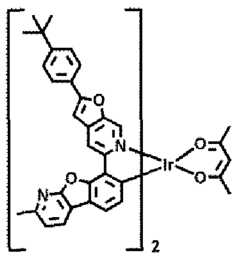
86

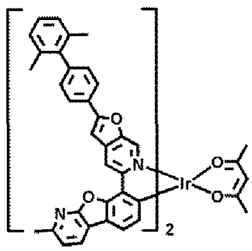
87

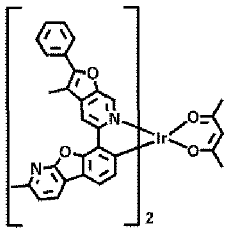
88

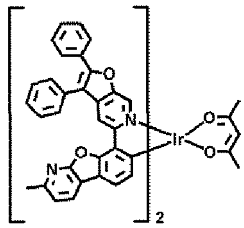
89

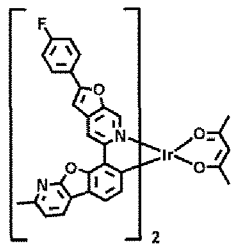
90

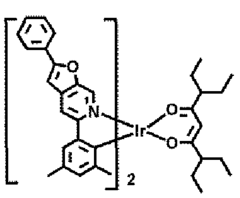
91

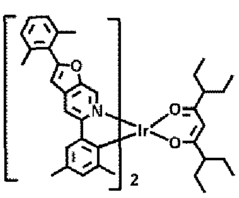
92

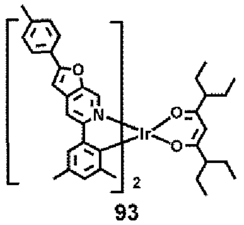
93

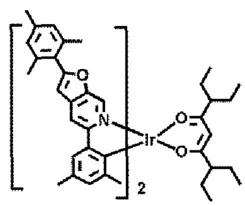
94

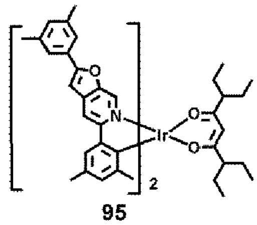
95

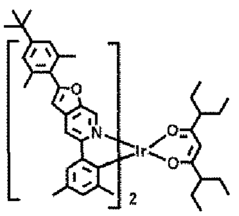
96

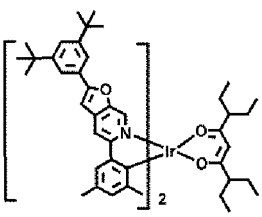
97

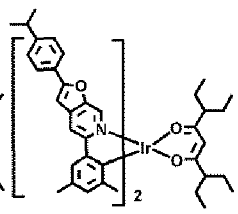
98

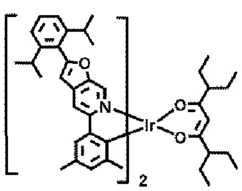
99

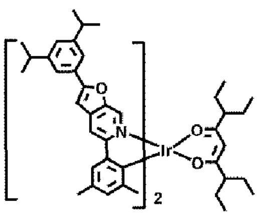
100

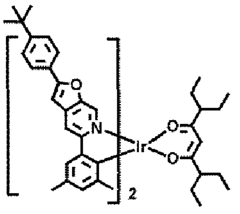
101

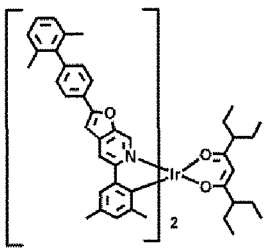
102

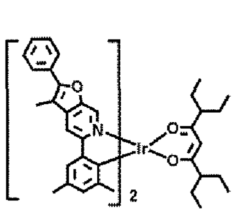
103

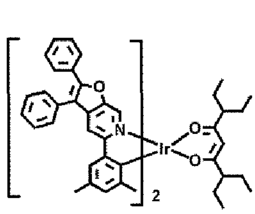
104

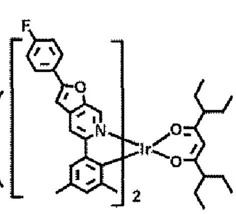
105

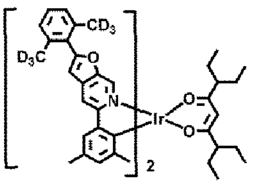

106

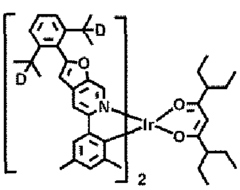

107

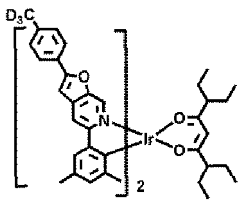

108

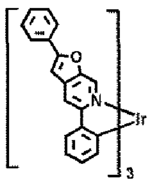

109

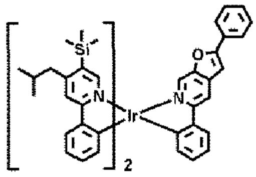

110

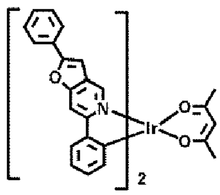

111

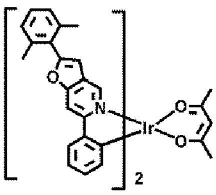

112

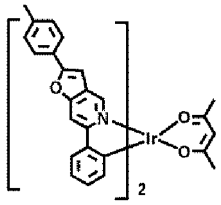

113

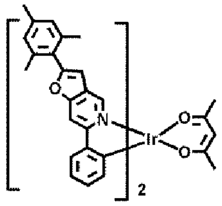

114

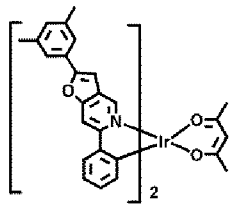

115

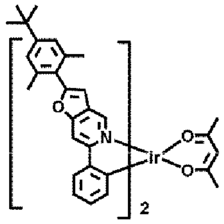

116

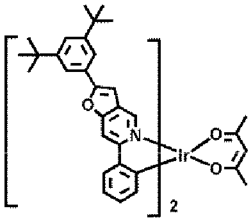

117

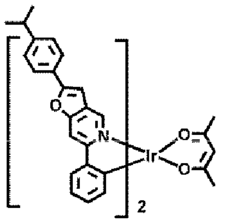

118

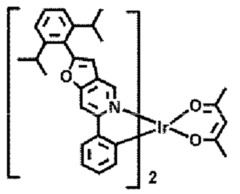

119

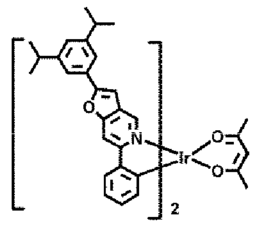

120

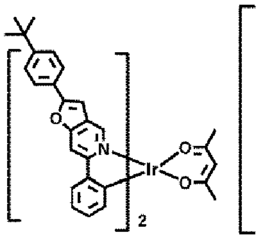

121

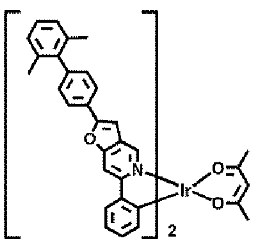

122

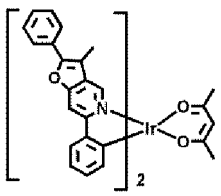

123

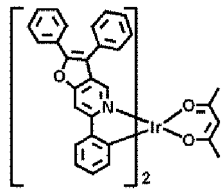

124

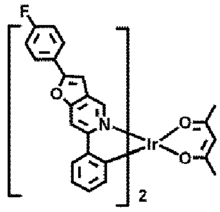

125

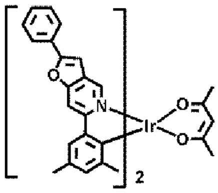

126

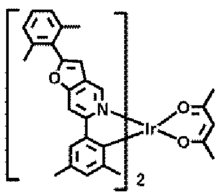

127

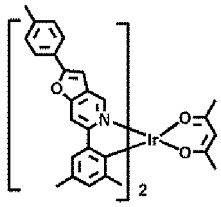

128

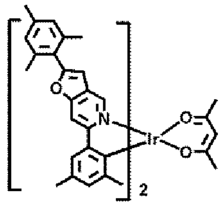

129

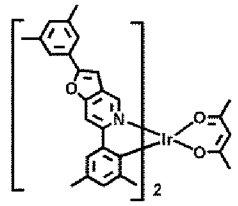

130

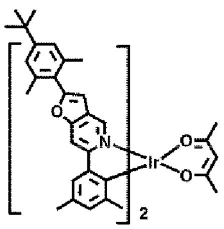

131

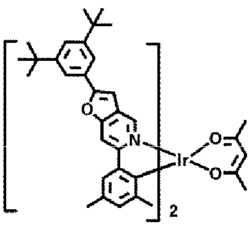

132

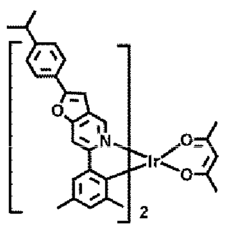

133

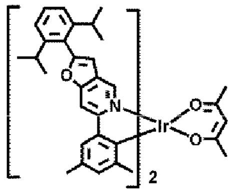

134

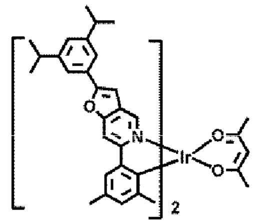

135

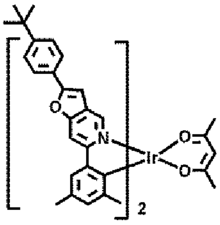
136
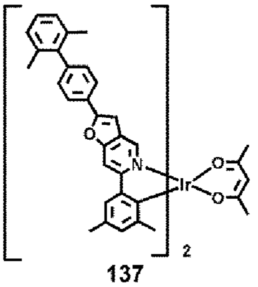
137
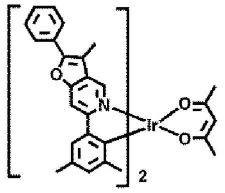
138
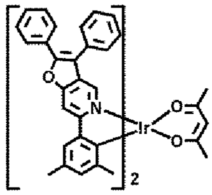
139
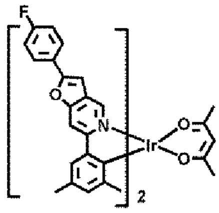
140

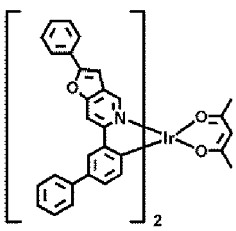
141
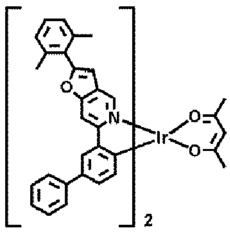
142
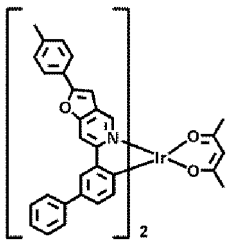
143
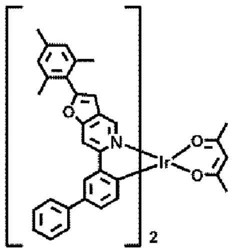
144
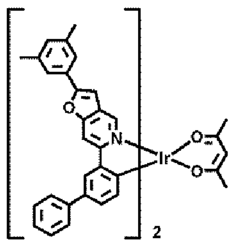
145

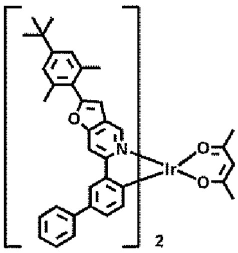
146
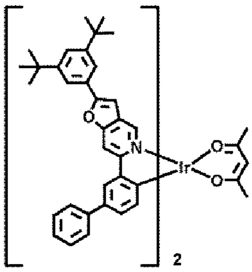
147
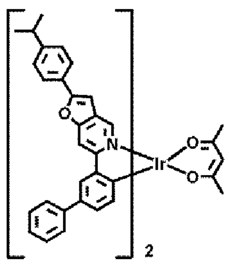
148
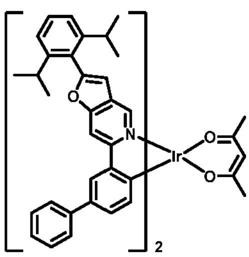
149
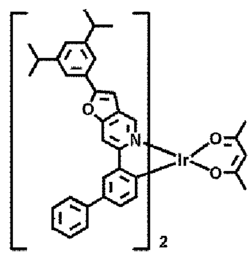
150

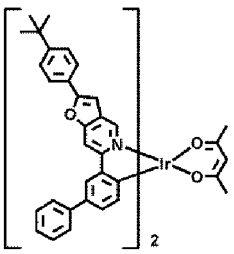
151
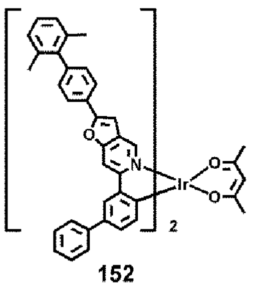
152
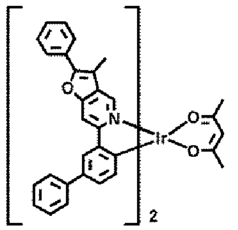
153
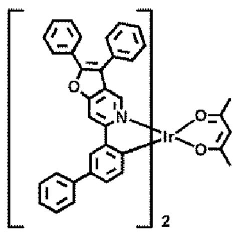
154
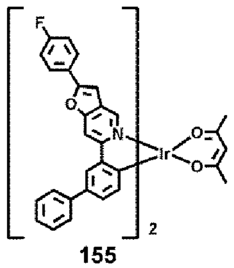
155

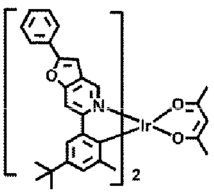
156
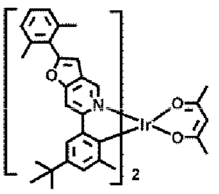
157
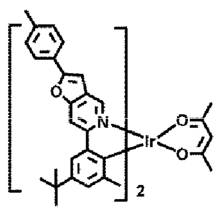
158
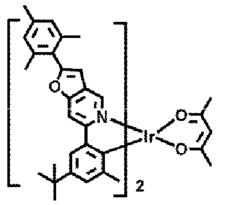
159
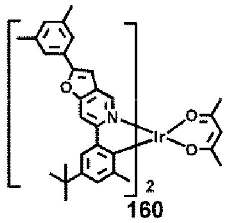
160

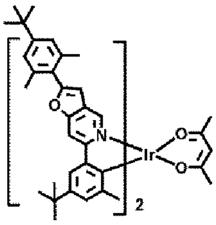
161
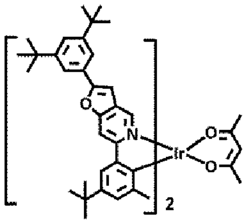
162
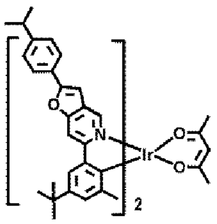
163
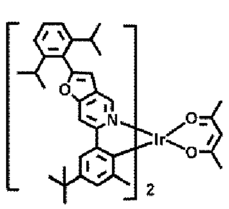
164
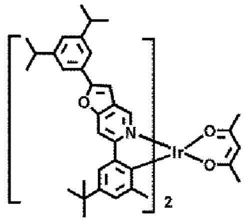
165

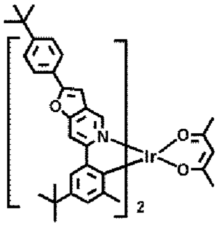
166
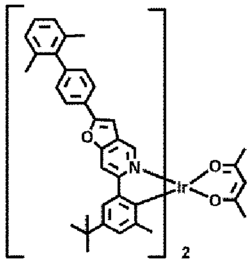
167
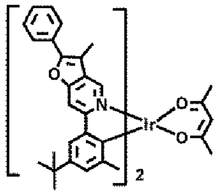
168
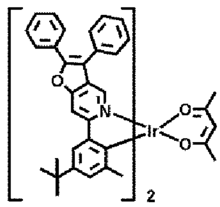
169
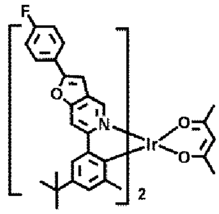
170

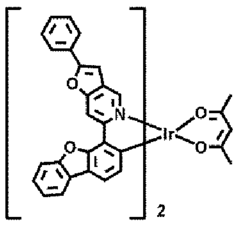
171
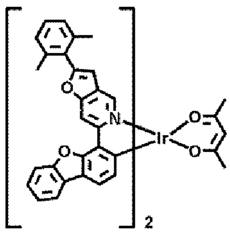
172
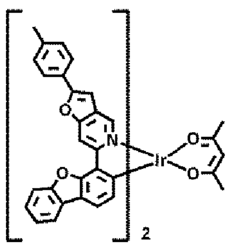
173
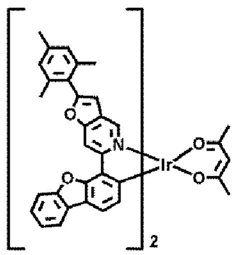
174
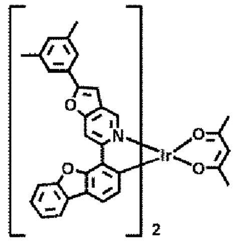
175

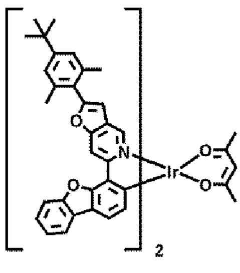
176
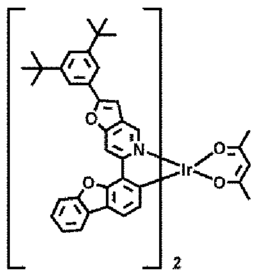
177
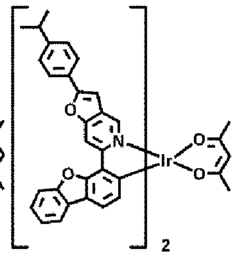
178
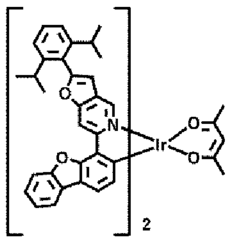
179
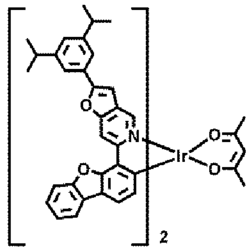
180

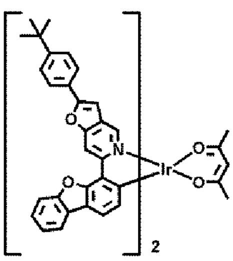
181
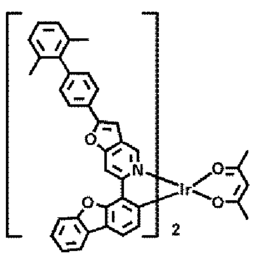
182
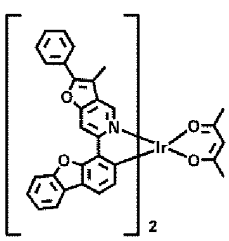
183
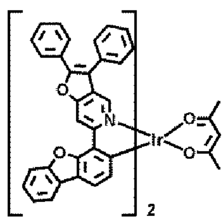
184
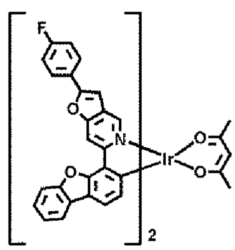
185

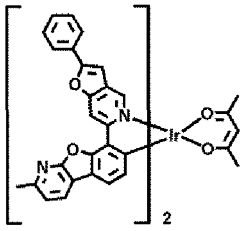
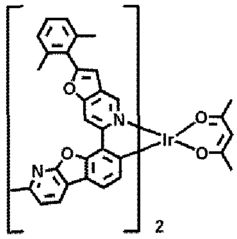
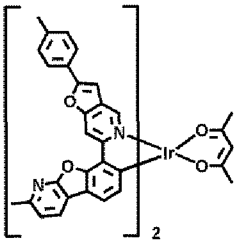
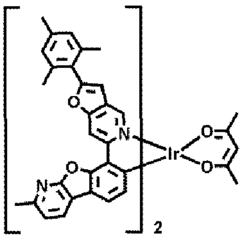
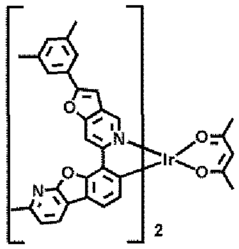

186 187 188 189 190

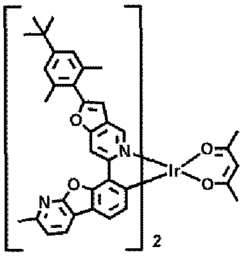
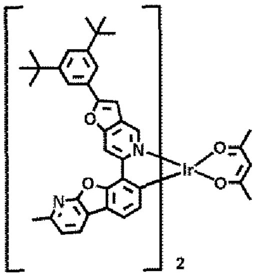
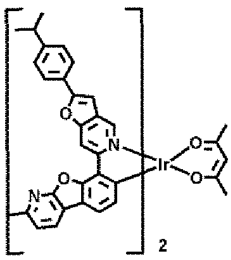
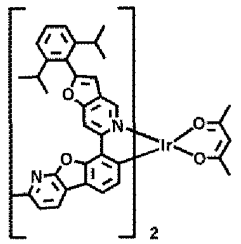
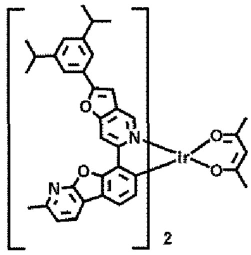

191 192 193 194 195

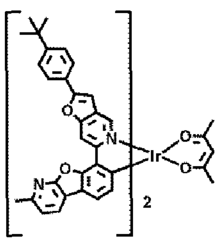
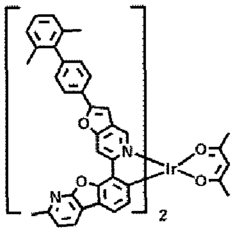
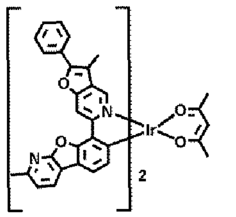
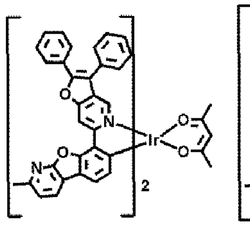
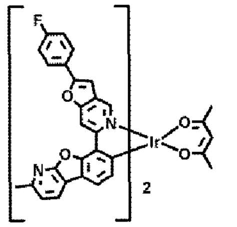

196 197 198 199 200

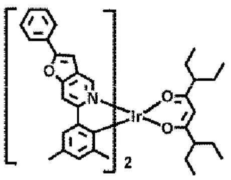
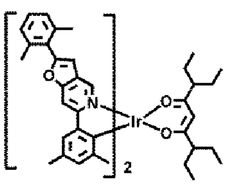
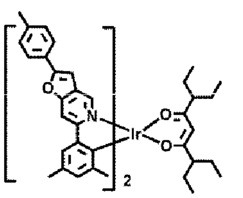
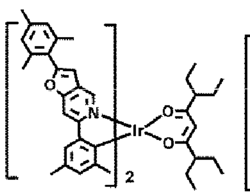
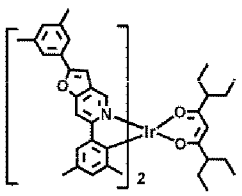

201 202 203 204 205

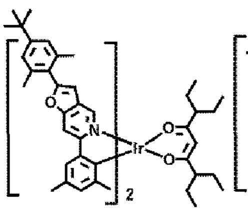
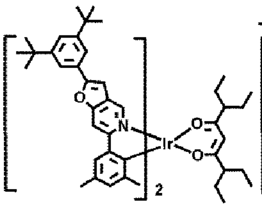
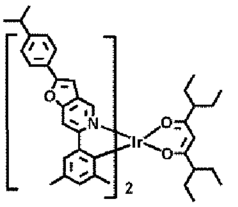
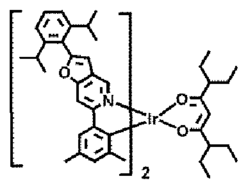
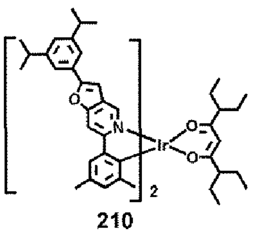

206 207 208 209 210

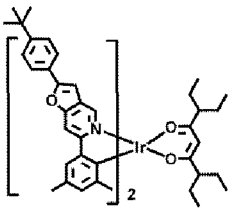
211

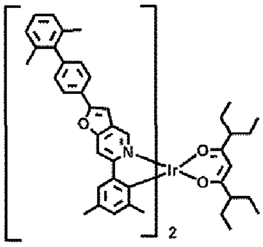
212

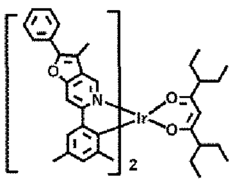
213

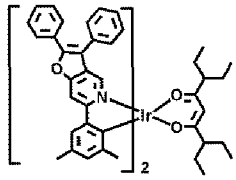
214

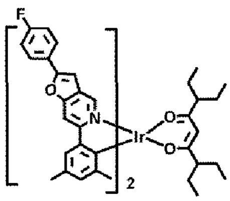
215

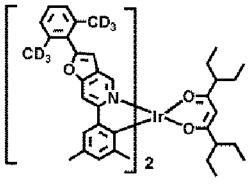
216

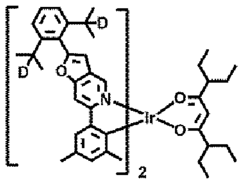
217

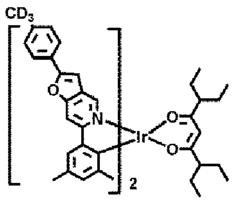
218

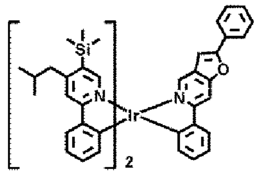
220

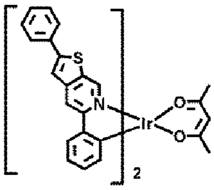
221

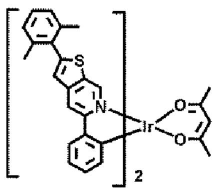
222

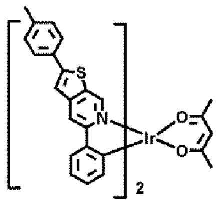
223

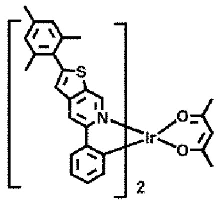
224

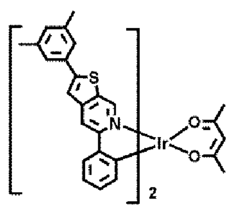
225

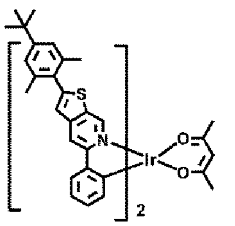
226

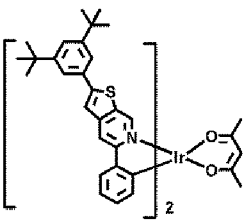
227

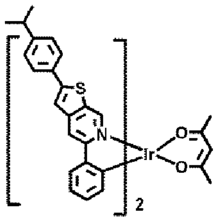
228

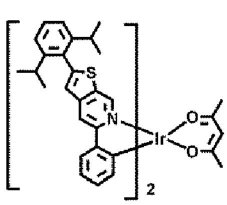
229

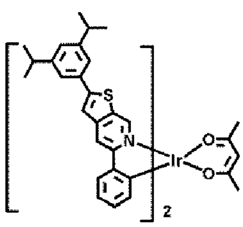
230

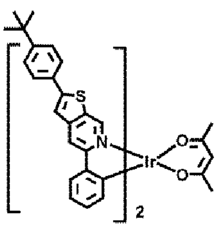
231

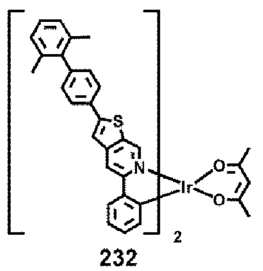
232

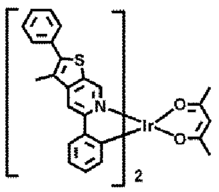
233

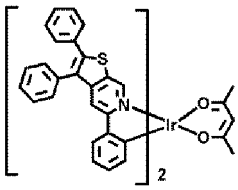
234

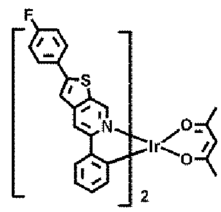
235

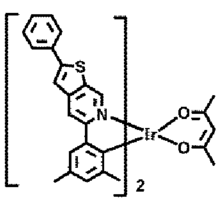
236

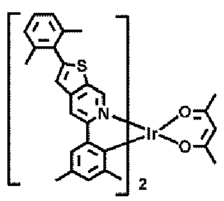
237

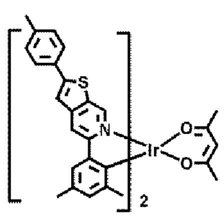
238

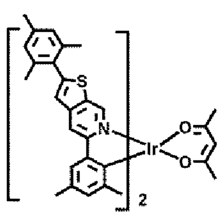
239

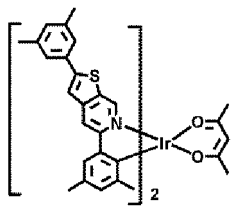
240

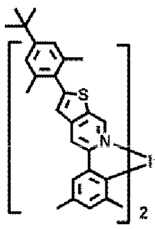
241

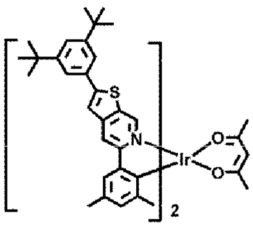
242

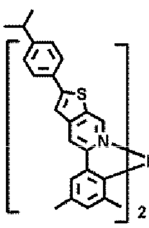
243

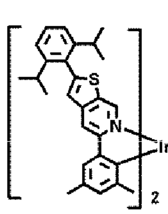
244

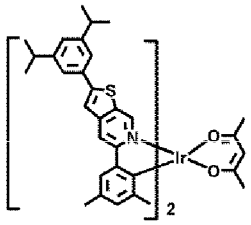
245

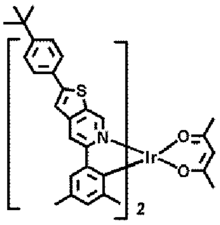
246

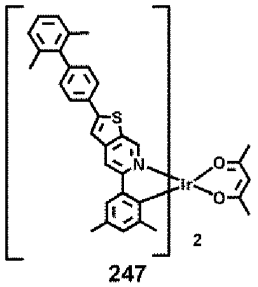
247

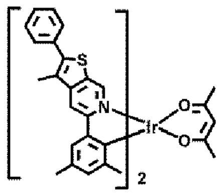
248

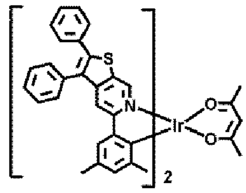
249

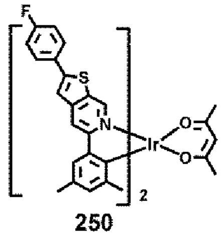
250

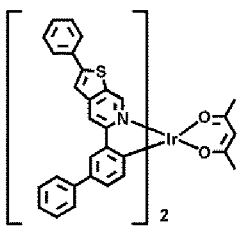
251

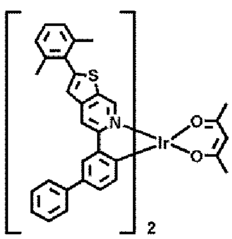
252

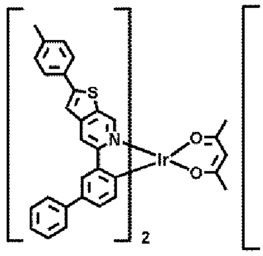
253

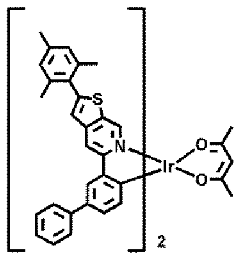
254

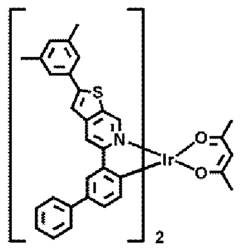
255

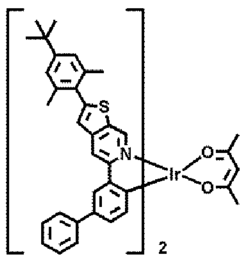
256

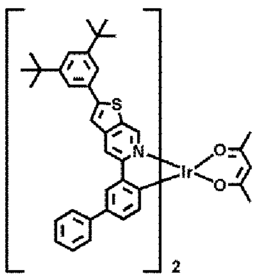
257

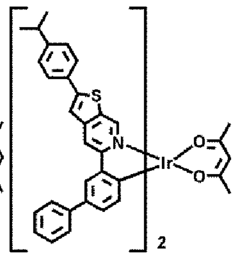
258

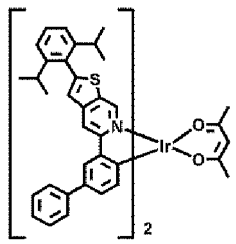
259

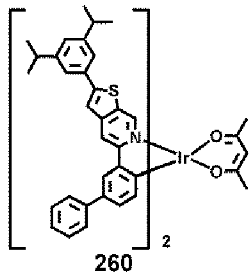
260

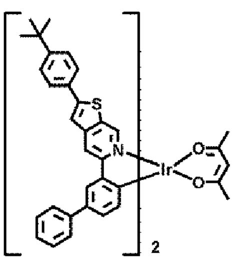
261

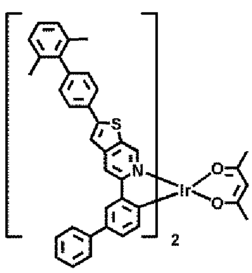
262

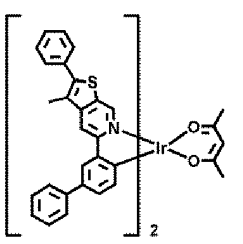
263

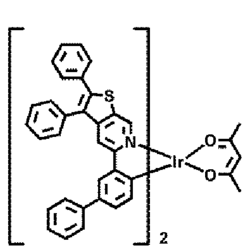
264

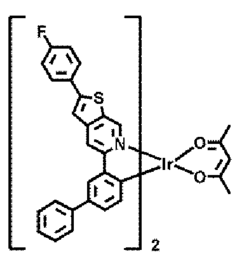
265

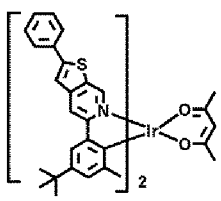
266

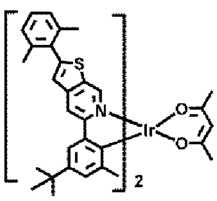
267

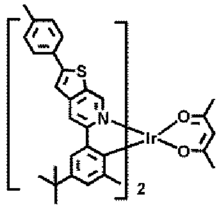
268

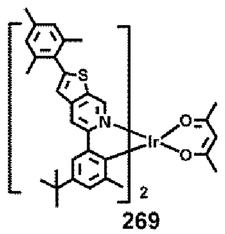
269

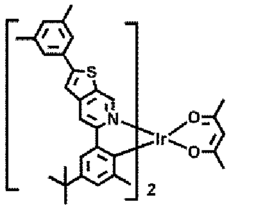
270

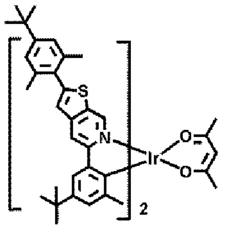
271

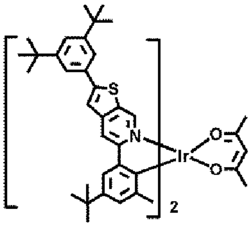
272

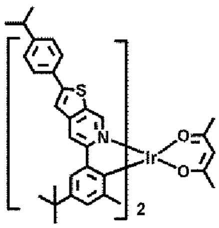
273

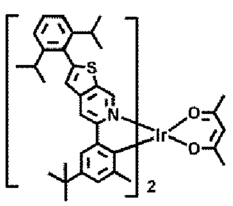
274

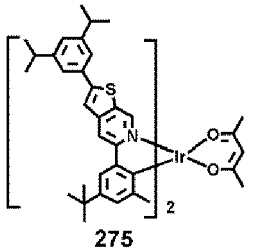
275

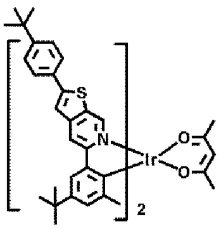
276

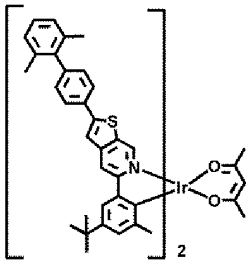
277

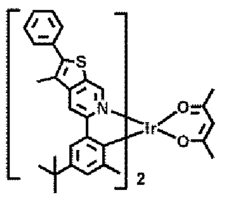
278

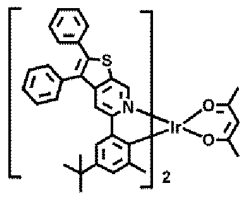
279

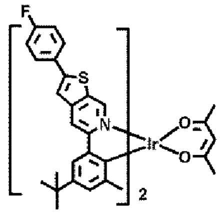
280

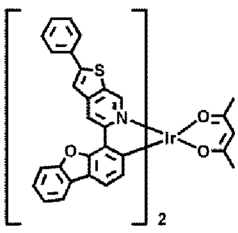
281

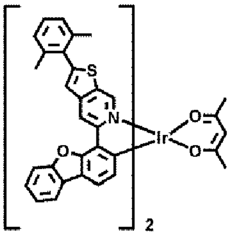
282

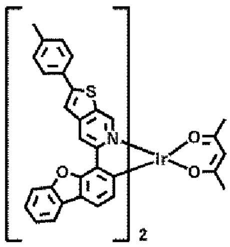
283

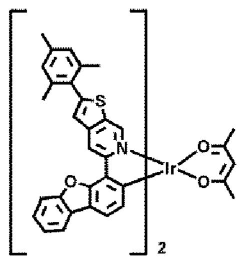
284

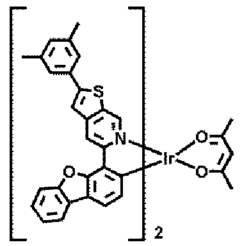
285

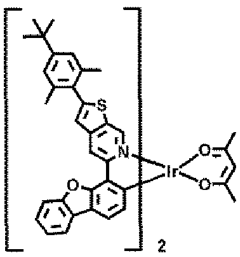
286

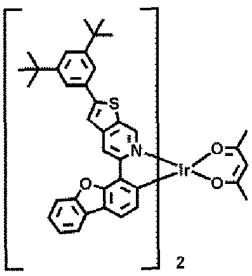
287

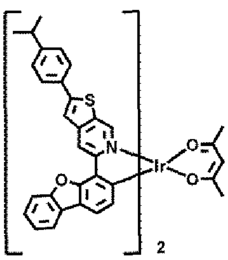
288

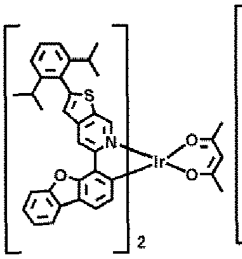
289

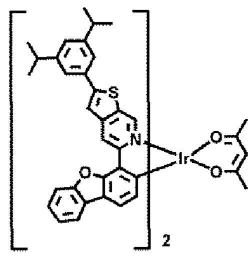
290

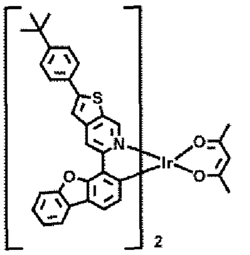

291

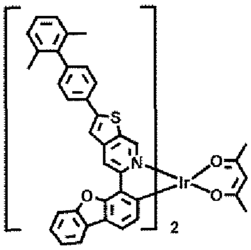

292

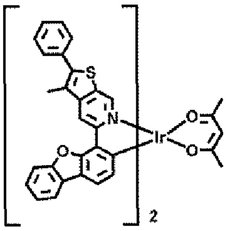

293

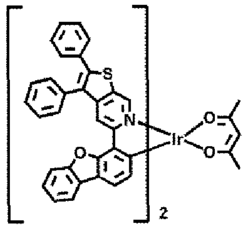

294

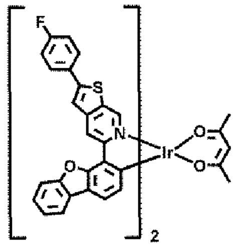

295

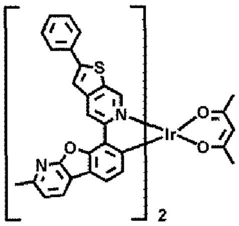

296

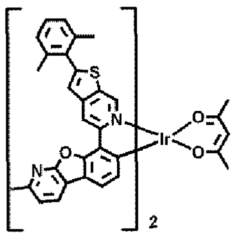

297

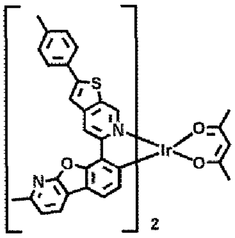

298

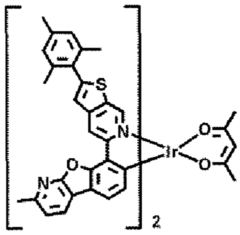

299

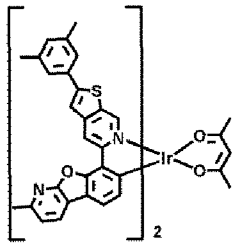

300

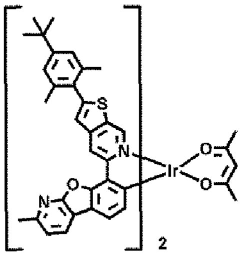

301

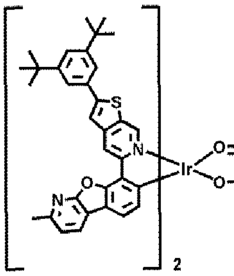

302

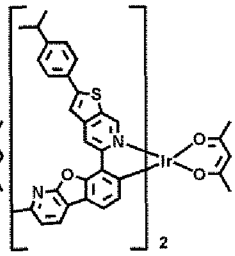

303

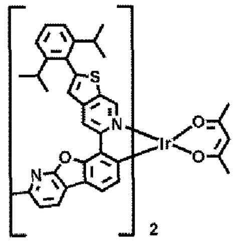

304

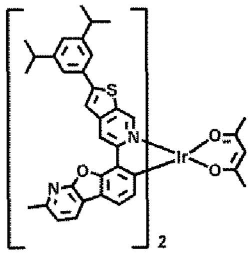

305

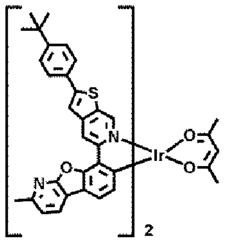

306

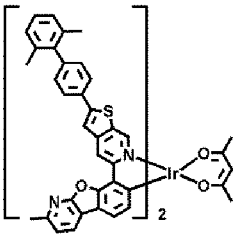

307

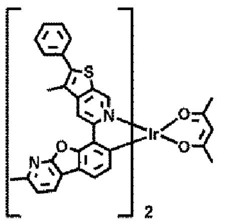

308

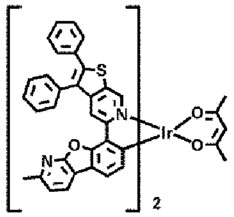

309

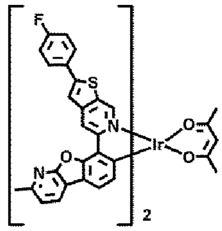

310

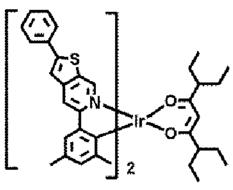

311

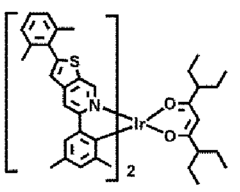

312

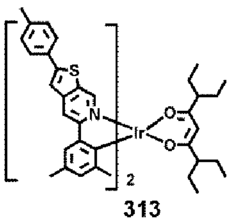

313

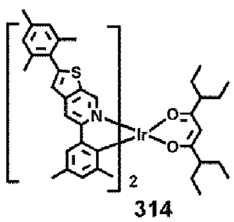

314

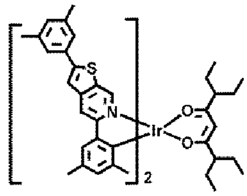

315

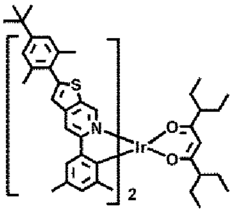

316

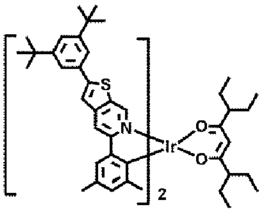

317

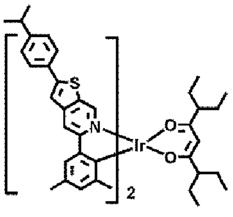

318

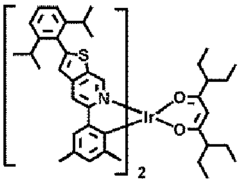

319

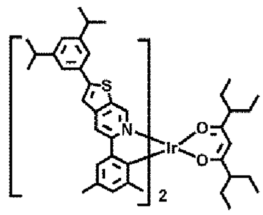

320

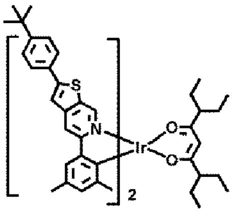

321

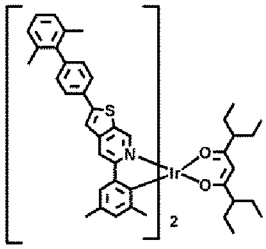

322

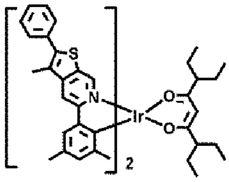

323

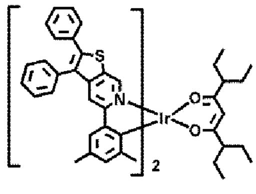

324

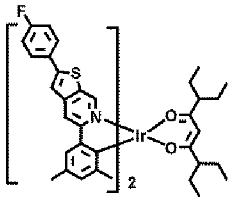

325

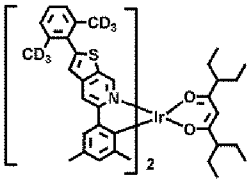

326

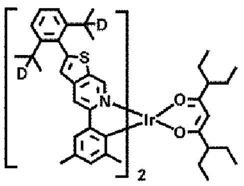

327

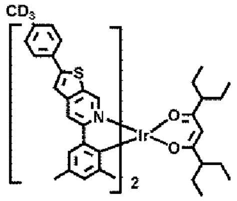

328

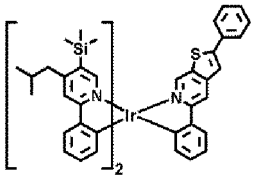

330

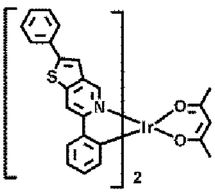

331

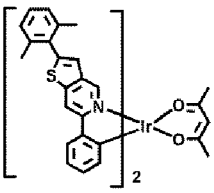

332

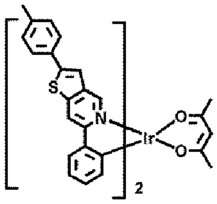

333

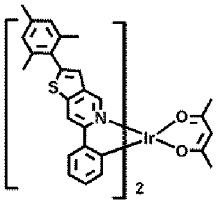

334

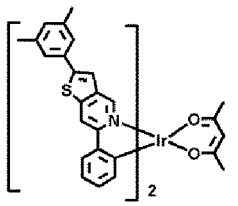

335

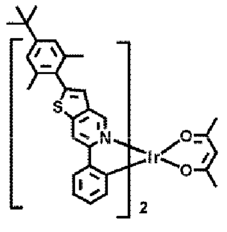

336

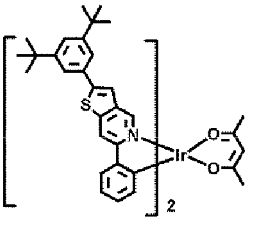

337

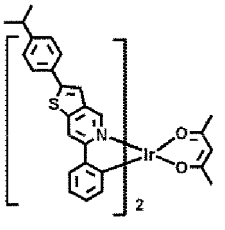

338

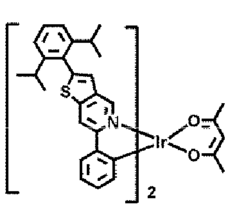

339

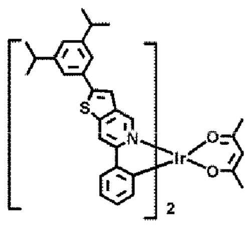

340

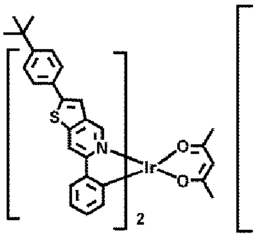

341

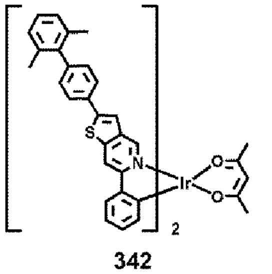

342

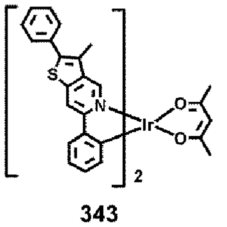

343

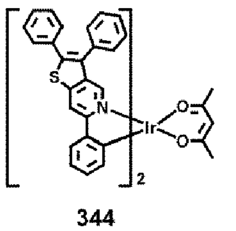

344

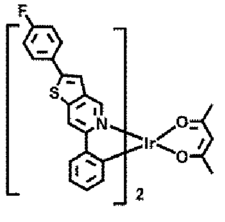

345

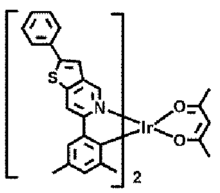
346

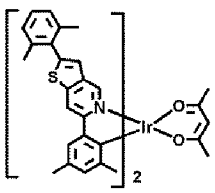
347

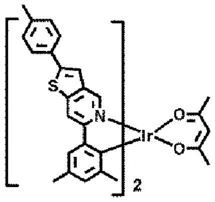
348

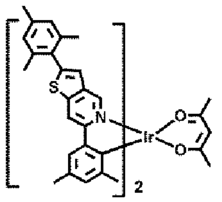
349

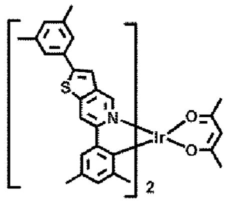
350

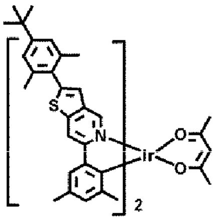
351

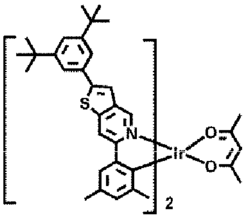
352

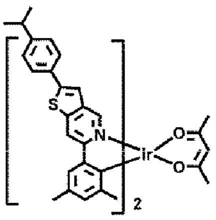
353

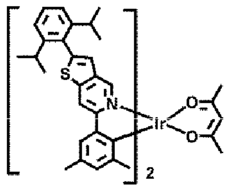
354

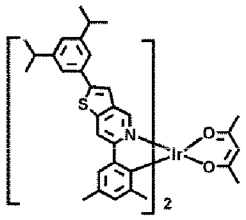
355

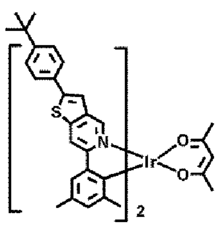
356

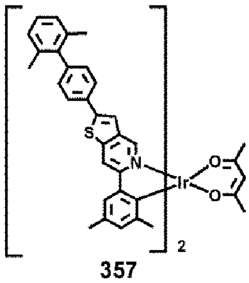
357

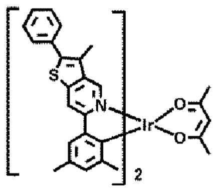
358

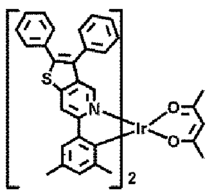
359

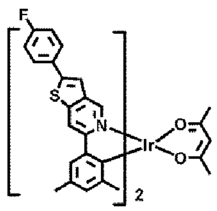
360

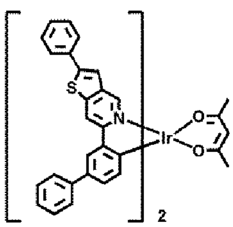
361

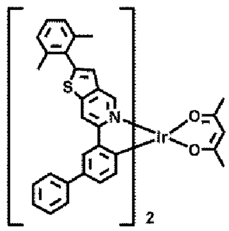
362

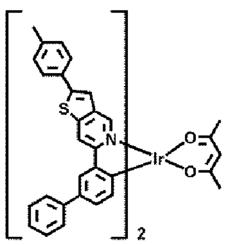
363

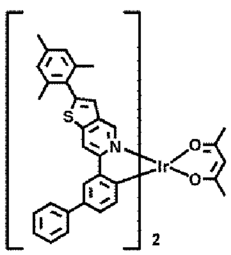
364

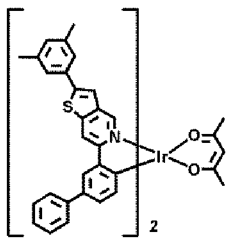
365

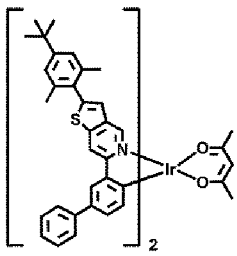
366

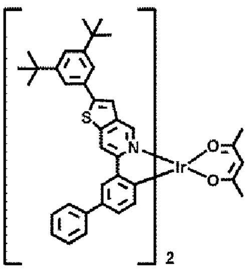
367

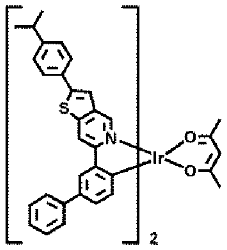
368

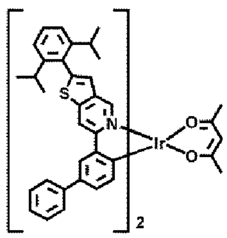
369

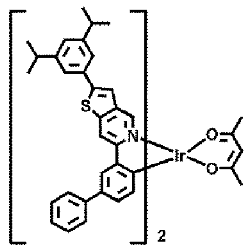
370

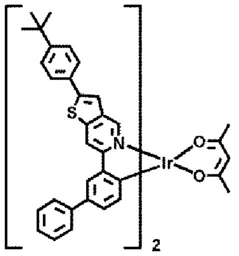
371
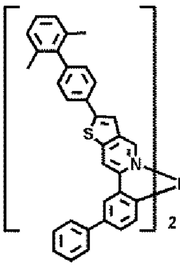
372
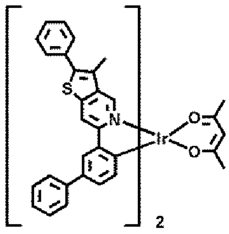
373
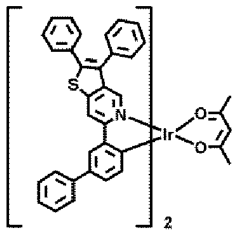
374
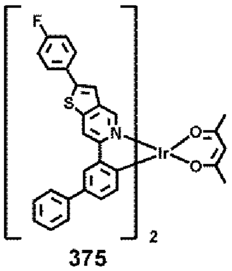
375

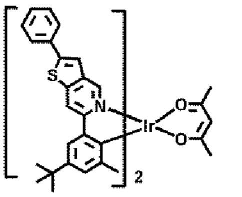
376
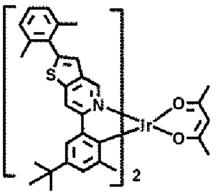
377
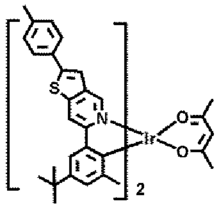
378
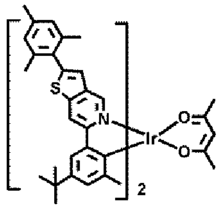
379
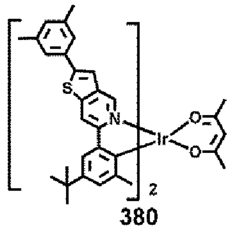
380

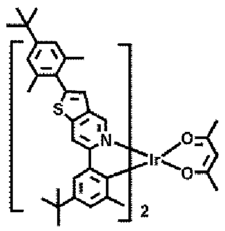
381
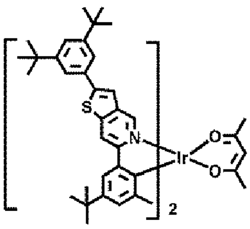
382
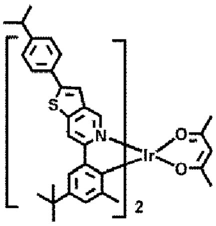
383
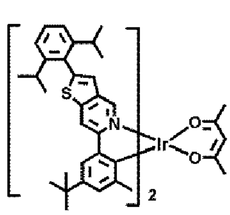
384
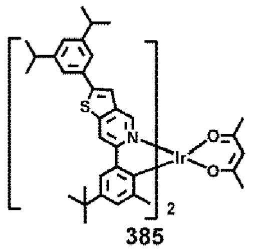
385

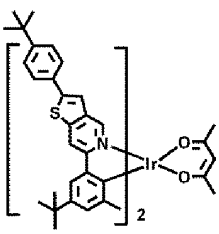
386
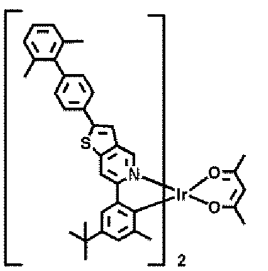
387
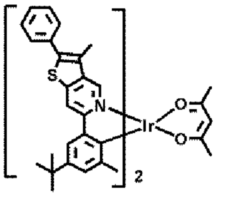
388
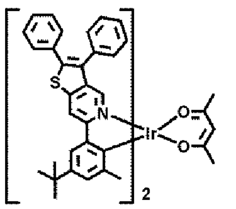
389
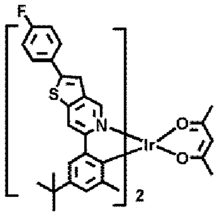
390

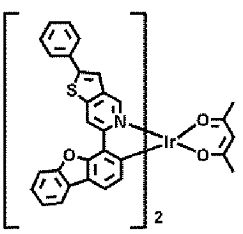
391
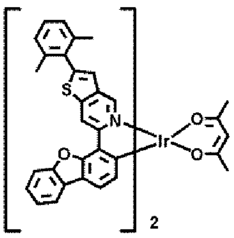
392
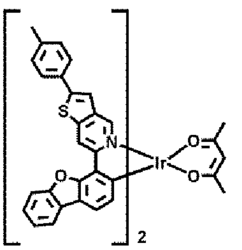
393
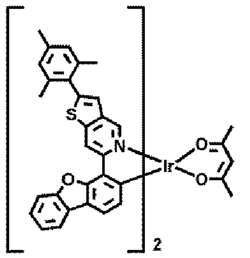
394
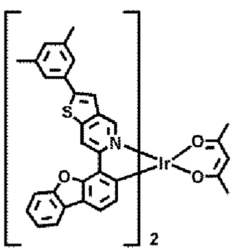
395

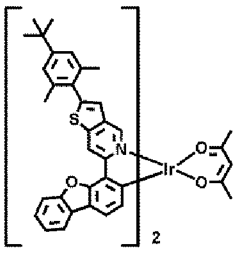
396

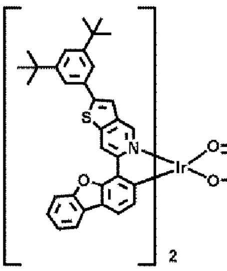
397

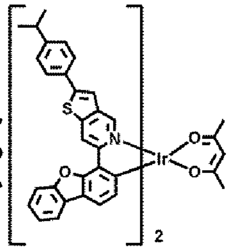
398

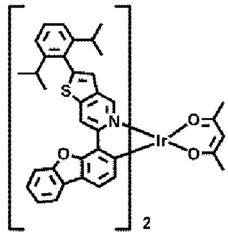
399

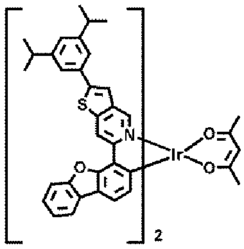
400

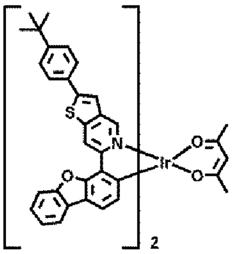
401

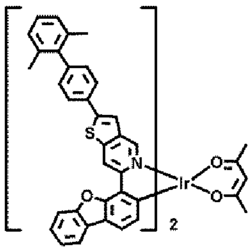
402

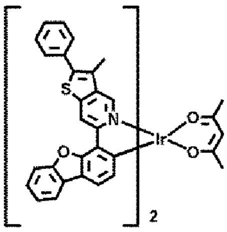
403

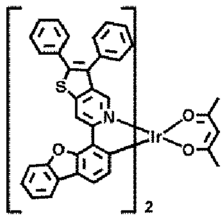
404

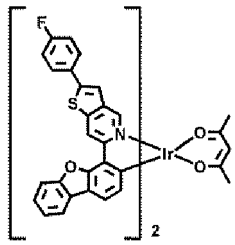
405

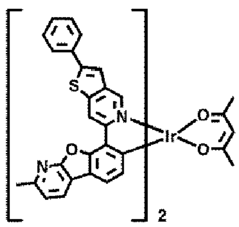
406

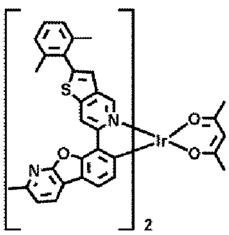
407

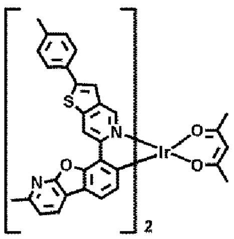
408

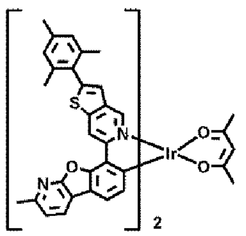
409

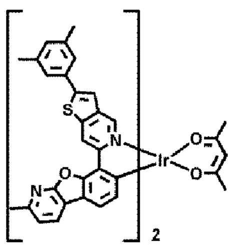
410

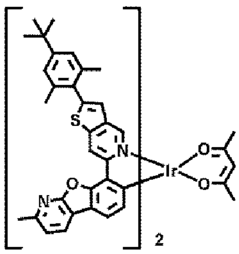
411

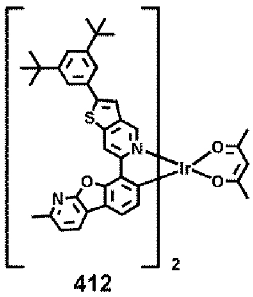
412

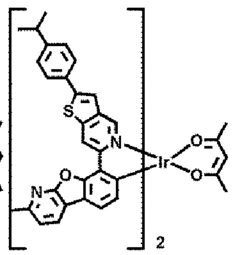
413

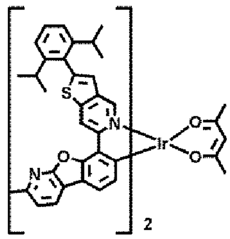
414

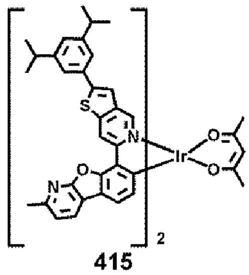
415

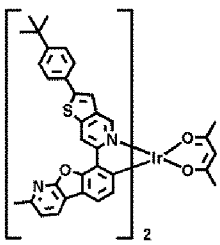
416

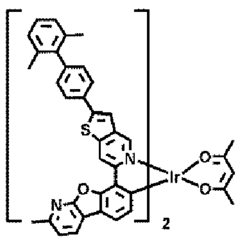
417

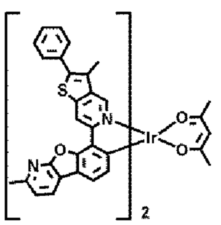
418

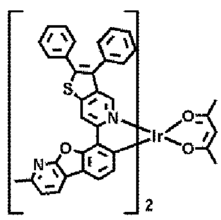
419

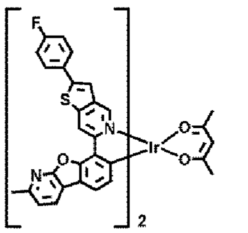
420

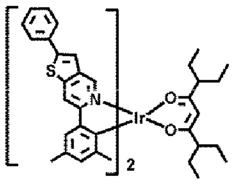
421
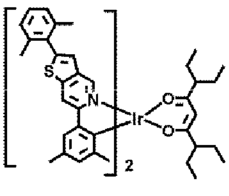
422
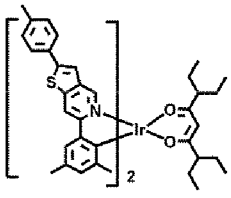
423
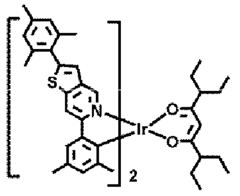
424
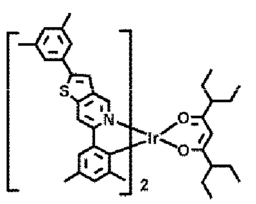
425

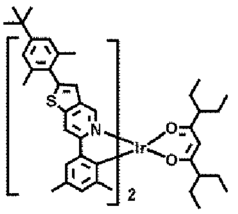
426
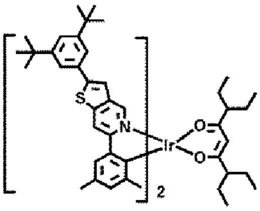
427
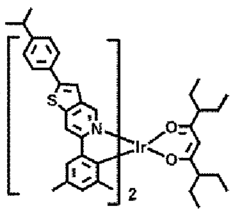
428
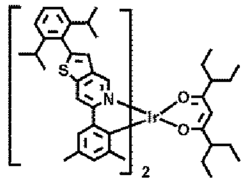
429
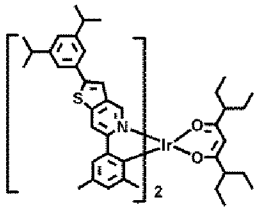
430

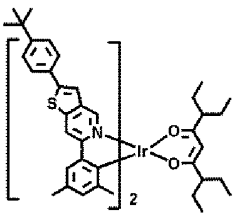
431
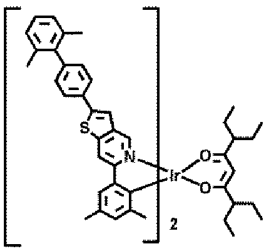
432
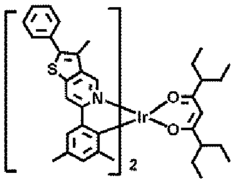
433
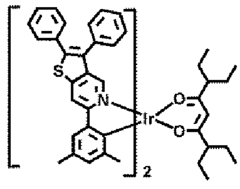
434
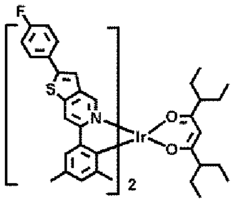
435

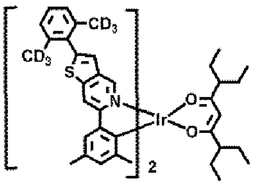
436
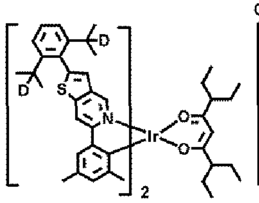
437
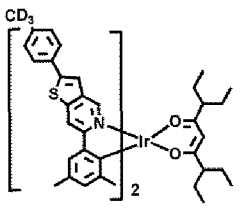
438
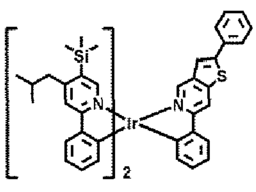
440

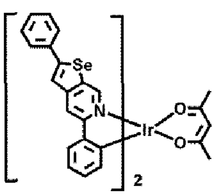
441
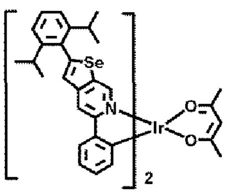
442
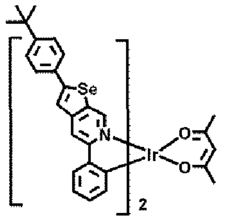
443
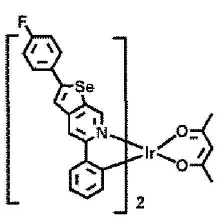
444
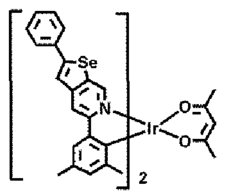
445

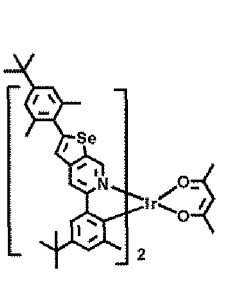
446
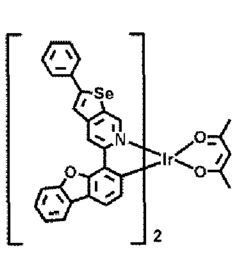
447
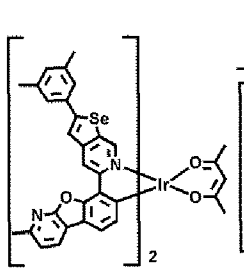
448
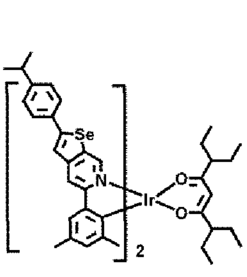
449
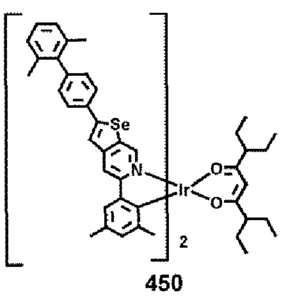
450

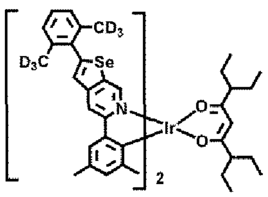
451
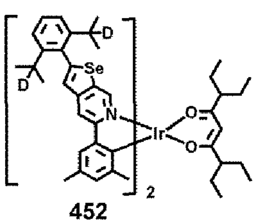
452
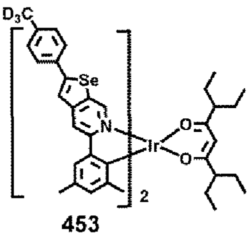
453
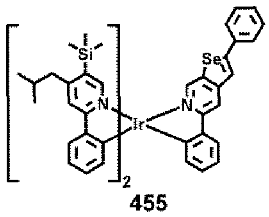
455

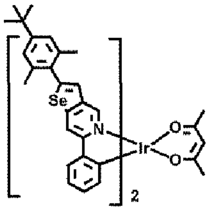
456
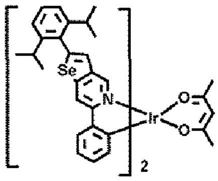
457
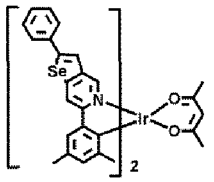
458
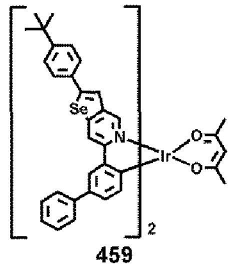
459
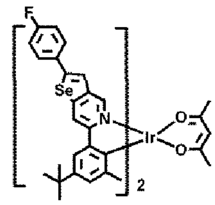
460

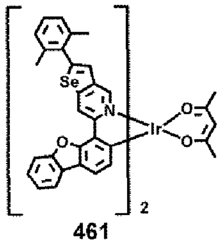
461
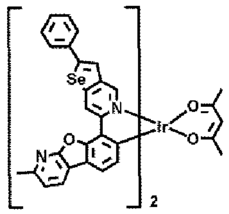
462
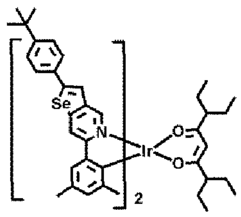
463
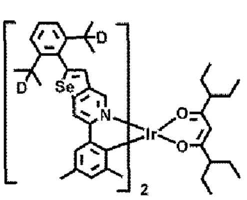
464
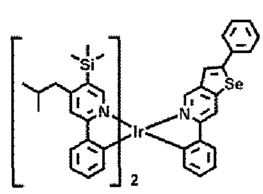
465

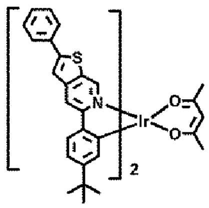
466
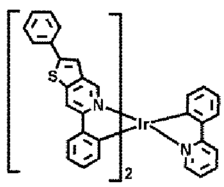
468
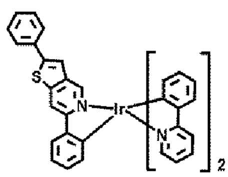
469

$L_1$ of the organometallic compound represented by Formula 1 may be a ligand represented by Formula 2A, and n1 which indicates the number of $L_1$ may be 1, 2, or 3. That is, the organometallic compound includes, as ligands linked to metal M, at least one ligand represented by Formula 2.

$X_2$ and $X_3$ in Formula 2 may each independently be O, S, Se, or $C(R_2)$, wherein $X_2$ or $X_3$ may be O, S, or Se. That is, in Formula 2, the 5-membered ring (see Formula 2') does not include *=N-*' (* and *' each indicate a binding site to a neighboring atom) as a ring-forming atom, and includes O, S, or Se. In addition, in Formula 2, the 5-membered ring is condensed with the 6-membered ring while sharing carbon 1 and carbon 2 (see Formula 2'). Therefore, a reduction in the intermolecular bonding force of the organometallic compound represented by Formula 1 may be prevented. Therefore, a reduction in the lifespan of an electronic device, for example, an organic light-emitting device, which includes the organometallic compound represented by Formula 1, may be prevented.

Formula 2'

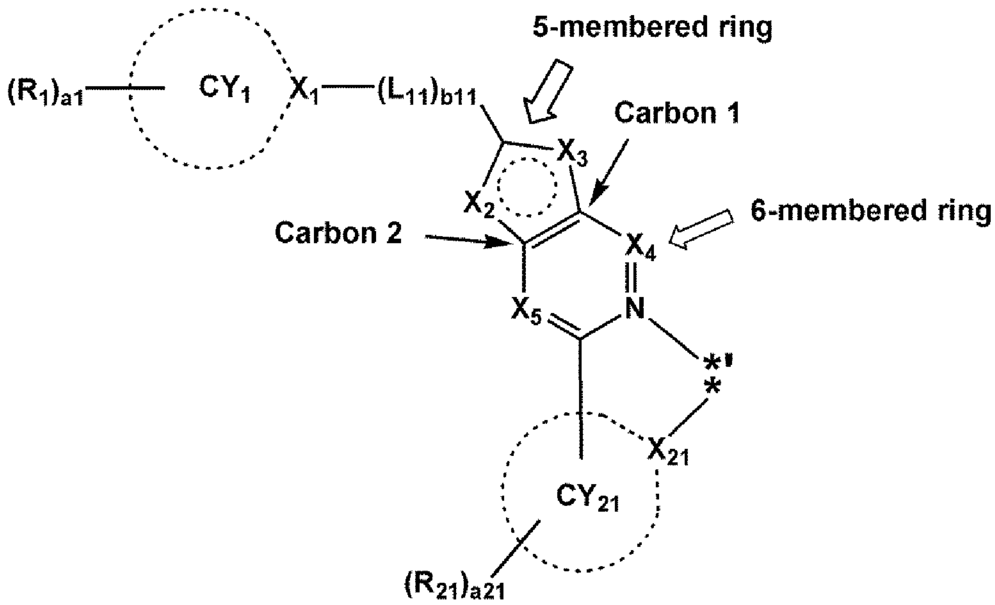

The ligand represented by Formula 2 includes "ring $CY_1$". Therefore, a transition dipole moment increases in an alignment axis direction of Formula 1, and the alignment of the organometallic compound represented by Formula 1 may be improved, thereby increasing the luminescence efficiency of an electronic device, for example, an organic light-emitting device, which includes the organometallic compound represented by Formula 1.

Meanwhile, in Formula 2, ring $CY_1$ and $R_2$ are not linked to each other, and $R_1$ and $R_2$ are not linked to each other. Therefore, it is possible to prevent the transition dipole moment of Formula 1 from being deviated to a direction other than the alignment axis direction of Formula 1, thereby increasing the luminescence efficiency of an electronic device, for example, an organic light-emitting device, which includes at least one organometallic compound represented by Formula 1.

A highest occupied molecular orbital (HOMO) energy level, a lowest unoccupied molecular orbital (LUMO) energy level, and a triplet ($T_1$) energy level of some compounds of the organometallic compound represented by Formula 1 are evaluated by a density functional theory (DFT) of Gaussian program with molecular structure optimization based on B3LYP, and results are shown in Table 1.

Table1

| Compound No. | HOMO (eV) | LUMO (eV) | $T_1$ (eV) |
|---|---|---|---|
| 1 | -4.623 | -1.748 | 2.253 |
| 16 (reference) | -4.469 | -1.747 | 2.121 |
| 31 | -4.614 | -1.809 | 2.215 |
| 236 | -4.517 | -1.786 | 2.103 |
| 346 | -4.567 | -1.598 | 2.208 |
| 439 (reference) | -4.618 | -1.678 | 2.235 |
| 466 | -4.625 | -1.770 | 2.203 |
| 467 (reference) | -4.480 | -1.677 | 2.187 |
| 468 | -4.811 | -1.660 | 2.278 |
| 469 | -4.836 | -1.637 | 2.287 |

Referring to Table 1, it is confirmed that the organometallic compound represented by Formula 1 has such electrical characteristics that are suitable for use in an electronic device, for example, an organic light-emitting device,

for use as a dopant.

Synthesis methods of the organometallic compound represented by Formula 1 may be understood by one of ordinary skill in the art by referring to Synthesis Examples provided below.

Therefore, the organometallic compound represented by Formula 1 may be suitable for use in an organic layer of an organic light-emitting device, for example, for use as a dopant in an emission layer of the organic layer. Another aspect of the present disclosure provides an organic light-emitting device including: a first electrode, a second electrode, and an organic layer disposed between the first electrode and the second electrode and including an emission layer, wherein the organic layer includes at least one organometallic compound represented by Formula 1.

The organic light-emitting device may have, due to the inclusion of an organic layer including the organometallic compound represented by Formula 1, a low driving voltage, high external quantum luminescence efficiency, a low roll-off ratio, and a long lifespan.

The organometallic compound represented by Formula 1 may be used between a pair of electrodes of an organic light-emitting device. For example, the organometallic compound represented by Formula 1 may be included in the emission layer. In this regard, the organometallic compound may act as a dopant, and the emission layer may further include a host (that is, an amount of the organometallic compound represented by Formula 1 is smaller than an amount of the host). The emission layer may emit, for example, green light or red light.

The expression "(an organic layer) includes at least one organometallic compound" as used herein may include a case in which "(an organic layer) includes identical organometallic compounds represented by Formula 1" and a case in which "(an organic layer) includes two or more different organometallic compounds represented by Formula 1".

For example, the organic layer may include, as the organometallic compound, only Compound 1. In this regard, Compound 1 may exist only in the emission layer of the organic light-emitting device. In one or more embodiments, the organic layer may include, as the organometallic compound, Compound 1 and Compound 2. In this regard, Compound 1 and Compound 2 may exist in an identical layer (for example, Compound 1 and Compound 2 both may exist in an emission layer).

The first electrode may be an anode, which is a hole injection electrode, and the second electrode may be a cathode, which is an electron injection electrode; or the first electrode may be a cathode, which is an electron injection electrode, and the second electrode may be an anode, which is a hole injection electrode.

For example, in the organic light-emitting device, the first electrode is an anode, and the second electrode is a cathode, and the organic layer further includes a hole transport region between the first electrode and the emission layer and an electron transport region between the emission layer and the second electrode, and the hole transport region includes a hole injection layer, a hole transport layer, an electron blocking layer, or any combination thereof, and the electron transport region includes a hole blocking layer, an electron transport layer, an electron injection layer, or any combination thereof.

The term "organic layer" as used herein refers to a single layer and/or a plurality of layers disposed between the first electrode and the second electrode of an organic light-emitting device. The "organic layer" may include, in addition to an organic compound, an organometallic complex including metal.

FIGURE is a schematic cross-sectional view of an organic light-emitting device 10 according to an embodiment. Hereinafter, the structure of an organic light-emitting device according to an embodiment and a method of manufacturing an organic light-emitting device according to an embodiment will be described in connection with the FIGURE. The organic light-emitting device 10 includes a first electrode 11, an organic layer 15, and a second electrode 19, which are sequentially stacked.

A substrate may be additionally disposed under the first electrode 11 or above the second electrode 19. For use as the substrate, any substrate that is used in general organic light-emitting devices may be used, and the substrate may be a glass substrate or a transparent plastic substrate, each having excellent mechanical strength, thermal stability, transparency, surface smoothness, ease of handling, and water resistance.

The first electrode 11 may be formed by depositing or sputtering a material for forming the first electrode 11 on the substrate. The first electrode 11 may be an anode. The material for forming the first electrode 11 may be a material with a high work function to facilitate hole injection. The first electrode 11 may be a reflective electrode, a semi-reflective electrode, or a transmissive electrode. The material for forming the first electrode may be, for example, indium tin oxide (ITO), indium zinc oxide (IZO), tin oxide ($SnO_2$), or zinc oxide (ZnO). In one or more embodiments, the material for forming the first electrode 11 may be metal or metal alloy, such as magnesium (Mg), aluminum (Al), aluminum-lithium (Al-Li), calcium (Ca), magnesium-indium (Mg-In), or magnesium-silver (Mg-Ag).

The first electrode 11 may have a single-layered structure or a multi-layered structure including two or more layers. For example, the first electrode 11 may have a three-layered structure of ITO/Ag/ITO, but the structure of the first electrode 11 is not limited thereto.

The organic layer 15 is disposed on the first electrode 11.

The organic layer 15 may include a hole transport region, an emission layer, and an electron transport region.

The hole transport region may be disposed between the first electrode 11 and the emission layer.

The hole transport region may include a hole injection layer, a hole transport layer, an electron blocking layer, a buffer layer, or any combination thereof.

The hole transport region may include only either a hole injection layer or a hole transport layer. In one or more embodiments, the hole transport region may have a hole injection layer/hole transport layer structure or a hole injection layer/hole transport layer/electron blocking layer structure, which are sequentially stacked in this stated order from the first electrode 11.

When the hole transport region includes a hole injection layer (HIL), the hole injection layer may be formed on the first electrode 11 by using one or more suitable methods, for example, vacuum deposition, spin coating, casting, and/or Langmuir-Blodgett (LB) deposition.

When a hole injection layer is formed by vacuum deposition, the deposition conditions may vary according to a material that is used to form the hole injection layer, and the structure and thermal characteristics of the hole injection layer. For example, the deposition conditions may include a deposition temperature of about 100 °C to about 500 °C, a vacuum pressure of about $10^{-8}$ torr to about $10^{-3}$ torr (1 Torr = 133.32 Pa) and a deposition rate of about 0.01 Å/sec to about 100 Å/sec. However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2,000 rpm to about 5,000 rpm, and a temperature at which a heat treatment is performed to remove a solvent after coating may be from about 80 °C to about 200 °C. However, the coating conditions are not limited thereto.

Conditions for forming a hole transport layer and an electron blocking layer may be understood by referring to conditions for forming the hole injection layer.

The hole transport region may include, for example, m-MTDATA, TDATA, 2-TNATA, NPB, β-NPB, TPD, spiro-TPD, spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), a compound represented by Formula 201 below, a compound represented by Formula 202 below, or any combination thereof:

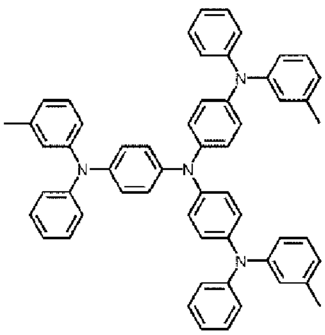

m-MTDATA

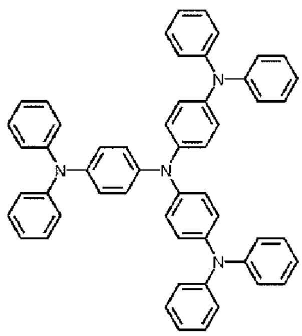

TDATA

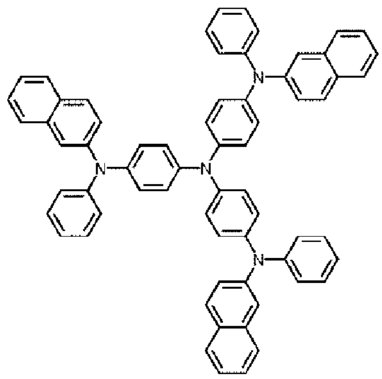

2-TNATA

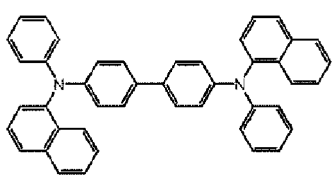

NPB

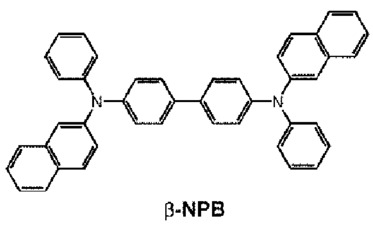

β-NPB

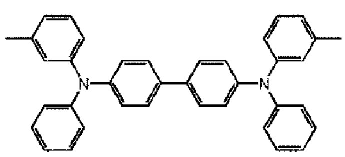

TPD

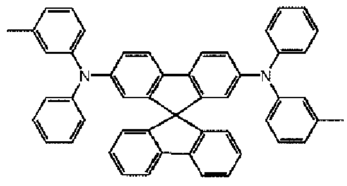

Spiro-TPD

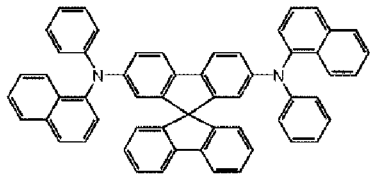

Spiro-NPB

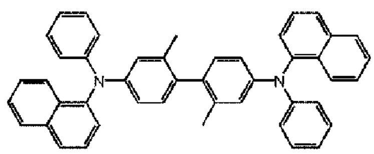

methylated NPB

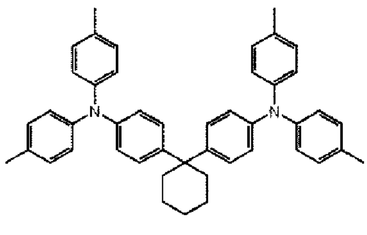

TAPC

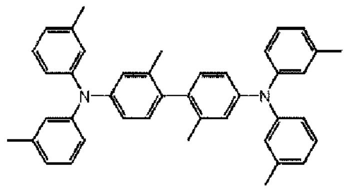

HMTPD

Formula 201

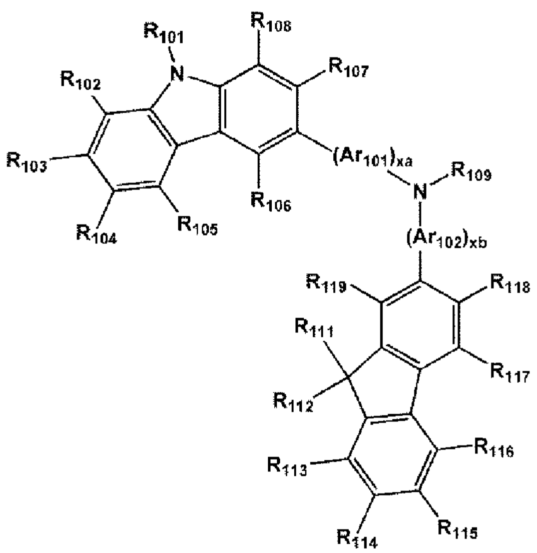

Formula 202

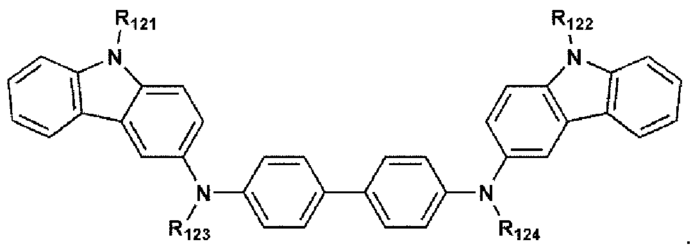

In Formula 201, $Ar_{101}$ and $Ar_{102}$ may each independently be a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group, each unsubstituted or substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl

group, a $C_3$-$C_{10}$ cycloalkenyl group, a $C_2$-$C_{10}$ heterocycloalkyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, or any combination thereof.

In Formula 201, xa and xb may each independently be an integer from 0 to 5, or may be 0, 1, or 2. For example, xa may be 1, and xb may be 0, but embodiments of the present disclosure are not limited thereto.

In Formulae 201 and 202, $R_{101}$ to $R_{108}$, $R_{111}$ to $R_{119}$, and $R_{121}$ to $R_{124}$ may each independently be:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group (for example, a methyl group, an ethyl group, a propyl group, a butyl group, pentyl group, a hexyl group, and the like), or a $C_1$-$C_{10}$ alkoxy group (for example, a methoxy group, an ethoxy group, a propoxy group, a butoxy group, a pentoxy group, and the like);

a $C_1$-$C_{10}$ alkyl group or a $C_1$-$C_{10}$ alkoxy group, each substituted with deuterium, - F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, or any combination thereof; or

a phenyl group, a naphthyl group, an anthracenyl group, a fluorenyl group, or a pyrenyl group, each unsubstituted or substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a $C_1$-$C_{10}$ alkoxy group, or any combination thereof,

but embodiments of the present disclosure are not limited thereto.

In Formula 201, $R_{109}$ may be a phenyl group, a naphthyl group, an anthracenyl group, or a pyridinyl group, each unsubstituted or substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a naphthyl group, an anthracenyl group, a pyridinyl group, or any combination thereof.

In one embodiment, the compound represented by Formula 201 may be represented by Formula 201A below, but embodiments of the present disclosure are not limited thereto:

Formula 201A

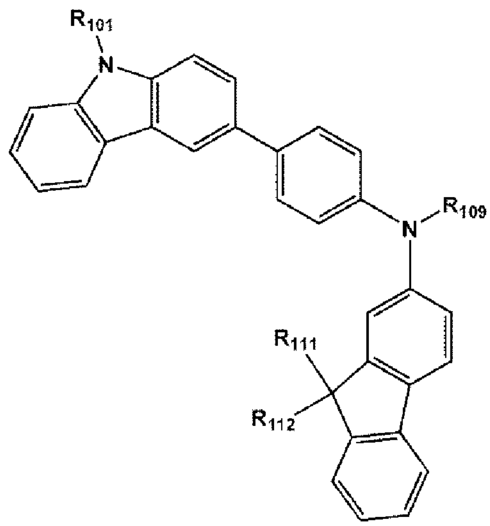

Detailed descriptions of $R_{101}$, $R_{111}$, $R_{112}$, and $R_{109}$ in Formula 201A are the same as described above.

For example, the compound represented by Formula 201 and the compound represented by Formula 202 may each include Compounds HT1 to HT20, but are not limited thereto:

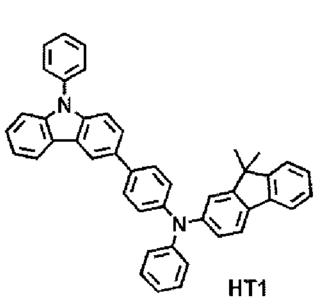
HT1

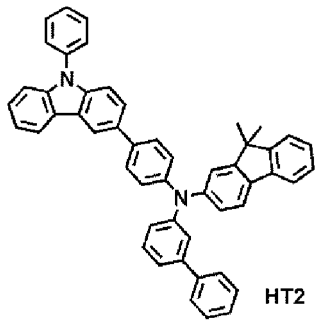
HT2

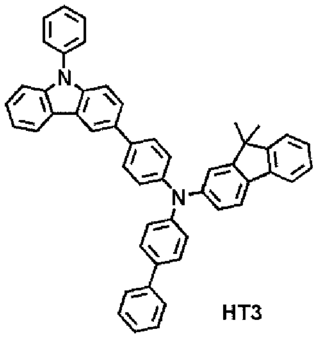
HT3

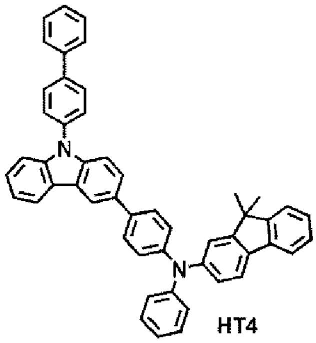
HT4

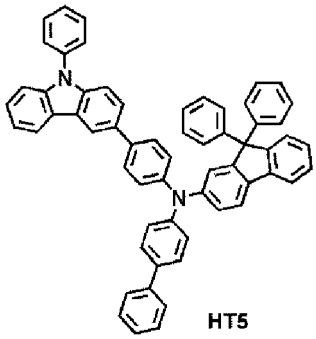
HT5

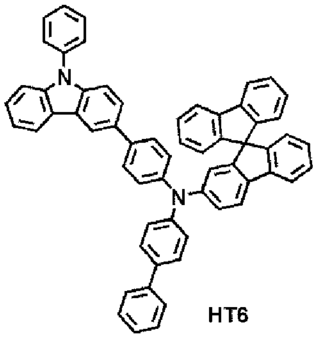
HT6

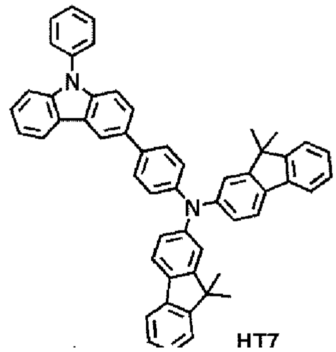
HT7

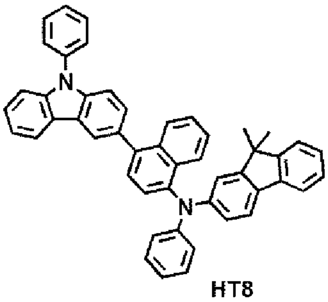
HT8

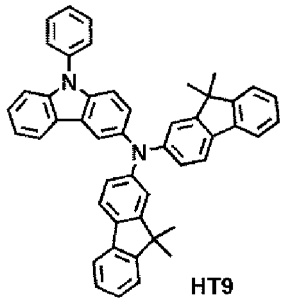
HT9

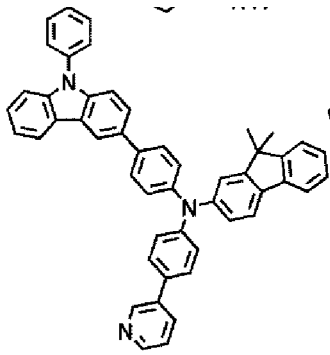
HT10

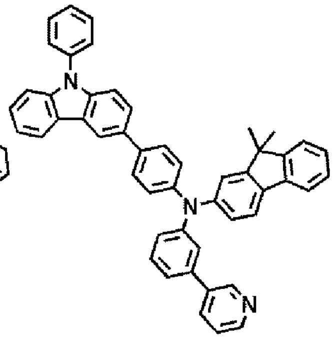
HT11

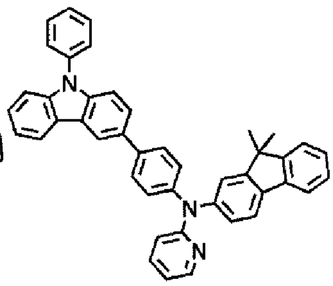
HT12

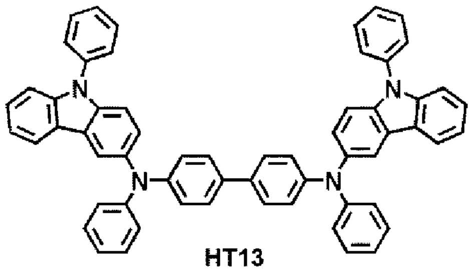

HT13

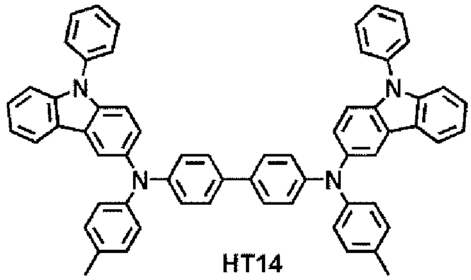

HT14

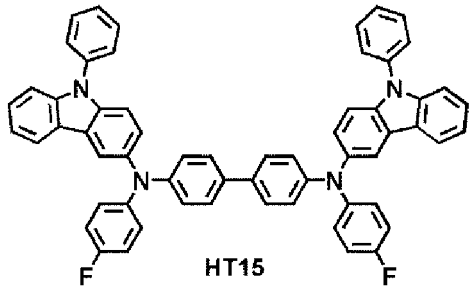

HT15

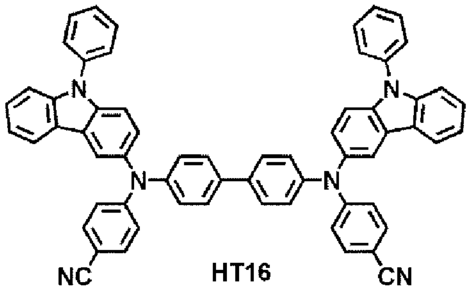

HT16

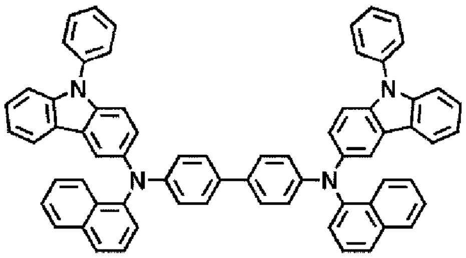

HT17

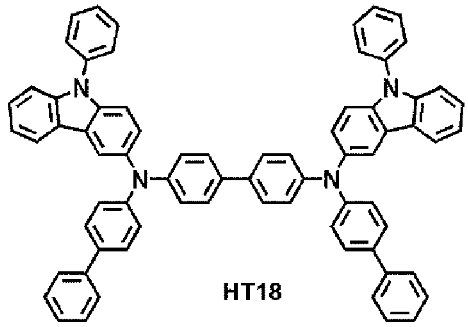

HT18

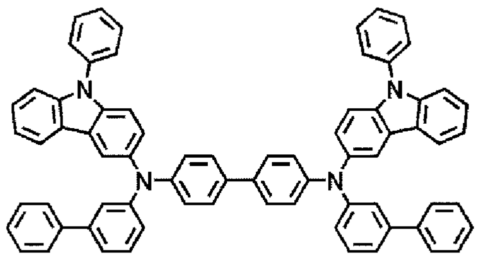

HT19

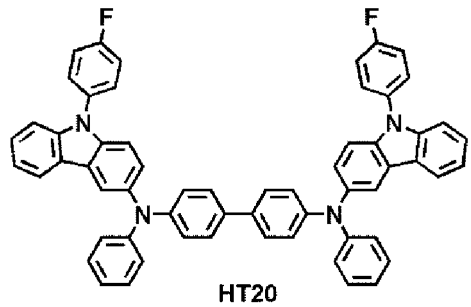

HT20

A thickness of the hole transport region may be from about 100 Å to about 10,000 Å, for example, about 100 Å to about 1,000 Å. When the hole transport region includes a hole injection layer, a hole transport layer, or any combination thereof, a thickness of the hole injection layer may be in a range of about 100 Å to about 10000 Å, for example, about 100 Å to about 1,000 Å, and a thickness of the hole transport layer may be in a range of about 50 Å to about 2,000 Å, for example about 100 Å to about 1,500 Å. When the thicknesses of the hole transport region, the hole injection layer, and the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be a quinone derivative, a metal oxide, a cyano group-containing compound, or any combination thereof, but embodiments of the present disclosure are not limited thereto. Non-limiting examples of the p-dopant are a quinone derivative, such as tetracyanoquinodimethane (TCNQ) or 2,3,5,6-tetrafluoro-tetracyano-1,4-benzoquinodimethane (F4-TCNQ); a metal oxide, such as a tungsten oxide or a molybdenum oxide; and a cyano group-containing compound, such as Compound HT-D1 below, but are not limited thereto:

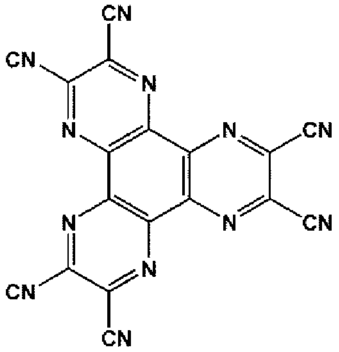

HT-D1

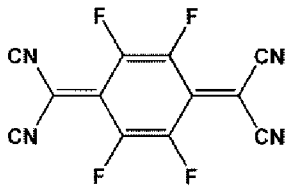

F4-TCNQ .

The hole transport region may include a buffer layer.

Also, the buffer layer may compensate for an optical resonance distance according to a wavelength of light emitted from the emission layer, and thus, efficiency of a formed organic light-emitting device may be improved.

Meanwhile, when the hole transport region includes an electron blocking layer, a material for the electron blocking layer may be a material as described for the hole transport region described above, a material for a host to be explained later, or any combination thereof. However, the material for the electron blocking layer is not limited thereto. For example, when the hole transport region includes an electron blocking layer, a material for the electron blocking layer may be mCP, which will be explained later.

An emission layer may be formed on the hole transport region by vacuum deposition, spin coating, casting, LB deposition, or the like. When the emission layer is formed by vacuum deposition or spin coating, the deposition or coating conditions may be similar to those applied in forming the hole injection layer although the deposition or coating conditions may vary according to a compound that is used to form the emission layer.

The emission layer may include a host and a dopant, and the dopant may include the organometallic compound represented by Formula 1.

The host may include TPBi, TBADN, ADN (also referred to as "DNA"), CBP, CDBP, TCP, mCP, one of Compounds H50 to H52, or any combination thereof:

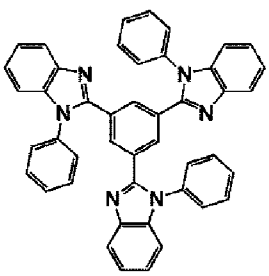

TPBi

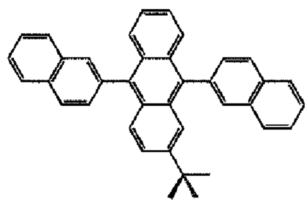

TBADN

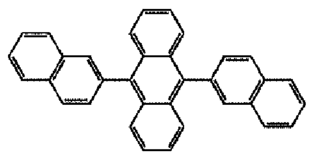

ADN

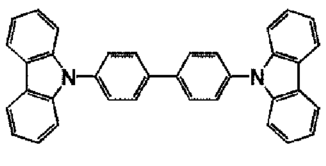

CBP

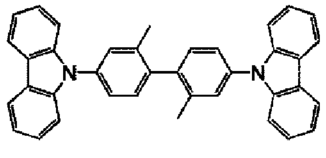

CDBP

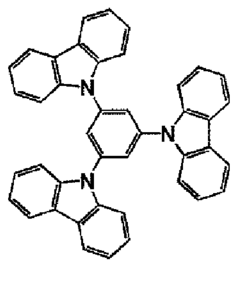

TCP

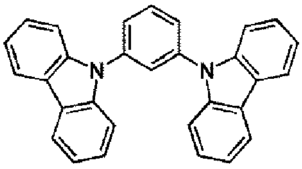
mCP

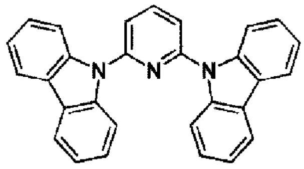
H50

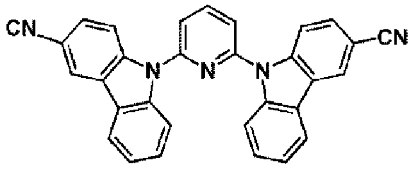
H51

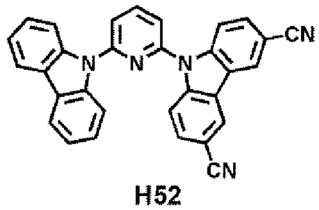
H52

When the organic light-emitting device is a full-color organic light-emitting device, the emission layer may be patterned into a red emission layer, a green emission layer, and/or a blue emission layer. In one or more embodiments, due to a stacked structure including a red emission layer, a green emission layer, and/or a blue emission layer, the emission layer may emit white light.

When the emission layer includes a host and a dopant, an amount of the dopant may be in a range of about 0.01 parts by weight to about 15 parts by weight based on 100 parts by weight of the host, but embodiments of the present disclosure are not limited thereto.

A thickness of the emission layer may be in a range of about 100 Å to about 1,000 Å, for example, about 200 Å to about 600 Å. When the thickness of the emission layer is within this range, excellent light-emission characteristics may be obtained without a substantial increase in driving voltage.

An electron transport region may be disposed on the emission layer.

The electron transport region may include a hole blocking layer, an electron transport layer, an electron injection layer, or any combination thereof.

For example, the electron transport region may have a hole blocking layer/electron transport layer/electron injection layer structure or an electron transport layer/electron injection layer structure, but the structure of the electron transport region is not limited thereto. The electron transport layer may have a single-layered structure or a multi-layered structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, BCP, Bphen, BAlq, or any combination thereof, but embodiments of the present disclosure are not limited thereto:

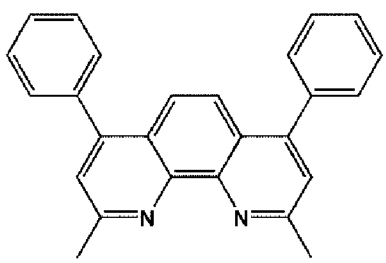
BCP

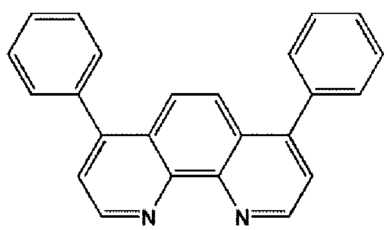
Bphen

A thickness of the hole blocking layer may be from about 20 Å to about 1,000 Å, for example, about 30 Å to about 300 Å. When the thickness of the hole blocking layer is within these ranges, the hole blocking layer may have excellent hole blocking characteristics without a substantial increase in driving voltage.

The electron transport layer may include BCP, Bphen, $Alq_3$, BAlq, TAZ, NTAZ, or any combination thereof:

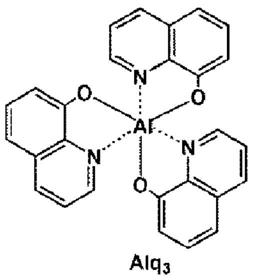

Alq$_3$

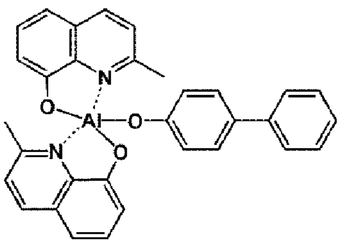

BAlq

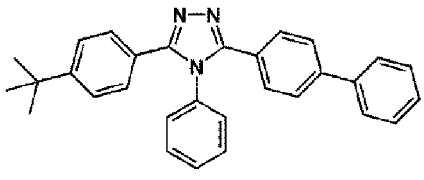

TAZ

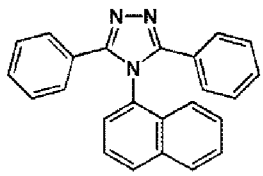

NTAZ

.

In one or more embodiments, the electron transport layer may include one of ET1 to ET25, but embodiments of the present disclosure are not limited thereto:

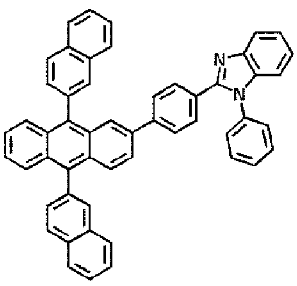

ET1

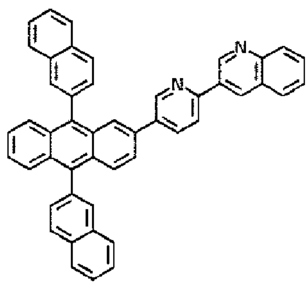

ET2

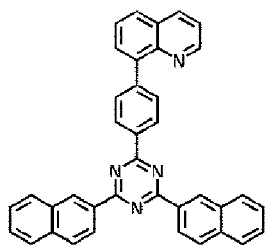

ET3

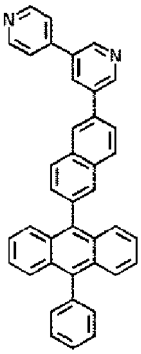

ET4

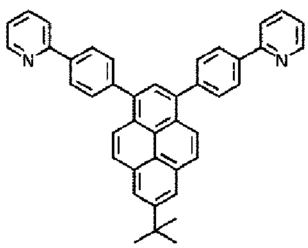

ET5

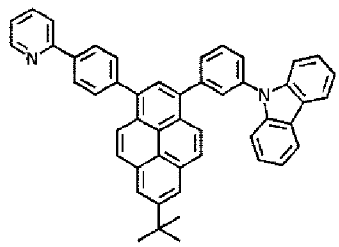

ET6

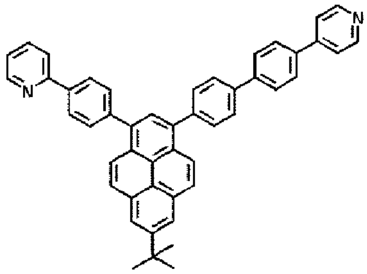

ET7

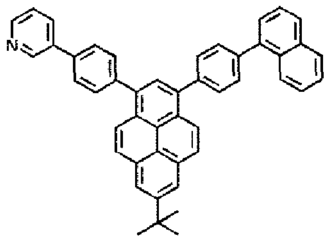

ET8

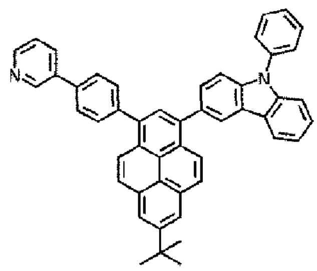

ET9

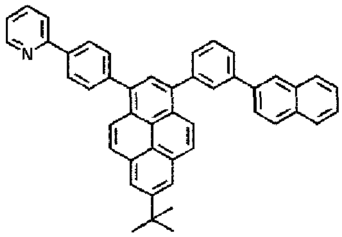
**ET10**

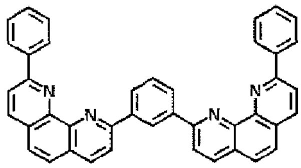
**ET11**

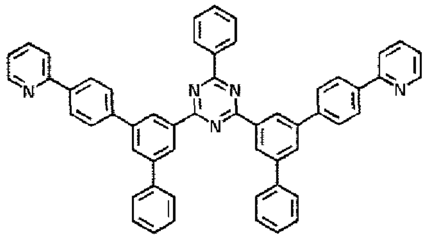
**ET12**

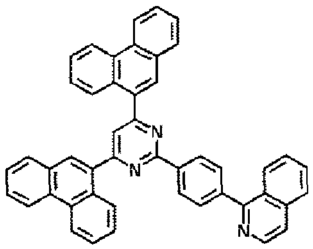
**ET13**

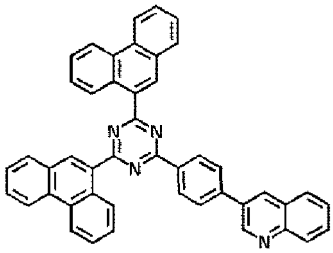
**ET14**

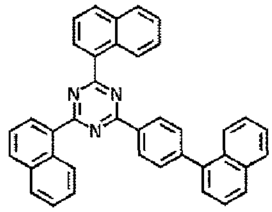
**ET15**

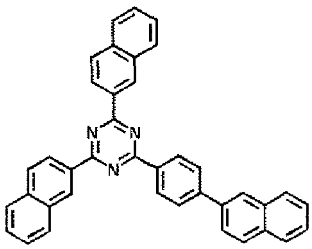
**ET16**

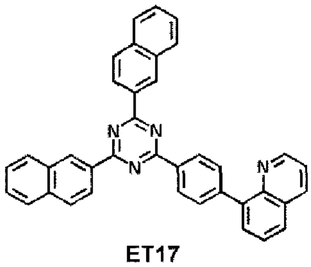
**ET17**

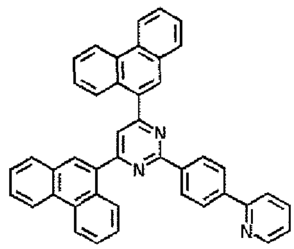
**ET18**

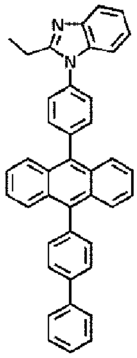
**ET19**

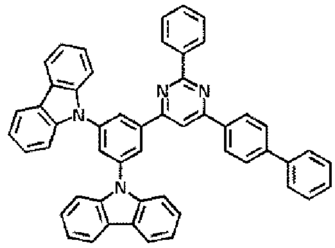
**ET20**

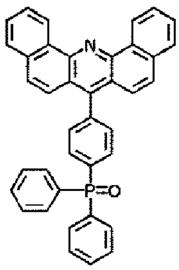
**ET21**

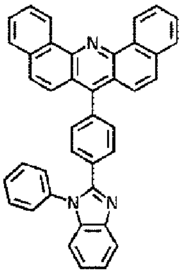

ET22

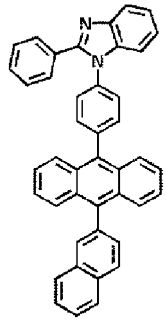

ET23

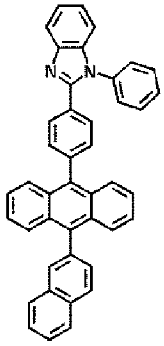

ET24

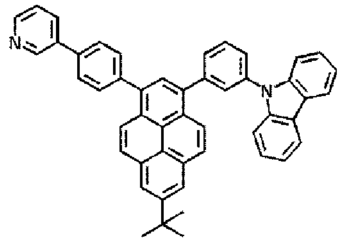

ET25

.

A thickness of the electron transport layer may be from about 100 Å to about 1,000 Å, for example, about 150 Å to about 500 Å. When the thickness of the electron transport layer is within the range described above, the electron transport layer may have satisfactory electron transport characteristics without a substantial increase in driving voltage.

Also, the electron transport layer may further include, in addition to the materials described above, a metal-containing material.

The metal-containing material may include a Li complex. The Li complex may include, for example, Compound ET-D1 (LiQ), ET-D2, or any combination thereof:

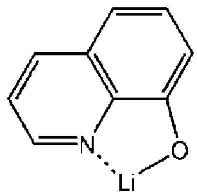

ET-D1

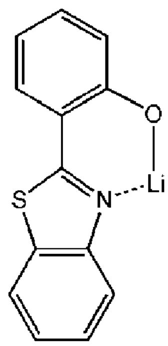

ET-D2

.

The electron transport region may include an electron injection layer that promotes the flow of electrons from the second electrode 19 thereinto.

The electron injection layer may include LiF, NaCl, CsF, $Li_2O$, BaO, or any combination thereof.

A thickness of the electron injection layer may be from about 1 Å to about 100 Å, for example, about 3 Å to about 90 Å. When a thickness of the electron injection layer is within these ranges, satisfactory electron injection characteristics may be obtained without substantial increase in driving voltage.

The second electrode 19 is disposed on the organic layer 15. The second electrode 19 may be a cathode. A material for forming the second electrode 19 may be metal, an alloy, an electrically conductive compound, or a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be used as the material for forming the second electrode 19. To manufacture a top-emission type light-emitting device, a transmissive electrode formed using ITO or IZO may be used as the second electrode 19.

Hereinbefore, the organic light-emitting device according to an embodiment has been described in connection with the FIGURE.

Another aspect of the present disclosure provides a diagnostic composition including at least one organometallic compound represented by Formula 1.

The organometallic compound represented by Formula 1 provides high luminescence efficiency. Accordingly, a diagnostic composition including the organometallic compound may have high diagnostic efficiency.

The diagnostic composition may be used in various applications including a diagnosis kit, a diagnosis reagent, a biosensor, and a biomarker.

The term "$C_1$-$C_{60}$ alkyl group" as used herein refers to a linear or branched saturated aliphatic hydrocarbon monovalent group having 1 to 60 carbon atoms, and examples thereof include a methyl group, an ethyl group, a propyl

group, an isobutyl group, a sec-butyl group, a tert-butyl group, a pentyl group, an isoamyl group, and a hexyl group. The term "$C_1$-$C_{60}$ alkylene group" as used herein refers to a divalent group having the same structure as the $C_1$-$C_{60}$ alkyl group.

The term "$C_1$-$C_{60}$ alkoxy group" as used herein refers to a monovalent group represented by -$OA_{101}$ (wherein $A_{101}$ is the $C_1$-$C_{60}$ alkyl group), and examples thereof include a methoxy group, an ethoxy group, and an isopropyloxy group.

The term "$C_2$-$C_{60}$ alkenyl group" as used herein refers to a hydrocarbon group having at least one carbon-carbon double bond in the middle or at the terminus of the $C_2$-$C_{60}$ alkyl group, and examples thereof include an ethenyl group, a propenyl group, and a butenyl group. The term "$C_2$-$C_{60}$ alkenylene group" as used herein refers to a divalent group having the same structure as the $C_2$-$C_{60}$ alkenyl group.

The term "$C_2$-$C_{60}$ alkynyl group" as used herein refers to a hydrocarbon group having at least one carbon-carbon triple bond in the middle or at the terminus of the $C_2$-$C_{60}$ alkyl group, and examples thereof include an ethynyl group, and a propynyl group. The term "$C_2$-$C_{60}$ alkynylene group" as used herein refers to a divalent group having the same structure as the $C_2$-$C_{60}$ alkynyl group.

The term "$C_3$-$C_{10}$ cycloalkyl group" as used herein refers to a monovalent saturated hydrocarbon monocyclic group having 3 to 10 carbon atoms, and non-limiting examples thereof include a cyclopropyl group, a cyclobutyl group, a cyclopentyl group, a cyclohexyl group, and a cycloheptyl group. The term "$C_3$-$C_{10}$ cycloalkylene group" as used herein refers to a divalent group having the same structure as the $C_3$-$C_{10}$ cycloalkyl group.

The term "$C_2$-$C_{10}$ heterocycloalkyl group" as used herein refers to a monovalent saturated monocyclic group having N, O, P, Si, Se, S, or a combination thereof and 2 to 10 carbon atoms as ring-forming atoms, and non-limiting examples thereof include a tetrahydrofuranyl group, and a tetrahydrothiophenyl group. The term "$C_2$-$C_{10}$ heterocycloalkylene group" as used herein refers to a divalent group having the same structure as the $C_2$-$C_{10}$ heterocycloalkyl group.

The term "$C_3$-$C_{10}$ cycloalkenyl group" as used herein refers to a monovalent monocyclic group that has 3 to 10 carbon atoms and at least one carbon-carbon double bond in the ring thereof and no aromaticity, and non-limiting examples thereof include a cyclopentenyl group, a cyclohexenyl group, and a cycloheptenyl group. The term "$C_3$-$C_{10}$ cycloalkenylene group" as used herein refers to a divalent group having the same structure as the $C_3$-$C_{10}$ cycloalkenyl group.

The term "$C_2$-$C_{10}$ heterocycloalkenyl group" as used herein refers to a monovalent monocyclic group that has N, O, P, Si, Se, S, or a combination thereof and 1 to 10 carbon atoms as ring-forming atoms, and a double bond in the ring. Examples of the $C_2$-$C_{10}$ heterocycloalkenyl group are a 2,3-dihydrofuranyl group, and a 2,3-dihydrothiophenyl group. The term "$C_2$-$C_{10}$ heterocycloalkenylene group" as used herein refers to a divalent group having the same structure as the $C_2$-$C_{10}$ heterocycloalkenyl group.

The term "$C_6$-$C_{60}$ aryl group" as used herein refers to a monovalent group having a carbocyclic aromatic system having 6 to 60 carbon atoms, and the term "$C_6$-$C_{60}$ arylene group" as used herein refers to a divalent group having a carbocyclic aromatic system having 6 to 60 carbon atoms. Non-limiting examples of the $C_6$-$C_{60}$ aryl group include a phenyl group, a naphthyl group, an anthracenyl group, a phenanthrenyl group, a pyrenyl group, and a chrysenyl group. When the $C_6$-$C_{60}$ aryl group and the $C_6$-$C_{60}$ arylene group each include two or more rings, the rings may be fused to each other.

The term "$C_1$-$C_{60}$ heteroaryl group" as used herein refers to a monovalent group having a heterocyclic aromatic system that has N, O, P, Si, Se, S, or a combination thereof and 2 to 60 carbon atoms as ring-forming atoms. The term "$C_1$-$C_{60}$ heteroarylene group" as used herein refers to a divalent group having a heterocyclic aromatic system that has N, O, P, Si, Se, S, or a combination thereof as ring-forming atoms. Non-limiting examples of the $C_1$-$C_{60}$ heteroaryl group include a pyridinyl group, a pyrimidinyl group, a pyrazinyl group, a pyridazinyl group, a triazinyl group, a quinolinyl group, and an isoquinolinyl group. When the $C_1$-$C_{60}$ heteroaryl group and the $C_1$-$C_{60}$ heteroarylene group each include two or more rings, the rings may be fused to each other.

The term "$C_6$-$C_{60}$ aryloxy group" as used herein indicates -$OA_{102}$ (wherein $A_{102}$ is the $C_6$-$C_{60}$ aryl group), and the term "$C_6$-$C_{60}$ arylthio group" as used herein indicates - $SA_{103}$ (wherein $A_{103}$ is the $C_6$-$C_{60}$ aryl group).

The term "monovalent non-aromatic condensed polycyclic group" as used herein refers to a monovalent group (for example, having 8 to 60 carbon atoms) having two or more rings condensed to each other, only carbon atoms as ring-forming atoms, and no aromaticity in its entire molecular structure. Examples of the monovalent non-aromatic condensed polycyclic group include a fluorenyl group. The term "divalent non-aromatic condensed polycyclic group" as used herein refers to a divalent group having the same structure as the monovalent non-aromatic condensed polycyclic group.

The term "monovalent non-aromatic condensed heteropolycyclic group" as used herein refers to a monovalent group (for example, having 2 to 60 carbon atoms) having two or more rings condensed to each other, N, O, P, Si, Se, S, or any combination thereof, other than carbon atoms, as ring-forming atoms, and no aromaticity in its entire molecular structure. Non-limiting examples of the monovalent non-aromatic condensed heteropolycyclic group include a carbazolyl group. The term "divalent non-aromatic condensed heteropolycyclic group" as used herein refers to a divalent group

having the same structure as the monovalent non-aromatic condensed heteropolycyclic group.

The term "$C_5$-$C_{30}$ carbocyclic group" as used herein refers to a saturated or unsaturated cyclic group having, as ring-forming atoms, 5 to 30 carbon atoms only. The $C_5$-$C_{30}$ carbocyclic group may be a monocyclic group or a polycyclic group.

The term "$C_2$-$C_{30}$ heterocyclic group" as used herein refers to a saturated or unsaturated cyclic group having, N, O, Si, P, Se, S, or any combination thereof and 1 to 30 carbon atoms as ring-forming atoms. The $C_2$-$C_{30}$ heterocyclic group may be a monocyclic group or a polycyclic group.

A substituent(s) of the substituted $C_5$-$C_{30}$ carbocyclic group, the substituted $C_2$-$C_{30}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_2$-$C_{10}$ heterocycloalkyl group, the substituted $C_3$-$C_{10}$ cycloalkenyl group, the substituted $C_2$-$C_{10}$ heterocycloalkenyl group, the substituted $C_6$-$C_{60}$ aryl group, the substituted $C_6$-$C_{60}$ aryloxy group, the substituted $C_6$-$C_{60}$ arylthio group, the substituted $C_1$-$C_{60}$ heteroaryl group, the substituted monovalent non-aromatic condensed polycyclic group, and the substituted monovalent non-aromatic condensed heteropolycyclic group may be:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;
a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_2$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, - $N(Q_{11})(Q_{12})$, $-Si(Q_{13})(Q_{14})(Q_{15})$, $-B(Q_{16})(Q_{17})$, $-P(=O)(Q_{18})(Q_{19})$, or any combination thereof;
a $C_3$-$C_{10}$ cycloalkyl group, a $C_2$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group;
a $C_3$-$C_{10}$ cycloalkyl group, a $C_2$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group, each substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_2$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, $-N(Q_{21})(Q_{22})$, - $Si(Q_{23})(Q_{24})(Q_{25})$, $-B(Q_{26})(Q_{27})$, $-P(=O)(Q_{28})(Q_{29})$, or any combination thereof;
$-N(Q_{31})(Q_{32})$, $-Si(Q_{33})(Q_{34})(Q_{35})$, $-B(Q_{36})(Q_{37})$, or $-P(=O)(Q_{38})(Q_{39})$; or any combination thereof.

In the present specification, $Q_1$ to $Q_9$, $Q_{11}$ to $Q_{19}$, $Q_{21}$ to $Q_{29}$, and $Q_{31}$ to $Q_{39}$ may each independently be hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_2$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryl group substituted with a $C_1$-$C_{60}$ alkyl group, a $C_6$-$C_{60}$ aryl group or any combination thereof, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, or any combination thereof.

Hereinafter, a compound and an organic light-emitting device according to embodiments are described in detail with reference to Synthesis Example and Examples. However, the organic light-emitting device is not limited thereto. The wording "B was used instead of A" used in describing Synthesis Examples means that an amount of A used was identical to an amount of B used, in terms of a molar equivalent.

Examples

Synthesis Example 1 (Compound 1)

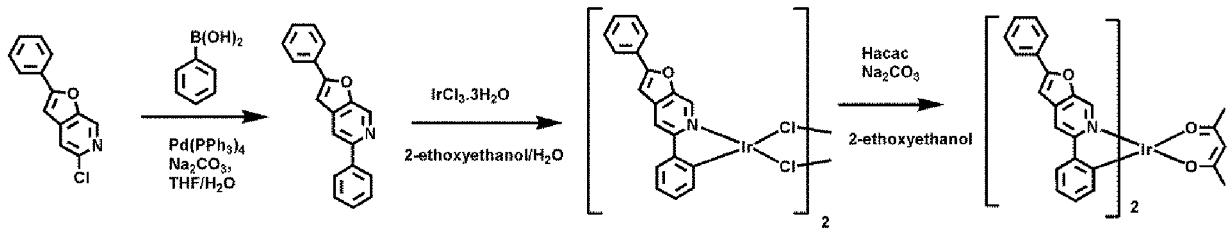


L1 L1-dimer 1

Synthesis of Intermediate L1

5-chloro-2-phenylfuro[2,3-c]pyridine (2.55 g, 11.1 mmol), phenylboronic acid (2.603 g, 16.64 mmol), $Pd(PPh_3)_4$ (1.03 g, 0.89 mmol), and $K_2CO_3$ (3.83 g, 27.7 mmol) were mixed with 60 mL of tetrahydrofuran and 30 mL of distilled water, stirred at a temperature of 90 °C for 18 hours, and then cooled to room temperature. The reaction mixture was extracted with ethyl acetate, dried by using anhydrous magnesium sulfate ($MgSO_4$), and filtered to obtain a filtrate. The filtrate was concentrated under vaccuum to obtain a residue. The residue was purified by column chromatography using ethyl acetate:hexane = 1:2 (V/V) as an eluent to obtain Intermediate L1 (2.90 g, 83 %). LC-MS m/z = 272.31 $(M+H)^+$.

Synthesis of Intermediate L1-dimer

Intermediate L1 (1.99 g, 7.32 mmol) and Iridium chloride hydrate (1.15 g, 3.25 mmol) were mixed with 21 mL of 2-ethoxy ethanol and 7 mL of distilled water, stirred at a temperature of 120 °C for 24 hours under reflux, and then cooled to room temperature. A solid obtained therefrom was filtered, and the filtered solid was sufficiently washed in the order of water/methanol/hexane. The solid was dried in a vacuum oven to obtain Intermediate L1-dimer (1.95 g, 78 %). The obtained compound was used in a next reaction without additional purification.

Synthesis of Compound 1

30 mL of 2-ethoxy ethanol was added to Intermediate L1-dimer (1.94 g, 1.26 mmol), acetyl acetone (1.26 g, 12.6 mmol), and $Na_2CO_3$ (1.33 g, 12.6 mmol), and stirred at room temperature for 12 hours. The reaction mixture was extracted using ethyl acetate, dried by using anhydrous magnesium sulfate ($MgSO_4$), and filtered to obtain a filtrate. The filtrate was concentrated under vaccuum to obtain a residue. The residue was purified by column chromatography using methylene chloride:hexane = 1:4 (V/V) as an eluent to obtain Compound 1 (0.453 g, 22 %). The obtained compound was identified by Mass Specrometry (MS) and HPLC analysis. HRMS(MALDI-TOF) calcd for $C_{43}H_{31}IrN_2O_4$: m/z 832.1913, Found: 832.1912.

Reference Synthesis Example 2 (Compound 16)

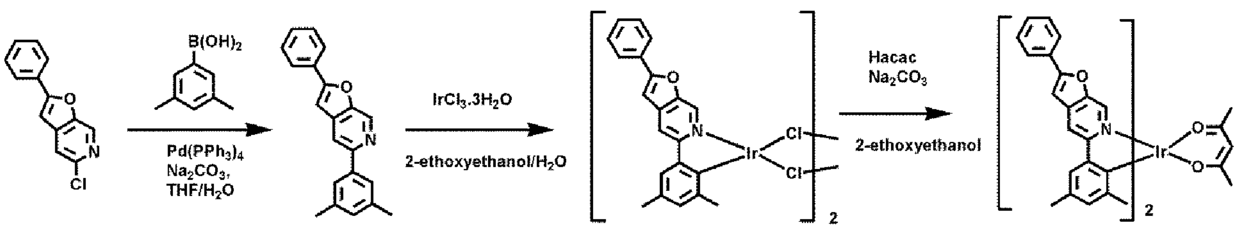


L16 L16-dimer 16

Synthesis of Intermediate L16

5-chloro-2-phenylfuro[2,3-c]pyridine (2.69 g, 11.71 mmol), (3,5-dimethylphenyl)boronic acid (2.64 g, 17.56 mmol), $Pd(PPh_3)_4$ (1.08 g, 0.94 mmol), and $K_2CO_3$ (4.05 g, 29.3 mmol) were mixed with 60 mL of tetrahydrofuran and 30 mL of distilled water, stirred at a temperature of 90 °C for 18 hours, and then cooled to room temperature. The reaction mixture was extracted using ethyl acetate, dried by using anhydrous magnesium sulfate ($MgSO_4$), and filtered to obtain a filtrate. The filtrate was concentrated under vaccuum to obtain a residue. The residue was purified by column chromatography using ethyl acetate:hexane = 1:2 (V/V) as an eluent to obtain Intermediate L16 (3.02 g, 86 %).

LC-MS m/z = 300.13 $(M+H)^+$.

Synthesis of Intermediate L16-dimer

Intermediate L16 (2.04 g, 6.82 mmol) and iridium chloride hydrate (1.07 g, 3.03 mmol) were mixed with 21 mL of 2-ethoxy ethanol and 7 mL of distilled water, stirred at a temperature of 120 °C for 24 hours under reflux, and then cooled to room temperature. A solid obtained therefrom was filtered, and the filtered solid sufficiently washed in the order of water/methanol/hexane. The solid was dried in a vacuum oven to obtain Intermediate L16-dimer (2.17 g, 87 %). The obtained compound was used in a next reaction without additional purification.

Synthesis of Compound 16

30 mL of 2-ethoxy ethanol was added to Intermediate L16-dimer (2.14 g, 1.29 mmol), acetyl acetone (1.29 g, 12.9 mmol), and $Na_2CO_3$ (1.37 g, 12.9 mmol), and stirred at room temperature for 12 hours. The reaction mixture was extracted using ethyl acetate, dried by using anhydrous magnesium sulfate ($MgSO_4$), and filtered to obtain a filtrate. The filtrate was concentrated under vaccuum to obtain a residue. The residue was purified by column chromatography using methylene chloride:hexane = 1:4 (V/V) as an eluent to obtain Compound 16 (0.47 g, 20 %). The obtained compound was identified by MS and HPLC analysis.

HRMS(MALDI-TOF) calcd for $C_{47}H_{39}IrN_2O_4$: m/z 888.2539, Found: 888.2541.

Synthesis Example 3 (Compound 31)

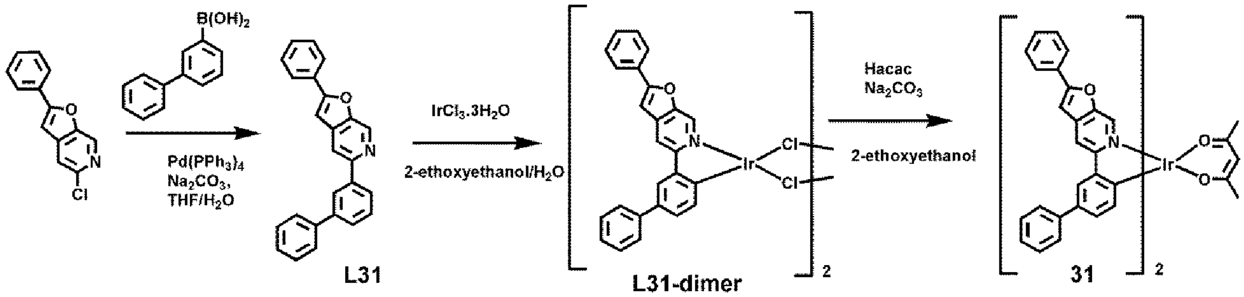


Synthesis of Intermediate L31

Intermediate L31 (2.54 g, 85 %) was obtained in the same manner as in Synthesis of Intermediate L1 of Synthesis Example 1, except that diphenyl 3-boronic acid ([1,1'-biphenyl]-3-ylboronic acid) (2.57 g, 12.9 mmol) was used instead of phenylboronic acid (2.603 g, 16.64 mmol).

LC-MS m/z = 348 $(M+H)^+$.

Synthesis of Intermediate L31-dimer

Intermediate L31-dimer (1.89 g, 76 %) was obtained in the same manner as in Synthesis of Intermediate $L_1$-dimer of Synthesis Example 1, except that Intermediate L31 (2.12 g, 6.11 mmol) was used instead of Intermediate L1.

Synthesis of Compound 31

Compound 31 (0.45 g, 23 %) was obtained in the same manner as in Synthesis of Compound 1 of Synthesis

Example 1, except that Intermediate L31-dimer(1.87 g, 1.25 mmol) was used instead of Intermediate L1-dimer. The obtained compound was identified by MS and HPLC analysis.

HRMS(MALDI-TOF) calcd for $C_{55}H_{39}IrN_2O_4$: m/z 984.2539, Found: 984.2539.

Synthesis Example 4 (Compound 236)

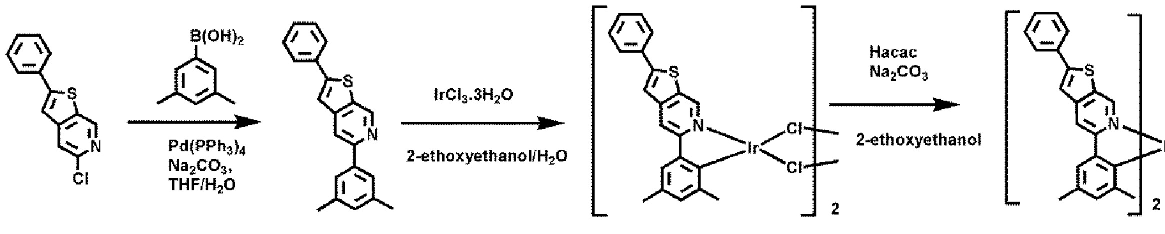


L236 L236-dimer 236

Synthesis of Intermediate L236

Intermediate L236 (2.54 g, 85 %) was obtained in the same manner as in Synthesis of Intermediate L16 of Synthesis Example 2, except that 5-chloro-2-phenylthieno[2,3-c]pyridine (2.34 g, 9.51 mmol) was used instead of 5-chloro-2-phenylfuro[2,3-c]pyridine (2.69 g, 11.71 mmol).

LC-MS m/z = 316 $(M+H)^+$.

Synthesis of Intermediate L236-dimer

Intermediate L236-dimer (2.11 g, 84 %) was obtained in the same manner as in Synthesis of Intermediate L16-dimer of Synthesis Example 2, except that Intermediate L236 (2.07 g, 6.57 mmol) was used instead of Intermediate L16.

Synthesis of Compound 236

Compound 236 (0.41 g, 19 %) was obtained in the same manner as in Synthesis of Compound 16 of Synthesis Example 2, except that Intermediate L236-dimer (2.05 g, 1.20 mmol) was used instead of Intermediate L16-dimer. The obtained compound was identified by MS and HPLC analysis.

HRMS (MALDI-TOF) calcd for $C_{47}H_{39}IrN_2O_2 S_2$: m/z 920.2082, Found: 920.2080.

Synthesis Example 5 (Compound 346)

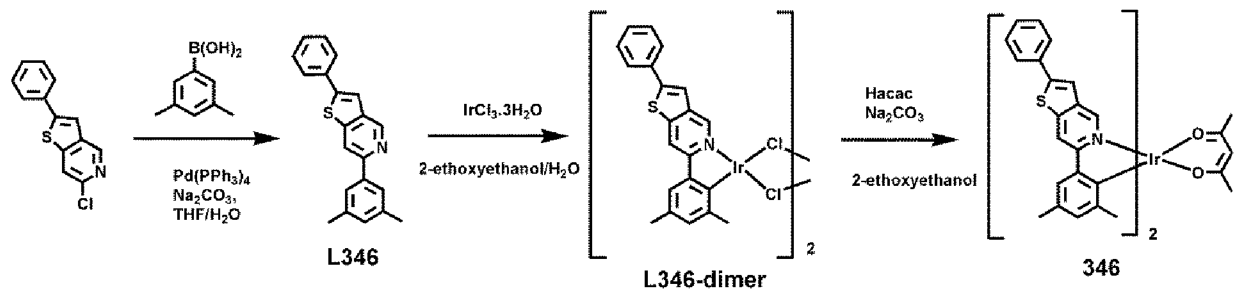


L346 L346-dimer 346

Synthesis of Intermediate L346

Intermediate L346 (2.67 g, 87 %) was obtained in the same manner as in Synthesis of Intermediate L16 of Synthesis Example 2, except that 6-chloro-2-phenylthieno[3,2-c]pyridine (2.34 g, 9.51 mmol) was used instead of 5-chloro-2-phenylfuro[2,3-c]pyridine (2.69 g, 11.71 mmol).

LC-MS m/z = 316 $(M+H)^+$.

Synthesis of Intermediate L346-dimer

Intermediate L346-dimer (2.25 g, 80 %) was obtained in the same manner as in Synthesis of Intermediate L16-dimer of Synthesis Example 2, except that Intermediate L346 (2.32 g, 7.36 mmol) was used instead of Intermediate L16.

Synthesis of Compound 346

Compound 346 (0.526 g, 23 %) was obtained in the same manner as in Synthesis of Compound 16 of Synthesis Example 2, except that Intermediate L346-dimer (2.14 g, 1.25 mmol) was used instead of Intermediate L16-dimer. The obtained compound was identified by MS and HPLC analysis.

HRMS (MALDI-TOF) calcd for $C_{47}H_{39}IrN_2O_2S_2$: m/z 920.2082, Found: 920.2081.

Synthesis Example 6 (Compound 468)

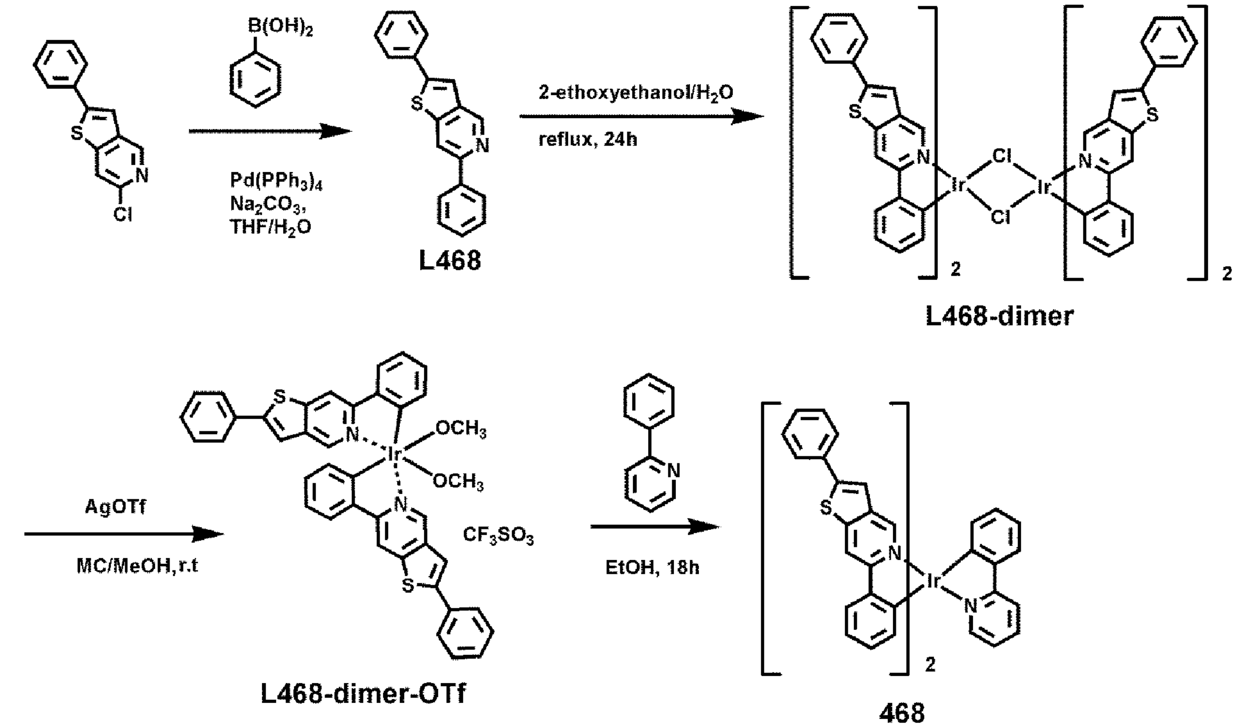


Synthesis of Intermediate L468

Intermediate L468 (3.02 g, 74 %) was obtained in the same manner as in Synthesis of Intermediate L1 of Synthesis Example 1, except that 6-chloro-2-phenylthieno[3,2-c]pyridine (3.51 g, 14.3 mmol) was used instead of 5-chloro-2-phenylfuro[2,3-c]pyridine (2.69 g, 11.71 mmol).

Synthesis of Intermediate L468-dimer

Intermediate L468-dimer (2.11 g, 96 %) was obtained in the same manner as in Synthesis of Intermediate L1-dimer of Synthesis Example 1, except that Intermediate L468 (1.78 g, 6.19 mmol) was used instead of Intermediate L1.

Synthesis of Intermediate L468-dimer-OTf 60 mL of methylene chloride (MC) was mixed with Intermediate L468-dimer (1.97 g, 1.23 mmol), and AgOTf (0.631 g, 2.46 mmol) was dissolved in 20 mL of methanol and added thereto. Then, the reaction proceeded with stirring at room temperature for 18 hours in a state in which light was blocked by an aluminum foil. The reaction mixture was filtered through celite, and a filtrate was concentrated under vaccuum to obtain Intermediate L468-dimer-OTf. Intermediate L468-dimer-OTf was used in a next reaction without additional purification.

Synthesis of Compound 468

Intermediate L468-dimer-OTf (2.23 g, 2.28 mmol) and 2-phenylpyridine (0.39 g, 2.51 mmol) were mixed with 100 mL of ethanol, stirred for 18 hours under reflux, and then cooled. A mixture obtained therefrom was filtered to obtain a solid. The solid was sufficiently washed with ethanol and hexane, and column chromatography using MC:hexane = 40:60 (V/V) as an eluent was performed thereon to obtain Compound 468 (0.32 g, 19 %). The obtained compound was identified by MS and HPLC analysis.

HRMS (MALDI-TOF) calcd for $C_{49}H_{32}IrN_3S_2$: m/z 919.1667, Found: 919.1666.

Synthesis Example 7 (Compound 469)

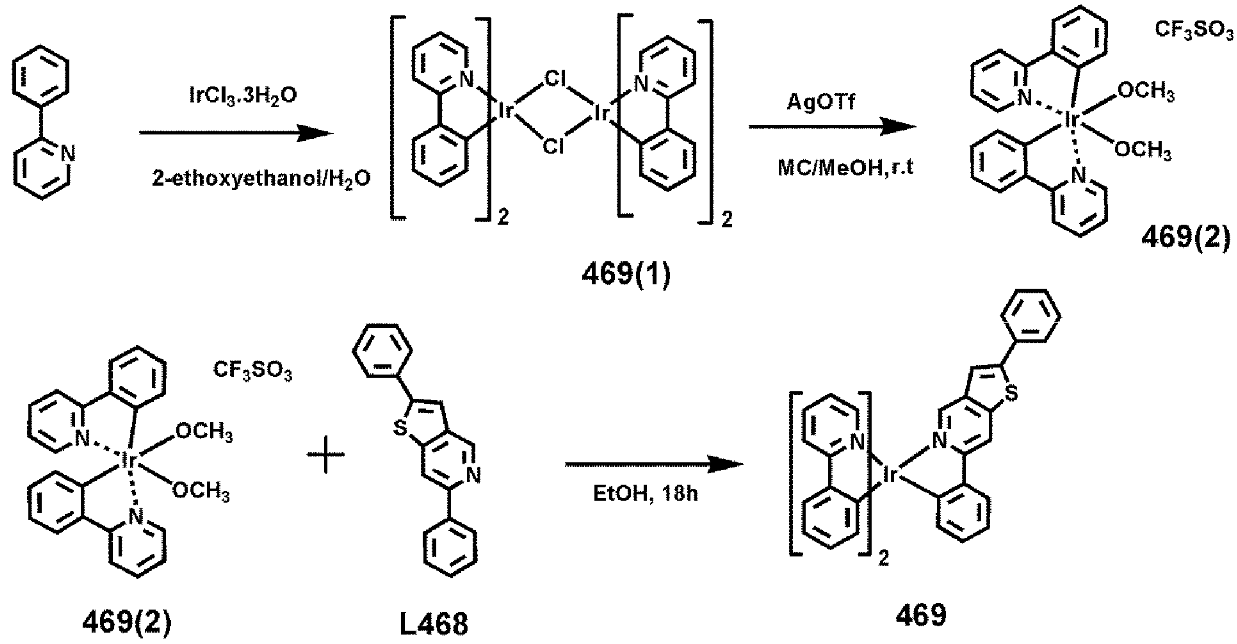


469(1)

469(2)

469(2)

L468

469

Synthesis of Intermediate 469(1)

2-phenylpyridine (14.66 g, 94.44 mmol) and Iridium chloride (14.80 g, 41.97 mmol) were mixed with 210 mL of 2-ethoxy ethanol and 70 mL of distilled water, stirred for 24 hours under reflux, and then cooled to room temperature. A solid obtained therefrom was filtered, and sufficiently washed in the order of water/methanol/hexane. The solid was dried in a vacuum oven to obtain Intermediate 469(1) (19.5 g, 87 %).

Synthesis of Intermediate 469(2)

60 mL of MC was mixed with Intermediate 469(1) (1.88 g, 1.75 mmol), and AgOTf (0.90 g, 3.50 mmol) was dissolved in 20 mL of methanol and added thereto. Then, the reaction proceeded while stirring at room temperature for 18 hours in a state in which light was blocked by an aluminum foil. The reaction mixture was filtered through celite, and a filtrate was concentrated under vaccuum to obtain Intermediate L469(2). Intermediate L469(2) was used in a next reaction without additional purification.

Synthesis of Compound 469

Intermediate 469(2) (1.27 g, 1.78 mmol) and Intermediate L468 (0.562 g, 1.96 mmol) were mixed with 40 mL of ethanol, stirred for 18 hours under reflux, and then cooled. A mixture obtained therefrom was filtered to obtain a solid. The solid was sufficiently washed with ethanol and hexane, and column chromatography using MC:hexane = 40:60 (V/V) as an eluent was performed thereon to obtain Compound 469 (0.31 g, 22 %). The obtained compound was identified by MS and HPLC analysis.

HRMS (MALDI-TOF) calcd for $C_{41}H_{28}IrN_3S$: m/z 787.1633, Found: 787.1633.

Evaluation Example 1 : Evaluation of radiative decay rate

CBP and Compound 1 were co-deposited at a weight ratio of 9:1 under a vacuum pressure of $10^{-7}$ torr (1 torr = 133.32 Pa) to manufacture a film having a thickness of 40 nm.

A PL spectrum of the film was evaluated at room temperature by using a PicoQuant TRPL measurement system FluoTime 300 and a PicoQuant pumping source PLS340 (excitation wavelength = 340 nm, spectral width = 20 nm), a wavelength of a main peak of the spectrum was determined, and PLS340 repeatedly measured the number of photons emitted from the film at the wavelength of the main peak due to a photon pulse (pulse width = 500 ps) applied to the film according to time based on time-correlated single photon counting (TCSPC), thereby obtaining a sufficiently fittable TRPL curve. A decay time $T_{decay}$ of the film was obtained by fitting one or more exponential decay functions to the result obtained therefrom. The function used for fitting is expressed by Equation 10, and the greatest value among the values obtained from each exponential decay function used for fitting was taken as $T_{decay}$. At this time, a baseline or background signal curve was obtained by repeating the same measurement once more for the same measurement time as the measurement time for obtaining the TRPL curve in a dark state (a state in which a pumping signal applied to the predetermined film was blocked), and the baseline or background signal curve was used for fitting as a baseline.

Equation 10

$$f(t) = \sum_{i=1}^{n} A_i \exp(-t/T_{decay,i})$$

Then, the quantum efficiency of the film was measured by using a Hamamatsu Quantaurus-QY Absolute PL quantum yield spectrometer (provided with a xenon light source, a monochromator, a photonic multichannel analyzer, and an integrating sphere and using PLQY measurement software (Hamamatsu Photonics, Ltd., Shizuoka, Japan)). Upon measurement of the quantum efficiency, the excitation wavelength was measured while scanning from 320 nm to 380 nm at an interval of 10 nm, and the greatest value was taken as the quantum efficiency (Φ).

The radiative decay rate ($k_r$) of Compound 1 was obtained by substituting $T_{decay}$ and Φ into Equation 11, and results thereof are shown in Table 2.

Equation 11

$$k_r = \Phi / T_{decay}.$$

The measurement of the radiative decay rate was repeated on Compounds 16, 31, 236, 346, 468, 469, A1, A2, B, C1, C2, and D, and results thereof are shown in Table 2.

Table 2

| Compound No. | Radiative decay rate ($s^{-1}$) |
|---|---|
| 1 | $6.70 \times 10^5$ |
| 16 (reference) | $8.94 \times 10^5$ |
| 31 | $8.26 \times 10^5$ |
| 236 | $1.36 \times 10^6$ |
| 346 | $1.17 \times 10^6$ |

(continued)

| Compound No. | Radiative decay rate ($s^{-1}$) |
|---|---|
| 468 | $6.46 \times 10^5$ |
| 469 | $5.56 \times 10^5$ |
| A1 | $3.17 \times 10^5$ |
| A2 | $3.32 \times 10^5$ |
| B | $4.62 \times 10^5$ |
| C1 | $1.27 \times 10^5$ |
| C2 | $2.93 \times 10^5$ |
| D | $3.25 \times 10^5$ |

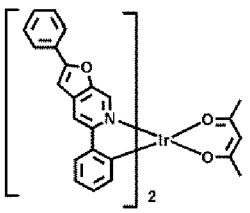
1

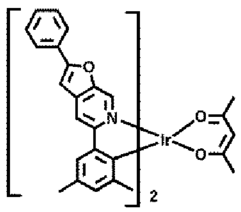
16

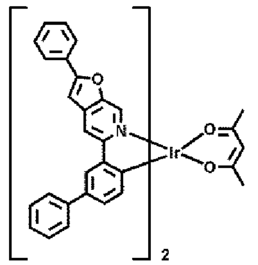
31

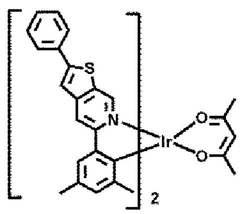
236

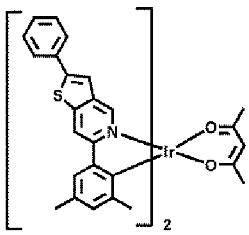
346

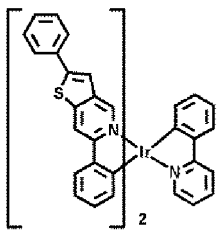
468

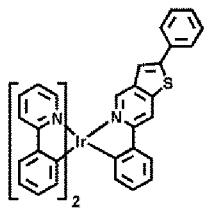
469

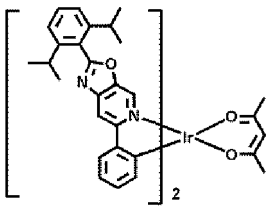
A1

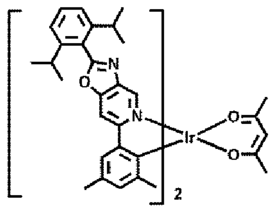
A2

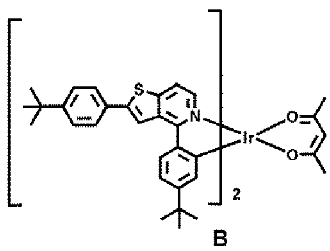
B

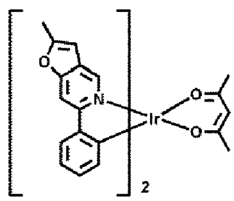
C1

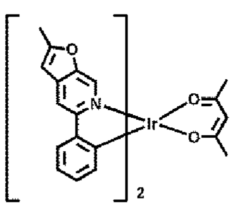
C2

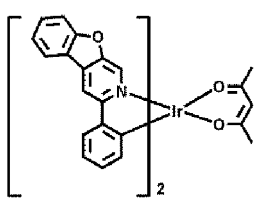
D

From Table 2, it is confirmed that Compounds 1, 31, 236, 346, 468, 469 and Reference Compound 16 have high radiative decay rates, as compared with those of Compounds A1, A2, B, C1, C2, and D.

Example 1

As an anode, a glass substrate, on which ITO/Ag/ITO were deposited to thicknesses of 70 Å /1,000 Å /70 Å, was cut to a size of 50 mm × 50 mm × 0.5 mm, sonicated with isopropyl alcohol and pure water each for 5 minutes, and then cleaned by exposure to ultraviolet rays and ozone for 30 minutes. Then, the glass substrate was provided to a vacuum deposition apparatus.

2-TNATA was vacuum-deposited on the anode to form a hole injection layer having a thickness of 600 Å, and 4,4'-bis[N-(1-naphthyl)-N-phenylamino]biphenyl (NPB) was vacuum-deposited on the hole injection layer to form a hole transport layer having a thickness of 1,350 Å.

Then, CBP (host) and Compound 1 (dopant) were co-deposited on the hole transport layer at a weight ratio of 98:2 to form an emission layer having a thickness of 400 Å.

Then, BCP was vacuum-deposited on the emission layer to form a hole blocking layer having a thickness of 50 Å, $Alq_3$ was vacuum-deposited on the hole blocking layer to form an electron transport layer having a thickness of 350 Å, LiF was vacuum-deposited on the electron transport layer to form an electron injection layer having a thickness of 10 Å, and Mg and Ag were co-deposited on the electron injection layer at a weight ratio of 90:10 to form a cathode having a thickness of 120 Å, thereby completing the manufacture of an organic light-emitting device (which emits red light).

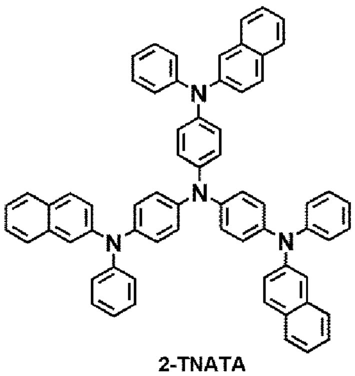

2-TNATA

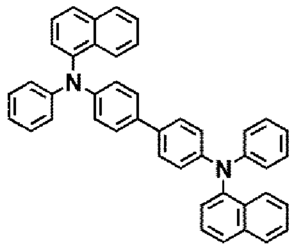

NPB

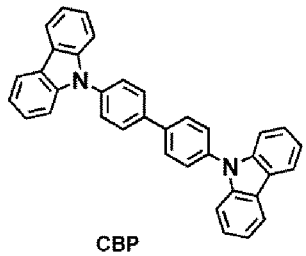

CBP

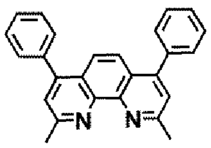

BCP

Reference Example 2, Examples 3 to 7 and Comparative Examples A1, A2, B, C1, C2, and D

Organic light-emitting devices were manufactured in the same manner as in Example 1, except that Compounds shown in Table 3 were each used instead of Compound 1 as a dopant in forming an emission layer.

Evaluation Example 2: Evaluation of characteristics of organic light-emitting devices

The driving voltage, maximum value of external quantum efficiency (Max EQE), roll-off ratio, maximum emission wavelength of main peak of EL spectrum, and lifespan ($T_{97}$) of the organic light-emitting devices manufactured according to Examples 1 to 7 and Comparative Examples A1, A2, B, C1, C2, and D were evaluated, and results thereof are shown in Table 3. A current-voltage meter (Keithley 2400) and a luminance meter (Minolta Cs-1000A) were used as the evaluation devices, and the lifespan ($T_{97}$) (at 3,500 nit) indicates an amount of time that lapsed when luminance was 97 % of initial luminance (100 %). The roll-off ratio was calculated by Equation 20:

Equation 20

Roll off ratio = {1- (efficiency (at 3,500 nit) / maximum luminescence efficiency)} X 100%. In the subsequent table, the non SI unit "nit" is used (1 nit = 1 cd/m2)

Table 3

| | Compound No. of dopant in emission layer | Driving voltage (V) | Max EQE (%) | Roll-Off ratio (%) | Maximum emission wavelength (nm) | $LT_{97}$ (hr) (at 3,500 nit) |
|---|---|---|---|---|---|---|
| Example 1 | 1 | 4.0 | 28.4 | 8 | 524 | 273 |
| Reference Example 2 | 16 | 3.7 | 30.1 | 9 | 558 | 205 |
| Example 3 | 31 | 3.8 | 26.6 | 6 | 533 | 155 |
| Example 4 | 236 | 3.5 | 31.9 | 7 | 582 | 320 |
| Example 5 | 346 | 3.7 | 31.7 | 10 | 565 | 232 |
| Example 6 | 468 | 3.6 | 28.4 | 9 | 540 | 253 |
| Example 7 | 469 | 4.0 | 26.8 | 8 | 538 | 212 |
| Comparative Example A1 | A1 | 5.2 | 24.8 | 13 | 530 | 120 |
| Comparative Example A2 | A2 | 4.8 | 24.0 | 11 | 540 | 65 |
| Comparative Example B | B | 4.3 | 26.0 | 12 | 580 | 60 |
| Comparative Example C1 | C1 | 4.8 | 21.8 | 12 | 515 | 108 |
| Comparative Example C2 | C2 | 5.4 | 22.9 | 15 | 510 | 34 |
| Comparative Example D | D | 4.6 | 22.0 | 12 | 552 | 33 |

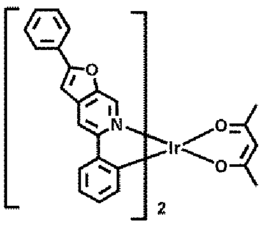

1

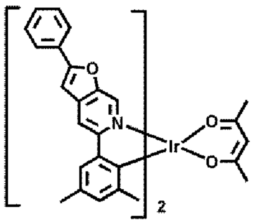

16

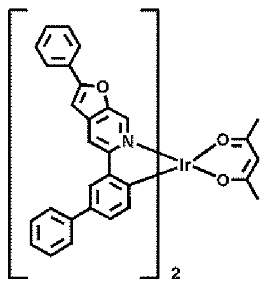

31

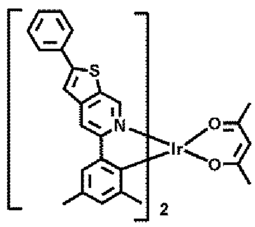

236

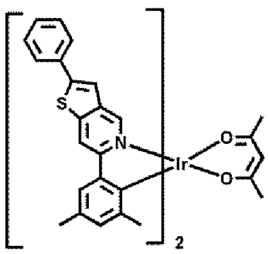

346

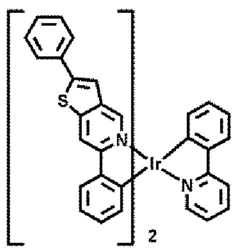

468

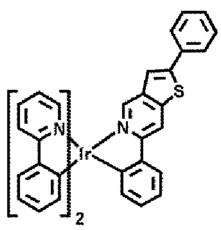

469

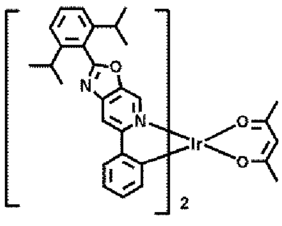

A1

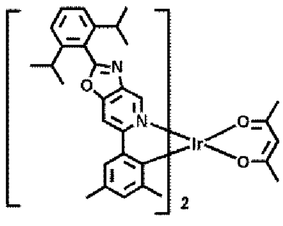

A2

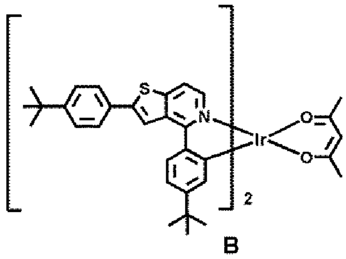

B

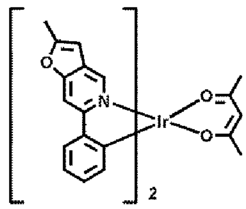

C1

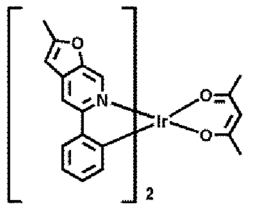

C2

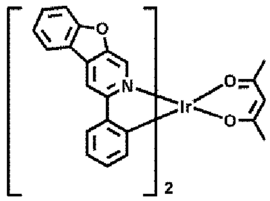

D

From Table 3, it is confirmed that the organic light-emitting devices of Examples 1 and 3 to 7 and Reference Example 2 emit red light and have improved driving voltage, improved external quantum efficiency, improved roll-off ratio, and improved lifespan characteristics, as compared with those of the organic light-emitting devices of Comparative Examples A1, A2, B, C1, C2, and D.

Since the organometallic compound represented by Formula 1 has a high radiative decay rate, an electronic device, for example, an organic light-emitting device, which includes the organometallic compound represented by Formula 1, may have improved driving voltage, improved external quantum luminescence efficiency, improved roll-off ratio, and improved lifespan characteristics. In addition, since the organometallic compound represented by Formula 1 has excellent phosphorescent luminescent characteristics, a diagnostic composition having high diagnostic efficiency may be provided by using the organometallic compound.

It should be understood that embodiments described herein should be considered in a descriptive sense only and not for purposes of limitation. Descriptions of features or aspects within each embodiment should typically be considered as available for other similar features or aspects in other embodiments. The claimed subject-matter is limited to the claims as following hereafter:

## Claims

1. An organometallic compound represented by Formula 1:

Formula 1
$M(L_1)_{n1}(L_2)_{n2}$,
wherein, in Formula 1,
$L_1$ is a ligand represented by Formula 2,
n1 is 1, 2, or 3, wherein, when n1 is 2 or more, two or more $L_1$ are identical to or different from each other,
$L_2$ is a bidentate ligand represented by Formula 3,
n2 is 1, 2, 3, or 4, wherein, when n2 is 2 or more, two or more $L_2$ are identical to or different from each other, and
$L_1$ and $L_2$ are different from each other:

M is Ir or Os, and the sum of n1 and n2 is 3 or 4; or M is Pt, and the sum of n1 and n2 is 2,

Formula 2

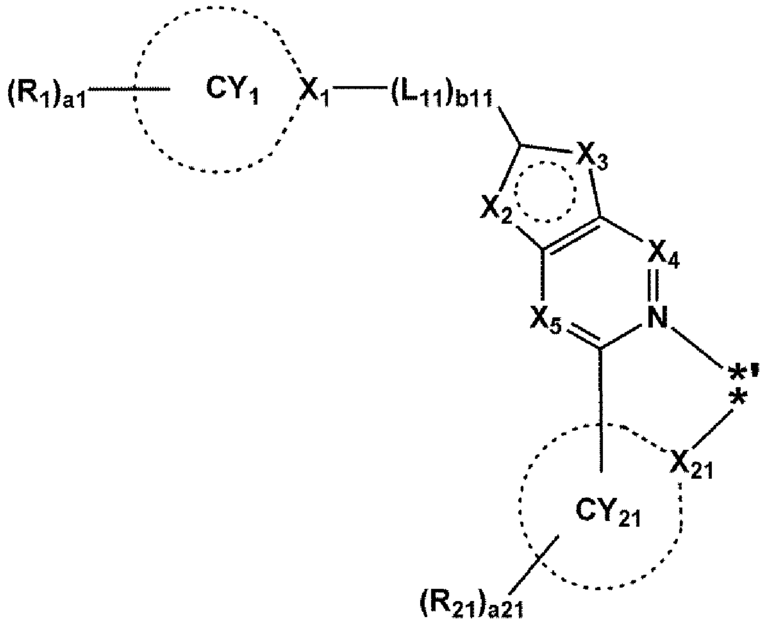

,

wherein, in Formula 2,
$X_1$ is C, N, Si, or P,
$X_{21}$ is C or N,
ring $CY_1$ and ring $CY_{21}$ are each independently a $C_5$-$C_{30}$ carbocyclic group or a $C_2$-$C_{30}$ heterocyclic group,
$X_2$ and $X_3$ are each independently O, S, Se, or $C(R_2)$, wherein $X_2$ or $X_3$ is O, S, or Se,
$X_4$ is N or $C(R_4)$,
$X_5$ is N or $C(R_5)$,
$R_1$, $R_2$, $R_4$, $R_5$, and $R_{21}$ are each independently hydrogen, deuterium, -F, -Cl, -Br, -I, $-SF_5$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_2$-$C_{10}$ heterocycloalkyl group, a substituted or unsubstituted $C_3$-$C_{10}$ cycloalkenyl group, a substituted or unsubstituted $C_2$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted monovalent non-aromatic condensed polycyclic group, a substituted or unsubstituted monovalent non-aromatic condensed heteropolycyclic group, $-N(Q_1)(Q_2)$, - $Si(Q_3)(Q_4)(Q_5)$, $-B(Q_6)(Q_7)$, $-P(=O)(Q_8)(Q_9)$ or $-P(Q_8)(Q_9)$,
a1 and a21 are each independently an integer from 0 to 20,
ring $CY_1$ and $R_2$ are not linked to each other, and $R_1$ and $R_2$ are not linked to each other,
$L_{11}$ is a $C_5$-$C_{30}$ carbocyclic group that is unsubstituted or substituted with at least one $R_{10a}$ or a $C_2$-$C_{30}$ heterocyclic group that is unsubstituted or substituted with at least one $R_{10a}$,
b11 is an integer from 0 to 10, wherein, when b11 is 0, a group represented by $*-(L_{11})_{b11}-*'$ is a single bond, and when b11 is 2 or more, two or more $L_{11}$ are identical to or different from each other,
two of a plurality of neighboring $R_{21}$ are optionally linked to form a $C_5$-$C_{30}$ carbocyclic group that is unsubstituted or substituted with at least one $R_{10a}$ or a $C_2$-$C_{30}$ heterocyclic group that is unsubstituted or substituted with at least one $R_{10a}$,
$R_{10a}$ is the same as defined in connection with $R_{21}$,
* and *' each indicate a binding site to M in Formula 1,
a substituent of the substituted $C_5$-$C_{30}$ carbocyclic group, the substituted $C_2$-$C_{30}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_2$-$C_{10}$ heterocycloalkyl group, the substituted $C_3$-$C_{10}$ cycloalkenyl group, the substituted $C_2$-$C_{10}$ heterocycloalkenyl

group, the substituted $C_6$-$C_{60}$ aryl group, the substituted $C_6$-$C_{60}$ aryloxy group, the substituted $C_6$-$C_{60}$ arylthio group, the substituted $C_1$-$C_{60}$ heteroaryl group, the substituted monovalent non-aromatic condensed polycyclic group, and the substituted monovalent non-aromatic condensed heteropolycyclic group is:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;
a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_2$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, - $N(Q_{11})(Q_{12})$, $-Si(Q_{13})(Q_{14})(Q_{15})$, $-B(Q_{16})(Q_{17})$, $-P(=O)(Q_{18})(Q_{19})$, or any combination thereof;
a $C_3$-$C_{10}$ cycloalkyl group, a $C_2$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group;
a $C_3$-$C_{10}$ cycloalkyl group, a $C_2$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group, each substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_2$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, $-N(Q_{21})(Q_{22})$, - $Si(Q_{23})(Q_{24})(Q_{25})$, $-B(Q_{26})(Q_{27})$, $-P(=O)(Q_{28})(Q_{29})$, or any combination thereof;
$-N(Q_{31})(Q_{32})$, $-Si(Q_{33})(Q_{34})(Q_{35})$, $-B(Q_{36})(Q_{37})$, or $-P(=O)(Q_{38})(Q_{39})$; or
any combination thereof, and
$Q_1$ to $Q_9$, $Q_{11}$ to $Q_{19}$, $Q_{21}$ to $Q_{29}$, and $Q_{31}$ to $Q_{39}$ are each independently hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_2$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryl group substituted with a $C_1$-$C_{60}$ alkyl group, a $C_6$-$C_{60}$ aryl group, or any combination thereof, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group;

Formula 3

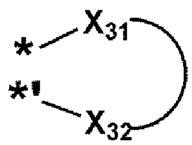

,

wherein, in Formula 3,
$X_{31}$ and $X_{32}$ are each O;
$X_{31}$ is O and $X_{32}$ is N; or
$X_{31}$ is N and $X_{32}$ is C,

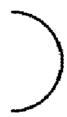

indicates any atomic group linking $X_{31}$ and $X_{32}$ to each other, and * and *' each indicate a binding site to M in Formula 1; and
wherein the organometallic compound is not the following compound:

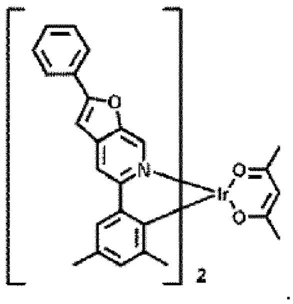

2. The organometallic compound of claim 1, wherein,
ring $CY_1$ and ring $CY_{21}$ are each independently a cyclopentene group, a cyclohexene group, a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group.

3. The organometallic compound of claims 1 or 2, wherein
$R_1$, $R_2$, $R_4$, $R_5$, $R_{21}$, and $R_{10a}$ are each independently:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, $-SF_5$, a $C_1$-$C_{20}$ alkyl group, or a $C_1$-$C_{20}$ alkoxy group;
a $C_1$-$C_{20}$ alkyl group or a $C_1$-$C_{20}$ alkoxy group, each substituted with deuterium, - F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a cyclopentyl group, a cyclohexyl group, a cycloheptyl group, a cycloctyl group, an adamantyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a biphenyl group, a terphenyl group, a naphthyl group, a pyridinyl group, a pyrimidinyl group, or any combination thereof;
a cyclopentyl group, a cyclohexyl group, a cycloheptyl group, a cycloctyl group, an adamantyl group, a norbornenyl group, a cyclopentenyl group, a cyclohexenyl group, a cycloheptenyl group, a cyclooctenyl group, a bicyclo[1.1.1]pentyl group, a bicyclo[2.1.1]hexyl group, a bicyclo[2.2.1]heptyl group, a bicyclo[2.2.2]octyl group,

a phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a cyclopentyl group, a cyclohexyl group, a cycloheptyl group, a cycloctyl group, an adamantyl group, a norbornenyl group, a cyclopentenyl group, a cyclohexenyl group, a cycloheptenyl group, a cyclooctenyl group, a bicyclo[1.1.1]pentyl group, a bicyclo[2.1.1]hexyl group, a bicyclo[2.2.1]heptyl group, a bicyclo[2.2.2]octyl group, a phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, $-Si(Q_{33})(Q_{34})(Q_{35})$, or any combination thereof; or
$-N(Q_1)(Q_2)$, $-Si(Q_3)(Q_4)(Q_5)$, $-B(Q_6)(Q_7)$, $-P(=O)(Q_8)(Q_9)$, or $-P(Q_8)(Q_9)$, and
$Q_1$ to $Q_9$ and $Q_{33}$ to $Q_{35}$ are each independently:
$-CH_3$, $-CD_3$, $-CD_2H$, $-CDH_2$, $-CH_2CH_3$, $-CH_2CD_3$, $-CH_2CD_2H$, $-CH_2CDH_2$, $-CHDCH_3$, $-CHDCD_2H$, $-CHDCDH_2$, $-CHDCD_3$, $-CD_2CD_3$, $-CD_2CD_2H$, or $-CD_2CDH_2$; or
an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group, each unsubstituted or substituted with deuterium, a $C_1$-$C_{10}$ alkyl group, a phenyl group, or any combination thereof.

**4.** The organometallic compound of any of claims 1-3, wherein a group represented by

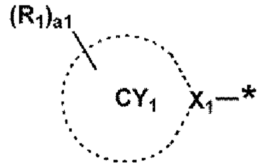

in Formula 2 is a $C_3$-$C_{10}$ cycloalkenyl group, a $C_2$-$C_{10}$ heterocycloalkenyl group, a $C_6$-$C_{30}$ aryl group, a $C_2$-$C_{30}$ heteroaryl group, a monovalent non-aromatic condensed polycyclic group, or a monovalent non-aromatic condensed heteropolycyclic group, each unsubstituted or substituted with $R_1$ in the number of a1,
in Formula 2, $R_1$ and $R_2$ are each independently:

hydrogen, deuterium, -F, -Cl, -Br, -I, a cyano group, or $-SF_5$; or
a $C_1$-$C_{60}$ alkyl group, a $C_1$-$C_{60}$ alkoxy group, a $C_3$-$C_{10}$ cycloalkyl group, a $C_2$-$C_{10}$ heterocycloalkyl group, a $C_3$-$C_{10}$ cycloalkenyl group, or $C_2$-$C_{10}$ heterocycloalkenyl group, each unsubstituted or substituted with deuterium, -F, -Cl, -Br, -I, $-CD_3$, $-CD_2H$, $-CDH_2$, $-CF_3$, $-CF_2H$, $-CFH_2$, a cyano group, a $C_1$-$C_{20}$ alkyl group, a cyclopentyl group, a cyclohexyl group, a cycloheptyl group, a cycloctyl group, an adamantyl group, a norbornyl group, a norbornenyl group, a cyclopentenyl group, a cyclohexenyl group, a cycloheptenyl group, a phenyl group, a biphenyl group, a naphthyl group, a pyridinyl group, a pyrimidinyl group, or any combination thereof, and
a1 is an integer from 0 to 10.

**5.** The organometallic compound of any of claims 1-4, wherein

a group represented by

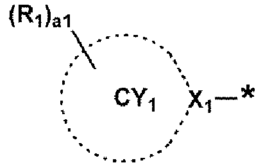

in Formula 2 is a phenyl group, a biphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group, each unsubstituted or substituted with $R_1$ in the number of a1,
in Formula 2, $R_1$ and $R_2$ are each independently:

hydrogen, deuterium, -F, -Cl, -Br, -I, a cyano group, or $-SF_5$; or
a methyl group, an ethyl group, a propyl group, an n-butyl group, an isobutyl group, a see-butyl group, a tert-butyl group, an n-pentyl group, an isopentyl group, a sec-pentyl group, a tert-pentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonanyl group, an isononanyl group, a sec-nonanyl group, a tert-nonanyl group, an n-decanyl group, an isodecanyl group, a sec-decanyl group, a tert-decanyl group, a $C_1$-$C_{10}$ alkoxy, a cyclopentyl group, a cyclohexyl group, a cycloheptyl group, a cycloctyl group, an adamantyl group, a norbornyl group, a norbornenyl group, a cyclopentenyl group, a cyclohexenyl group, or a cycloheptenyl group, each unsubstituted or substituted with deuterium, -F, -Cl, -Br, -I, $-CD_3$, $-CD_2H$, $-CDH_2$, $-CF_3$, $-CF_2H$, $-CFH_2$, a cyano group, a $C_1$-$C_{20}$ alkyl group, a cyclopentyl group, a cyclohexyl group, a cycloheptyl group, a cycloctyl group, an adamantyl group, a norbornyl group, a norbornenyl group, a cyclopentenyl group, a cyclohexenyl group, a cycloheptenyl group, a phenyl group, a biphenyl group, a naphthyl group, a pyridinyl group, a pyrimidinyl group, or any combination thereof, and
a1 is an integer from 0 to 5.

**6.** The organometallic compound of any of claims 1-5, wherein a group represented by

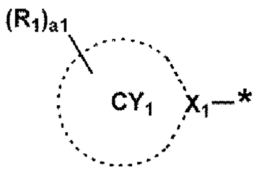

is a group represented by one of Formulae 10-13(1) to 10-13(18) and 10-13:

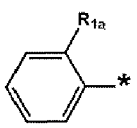

10-13(1)

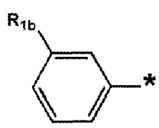

10-13(2)

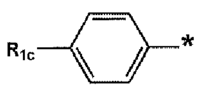

10-13(3)

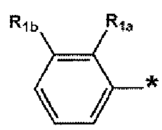

10-13(4)

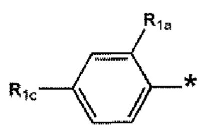

10-13(5)

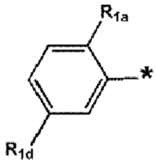
10-13(6)

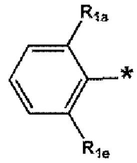
10-13(7)

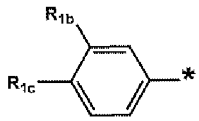
10-13(8)

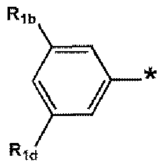
10-13(9)

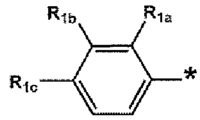
10-13(10)

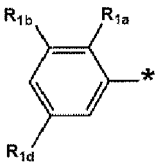
10-13(11)

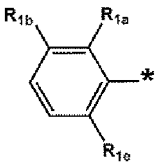
10-13(12)

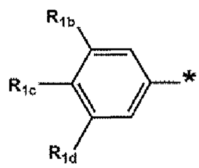
10-13(13)

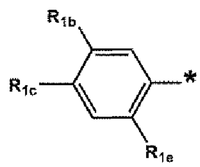
10-13(14)

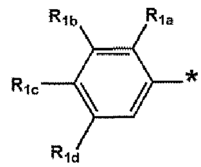
10-13(15)

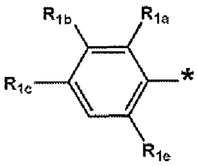
10-13(16)

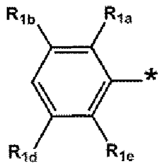
10-13(17)

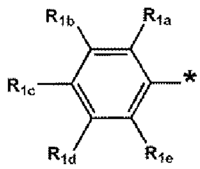
10-13(18)

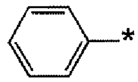
10-13

wherein, in Formulae 10-13(1) to 10-13(18) and 10-13, $R_{1a}$ to $R_{1e}$ are each independently the same as defined in connection with $R_1$ in claim 1, wherein $R_{1a}$ to $R_{1e}$ are each not hydrogen, and * indicates a binding site to a neighboring atom.

**7.** The organometallic compound of any of claims 1-6, wherein

a group represented by

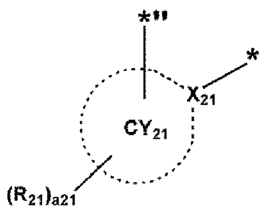

in Formula 2 is a group represented by one of Formulae one of CY21-1 to CY21-25:

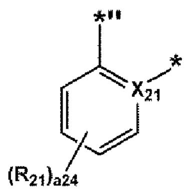
CY21-1

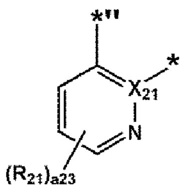
CY21-2

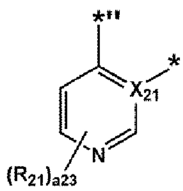
CY21-3

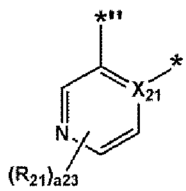
CY21-4

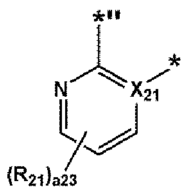
CY21-5

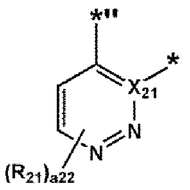
CY21-6

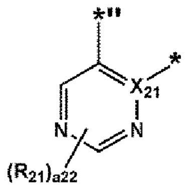
CY21-7

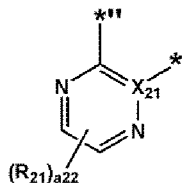
CY21-8

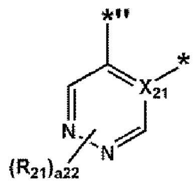
CY21-9

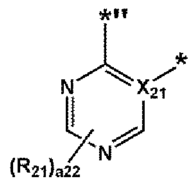
CY21-10

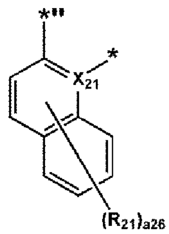
CY21-11

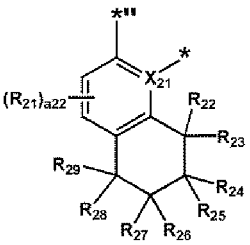
CY21-12

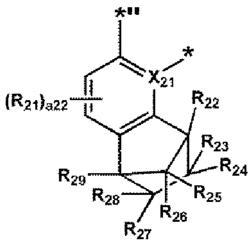
CY21-13

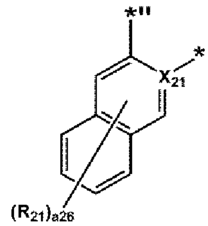
CY21-14

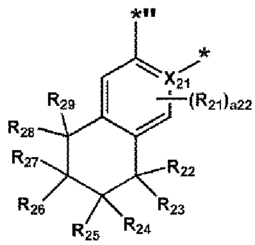
CY21-15

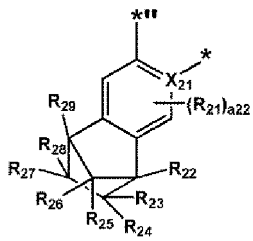
CY21-16

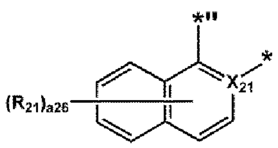
CY21-17

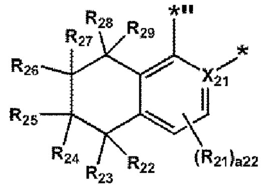
CY21-18

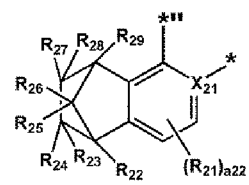
CY21-19

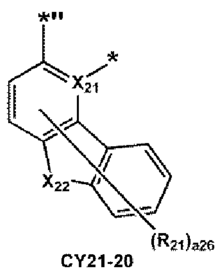
CY21-20

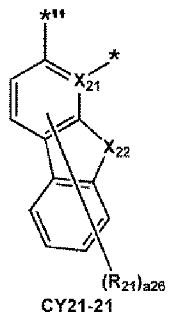
CY21-21

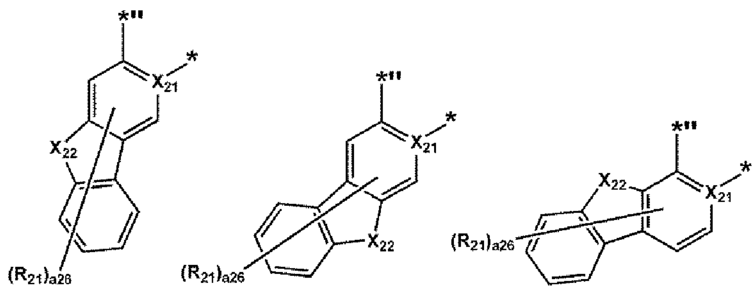
CY21-22

CY21-23

CY21-24

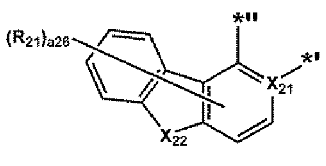
CY21-25

wherein, in Formulae CY21-1 to CY21-25,
$X_{21}$ and $R_{21}$ are each independently the same as described in claim 1,
$X_{22}$ is $C(R_{22})(R_{23})$, $N(R_{22})$, O, S, or $Si(R_{22})(R_{23})$,
$R_{22}$ to $R_{29}$ are each independently the same as defined in connection with $R_{21}$ in claim 1,
a26 is an integer from 0 to 6,
a24 is an integer from 0 to 4,
a23 is an integer from 0 to 3,
a22 is an integer from 0 to 2,
*" indicates a binding site to a carbon atom of a neighboring 6-membered ring in Formula 2, and

* indicates a binding site to M in Formula 1;
preferably wherein
a group represented by

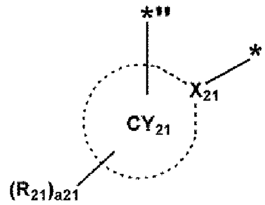

in Formula 2 is a group represented by one of Formulae CY21(1) to CY21(56) or a group represented by one of Formulae CY21-20 to CY21-26:

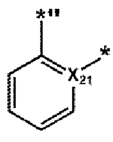
CY21(1)

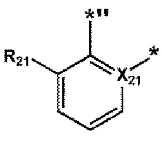
CY21(2)

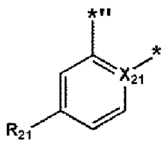
CY21(3)

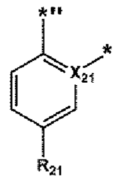
CY21(4)

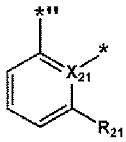
CY21(5)

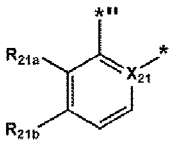
CY21(6)

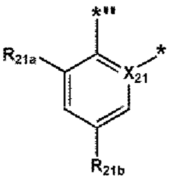
CY21(7)

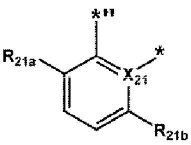
CY21(8)

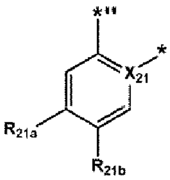
CY21(9)

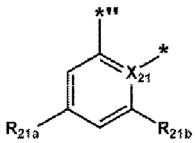
CY21(10)

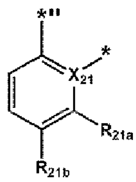
CY21(11)

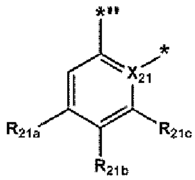
CY21(12)

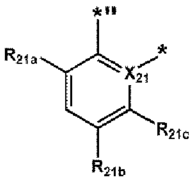
CY21(13)

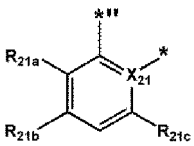
CY21(14)

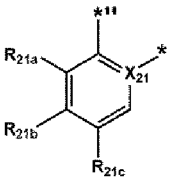
CY21(15)

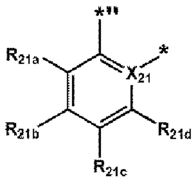
CY21(16)

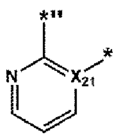
CY21(17)

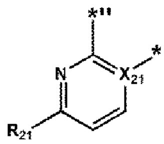
CY21(18)

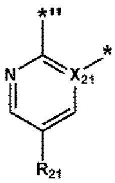
CY21(19)

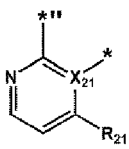
CY21(20)

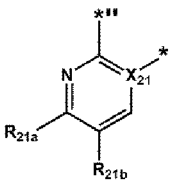
CY21(21)

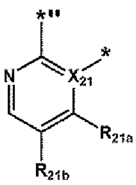
CY21(22)

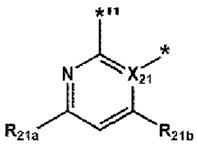
CY21(23)

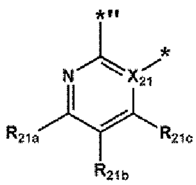
CY21(24)

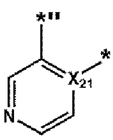
CY21(25)

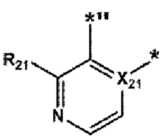
CY21(26)

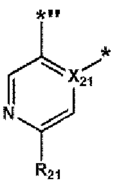
CY21(27)

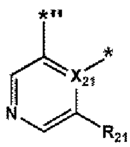
CY21(28)

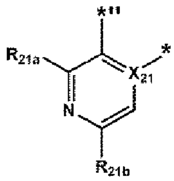
CY21(29)

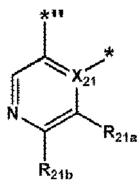
CY21(30)

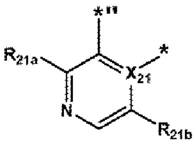
CY21(31)

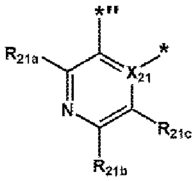
CY21(32)

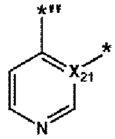
CY21(33)

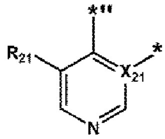
CY21(34)

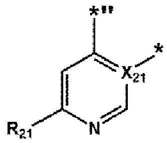
CY21(35)

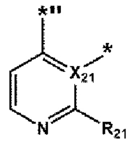
CY21(36)

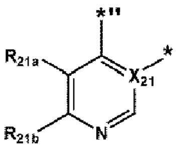
CY21(37)

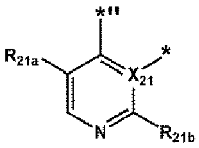
CY21(38)

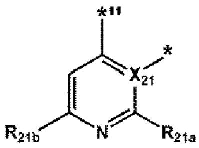
CY21(39)

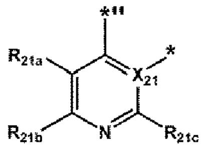
CY21(40)

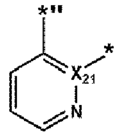
CY21(41)

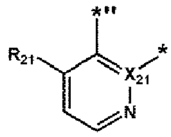
CY21(42)

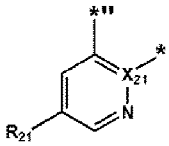
CY21(43)

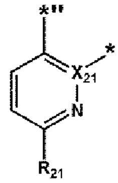
CY21(44)

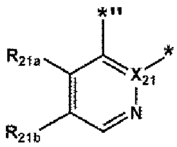
CY21(45)

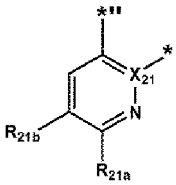
CY21(46)

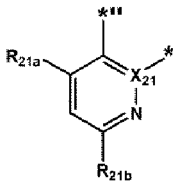
CY21(47)

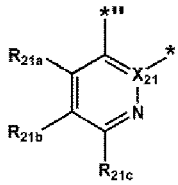
CY21(48)

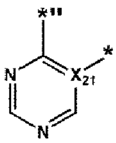
CY21(49)

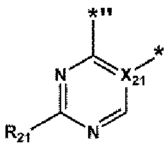
CY21(50)

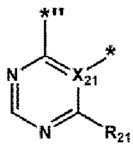
CY21(51)

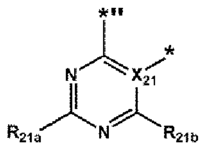
CY21(52)

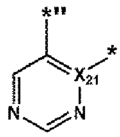
CY21(53)

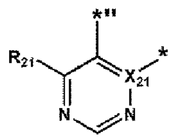
CY21(54)

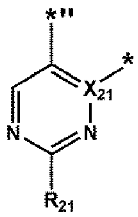
CY21(55)

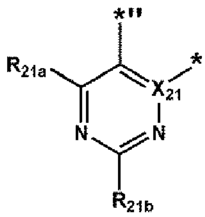
CY21(56)

,

wherein, in Formulae $CY_{21}$ (1) to CY21(56),

$X_{21}$ and $R_{21}$ are each independently the same as described in claim 1,

$R_{21a}$ to $R_{21d}$ are each independently the same as defined in connection with $R_{21}$ in claim 1, wherein $R_{21}$ and $R_{21a}$ to $R_{21d}$ are each not hydrogen,

*" indicates a binding site to a carbon atom of a neighboring 6-membered ring in Formula 2, and

* indicates a binding site to M in Formula 1.

**8.** The organometallic compound of any of claims 1-7, wherein $L_1$ is a ligand represented by Formula 2A or 2B:

Formula 2A

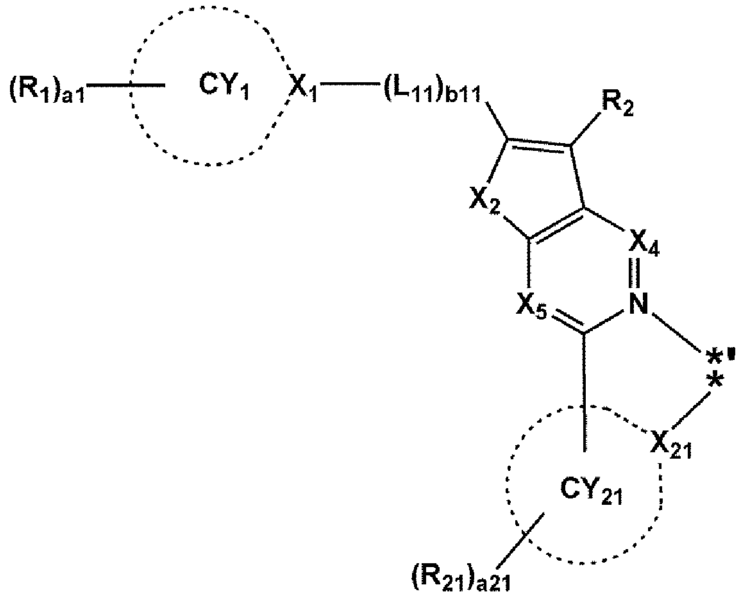

Formula 2B

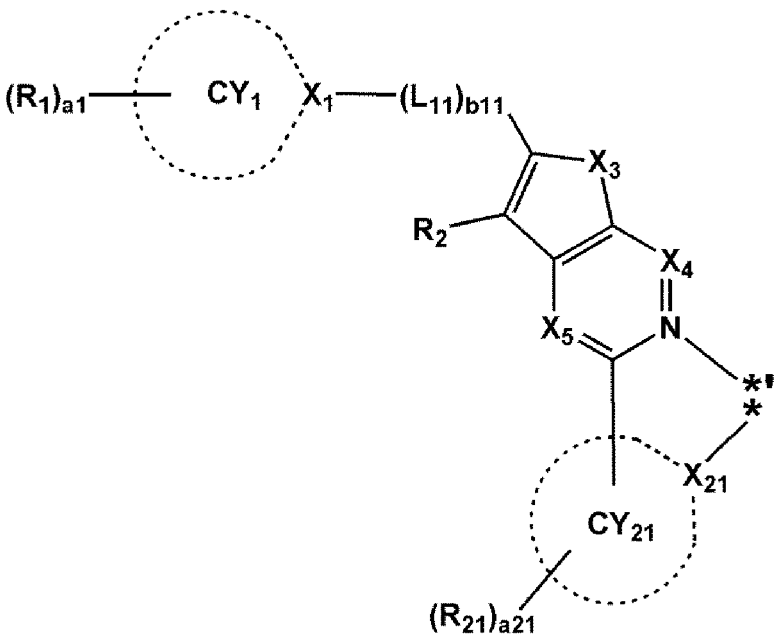

,

wherein, in Formulae 2A and 2B, $X_1$, $X_{21}$, ring $CY_1$, ring $CY_{21}$, $X_4$, $X_5$, $R_1$, $R_2$, $R_{21}$, a1, a21, $L_{11}$, b11, *, and *' are each independently the same as described in claim 1, wherein $X_2$ and $X_3$ are each independently O, S, or Se.

**9.** The organometallic compound of any of claims 1-8, wherein

in Formula 1, $L_2$ is a group represented by one of Formulae 3A to 3D:

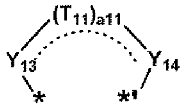
3A
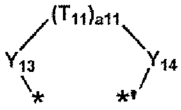
3B
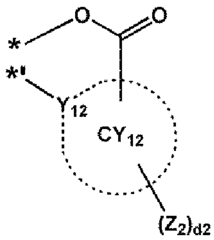
3C
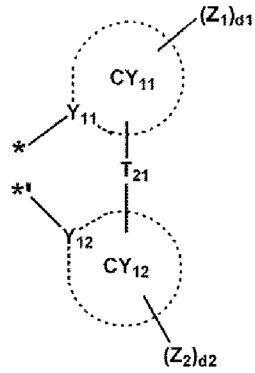
3D
,

wherein, in Formulae 3A to 3D,
$Y_{13}$ is O, N, or $N(Z_1)$,
$Y_{14}$ is O, N, or $N(Z_3)$,
$T_{11}$ is a single bond, a double bond, *-$C(Z_{11})(Z_{12})$-*', *-$C(Z_{11})=C(Z_{12})$-*', *=C(Zn)-*', *-$C(Z_{11})$=*', *=$C(Z_{11})-C(Z_{12})=C(Z_{13})$-*', *-$C(Z_{11})=C(Z_{12})-C(Z_{13})$=*', *-$N(Z_{11})$-*', or a $C_5$-$C_{30}$ carbocyclic group unsubstituted or substituted with at least one $Z_{11}$,
a11 is an integer from 1 to 10,
$Y_{11}$ and $Y_{12}$ are each independently C or N,
$T_{21}$ is a single bond, a double bond, O, S, $C(Z_{11})(Z_{12})$, $Si(Z_{11})(Z_{12})$, or $N(Z_{11})$,
ring $CY_{11}$ and ring $CY_{12}$ are each independently a $C_5$-$C_{30}$ carbocyclic group or a $C_2$-$C_{30}$ heterocyclic group,
$Z_1$ to $Z_3$ and $Z_{11}$ to $Z_{13}$ are each independently the same as defined in connection with $R_{21}$ in claim 1,
d1 and d2 are each independently an integer from 0 to 10, and
* and *' each indicate a binding site to M in Formula 1.

**10.** The organometallic compound of any of claims 1-9, wherein,

in Formula 1, $L_2$ is a group represented by Formulae one of 3-1(1) to 3-1(66), 3-1(301) and 3-1(308):

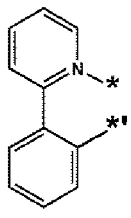
3-1(1)
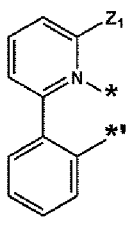
3-1(2)
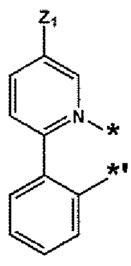
3-1(3)
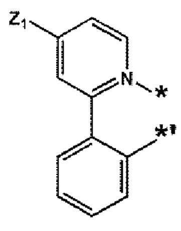
3-1(4)
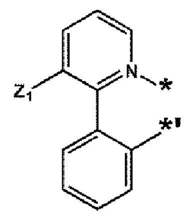
3-1(5)

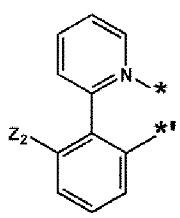
3-1(6)
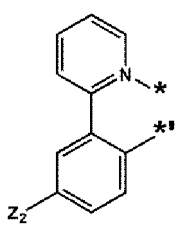
3-1(7)
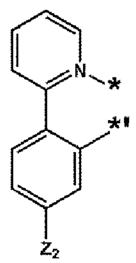
3-1(8)
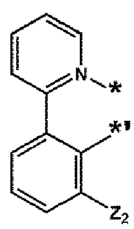
3-1(9)
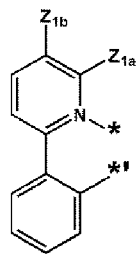
3-1(10)

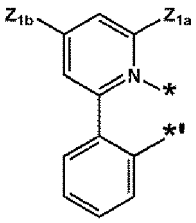
3-1(11)

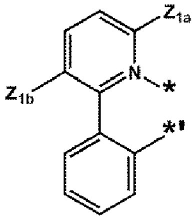
3-1(12)

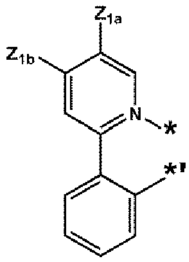
3-1(13)

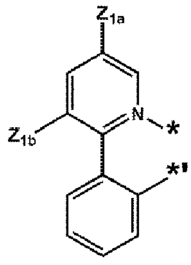
3-1(14)

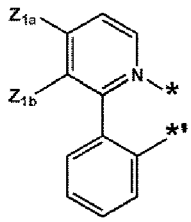
3-1(15)

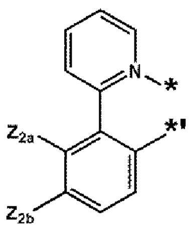
3-1(16)

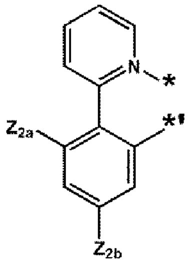
3-1(17)

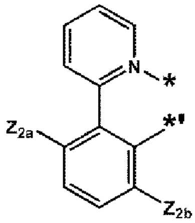
3-1(18)

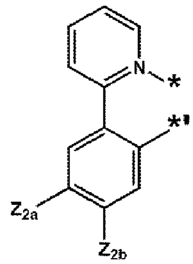
3-1(19)

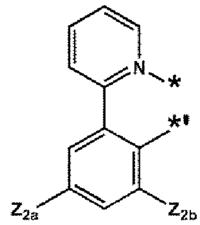
3-1(20)

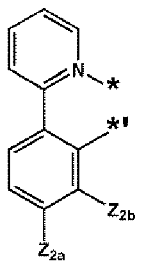
3-1(21)

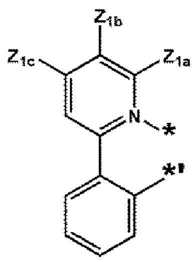
3-1(22)

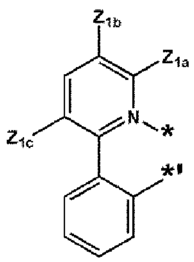
3-1(23)

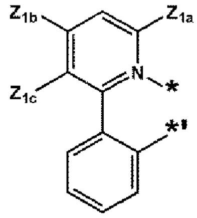
3-1(24)

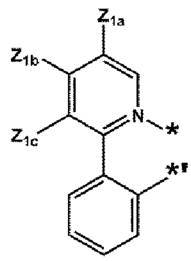
3-1(25)

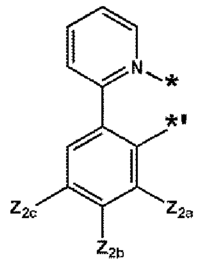
3-1(26)

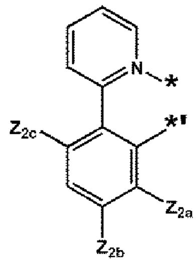
3-1(27)

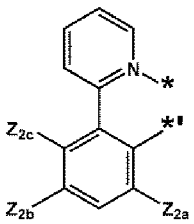
3-1(28)

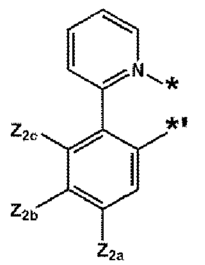
3-1(29)

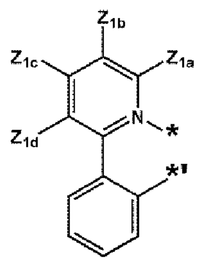
3-1(30)

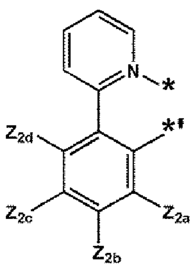
3-1(31)

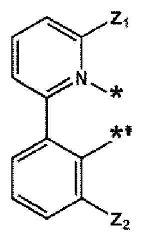
3-1(32)

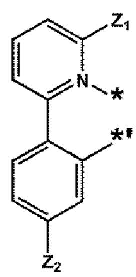
3-1(33)

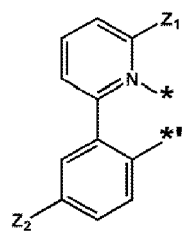
3-1(34)

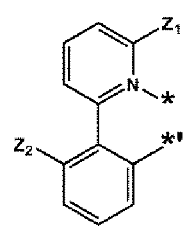
3-1(35)

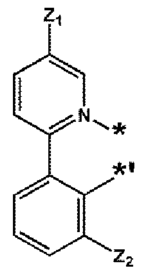
3-1(36)

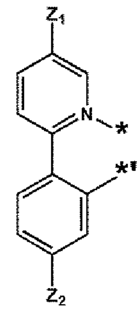
3-1(37)

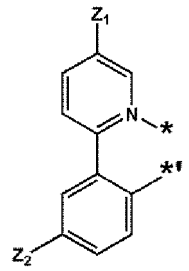
3-1(38)

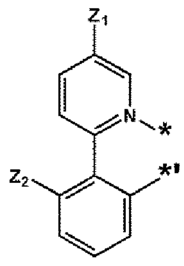
3-1(39)

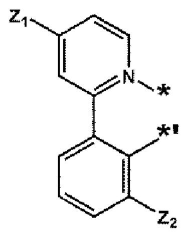
3-1(40)

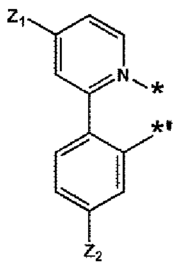
3-1(41)

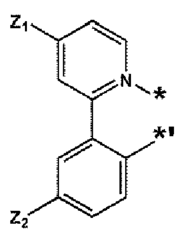
3-1(42)

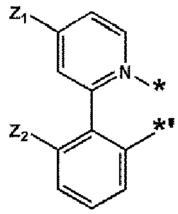
3-1(43)

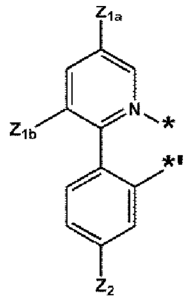
3-1(44)

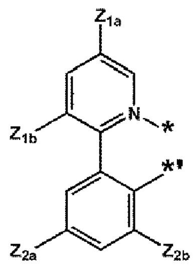
3-1(45)

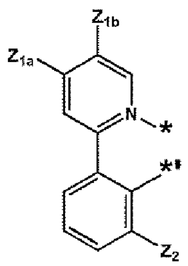
3-1(46)

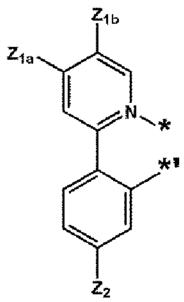
3-1(47)

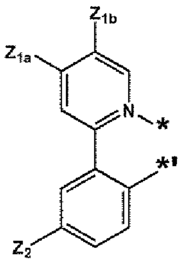
3-1(48)

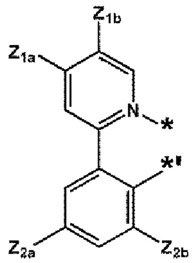
3-1(49)

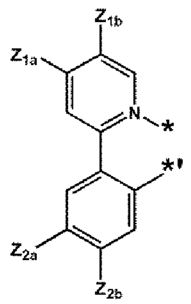
3-1(50)

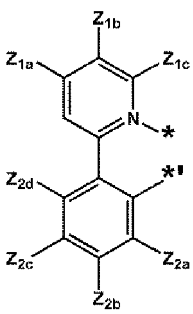
3-1(51)

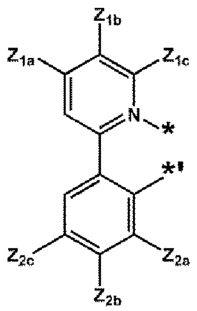
3-1(52)

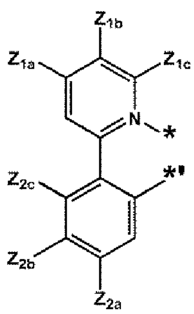
3-1(53)

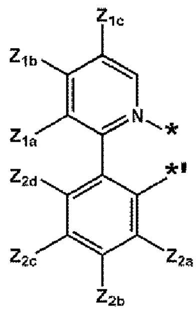
3-1(54)

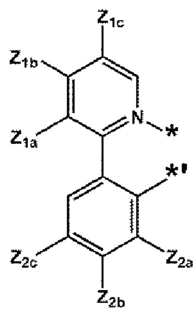
3-1(55)

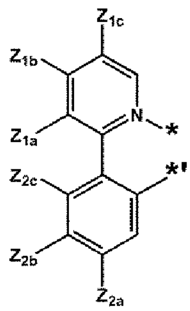

3-1(56)

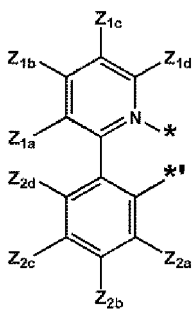

3-1(57)

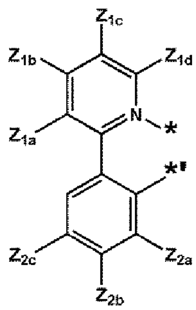

3-1(58)

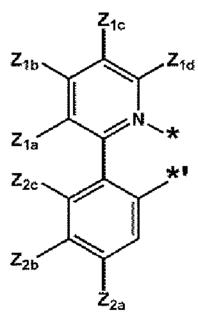

3-1(59)

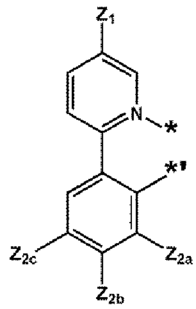

3-1(60)

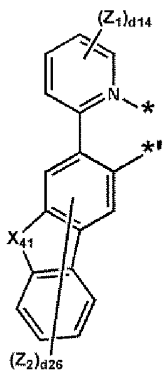

3-1(61)

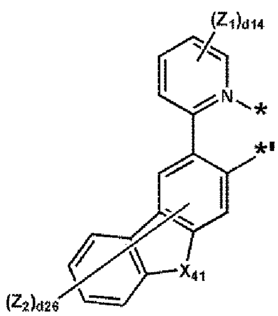

3-1(62)

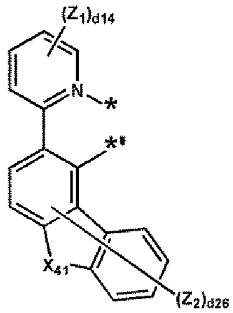

3-1(63)

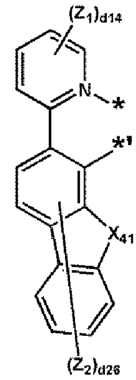

3-1(64)

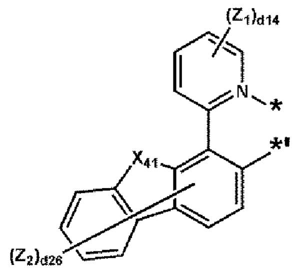

3-1(65)

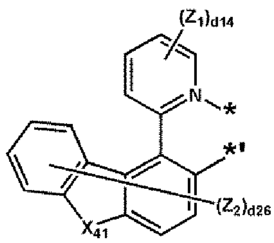

3-1(66)

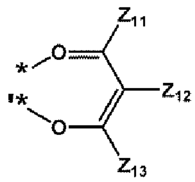

3-1(301)

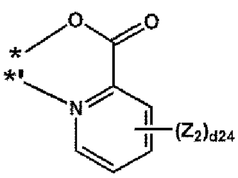

3-1(308)

wherein, in Formulae 3-1(1) to 3-1(66), 3-1(301) and 3-1(308),
$X_{41}$ is O, S, $N(Z_{21})$, $C(Z_{21})(Z_{22})$, or $Si(Z_{21})(Z_{22})$,
$Z_1$, $Z_2$, $Z_{1a}$, $Z_{1b}$, $Z_{1c}$, $Z_{1d}$, $Z_{2a}$, $Z_{2b}$, $Z_{2c}$, $Z_{2d}$, $Z_{11}$ to $Z_{13}$, $Z_{21}$ and $Z_{22}$ are each independently the same as defined in connection with $R_{21}$ in claim 1,

d14 is an integer from 0 to 4,
d26 is an integer from 0 to 6, and
* and *' each indicate a binding site to M in Formula 1.

**11.** The organometallic compound of claim 1, wherein the organometallic compound is one of Compounds 1 to 15, 17-218, 220-328, 330-438, 440-453, 455-466, 468 and 469:

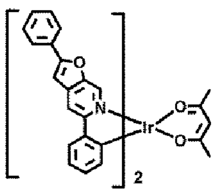
1

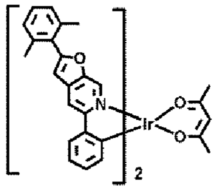
2

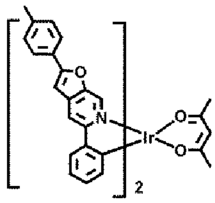
3

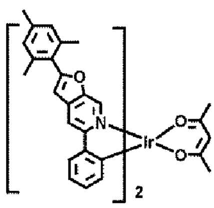
4

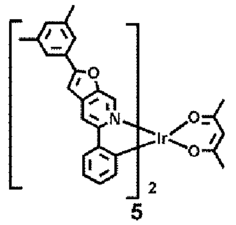
5

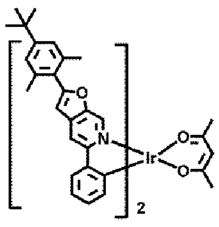
6

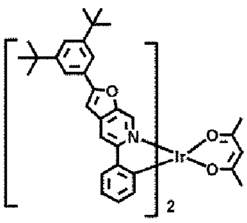
7

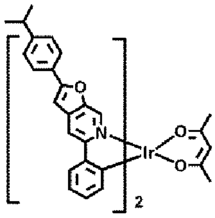
8

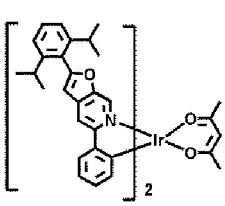
9

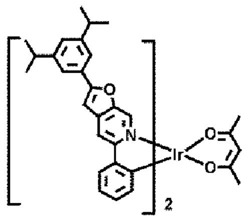
10

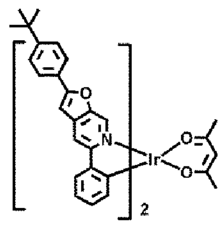
11

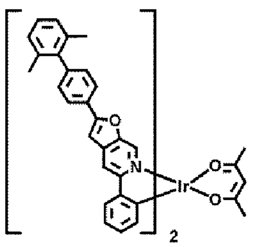
12

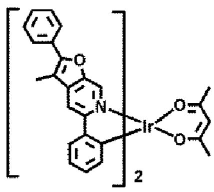
13

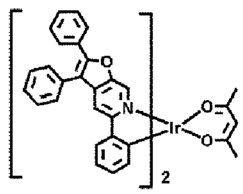
14

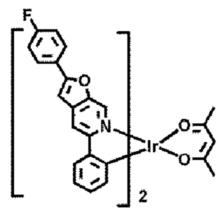
15

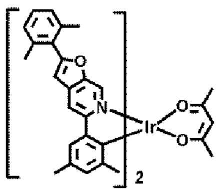
17

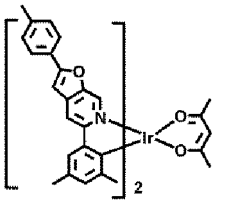
18

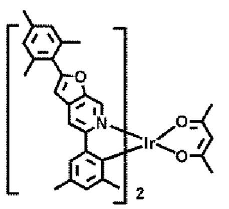
19

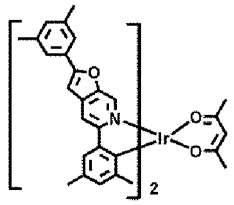
20

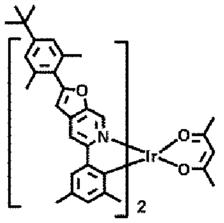
21

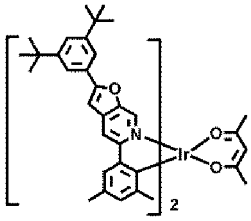
22

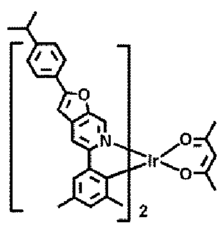
23

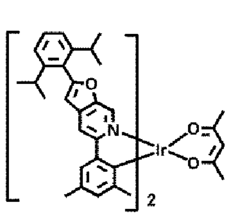
24

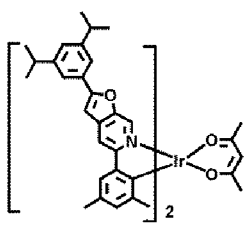
25

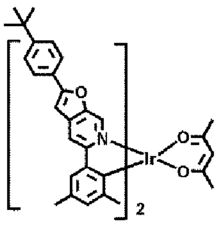
26

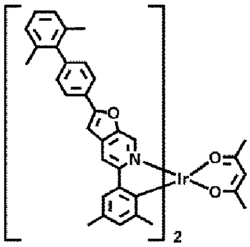
27

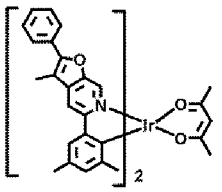
28

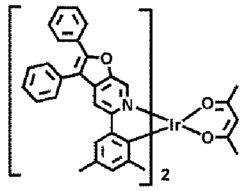
29

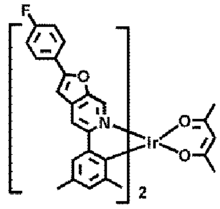
30

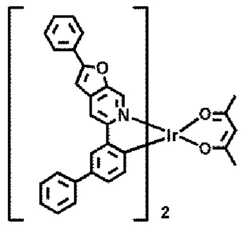
31

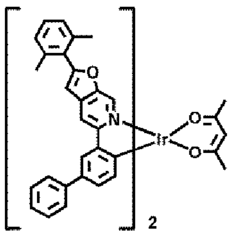
32

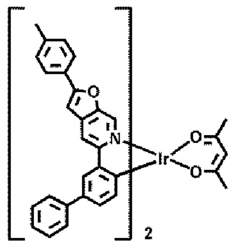
33

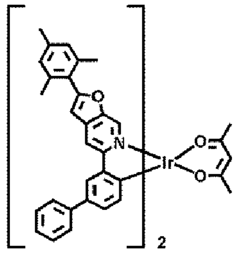
34

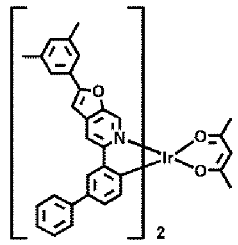
35

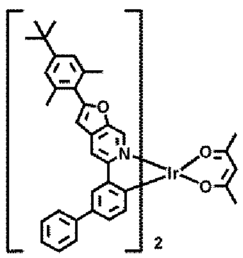
36

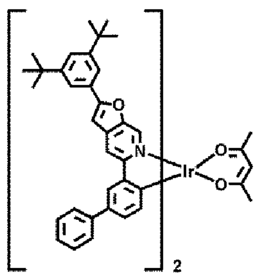
37

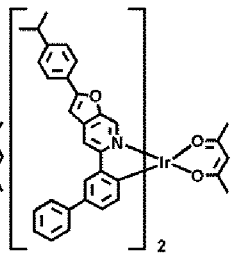
38

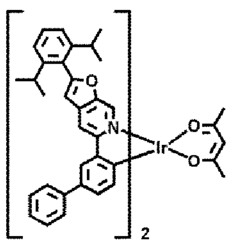
39

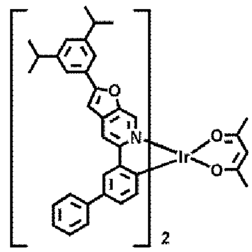
40

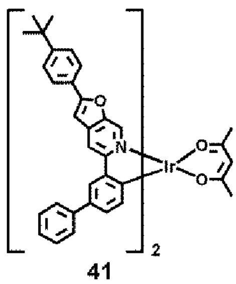
41

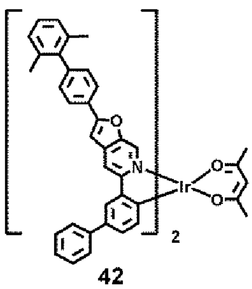
42

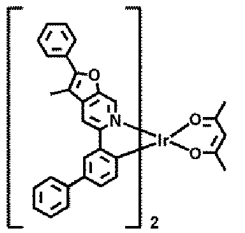
43

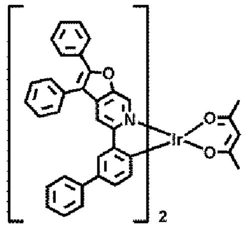
44

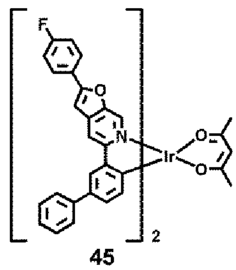
45

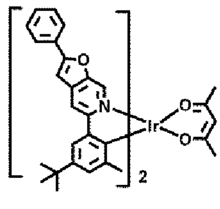
46

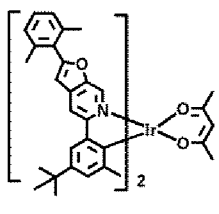
47

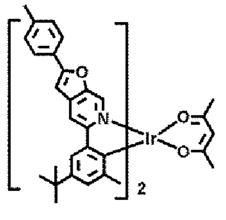
48

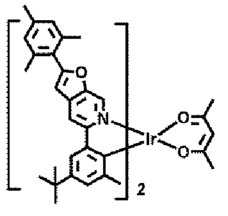
49

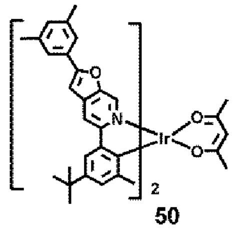
50

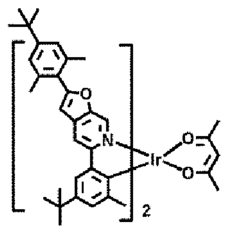
51

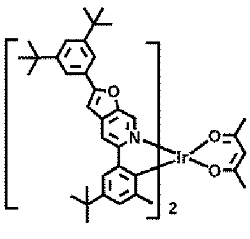
52

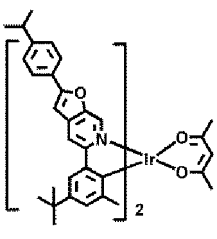
53

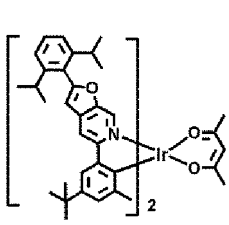
54

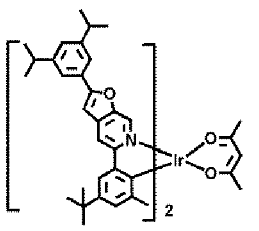
55

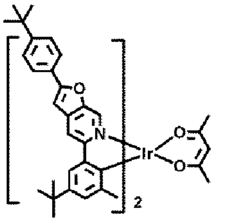
56

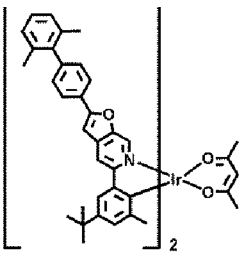
57

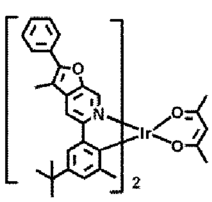
58

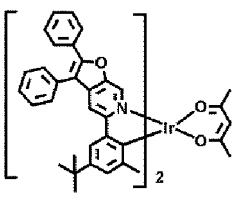
59

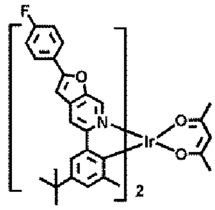
60

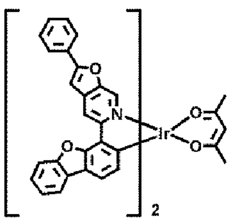
61

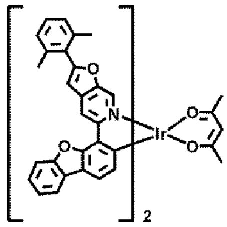
62

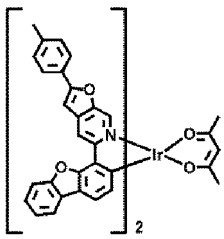
63

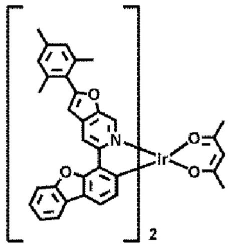
64

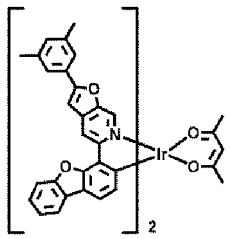
65

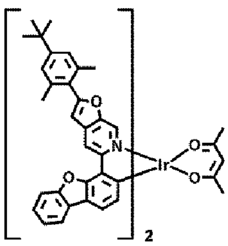
66

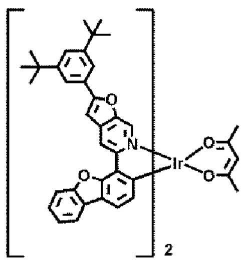
67

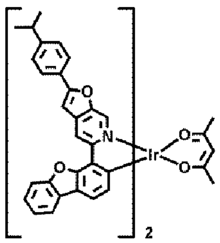
68

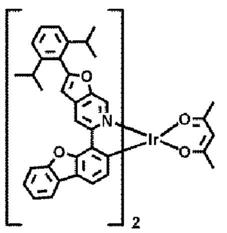
69

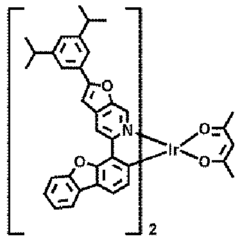
70

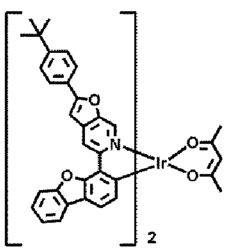
71

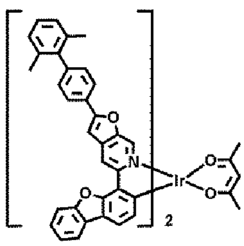
72

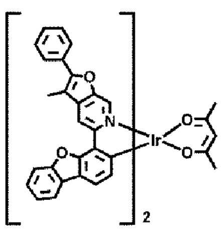
73

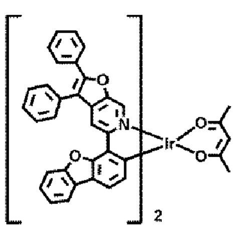
74

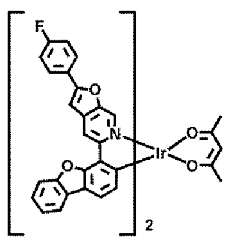
75

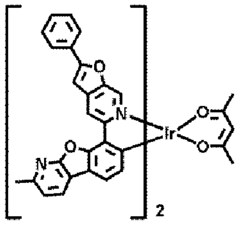
**76**

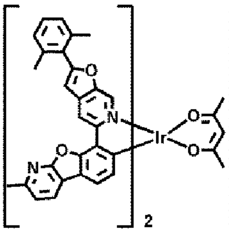
**77**

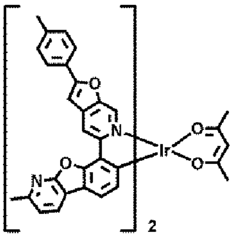
**78**

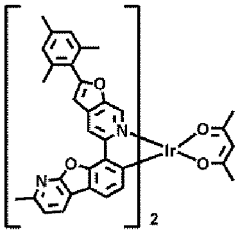
**79**

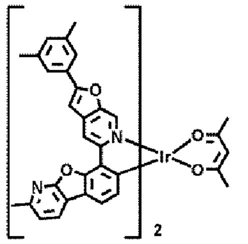
**80**

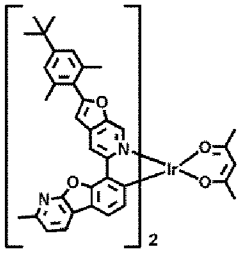
**81**

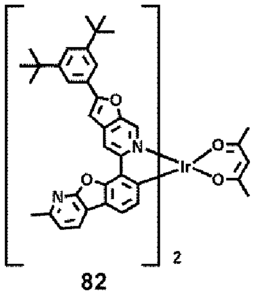
**82**

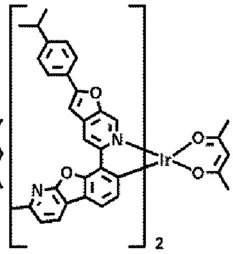
**83**

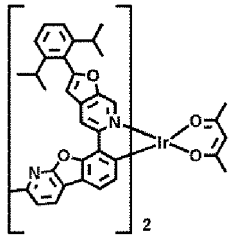
**84**

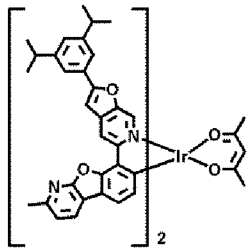
**85**

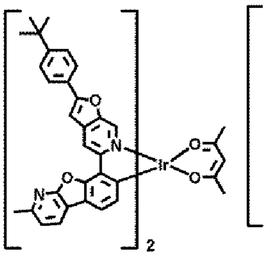
**86**

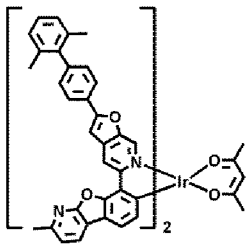
**87**

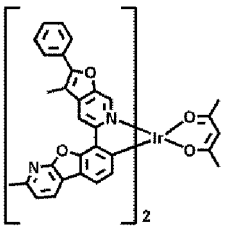
**88**

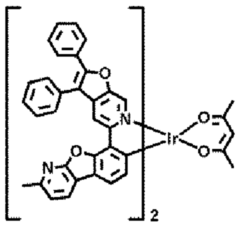
**89**

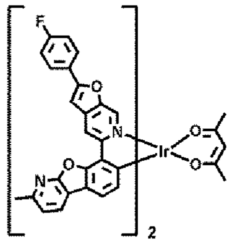
**90**

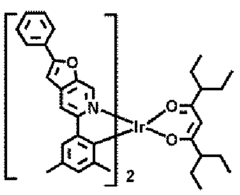
**91**

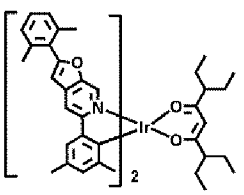
**92**

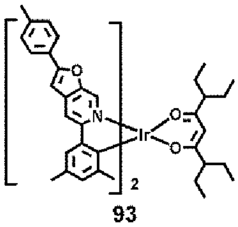
**93**

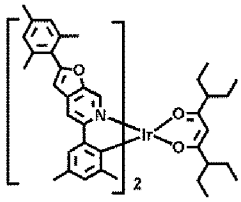
**94**

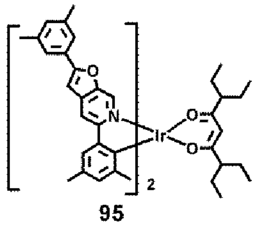
**95**

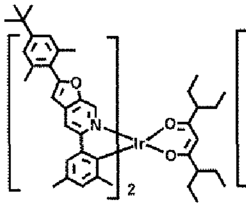
**96**

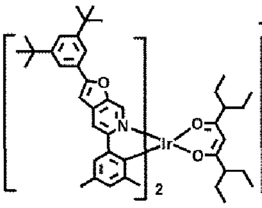
**97**

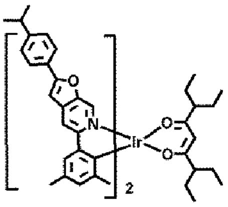
**98**

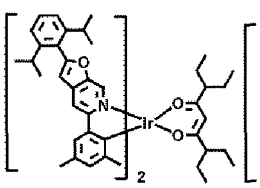
**99**

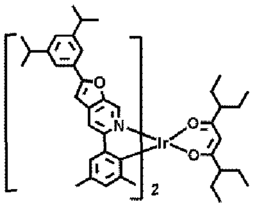
**100**

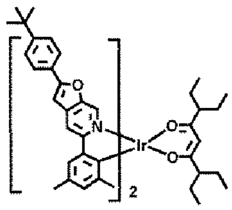
101

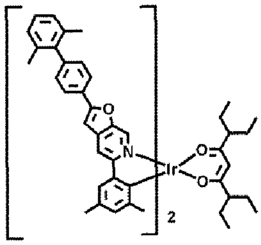
102

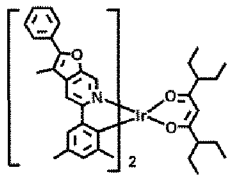
103

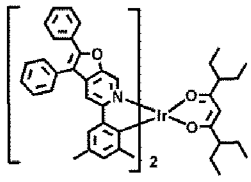
104

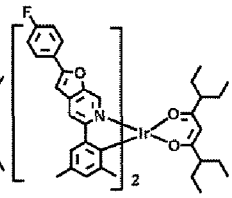
105

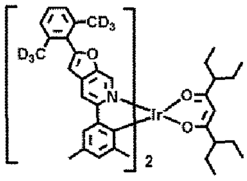
106

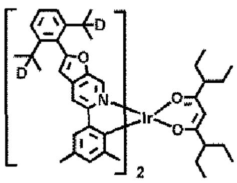
107

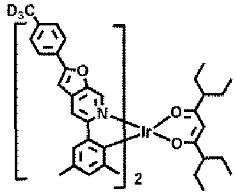
108

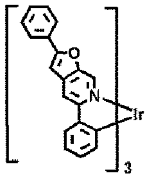
109

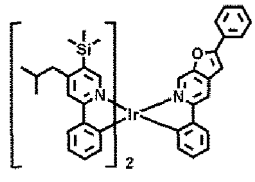
110

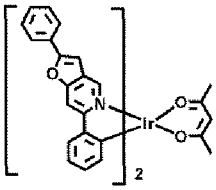
111

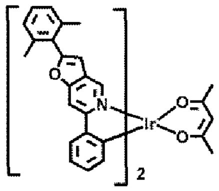
112

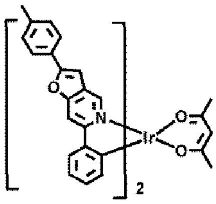
113

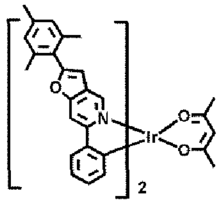
114

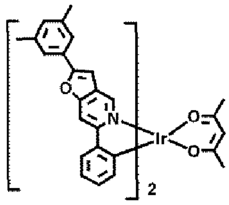
115

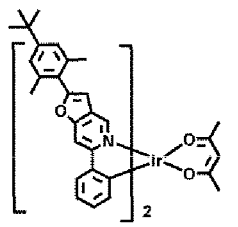
116

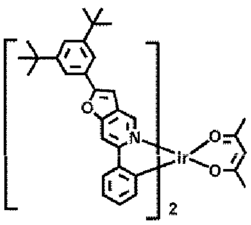
117

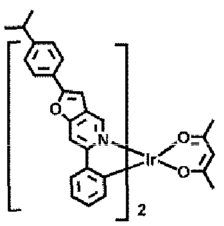
118

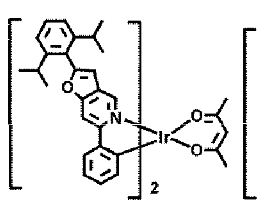
119

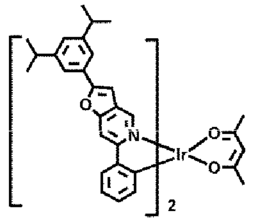
120

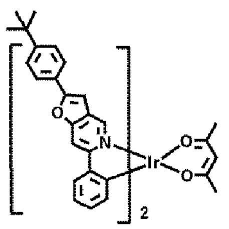
121

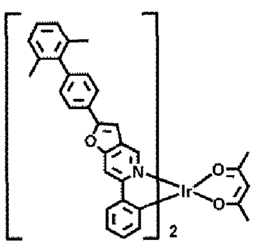
122

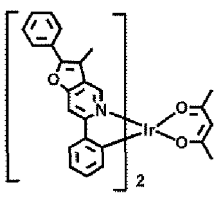
123

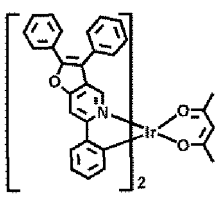
124

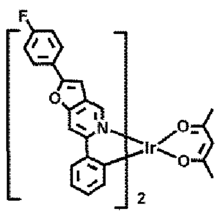
125

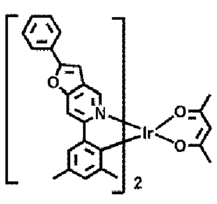
126

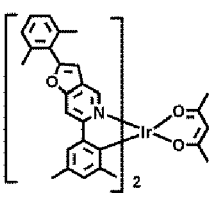
127

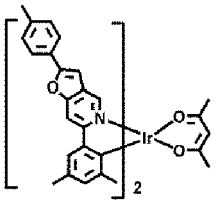
128

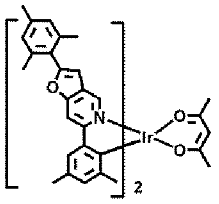
129

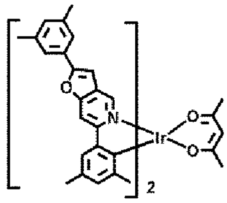
130

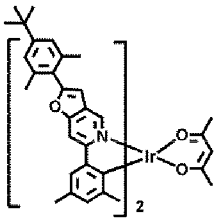
131

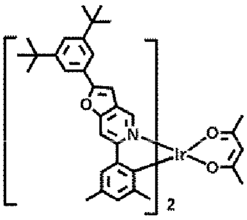
132

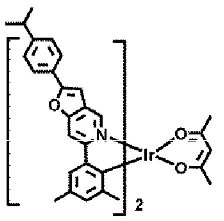
133

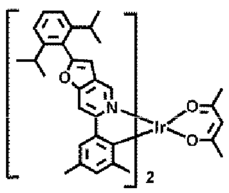
134

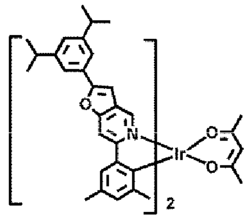
135

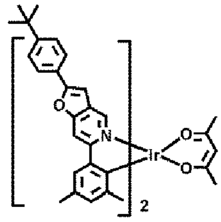
136

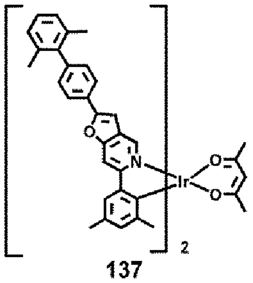
137

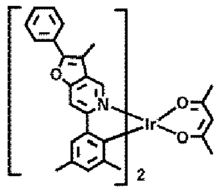
138

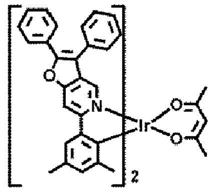
139

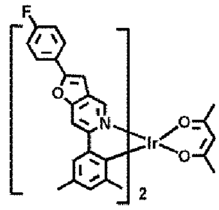
140

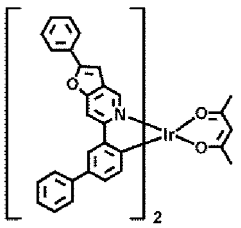
141

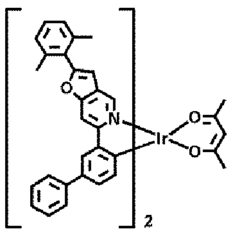
142

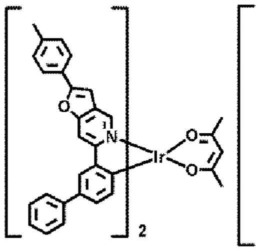
143

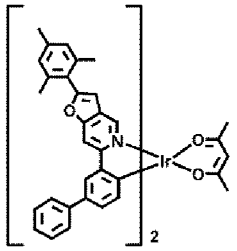
144

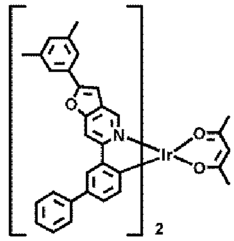
145

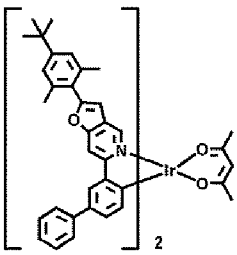
146

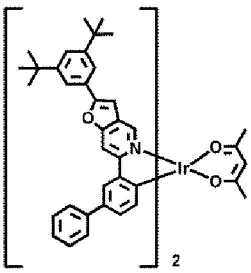
147

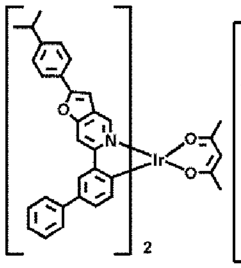
148

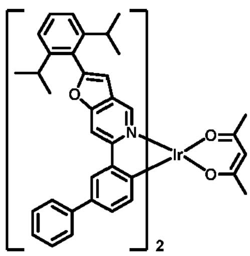
149

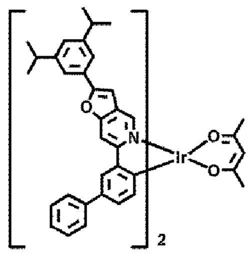
150

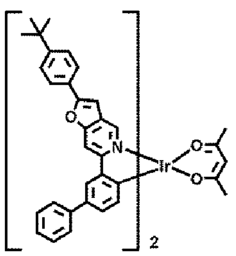
151

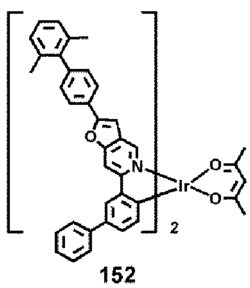
152

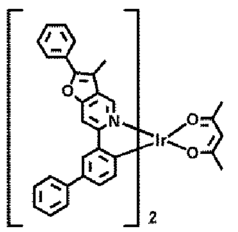
153

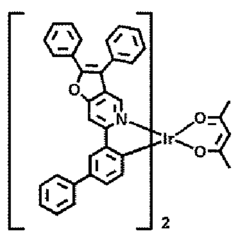
154

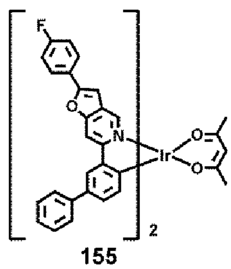
155

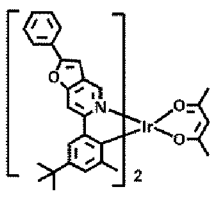
156

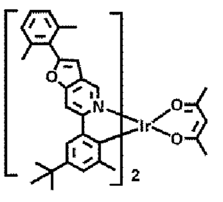
157

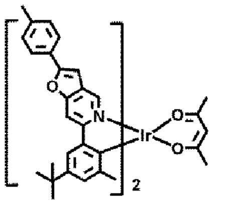
158

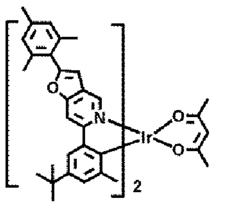
159

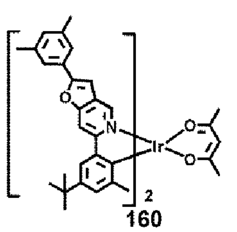
160

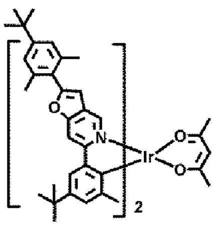
161

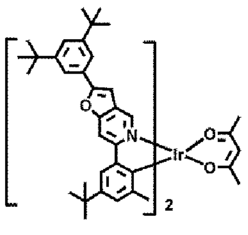
162

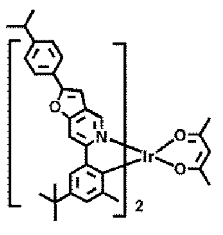
163

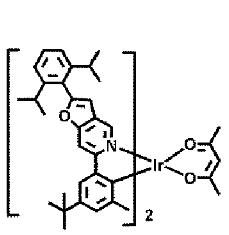
164

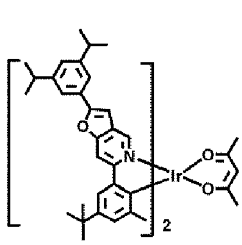
165

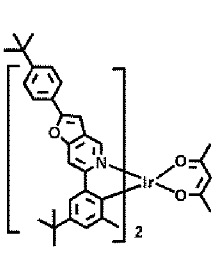
166

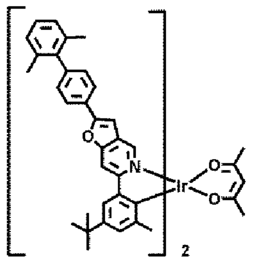
167

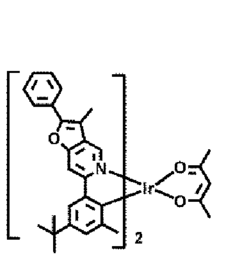
168

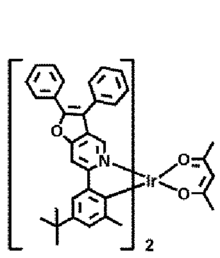
169

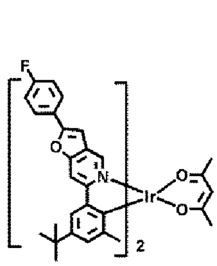
170

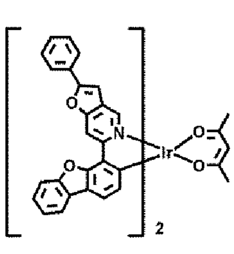
171

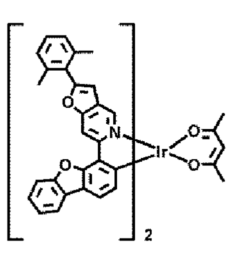
172

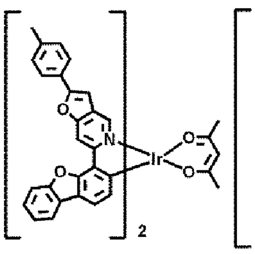
173

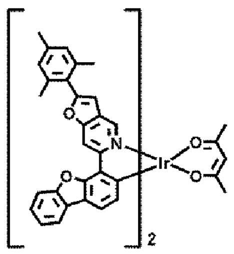
174

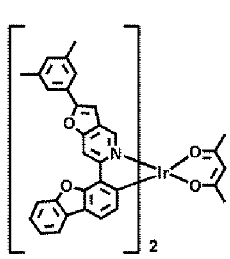
175

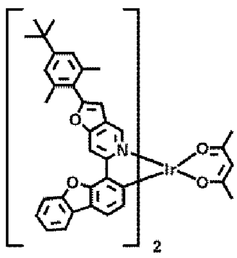
176

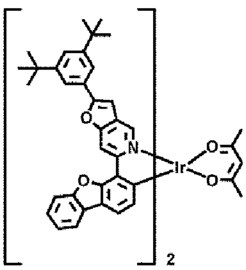
177

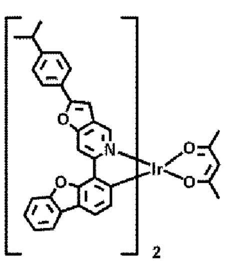
178

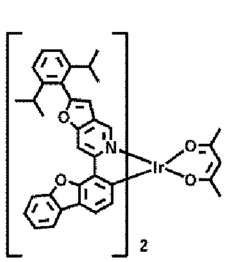
179

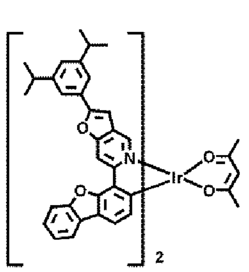
180

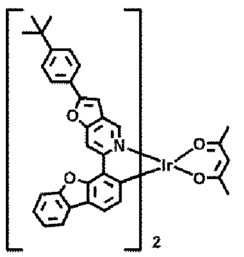
181

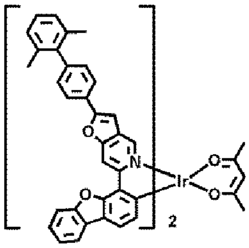
182

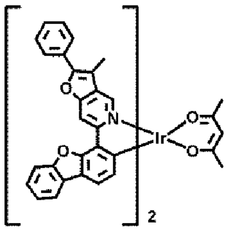
183

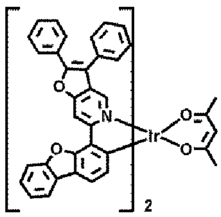
184

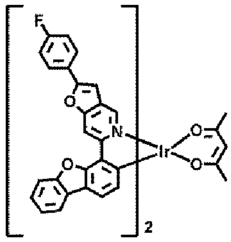
185

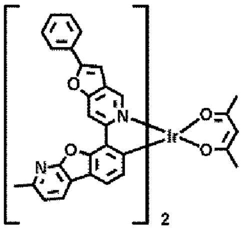
186

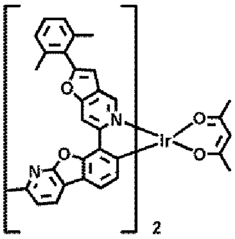
187

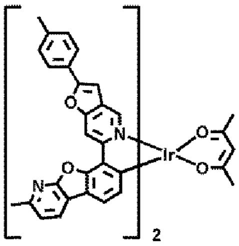
188

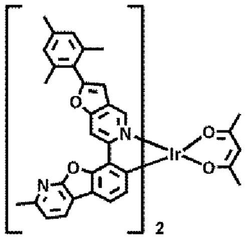
189

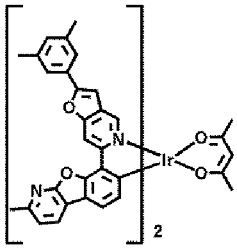
190

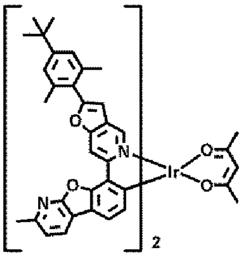
191

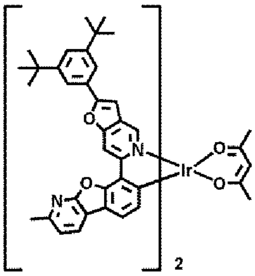
192

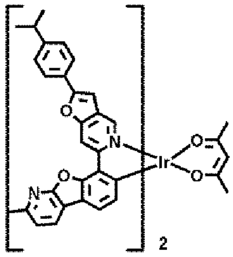
193

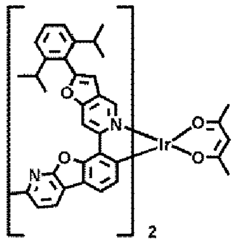
194

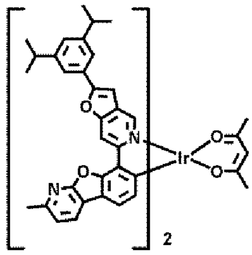
195

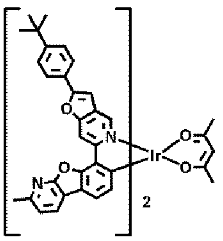
196

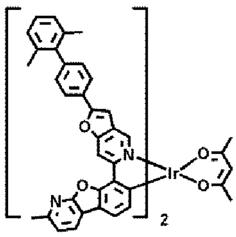
197

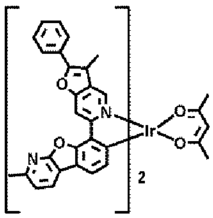
198

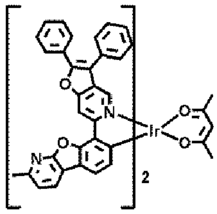
199

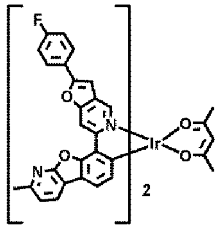
200

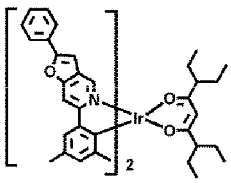
201

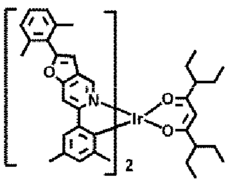
202

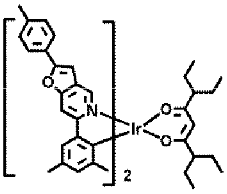
203

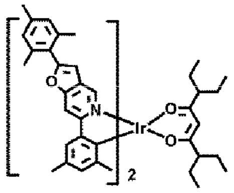
204

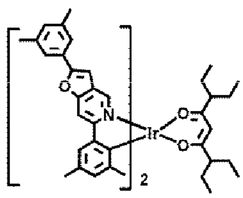
205

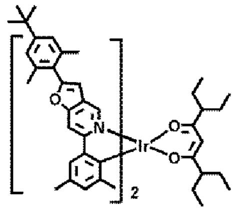
206
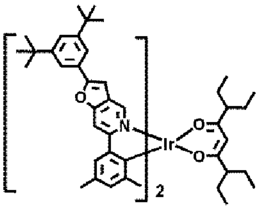
207
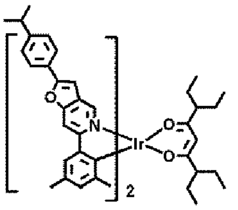
208
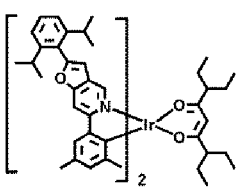
209
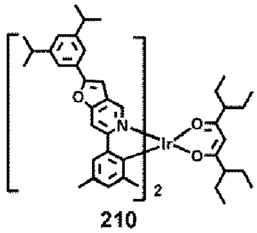
210

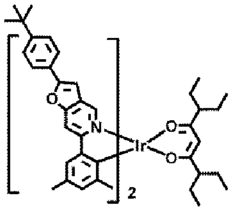
211
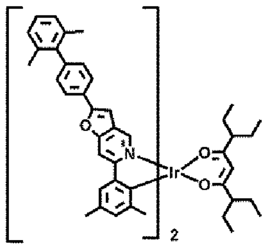
212
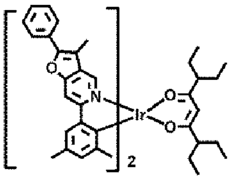
213
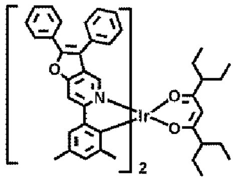
214
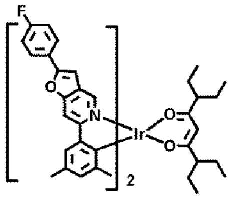
215

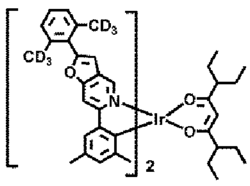
216
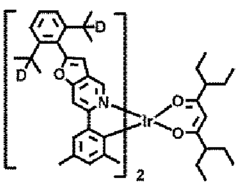
217
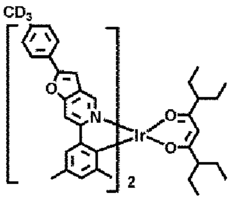
218
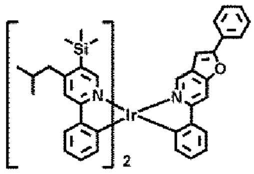
220

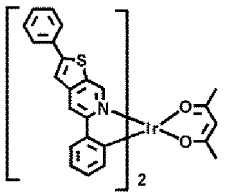
221
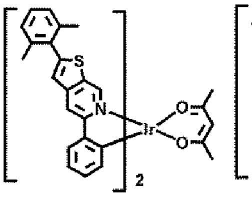
222
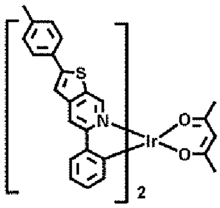
223
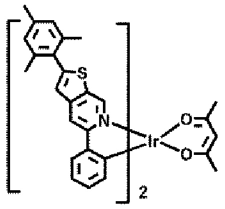
224
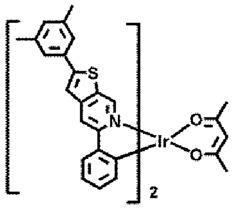
225

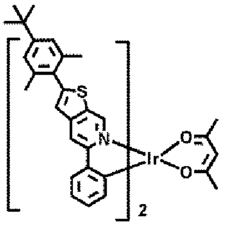
226
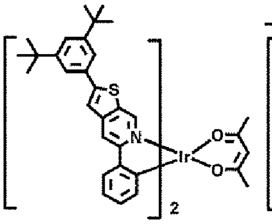
227
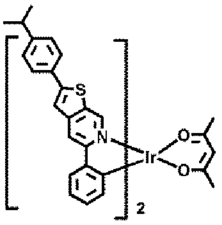
228
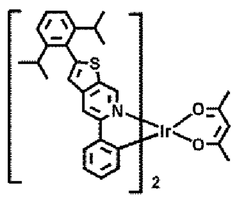
229
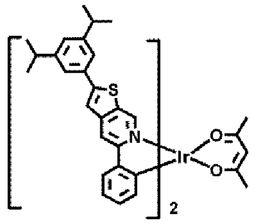
230

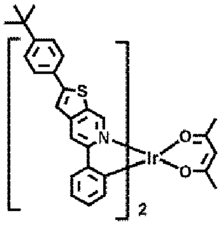
231
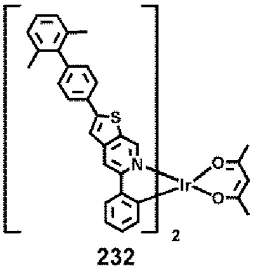
232
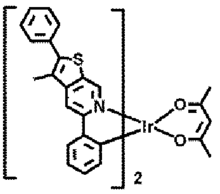
233
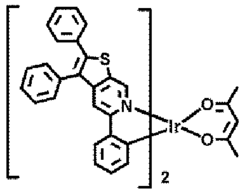
234
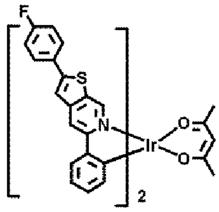
235

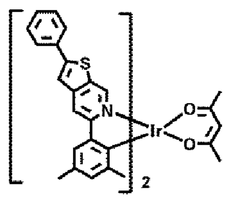
236

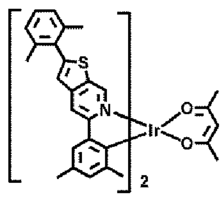
237

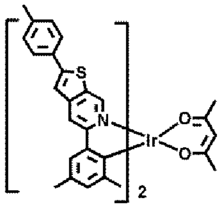
238

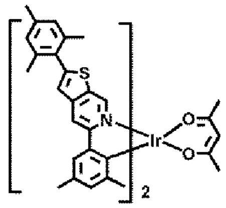
239

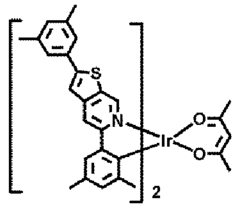
240

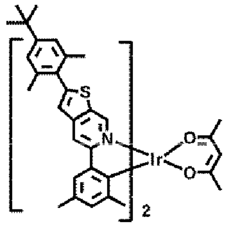
241

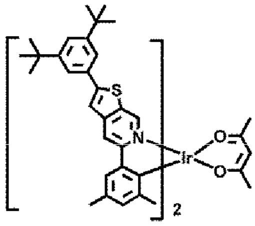
242

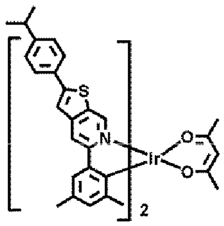
243

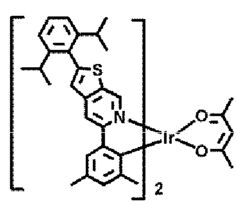
244

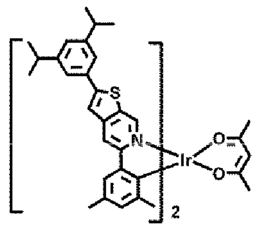
245

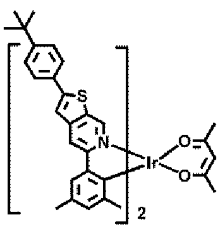
246

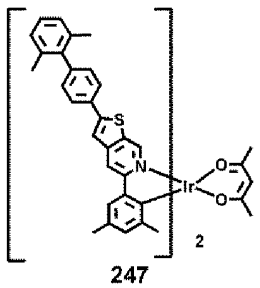
247

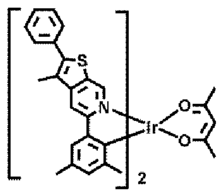
248

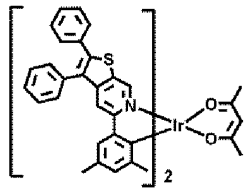
249

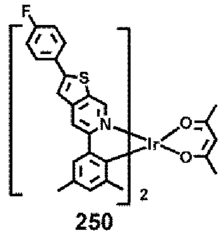
250

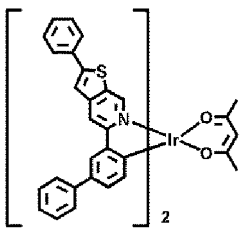
251

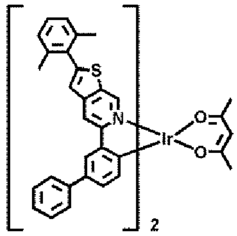
252

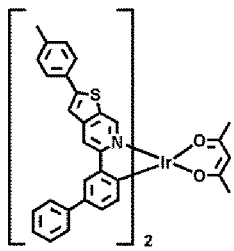
253

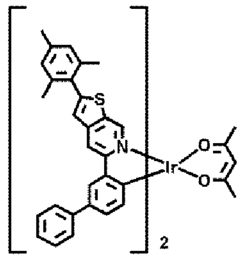
254

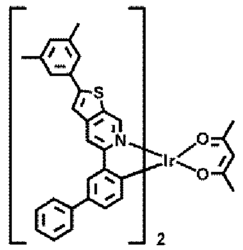
255

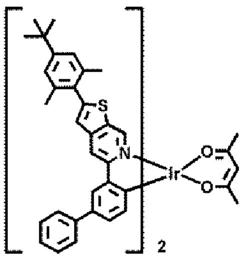
256

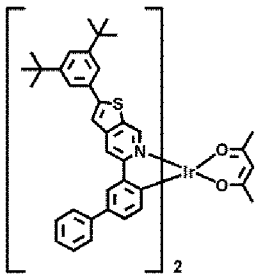
257

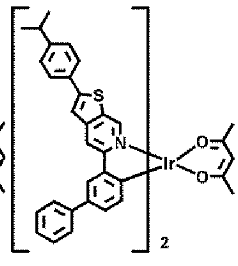
258

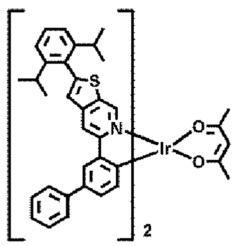
259

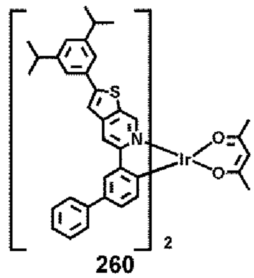
260

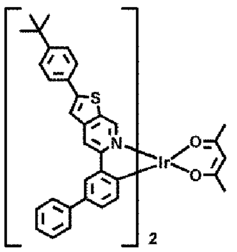
261

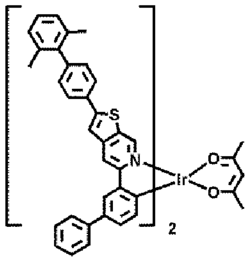
262

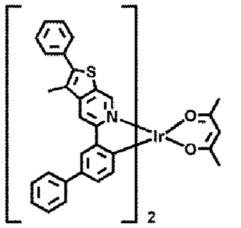
263

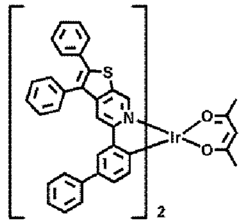
264

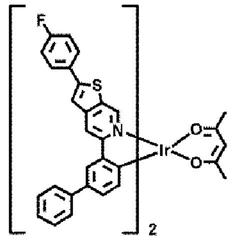
265

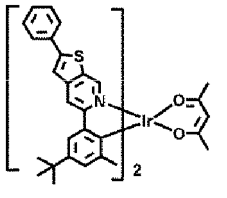
266

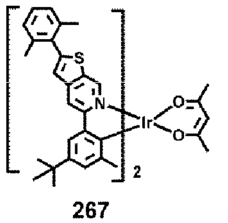
267

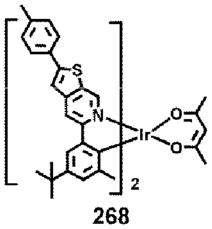
268

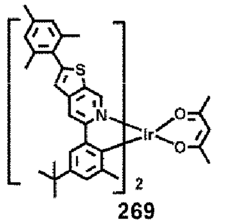
269

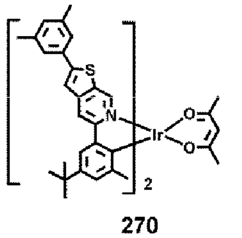
270

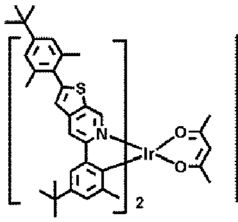
271

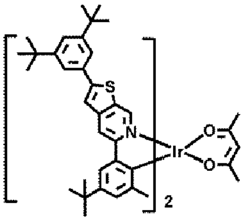
272

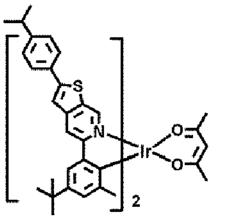
273

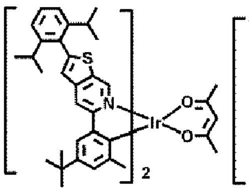
274

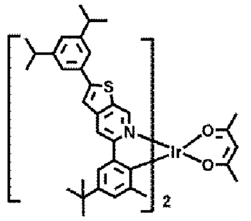
275

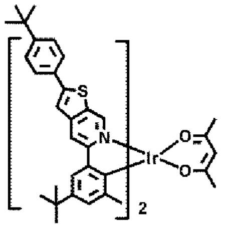
276

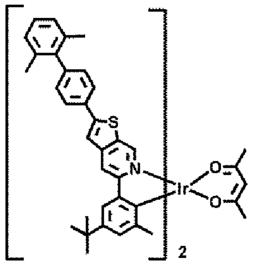
277

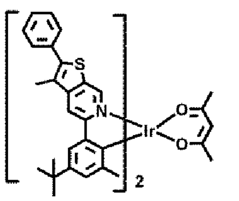
278

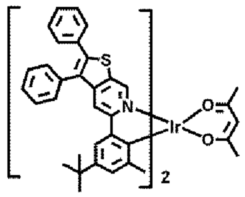
279

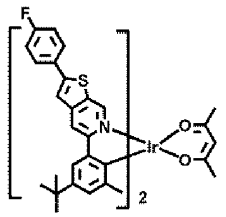
280

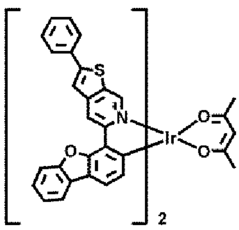
281

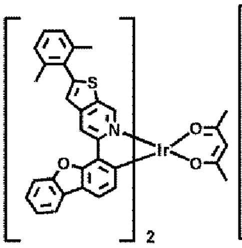
282

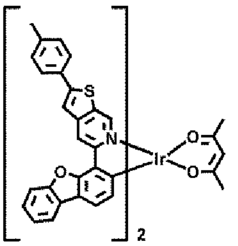
283

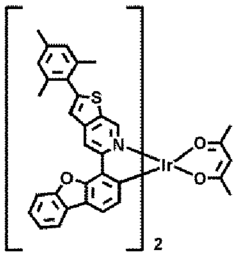
284

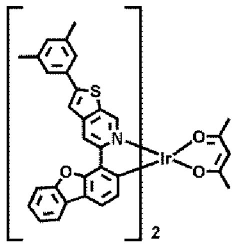
285

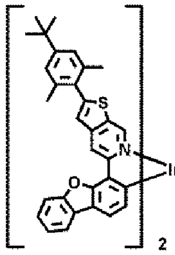
**286**

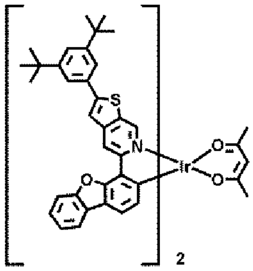
**287**

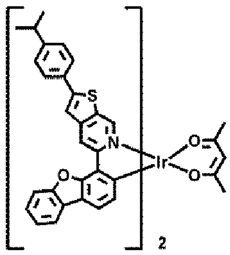
**288**

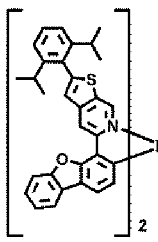
**289**

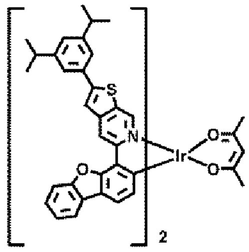
**290**

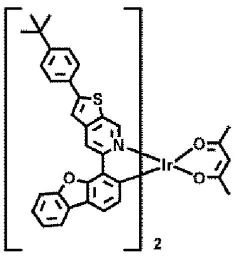
**291**

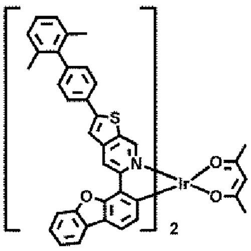
**292**

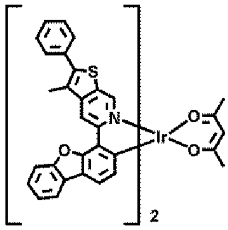
**293**

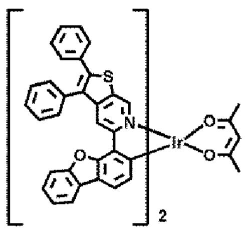
**294**

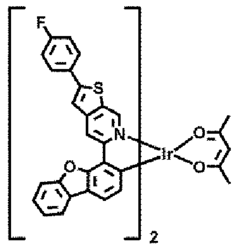
**295**

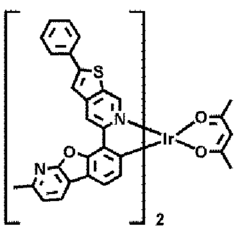
**296**

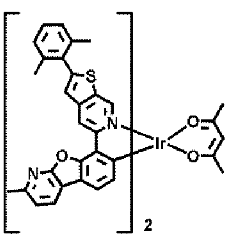
**297**

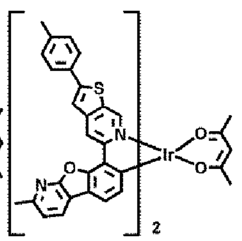
**298**

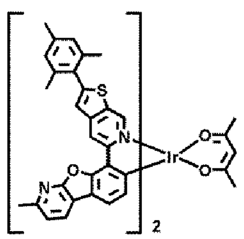
**299**

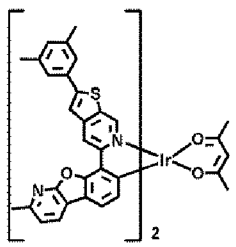
**300**

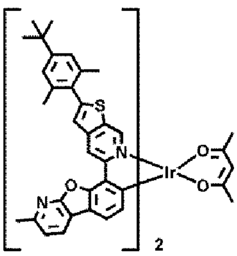
**301**

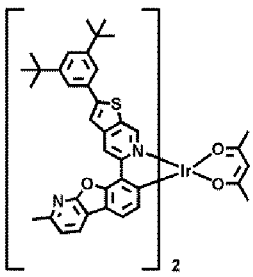
**302**

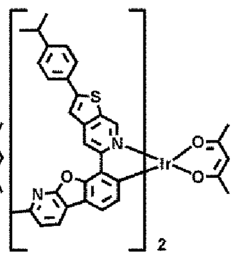
**303**

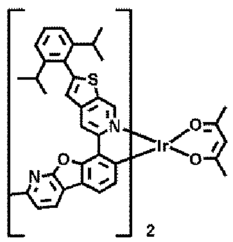
**304**

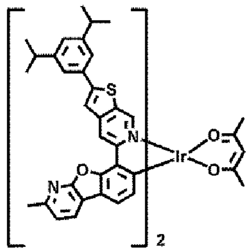
**305**

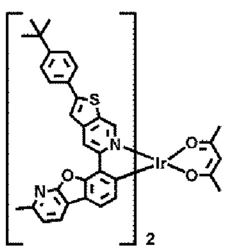
**306**

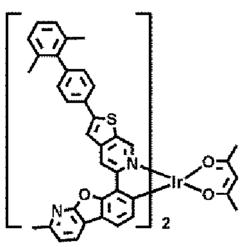
**307**

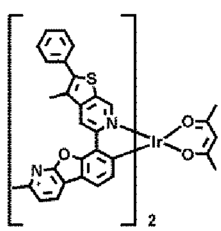
**308**

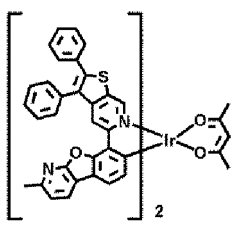
**309**

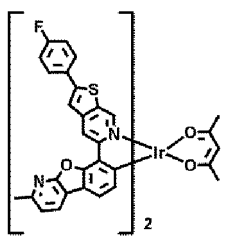
**310**

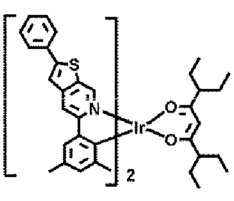

311

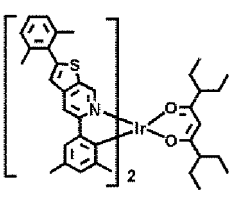

312

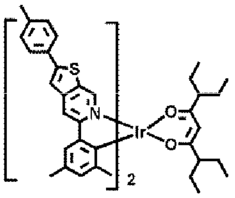

313

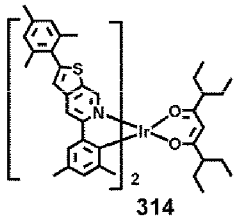

314

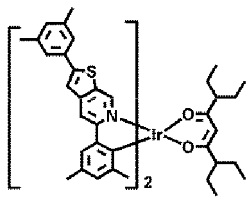

315

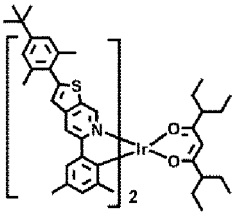

316

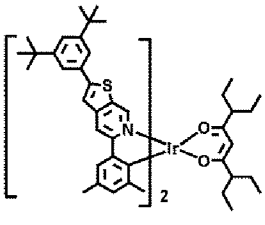

317

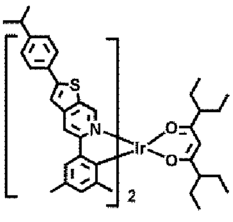

318

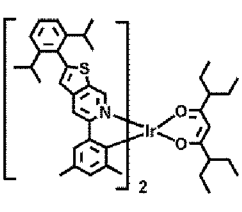

319

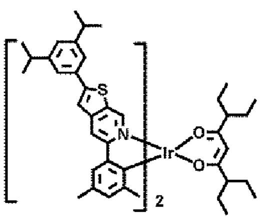

320

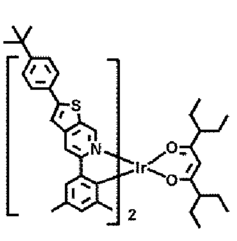

321

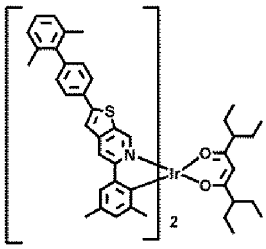

322

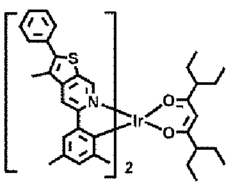

323

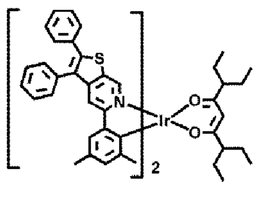

324

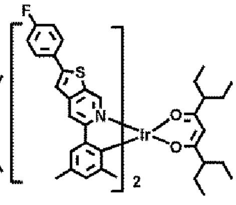

325

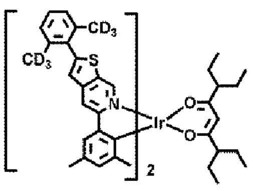

326

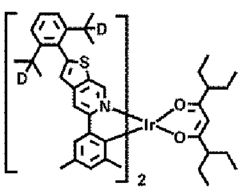

327

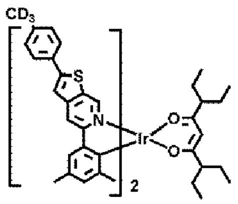

328

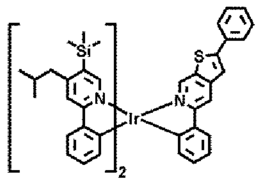

330

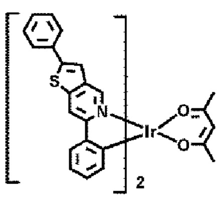

331

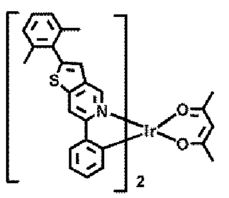

332

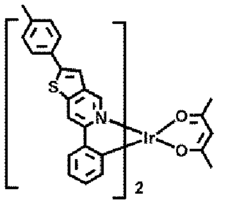

333

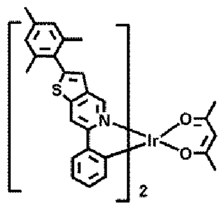

334

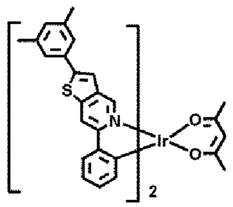

335

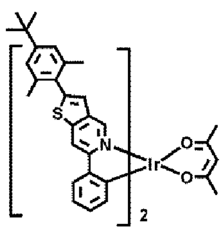

336

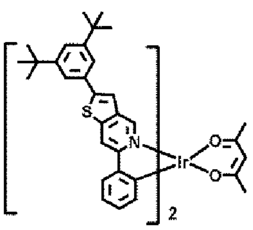

337

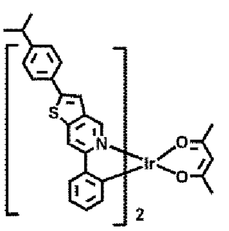

338

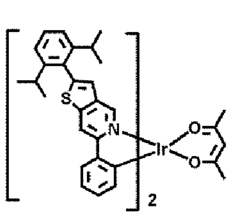

339

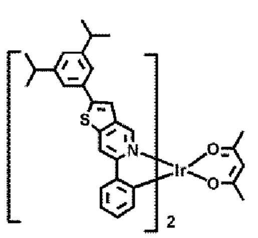

340

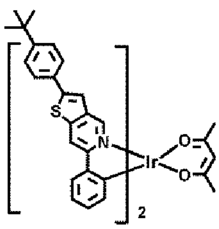
341
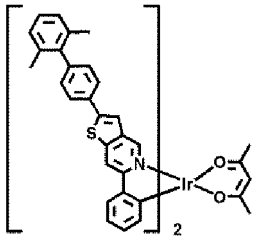
342
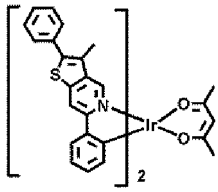
343
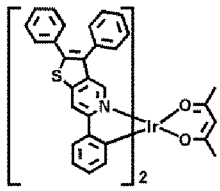
344
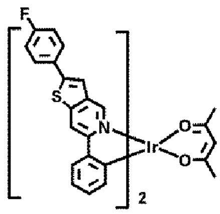
345

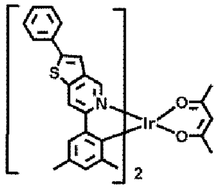
346
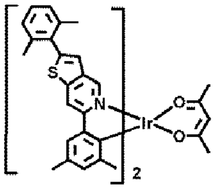
347
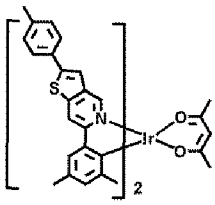
348
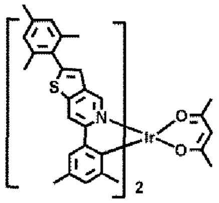
349
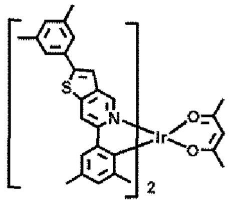
350

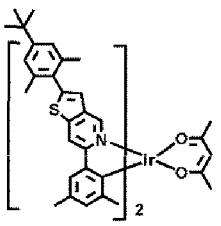
351
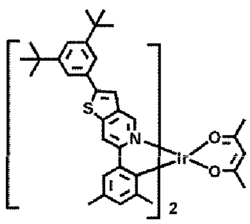
352
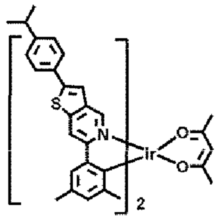
353
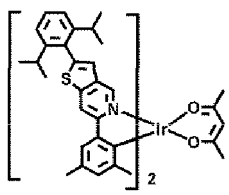
354
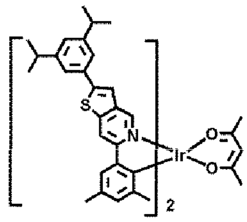
355

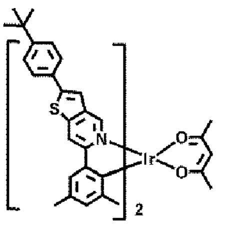
356
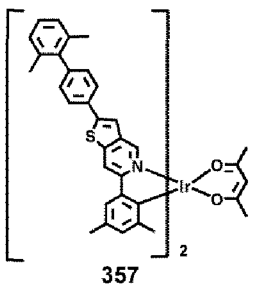
357
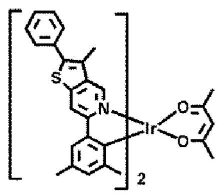
358
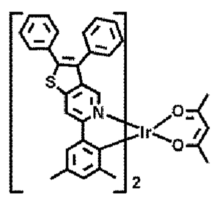
359
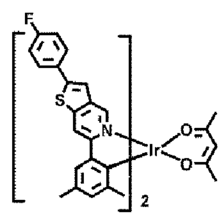
360

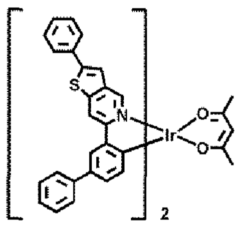
361
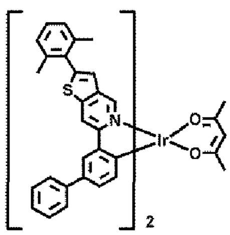
362
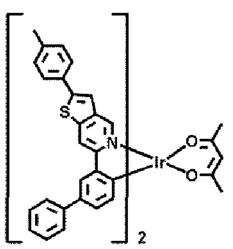
363
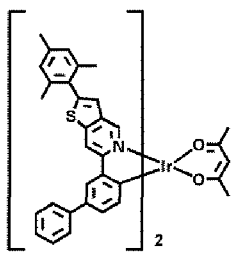
364
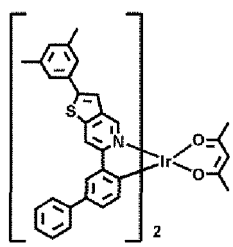
365

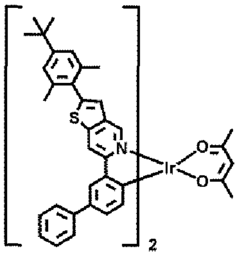
366

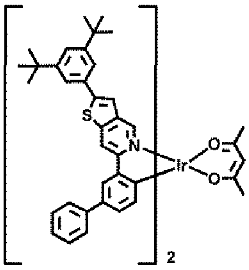
367

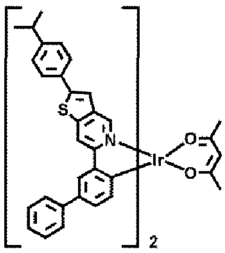
368

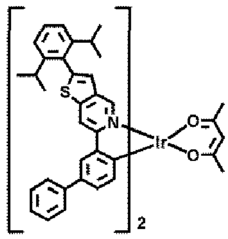
369

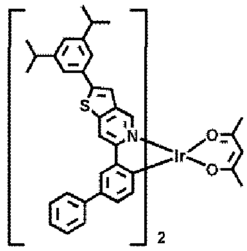
370

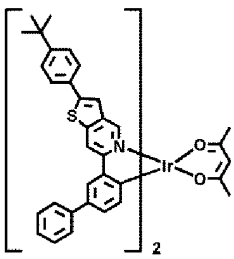
371

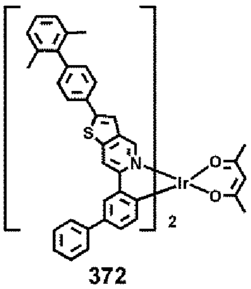
372

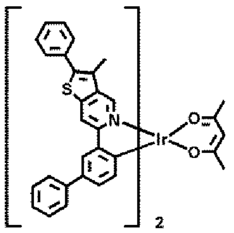
373

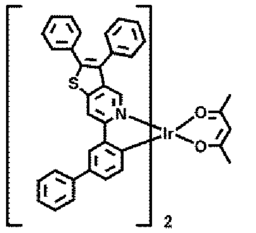
374

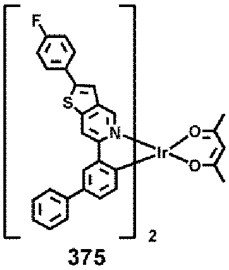
375

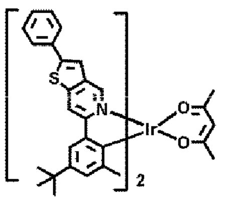
376

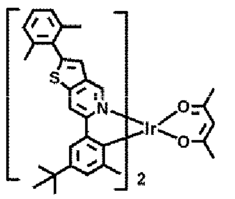
377

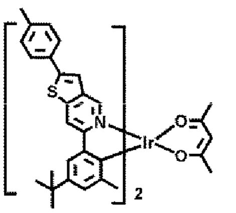
378

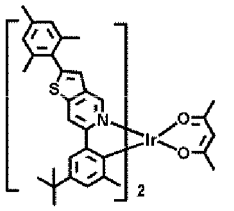
379

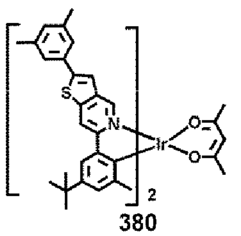
380

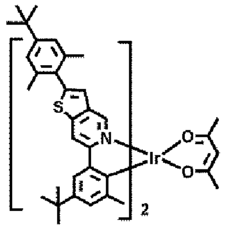
381

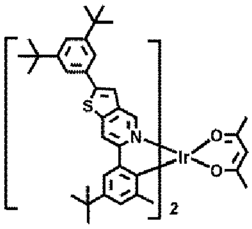
382

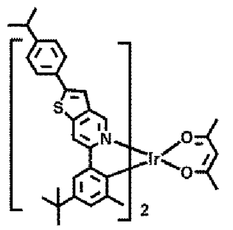
383

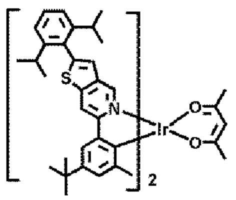
384

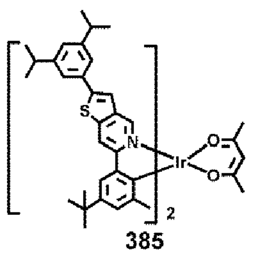
385

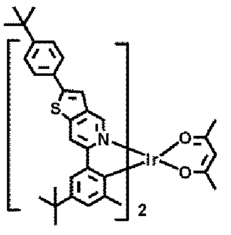
386

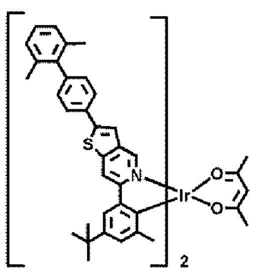
387

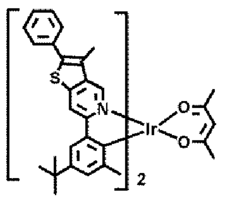
388

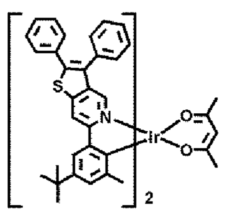
389

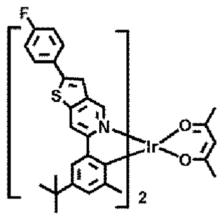
390

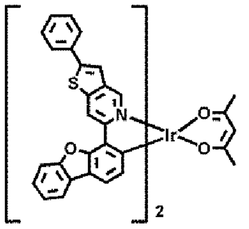
391
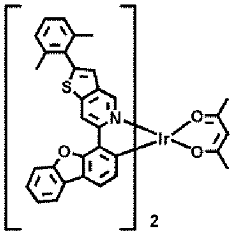
392
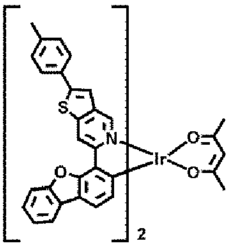
393
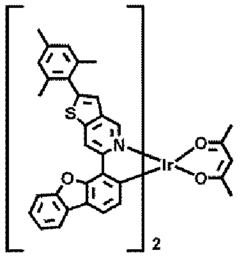
394
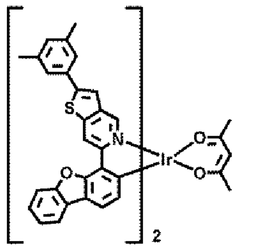
395

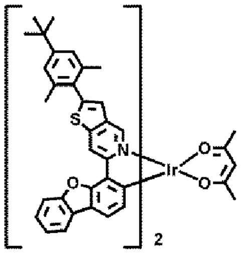
396
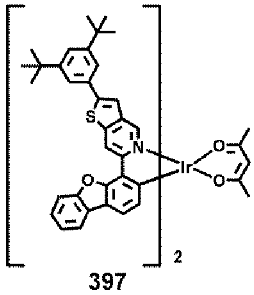
397
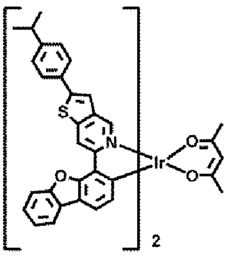
398
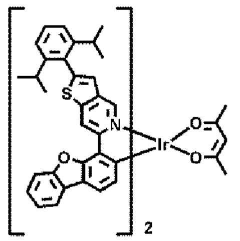
399
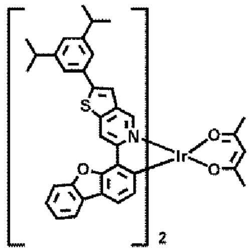
400

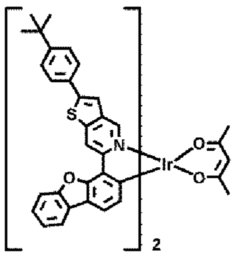
401
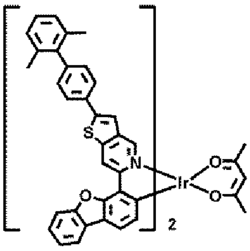
402
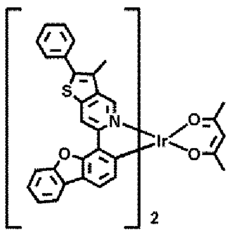
403
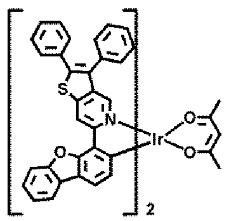
404
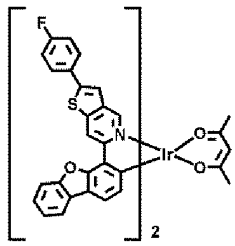
405

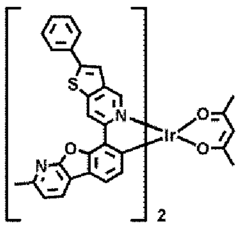
406
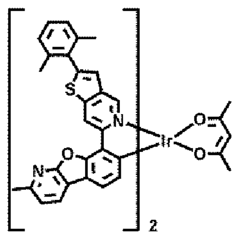
407
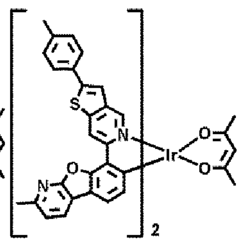
408
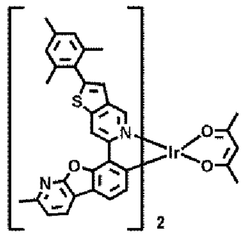
409
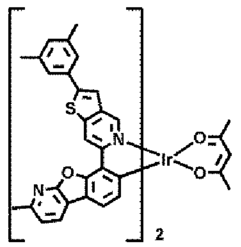
410

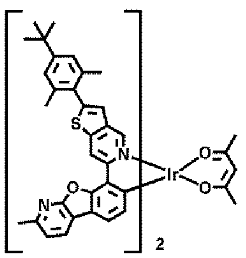
411
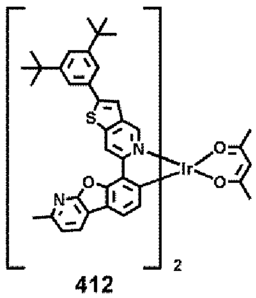
412
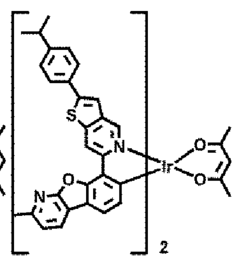
413
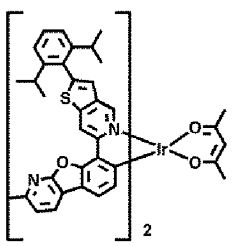
414
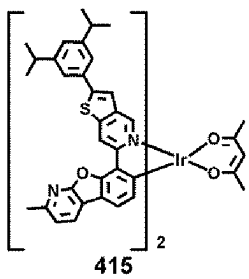
415

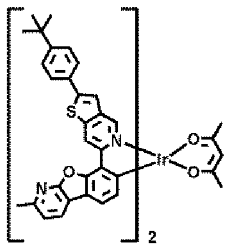
416

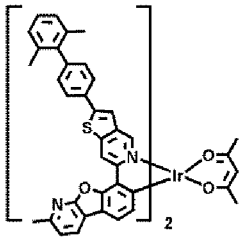
417

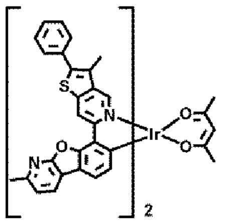
418

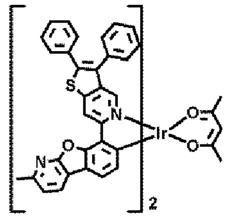
419

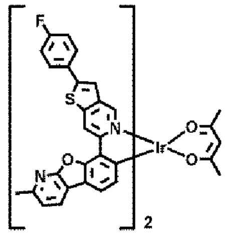
420

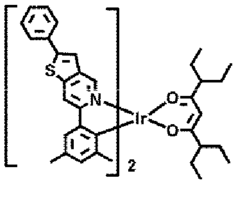
421

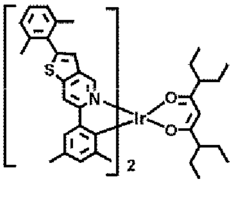
422

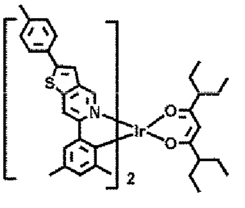
423

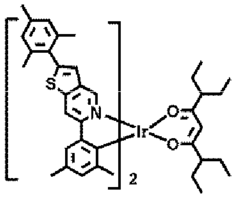
424

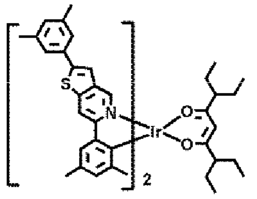
425

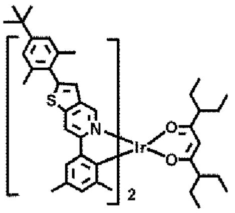
426

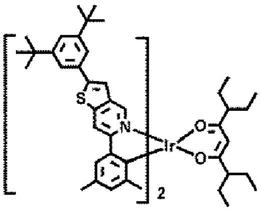
427

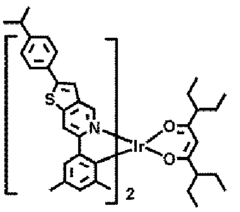
428

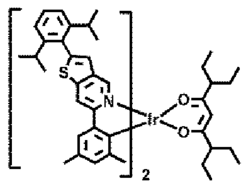
429

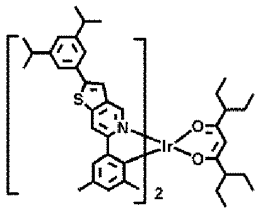
430

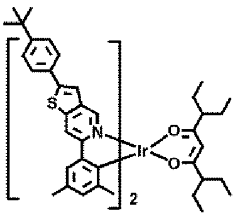
431

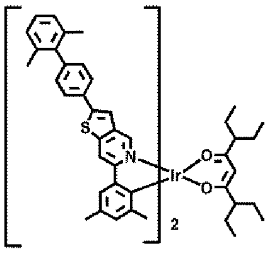
432

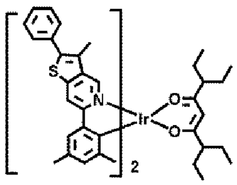
433

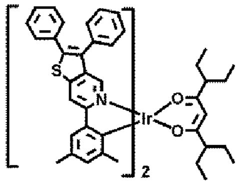
434

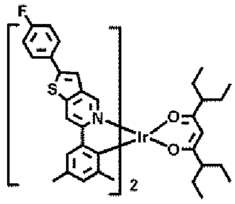
435

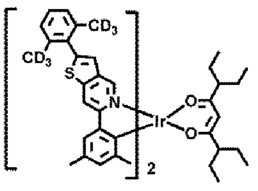
436

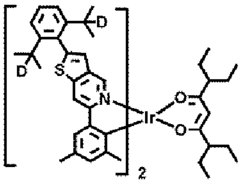
437

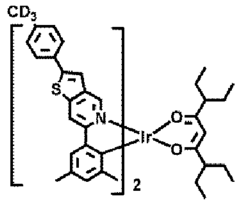
438

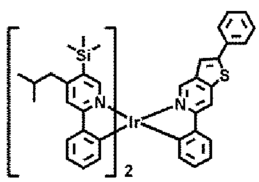
440

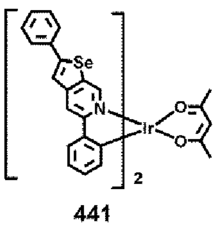
441

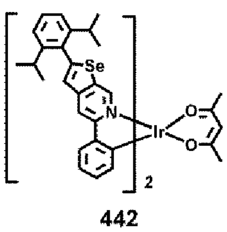
442

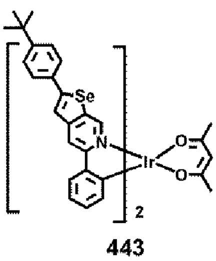
443

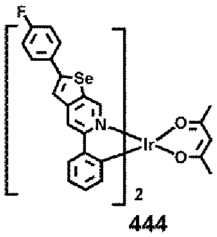
444

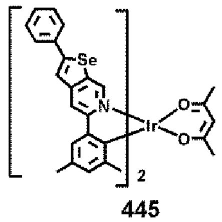
445

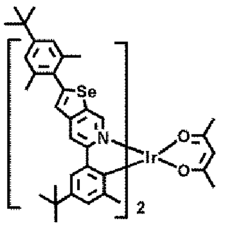
446
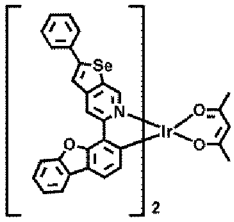
447
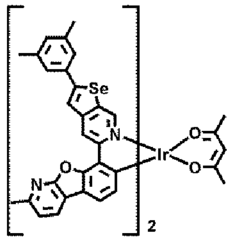
448
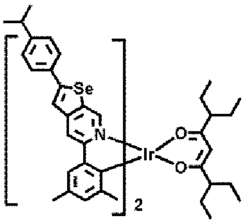
449
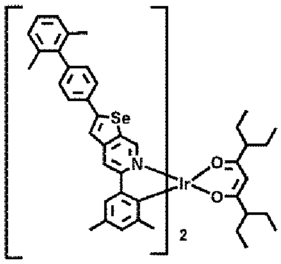
450

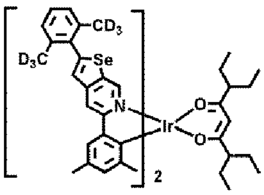
451
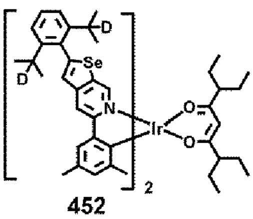
452
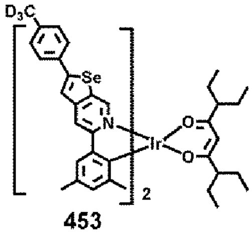
453
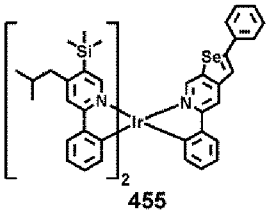
455

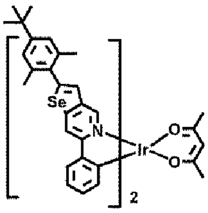
456
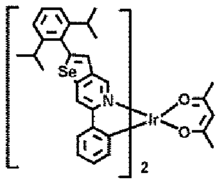
457
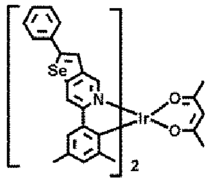
458
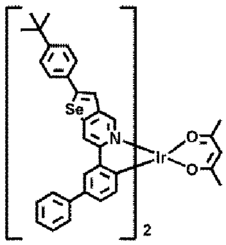
459
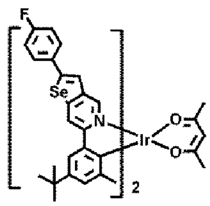
460

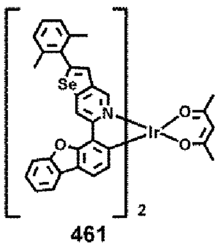
461
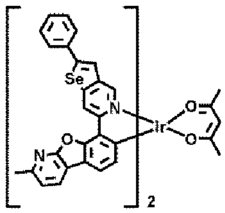
462
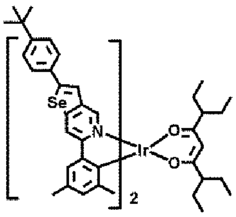
463
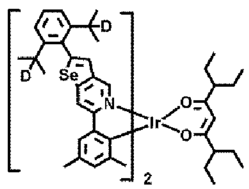
464
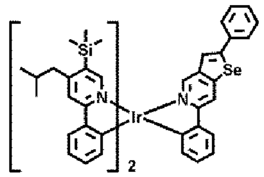
465

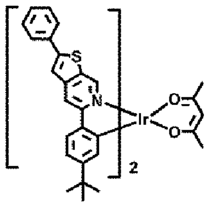
466
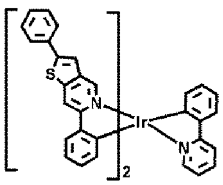
468
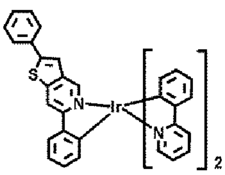
469

**12.** An organic light-emitting device comprising:

a first electrode;
a second electrode; and
an organic layer disposed between the first electrode and the second electrode and comprising an emission layer, wherein the organic layer comprises at least one organometallic compound of any of claims 1-11.

**13.** The organic light-emitting device of claim 12, wherein

the first electrode is an anode,

the second electrode is a cathode,
the organic layer further comprises a hole transport region between the first electrode and the emission layer and an electron transport region between the emission layer and the second electrode,
the hole transport region comprises a hole injection layer, a hole transport layer, an electron blocking layer, a buffer layer, or any combination thereof, and
the electron transport region comprises a hole blocking layer, an electron transport layer, an electron injection layer, or any combination thereof; and/or
wherein the emission layer comprises the organometallic compound, preferably wherein the emission layer further comprises a host, and an amount of the host is larger than an amount of the organometallic compound.

**14.** A diagnostic composition comprising the organometallic compound of any of claims 1-11.

**Patentansprüche**

**1.** Organometallische Verbindung, dargestellt durch Formel 1:

Formel 1
$M(L_1)_{n1}(L_2)_{n2}$,
wobei, in Formel 1,
$L_1$ ein durch Formel 2 dargestellter Ligand ist,
n1 1, 2 oder 3 ist, wobei, wenn n1 2 oder mehr ist, zwei oder mehr $L_1$ identisch zueinander oder unterschiedlich voneinander sind,
$L_2$ ein durch Formel 3 dargestellter zweizähniger Ligand ist,
n2 1, 2, 3 oder 4 ist, wobei, wenn n2 2 oder mehr ist, zwei oder mehr $L_2$ identisch oder unterschiedlich voneinander sind, und
$L_1$ und $L_2$ unterschiedlich voneinander sind:

M Ir oder Os ist und die Summe von n1 und n2 3 oder 4 ist; oder M Pt ist und die Summe von n1 und n2 2 ist,

Formel 2

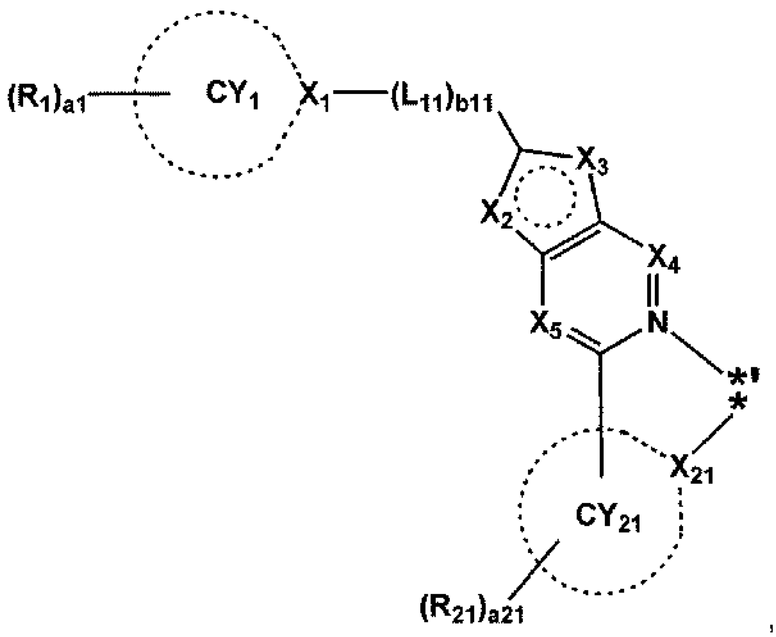

,

wobei, in Formel 2,
$X_1$ C, N, Si oder P ist,
$X_{21}$ C oder N ist,
Ring $CY_1$ und Ring $CY_{21}$ j eweils unabhängig eine carbocyclische $C_5$-$C_{30}$-Gruppe oder eine heterocyclische $C_2$-$C_{30}$-Gruppe sind,

$X_2$ und $X_3$ jeweils unabhängig O, S, Se oder $C(R_2)$ sind, wobei $X_2$ oder $X_3$ O, S oder Se ist,
$X_4$ N oder $C(R_4)$ ist,
$X_5$ N oder $C(R_5)$ ist,
$R_1$, $R_2$, $R_4$, $R_5$ und $R_{21}$ jeweils unabhängig Wasserstoff, Deuterium, - F, -Cl, -Br, -I, $-SF_5$, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäuregruppe oder ein Salz davon, eine Sulfonsäuregruppe oder ein Salz davon, eine Phosphorsäuregruppe oder ein Salz davon, eine substituierte oder unsubstituierte $C_1$-$C_{60}$-Alkylgruppe, eine substituierte oder unsubstituierte $C_2$-$C_{60}$-Alkenylgruppe, eine substituierte oder unsubstituierte $C_2$-$C_{60}$-Alkinylgruppe, eine substituierte oder unsubstituierte $C_1$-$C_{60}$-Alkoxygruppe, eine substituierte oder unsubstituierte $C_3$-$C_{10}$-Cycloalkylgruppe, eine substituierte oder unsubstituierte $C_2$-$C_{10}$-Heterocycloalkylgruppe, eine substituierte oder unsubstituierte $C_3$-$C_{10}$-Cycloalkenylgruppe, eine substituierte oder unsubstituierte $C_2$-$C_{10}$-Heterocycloalkenylgruppe, eine substituierte oder unsubstituierte $C_6$-$C_{60}$-Arylgruppe, eine substituierte oder unsubstituierte $C_6$-$C_{60}$-Aryloxygruppe, eine substituierte oder unsubstituierte $C_6$-$C_{60}$Arylthiogruppe, eine substituierte oder unsubstituierte $C_1$-$C_{60}$-Heteroarylgruppe, eine substituierte oder unsubstituierte monovalente nichtaromatische kondensierte polycyclische Gruppe, eine substituierte oder unsubstituierte monovalente nichtaromatische kondensierte heteropolycyclische Gruppe, $-N(Q_1)(Q_2)$, - $Si(Q_3)(Q_4)(Q_5)$, $-B(Q_6)(Q_7)$, $-P(=O)(Q_8)(Q_9)$ oder $-P(Q_8)(Q_9)$ sind,
a1 und a21 jeweils unabhängig eine ganze Zahl von 0 bis 20 sind,
Ring $CY_1$ und $R_2$ nicht miteinander verknüpft sind und $R_1$ und $R_2$ nicht miteinander verknüpft sind,
$L_{11}$ eine carbocyclische $C_5$-$C_{30}$-Gruppe ist, die unsubstituiert oder mit mindestens einer $R_{10a}$ substituiert ist, oder eine heterocyclische $C_2$-$C_{30}$-Gruppe, die unsubstituiert oder mit mindestens einer $R_{10a}$ substituiert ist,
b11 eine ganze Zahl von 0 bis 10 ist, wobei, wenn b11 0 ist, eine durch $*-(L_{11})_{b11}-*'$ dargestellte Gruppe eine Einfachbindung ist, und wenn b11 2 oder mehr ist, zwei oder mehr $L_{11}$ identisch zueinander oder unterschiedlich voneinander sind,
zwei einer Vielzahl von benachbarten $R_{21}$ optional miteinander verknüpft sind, um eine carbocyclische $C_5$-$C_{30}$-Gruppe, die unsubstituiert oder mit mindestens einer $R_{10a}$ substituiert ist, oder eine heterocyclische $C_2$-$C_{30}$-Gruppe, die unsubstituiert oder mit mindestens einer $R_{10a}$ substituiert ist, zu bilden,
$R_{10a}$ die gleiche wie in Verbindung mit $R_{21}$ definiert ist,
* und *' jeweils eine Bindungsstelle an M in Formel 1 angeben,
ein Substituent der substituierten carbocyclischen $C_5$-$C_{30}$-Gruppe, der substituierten heterocyclischen $C_2$-$C_{30}$-Gruppe, der substituierten $C_1$-$C_{60}$-Alkylgruppe, der substituierten $C_2$-$C_{60}$-Alkenylgruppe, der substituierten $C_2$-$C_{60}$-Alkinylgruppe, der substituierten $C_1$-$C_{60}$-Alkoxygruppe, der substituierten $C_3$-$C_{10}$-Cycloalkylgruppe, der substituierten $C_2$-$C_{10}$-Heterocycloalkylgruppe, der substituierten $C_3$-$C_{10}$-Cycloalkenylgruppe, der substituierten $C_2$-$C_{10}$-Heterocycloalkenylgruppe, der substituierten $C_6$-$C_{60}$-Arylgruppe, der substituierten $C_6$-$C_{60}$-Aryloxygruppe, der substituierten $C_6$-$C_{60}$-Arylthiogruppe, der substituierten $C_1$-$C_{60}$-Heteroarylgruppe, der substituierten monovalenten nichtaromatischen kondensierten polycyclischen Gruppe und der substituierten monovalenten nichtaromatischen kondensierten heteropolycyclischen Gruppe ist:

Deuterium, -F, -Cl, -Br, -I, $-CD_3$, $-CD_2H$, $-CDH_2$, $-CF_3$, $-CF_2H$, $-CFH_2$, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäuregruppe oder ein Salz davon, eine Sulfonsäuregruppe oder ein Salz davon, eine Phosphorsäuregruppe oder ein Salz davon, eine $C_1$-$C_{60}$-Alkylgruppe, eine $C_2$-$C_{60}$-Alkenylgruppe, eine $C_2$-$C_{60}$-Alkynylgruppe oder eine $C_1$-$C_{60}$-Alkoxygruppe;
eine $C_1$-$C_{60}$-Alkylgruppe, eine $C_2$-$C_{60}$-Alkenylgruppe, eine $C_2$-$C_{60}$-Alkinylgruppe oder eine $C_1$-$C_{60}$-Alkoxygruppe, jeweils substituiert mit Deuterium, -F, -Cl, -Br, -I, $-CD_3$, $-CD_2H$, - $CDH_2$, $-CF_3$, $-CF_2H$, $-CFH_2$, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäuregruppe oder ein Salz davon, eine Sulfonsäuregruppe oder ein Salz davon, eine Phosphorsäuregruppe oder ein Salz davon, eine $C_3$-$C_{10}$-Cycloalkylgruppe, eine $C_2$-$C_{10}$-Heterocycloalkylgruppe, eine $C_3$-$C_{10}$-Cycloalkenylgruppe, eine $C_2$-$C_{10}$-Heterocycloalkenylgruppe, eine $C_6$-$C_{60}$-Arylgruppe, eine $C_6$-$C_{60}$-Aryloxygruppe, eine $C_6$-$C_{60}$-Arylthiogruppe, eine $C_1$-$C_{60}$-Heteroarylgruppe, eine monovalente nichtaromatische kondensierte polycyclische Gruppe, eine monovalente nichtaromatische kondensierte heteropolycyclische Gruppe, $-N(Q_{11})(Q_{12})$, $-Si(Q_{13})(Q_{14})(Q_{15})$, $-B(Q_{16})(Q_{17})$, $-P(=O)(Q_{18})(Q_{19})$ oder eine beliebige Kombination davon;
eine $C_3$-$C_{10}$-Cycloalkylgruppe, eine $C_2$-$C_{10}$-Heterocycloalkylgruppe, eine $C_3$-$C_{10}$-Cycloalkenylgruppe, eine $C_2$-$C_{10}$-Heterocycloalkenylgruppe, eine $C_6$-$C_{60}$-Arylgruppe, eine $C_6$-$C_{60}$-Aryloxygruppe, eine $C_6$-$C_{60}$-Arylthiogruppe, eine $C_1$-$C_{60}$-Heteroarylgruppe, eine monovalente nichtaromatische kondensierte polycyclische Gruppe

und eine monovalente nichtaromatische kondensierte heteropolycyclische Gruppe;
eine $C_3$-$C_{10}$-Cycloalkylgruppe, eine $C_2$-$C_{10}$-Heterocycloalkylgruppe, eine $C_3$-$C_{10}$-Cycloalkenylgruppe, eine $C_2$-$C_{10}$-Heterocycloalkenylgruppe, eine $C_6$-$C_{60}$-Arylgruppe, eine $C_6$-$C_{60}$-Aryloxygruppe, eine $C_6$-$C_{60}$-Arylthiogruppe, eine $C_1$-$C_{60}$-Heteroarylgruppe, eine monovalente nichtaromatische kondensierte polycyclische Gruppe oder eine monovalente nichtaromatische kondensierte heteropolycyclische Gruppe, jeweils substituiert mit Deuterium, - F, -Cl, -Br, -I, $-CD_3$, $-CD_2H$, $-CDH_2$, $-CF_3$, $-CF_2H$, $-CFH_2$, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäuregruppe oder ein Salz davon, eine Sulfonsäuregruppe oder ein Salz davon, eine Phosphorsäuregruppe oder ein Salz davon, eine $C_1$-$C_{60}$-Alkylgruppe, eine $C_2$-$C_{60}$-Alkenylgruppe, eine $C_2$-$C_{60}$-Alkinylgruppe, eine $C_1$-$C_{60}$-Alkoxygruppe, eine $C_3$-$C_{10}$-Cycloalkylgruppe, eine $C_2$-$C_{10}$-Heterocycloalkylgruppe, eine $C_3$-$C_{10}$-Cycloalkenylgruppe, eine $C_2$-$C_{10}$-Heterocycloalkenylgruppe, eine $C_6$-$C_{60}$-Arylgruppe, eine $C_6$-$C_{60}$-Aryloxygruppe, eine $C_6$-$C_{60}$-Arylthiogruppe, eine $C_1$-$C_{60}$-Heteroarylgruppe, eine monovalente nichtaromatische kondensierte polycyclische Gruppe, eine monovalente nichtaromatische kondensierte heteropolycyclische Gruppe, $-N(Q_{21})(Q_{22})$, $-Si(Q_{23})(Q_{24})(Q_{25})$, $-B(Q_{26})(Q_{27})$, $-P(=O)(Q_{28})(Q_{29})$ oder eine beliebige Kombination davon;
$-N(Q_{31})(Q_{32})$, $-Si(Q_{33})(Q_{34})(Q_{35})$, $-B(Q_{36})(Q_{37})$ oder $-P(=O)(Q_{38})(Q_{39})$; oder
eine beliebige Kombination davon, und
$Q_1$ bis $Q_9$, $Q_{11}$ bis $Q_{19}$, $Q_{21}$ bis $Q_{29}$ und $Q_{31}$ bis $Q_{39}$ jeweils unabhängig Wasserstoff, Deuterium, -F, -Cl, -Br, -I, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäuregruppe oder ein Salz davon, eine Sulfonsäuregruppe oder ein Salz davon, eine Phosphorsäuregruppe oder ein Salz davon, eine $C_1$-$C_{60}$-Alkylgruppe, eine $C_2$-$C_{60}$-Alkenylgruppe, eine $C_2$-$C_{60}$-Alkinylgruppe, eine $C_1$-$C_{60}$-Alkoxygruppe, eine $C_3$-$C_{10}$-Cycloalkylgruppe, eine $C_2$-$C_{10}$-Heterocycloalkylgruppe, eine $C_3$-$C_{10}$-Cycloalkenylgruppe, eine $C_2$-$C_{10}$-Heterocycloalkenylgruppe, eine $C_6$-$C_{60}$-Arylgruppe, eine $C_6$-$C_{60}$-Arylgruppe substituiert mit einer $C_1$-$C_{60}$-Alkylgruppe, eine $C_6$-$C_{60}$-Arylgruppe oder eine beliebige Kombination davon, eine $C_6$-$C_{60}$-Aryloxygruppe, eine $C_6$-$C_{60}$-Arylthiogruppe, eine $C_1$-$C_{60}$-Heteroarylgruppe, eine monovalente nichtaromatische kondensierte polycyclische Gruppe und eine monovalente nichtaromatische kondensierte heteropolycyclische Gruppe sind;

Formel 3

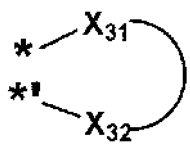
,

wobei, in Formel 3,
$X_{31}$ und $X_{32}$ jeweils O sind;
$X_{31}$ O ist und $X_{32}$ N ist; oder
$X_{31}$ N ist und $X_{32}$ C ist,

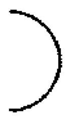

eine beliebige Atomgruppe angibt, die $X_{31}$ und $X_{32}$ miteinander verknüpft, und
* und *' jeweils eine Bindungsstelle an M in Formel 1 angeben; und
wobei die organometallische Verbindung nicht die folgende Verbindung ist:

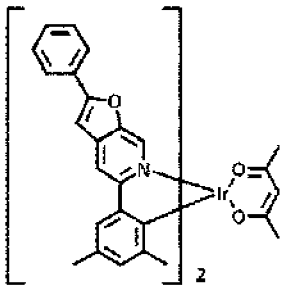

2. Organometallische Verbindung nach Anspruch 1, wobei Ring $CY_1$ und Ring $CY_{21}$ jeweils unabhängig eine Cyclopentengruppe, eine Cyclohexengruppe, eine Benzolgruppe, eine Naphthalengruppe, eine Anthracengruppe, eine Phenanthrengruppe, eine Triphenylengruppe, eine Pyrengruppe, eine Chrysengruppe, eine Cyclopentadiengruppe, eine 1,2,3,4-Tetrahydronaphthalengruppe, eine Thiophengruppe, eine Furangruppe, eine Indolgruppe, eine Benzoborolgruppe, eine Benzophospholgruppe, eine Indengruppe, eine Benzosilolgruppe, eine Benzogermolgruppe, eine Benzothiophengruppe, eine Benzoselenophengruppe, eine Benzofurangruppe, eine Carbazolgruppe, eine Dibenzoborolgruppe, eine Dibenzophospholgruppe, eine Fluorengruppe, eine Dibenzosilolgruppe, eine Dibenzogermolgruppe, eine Dibenzothiophengruppe, eine Dibenzoselenophengruppe, eine Dibenzofurangruppe, eine Dibenzothiophen-5-oxid-Gruppe, eine 9H-Fluoren-9-on-Gruppe, eine Dibenzothiophen-5,5-dioxid-Gruppe, eine Azaindolgruppe, eine Azabenzoborolgruppe, eine Azabenzophospholgruppe, eine Azaindengruppe, eine Azabenzosilolgruppe, eine Azabenzogermolgruppe, eine Azabenzothiophengruppe, eine Azabenzoselenophengruppe, eine Azabenzofurangruppe, eine Azacarbazolgruppe, eine Azadibenzoborolgruppe, eine Azadibenzophospholgruppe, eine Azafluorengruppe, eine Azadibenzosilolgruppe, eine Azadibenzogermolgruppe, eine Azadibenzothiophengruppe, eine Azadibenzoselenophengruppe, eine Azadibenzofurangruppe, eine Azadibenzothiophen-5-oxid-Gruppe, eine Aza-9H-fluoren-9-on-Gruppe, eine Azadibenzothiophen-5,5-dioxid-Gruppe, eine Pyridingruppe, eine Pyrimidingruppe, eine Pyrazingruppe, eine Pyridazingruppe, eine Triazingruppe, eine Chinolingruppe, eine Isochinolingruppe, eine Chinoxalingruppe, eine Chinazolingruppe, eine Phenanthrolingruppe, eine Pyrrolgruppe, eine Pyrazolgruppe, eine Imidazolgruppe, eine Triazolgruppe, eine Oxazolgruppe, eine Isoxazolgruppe, eine Thiazolgruppe, eine Isothiazolgruppe, eine Oxadiazolgruppe, eine Thiadiazolgruppe, eine Benzopyrazolgruppe, eine Benzimidazolgruppe, eine Benzoxazolgruppe, eine Benzothiazolgruppe, eine Benzoxadiazolgruppe, eine Benzothiadiazolgruppe, eine 5,6,7,8-Tetrahydroisochinolingruppe oder eine 5,6,7,8-Tetrahydrochinolingruppe sind.

3. Organometallische Verbindung nach Ansprüchen 1 oder 2, wobei $R_1$, $R_2$, $R_4$, $R_5$, $R_{21}$ und $R_{10a}$ jeweils unabhängig sind:

Wasserstoff, Deuterium, -F, -Cl, -Br, -I, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäuregruppe oder ein Salz davon, eine Sulfonsäuregruppe oder ein Salz davon, eine Phosphorsäuregruppe oder ein Salz davon, $-SF_5$, eine $C_1$-$C_{20}$-Alkylgruppe oder eine $C_1$-$C_{20}$-Alkoxygruppe;

eine $C_1$-$C_{20}$-Alkylgruppe oder eine $C_1$-$C_{20}$-Alkoxygruppe, jeweils substituiert mit Deuterium, -F, -Cl, -Br, -I, $-CD_3$, $-CD_2H$, $-CDH_2$, $-CF_3$, $-CF_2H$, - $CFH_2$, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäuregruppe oder ein Salz davon, eine Sulfonsäuregruppe oder ein Salz davon, eine Phosphorsäuregruppe oder ein Salz davon, eine $C_1$-$C_{10}$-Alkylgruppe, eine Cyclopentylgruppe, eine Cyclohexylgruppe, eine Cycloheptylgruppe, eine Cycloctylgruppe, eine Adamantanylgruppe, eine Norbomanylgruppe, eine Norbornenylgruppe, eine Cyclopentenylgruppe, eine Cyclohexenylgruppe, eine Cycloheptenylgruppe, eine Bicyclo[1.1.1]pentylgruppe, eine Bicyclo[2.1.1]hexylgruppe, eine Bicyclo[2.2.1]heptylgruppe, eine Bicyclo[2.2.2]octylgruppe, eine Phenylgruppe, eine Biphenylgruppe, eine Terphenylgruppe, eine Naphthylgruppe, eine Pyridinylgruppe, eine Pyrimidinylgruppe oder eine beliebige Kombination davon;

eine Cyclopentylgruppe, eine Cyclohexylgruppe, eine Cycloheptylgruppe, eine Cyclooctylgruppe, eine Adamantanylgruppe, eine Norbornenylgruppe, eine Cyclopentenylgruppe, eine Cyclohexenylgruppe, eine Cycloheptenylgruppe, eine Cyclooctenylgruppe, eine Bicyclo[1.1.1]pentylgruppe, eine Bicyclo[2.1.1]hexylgruppe, eine Bicyclo[2.2.1]heptylgruppe, eine Bicyclo[2.2.2]octylgruppe, eine Phenylgruppe,eine Biphenylgruppe, eine Terphenylgruppe, eine Naphthylgruppe, eine Fluorenylgruppe, eine Phenanthrenylgruppe, eine Anthracenylgruppe, eine Fluoranthenylgruppe, eine Triphenylenylgruppe, eine Pyrenylgruppe, eine Chrysenylgruppe, eine Pyrrolylgruppe, eine Thiophenylgruppe, eine Furanylgruppe, eine Imidazolylgruppe, Pyrazolylgruppe, eine Thiazolylgruppe, eine Isothiazolylgruppe, eine Oxazolylgruppe, eine Isoxazolylgruppe, eine Pyridinylgruppe, eine Pyrazinylgruppe, eine Pyrimidinylgruppe, eine Pyridazinylgruppe, eine Isoindolylgruppe, eine Indolylgruppe, eine Indazolylgruppe, eine Purinylgruppe, eine Chinolinylgruppe, eine Isochinolinylgruppe, eine Benzochinolinylgruppe, eine Chinoxalinylgruppe, eine Chinazolinylgruppe, eine Cinnolinylgruppe, eine Carbazolylgruppe, eine Phenanthrolinylgruppe, eine Benzoimidazolylgruppe, eine Benzofuranylgruppe, eine Benzothiophenylgruppe, eine Isobenzothiazolylgruppe, eine Benzoxazolylgruppe, eine Isobenzoxazolylgruppe, eine Triazolylgruppe, eine Tetrazolylgruppe, eine Oxadiazolylgruppe, eine Triazinylgruppe, eine Dibenzofuranylgruppe, eine Dibenzothiophenylgruppe, eine Benzocarbazolylgruppe, eine Dibenzocarbazolylgruppe, eine Imidazopyridinylgruppe oder eine Imidazopyrimidinylgruppe, jeweils unsubstituiert oder substituiert mit Deuterium, -F, -Cl, -Br, -I, $-CD_3$, $-CD_2H$, $-CDH_2$, $-CF_3$, $-CF_2H$, $-CFH_2$, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäureguppe oder ein Salz davon, eine Sulfonsäuregruppe oder ein Salz davon, eine Phosphorsäuregruppe oder ein Salz davon, eine $C_1$-$C_{20}$-Alylgruppe, eine $C_1$-$C_{20}$-Alkoxygruppe, eine Cyclopentylgruppe, eine Cyclohexylgruppe, eine Cycloheptylgruppe, eine Cycloctylgruppe, eine Adamantanylgruppe, eine Norbornenylgruppe, eine Cyclopentenylgruppe, eine Cyclohexenylgruppe, eine Cycloheptenylgruppe, eine Cyclooctenylgruppe, eine Bicyclo[1.1.1]pentylgruppe, eine Bicyclo[2.1.1]hexylgruppe, eine Bicyclo[2.2.1]heptylgruppe, eine Bicyclo[2.2.2]octylgruppe, eine Phenylgruppe, eine Biphenylgruppe, eine Terphenylgruppe, eine Naphthylgruppe, eine Fluorenylgruppe, eine Phenanthrenylgruppe, eine Anthracenylgruppe, eine Fluoranthenylgruppe, eine Triphenylenylgruppe, eine Pyrenylgruppe, eine Chrysenylgruppe, eine Pyrrolylgruppe, eine Thiophenylgruppe, eine Furanylgruppe, eine Imidazolylgruppe, eine Pyrazolylgruppe, eine Thiazolylgruppe, eine Isothiazolylgruppe, eine Oxazolylgruppe, eine Isoxazolylgruppe, eine Pyridinylgruppe, eine Pyrazinylgruppe, eine Pyrimidinylgruppe, eine Pyridazinylgruppe, eine Isoindolylgruppe, eine Indolylgruppe, eine Indazolylgruppe, eine Purinylgruppe, eine Chinolinylgruppe, eine Isochinolinylgruppe, eine Benzochinolinylgruppe, eine Chinoxalinylgruppe, eine Chinazolinylgruppe, eine Cinnolinylgruppe, eine Carbazolylgruppe, eine Phenanthrolinylgruppe, eine Benzimidazolylgruppe, eine Benzofuranylgruppe, eine Benzothiophenylgruppe, eine Isobenzothiazolylgruppe, eine Benzoxazolylgruppe, eine Isobenzoxazolylgruppe, eine Triazolylgruppe, eine Tetrazolylgruppe, eine Oxadiazolylgruppe, eine Triazinylgruppe, eine Dibenzofuranylgruppe, eine Dibenzothiophenylgruppe, eine Benzocarbazolylgruppe, eine Dibenzocarbazolylgruppe, eine Imidazopyridinylgruppe und eine Imidazopyrimidinylgruppe, $-Si(Q_{33})(Q_{34})(Q_{35})$ oder eine beliebige Kombination davon; oder
$-N(Q_1)(Q_2)$, $-Si(Q_3)(Q_4)(Q_5)$, $-B(Q_6)(Q_7)$, $-P(=O)(Q_8)(Q_9)$ oder $-P(Q_9)(Q_8)$, und
$Q_1$ bis $Q_9$ und $Q_{33}$ bis $Q_{35}$ jeweils unabhängig sind:
$-CH_3$, $-CD_3$, $-CD_2H$, $-CDH_2$, $-CH_2CH_3$, $-CH_2CD_3$, $-CH_2CD_2H$, $-CH_2CDH_2$, $-CHDCH_3$, $-CHDCD_2H$, $-CHDCDH_2$, $-CHDCD_3$, $-CD_2CD_3$, $-CD_2CD_2H$ oder $-CD_2CDH_2$; oder
eine n-Propylgruppe, eine Isopropylgruppe, eine n-Butylgruppe, eine Isobutylgruppe, eine sec-Butylgruppe, eine tert-Butylgruppe, eine n-Pentylgruppe, eine Isopentylgruppe, eine sec-Pentylgruppe, eine tert-Pentylgruppe, eine Phenylgruppe oder eine Naphthylgruppe, jeweils unsubstituiert oder substituiert mit Deuterium, eine $C_1$-$C_{10}$-Alkylgruppe, einer Phenylgruppe oder eine beliebige Kombination davon.

**4.** Organometallische Verbindung nach einem der Ansprüche 1-3, wobei

eine Gruppe, dargestellt durch

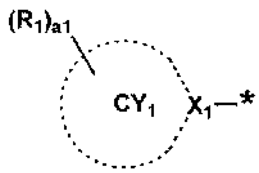

in Formel 2, eine $C_3$-$C_{10}$-Cycloalkenylgruppe, eine $C_2$-$C_{10}$-Heterocycloalkenylgruppe, eine $C_6$-$C_{30}$-Arylgruppe, eine $C_2$-$C_{30}$-Heteroarylgruppe, eine monovalente nichtaromatische kondensierte polycyclische Gruppe oder eine monovalente nichtaromatische kondensierte heteropolycyclische Gruppe, jeweils unsubstituiert oder substituiert mit $R_1$ in der Zahl von a1 ist,
in Formel 2, $R_1$ und $R_2$ jeweils unabhängig sind:

Wasserstoff, Deuterium, -F, -Cl, -Br, -I, eine Cyanogruppe oder $-SF_5$, oder
eine $C_1$-$C_{60}$-Alkylgruppe, eine $C_1$-$C_{60}$-Alkoxygruppe, eine $C_3$-$C_{10}$-Cycloalkylgruppe, eine $C_2$-$C_{10}$-Heterocycloalkylgruppe, eine $C_3$-$C_{10}$-Cycloalkenylgruppe oder eine $C_2$-$C_{10}$-Heterocycloalkenylgruppe, jeweils un-

substituiert oder substituiert mit Deuterium, -F, -Cl, -Br, -I, $-CD_3$, $-CD_2H$, $-CDH_2$, $-CF_3$, $-CF_2H$, $-CFH_2$, eine Cyanogruppe, eine $C_1$-$C_{20}$-Alkylgruppe, eine Cyclopentylgruppe, eine Cyclohexylgruppe, eine Cycloheptylgruppe, eine Cycloctylgruppe, eine Adamantylgruppe, eine Norbornylgruppe, eine Norbornenylgruppe, eine Cyclopentenylgruppe, eine Cyclohexenylgruppe, eine Cycloheptenylgruppe, eine Phenylgruppe, eine Biphenylgruppe, eine Naphthylgruppe, eine Pyridinylgruppe, eine Pyrimidinylgruppe oder eine beliebige Kombination davon, und

a1 eine ganze Zahl von 0 bis 10 ist.

**5.** Organometallische Verbindung nach einem der Ansprüche 1-4, wobei eine Gruppe, dargestellt durch

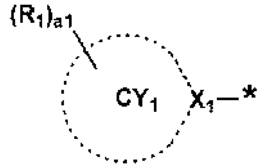

in Formel 2, eine Phenylgruppe, eine Biphenylgruppe, eine Naphthylgruppe, eine Fluorenylgruppe, eine Phenanthrenylgruppe, eine Anthracenylgruppe, eine Fluoranthenylgruppe, eine Triphenylenylgruppe, eine Pyrenylgruppe, eine Chrysenylgruppe, eine Pyrrolylgruppe, eine Thiophenylgruppe, eine Furanylgruppe, eine Imidazolylgruppe, eine Pyrazolylgruppe, eine Thiazolylgruppe, eine Isothiazolylgruppe, eine Oxazolylgruppe, eine Isoxazolylgruppe, eine Pyridinylgruppe, eine Pyrazinylgruppe, eine Pyrimidinylgruppe, eine Pyridazinylgruppe, eine Isoindolylgruppe, eine Indolylgruppe, eine Indazolylgruppe, eine Purinylgruppe, eine Chinolinylgruppe, eine Isochinolinylgruppe, eine Benzochinolinylgruppe, eine Chinoxalinylgruppe, eine Chinazolinylgruppe, eine Cinnolinylgruppe, eine Carbazolylgruppe, eine Phenanthrolinylgruppe, eine Benzoimidazolylgruppe, eine Benzofuranylgruppe, eine Benzothiophenylgruppe, eine Isobenzothiazolylgruppe, eine Benzoxazolylgruppe, eine Isobenzoxazolylgruppe, eine Triazolylgruppe, eine Tetrazolylgruppe, eine Oxadiazolylgruppe, eine Triazinylgruppe, eine Dibenzofuranylgruppe, eine Dibenzothiophenylgruppe, eine Benzocarbazolylgruppe, eine Dibenzocarbazolylgruppe, eine Imidazopyridinylgruppe oder eine Imidazopyrimidinylgruppe, jeweils unsubstituiert oder substituiert mit $R_1$ in der Zahl von a1, ist,

in Formel 2, $R_1$ und $R_2$ jeweils unabhängig sind:

Wasserstoff, Deuterium, -F, -Cl, -Br, -I, eine Cyanogruppe oder $-SF_5$, oder

eine Methylgruppe, eine Ethylgruppe, eine Propylgruppe, eine n-Butylgruppe, eine Isobutylgruppe, eine sec-Butylgruppe, eine tert-Butylgruppe, eine n-Pentylgruppe, eine Isopentylgruppe, eine sec-Pentylgruppe, eine tert-Pentylgruppe, eine n-Hexylgruppe, eine Isohexylgruppe, eine sec-Hexylgruppe, eine tert-Hexylgruppe, eine n-Heptylgruppe, eine Isoheptylgruppe, eine sec-Heptylgruppe, eine tert-Heptylgruppe, eine n-Octylgruppe, eine Isooctylgruppe, eine sec-Octylgruppe, eine tert-Octylgruppe, eine n-Nonanylgruppe, eine Isononanylgruppe, eine sec-Nonanylgruppe, eine tert-Nonanylgruppe, eine n-Decanylgruppe, eine Isodecanylgruppe, eine sec-Decanylgruppe, eine tert-Decanylgruppe, eine $C_1$-$C_{10}$-Alkoxy-, eine Cyclopentylgruppe, eine Cyclohexylgruppe, eine Cycloheptylgruppe, eine Cyclooctylgruppe, eine Adamantanylgruppe, eine Norbornylgruppe, eine Norbornenylgruppe, eine Cyclopentenylgruppe, eine Cyclohexenylgruppe oder eine Cycloheptenylgruppe, jeweils unsubstituiert oder substituiert mit Deuterium, -F, -Cl, -Br, -I, $-CD_3$, $-CD_2H$, $-CDH_2$, $-CF_3$, $-CF_2H$, $-CFH_2$, eine Cyanogruppe, eine $C_1$-$C_{20}$ -Alkylgruppe, eine Cyclopentylgruppe, eine Cyclohexylgruppe, eine Cycloheptylgruppe, eine Cyclooctylgruppe, eine Adamantanylgruppe, eine Norbornylgruppe, eine Norbornenylgruppe, eine Cyclopentenylgruppe, eine Cyclohexenylgruppe, eine Cycloheptenylgruppe, eine Phenylgruppe, eine Biphenylgruppe, eine Naphthylgruppe, eine Pyridinylgruppe, eine Pyrimidinylgruppe oder eine beliebige Kombination davon, und

a1 eine ganze Zahl von 0 bis 5 ist.

**6.** Organometallische Verbindung nach einem der Ansprüche 1-5, wobei

eine Gruppe, dargestellt durch

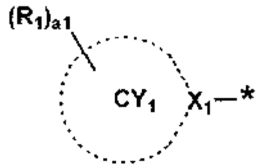

,

eine Gruppe ist, die durch eine der Formeln 10-13(1) bis 10-13(18) und 10-13 dargestellt ist:

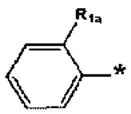

10-13(1)

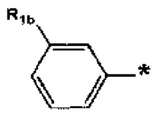

10-13(2)

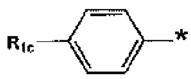

10-13(3)

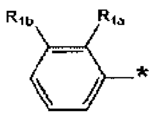

10-13(4)

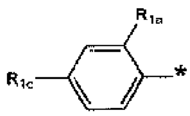

10-13(5)

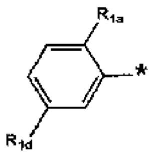

10-13(6)

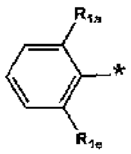

10-13(7)

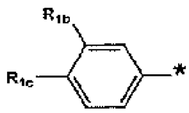

10-13(8)

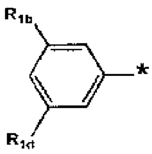

10-13(9)

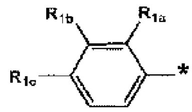

10-13(10)

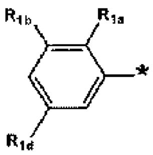

10-13(11)

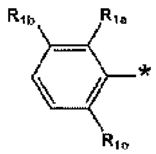

10-13(12)

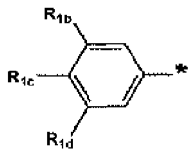

10-13(13)

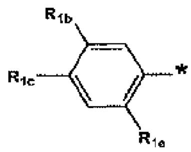

10-13(14)

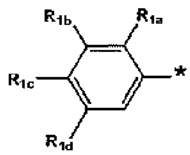

10-13(15)

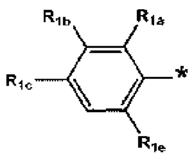

10-13(16)

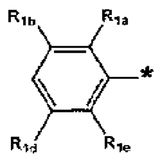

10-13(17)

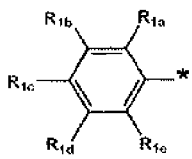

10-13(18)

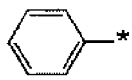

10-13

,

wobei, in Formeln 10-13(1) bis 10-13(18) und 10-13, $R_{1a}$ bis $R_{1e}$ jeweils unabhängig gleich sind, wie in Verbindung mit $R_1$ in Anspruch 1 definiert, wobei $R_{1a}$ bis $R_{1e}$ jeweils nicht Wasserstoff sind und * eine Bindungsstelle an ein Nachbaratom angibt.

**7.** Organometallische Verbindung nach einem der Ansprüche 1-6, wobei eine Gruppe, dargestellt durch

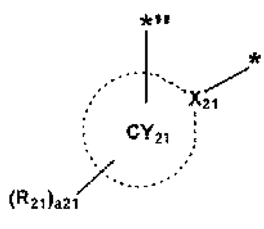

in Formel 2, eine Gruppe ist, die durch eine von Formeln eine von CY21-1 bis CY21-25 dargestellt ist:

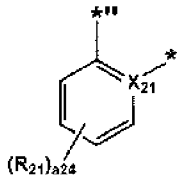
CY21-1

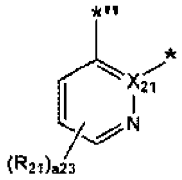
CY21-2

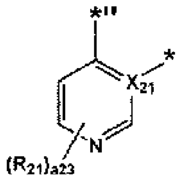
CY21-3

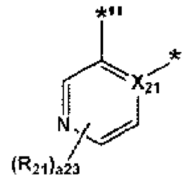
CY21-4

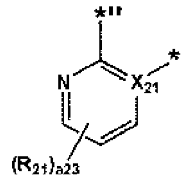
CY21-5

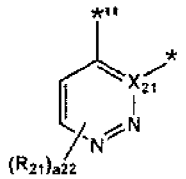
CY21-6

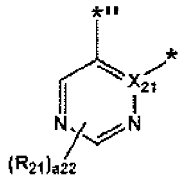
CY21-7

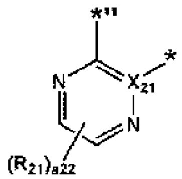
CY21-8

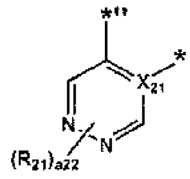
CY21-9

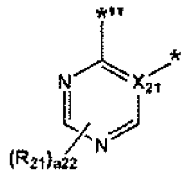
CY21-10

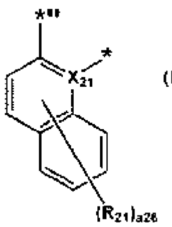
CY21-11

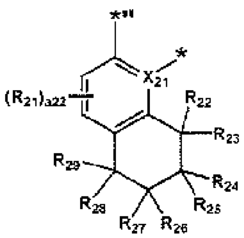
CY21-12

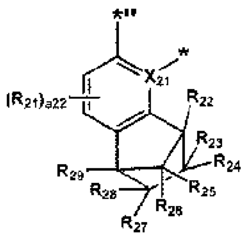
CY21-13

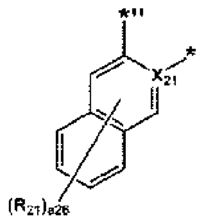
CY21-14

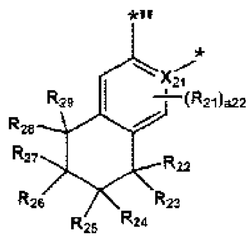
CY21-15

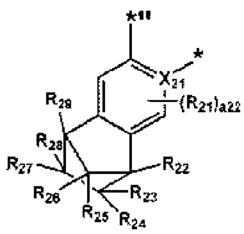
CY21-16

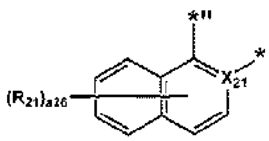
CY21-17

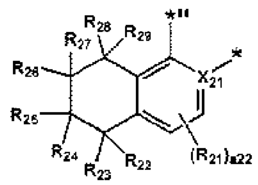
CY21-18

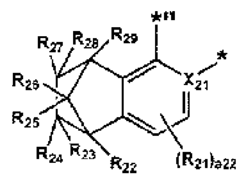
CY21-19

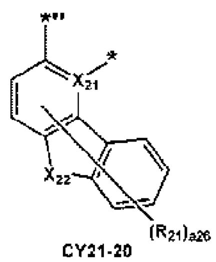
CY21-20

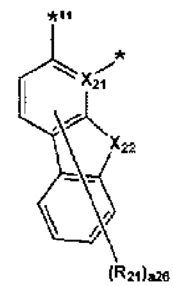
CY21-21

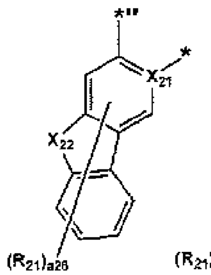
CY21-22

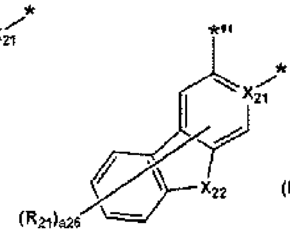
CY21-23

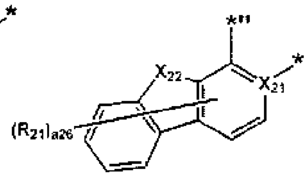
CY21-24

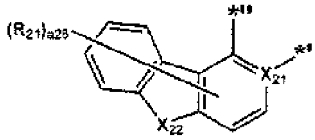
CY21-25

wobei, in Formeln CY21-1 bis CY21-25,

$X_{21}$ und R21 jeweils unabhängig gleich sind, wie in Anspruch 1 beschrieben,
$X_{22}$ $C(R_{22})(R_{23})$, $N(R_{22})$, O, S oder $Si(R_{22})(R_{23})$ ist,

$R_{22}$ bis $R_{29}$ jeweils unabhängig gleich sind, wie in Verbindung mit $R_{21}$ in Anspruch 1 definiert,
a26 eine ganze Zahl von 0 bis 6 ist,
a24 eine ganze Zahl von 0 bis 4 ist,
a23 eine ganze Zahl von 0 bis 3 ist,
a22 eine ganze Zahl von 0 bis 2 ist,
*" eine Bindungsstelle an ein Kohlenstoffatom eines benachbarten 6-gliedrigen Rings in Formel 2 angibt, und
* eine Bindungsstelle an M in Formel 1 angibt;
bevorzugt, wobei
eine Gruppe, dargestellt durch

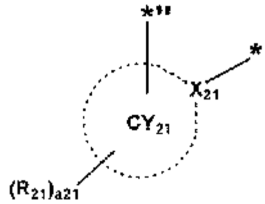

in Formel 2, eine Gruppe ist, die durch eine von Formeln CY21(1) bis CY21(56) dargestellt ist, oder eine Gruppe, die durch eine von Formeln CY21-20 bis CY21-26 dargestellt ist:

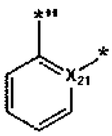
CY21(1)

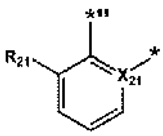
CY21(2)

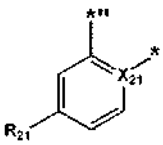
CY21(3)

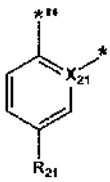
CY21(4)

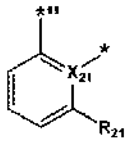
CY21(5)

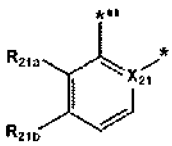
CY21(6)

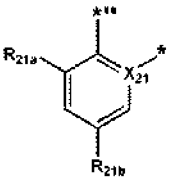
CY21(7)

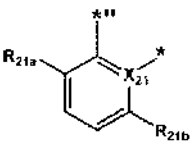
CY21(8)

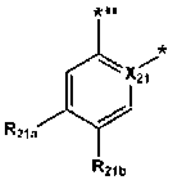
CY21(9)

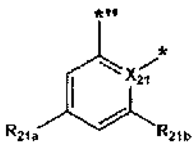
CY21(10)

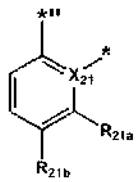
CY21(11)

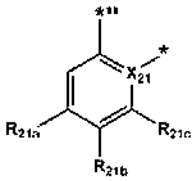
CY21(12)

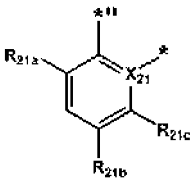
CY21(13)

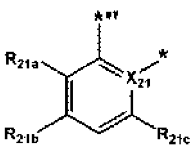
CY21(14)

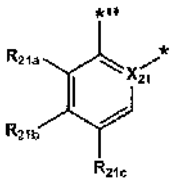
CY21(15)

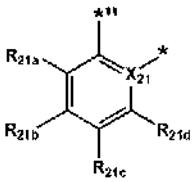
CY21(16)

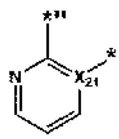
CY21(17)

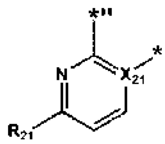
CY21(18)

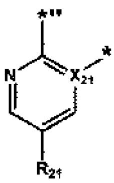
CY21(19)

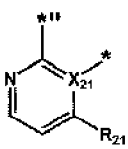
CY21(20)

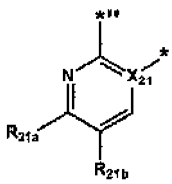
CY21(21)

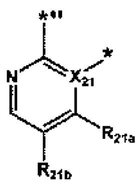
CY21(22)

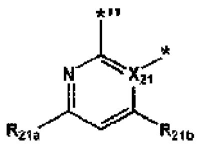
CY21(23)

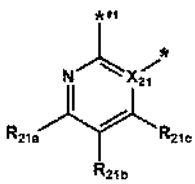
CY21(24)

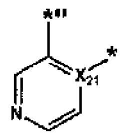
CY21(25)

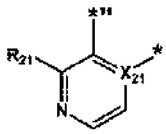
CY21(26)

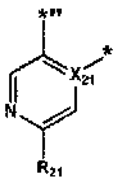
CY21(27)

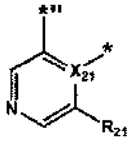
CY21(28)

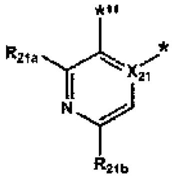
CY21(29)

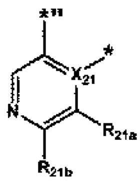
CY21(30)

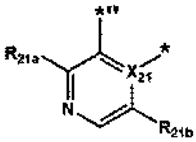
CY21(31)

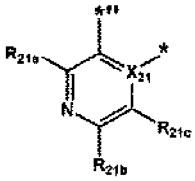
CY21(32)

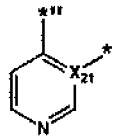
CY21(33)

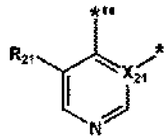
CY21(34)

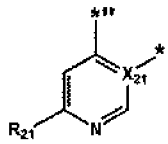
CY21(35)

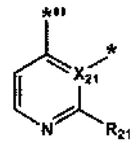
CY21(36)

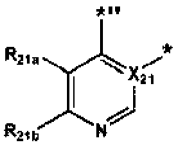
CY21(37)

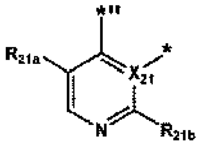
CY21(38)

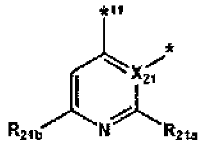
CY21(39)

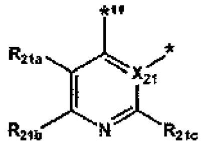
CY21(40)

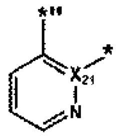
CY21(41)

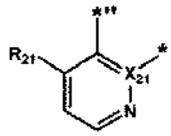
CY21(42)

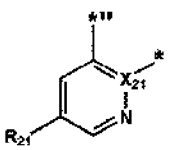
CY21(43)

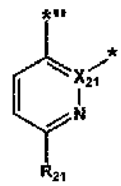
CY21(44)

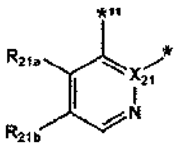
CY21(45)

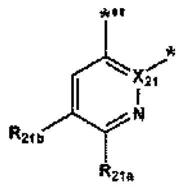
CY21(46)

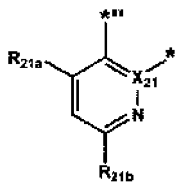
CY21(47)

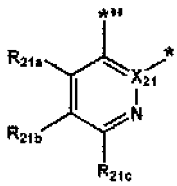
CY21(48)

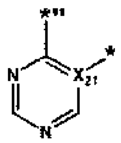
CY21(49)

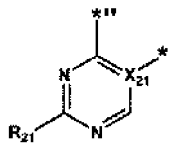
CY21(50)

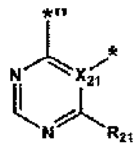
CY21(51)

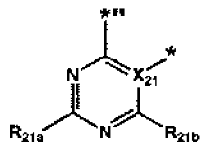
CY21(52)

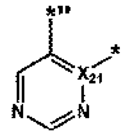
CY21(53)

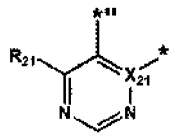
CY21(54)

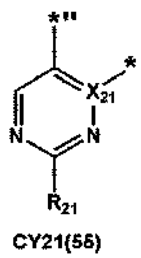
CY21(55)

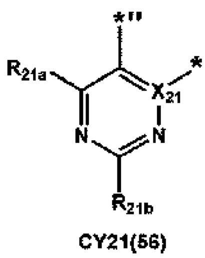
CY21(56)

,

wobei, in Formeln CY21(1) bis CY21(56),
$X_{21}$ und R21 jeweils unabhängig gleich sind, wie in Anspruch 1 beschrieben,
$R_{21a}$ bis $R_{21d}$ jeweils unabhängig gleich sind, wie in Verbindung mit $R_{21}$ in Anspruch 1 definiert, wobei $R_{21}$ und $R_{21d}$ bis $R_{21d}$ jeweils nicht Wasserstoff sind,
*" eine Bindungsstelle an ein Kohlenstoffatom eines benachbarten 6-gliedrigen Rings in Formel 2 angibt, und
* eine Bindungsstelle an M in Formel 1 angibt.

**8.** Organometallische Verbindung nach einem der Ansprüche 1-7, wobei $L_1$ ein durch Formel 2A oder 2B dargestellter Ligand ist:

Formel 2A

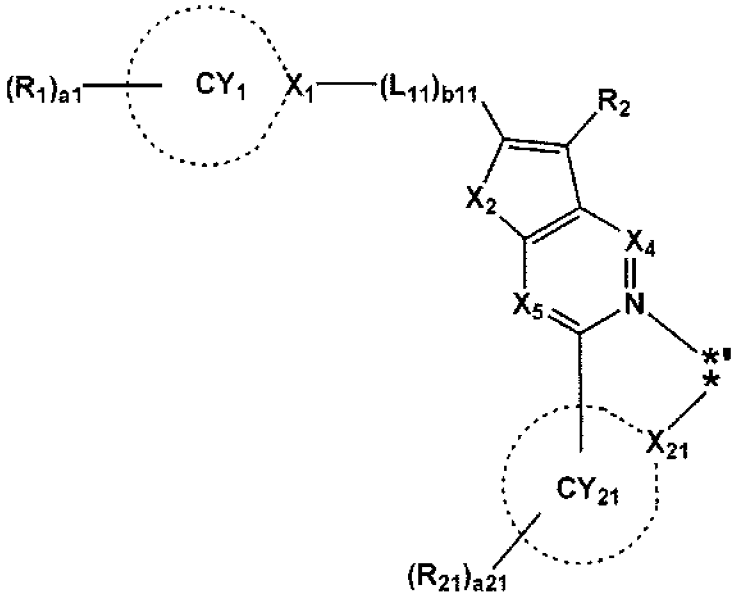

Formel 2B

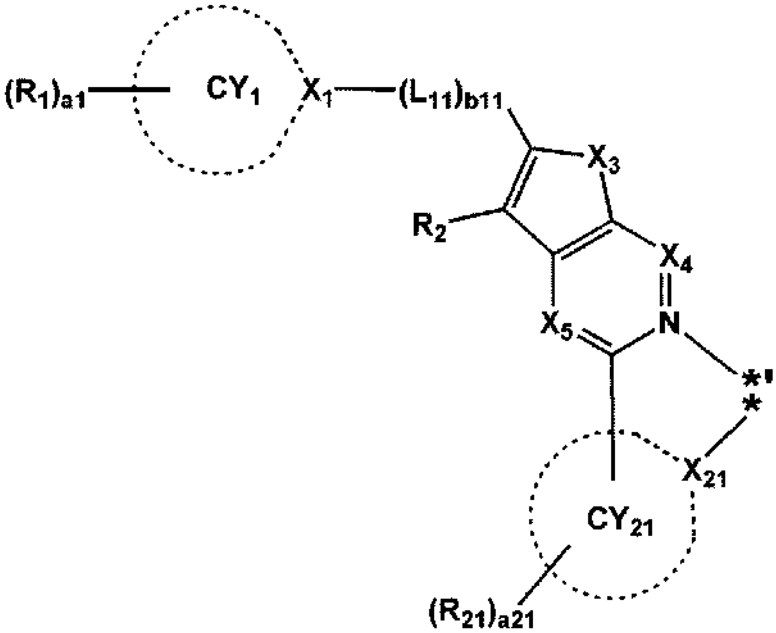

,

wobei, in Formeln 2A und 2B, $X_1$, $X_{21}$, Ring $CY_1$, Ring $CY_{21}$, $X_4$, $X_5$, $R_1$, $R_2$, $R_{21}$, a1, a21, $L_{11}$, b11, *, und *' jeweils unabhängig gleich sind, wie in Anspruch 1 beschrieben, wobei $X_2$ und $X_3$ jeweils unabhängig O, S oder Se sind.

**9.** Organometallische Verbindung nach einem der Ansprüche 1-8, wobei in Formel 1, $L_2$ eine Gruppe, dargestellt durch eine von Formeln 3A bis 3D, ist:

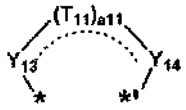

3A

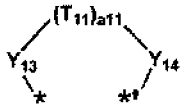

3B

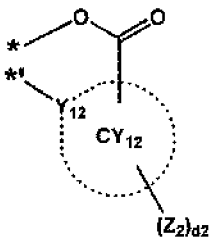

3C

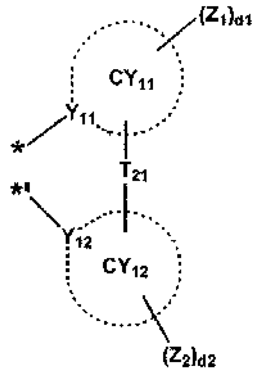

3D

,

wobei, in Formeln 3A bis 3D,

$Y_{13}$ O, N oder $N(Z_1)$ ist,
$Y_{14}$ O, N oder $N(Z_3)$ ist,
$T_{11}$ eine Einfachbindung, eine Doppelbindung, $*-C(Z_{11})(Z_{12})-*'$, $*-C(Z_{11})=C(Z_{12})-*'$, $*=C(Z_{11})-*'$, $*-C(Z_{11})=*'$, $*=C(Z_{11})-C(Z_{12})=C(Z_{13})-*'$, $*-C(Z_{11})=C(Z_{12})-C(Z_{13})=*'$, $*-N(Z_{11})-*'$ oder eine carbocyclische $C_5$-$C_{30}$-Gruppe, unsubstituiert oder substituiert mit mindestens einer $Z_{11}$, ist,
a11 eine ganze Zahl von 1 bis 10 ist,
$Y_{11}$ und $Y_{12}$ jeweils unabhängig C oder N sind,
$T_{21}$ eine Einfachbindung, eine Doppelbindung, O, S, $C(Z_{11})(Z_{12})$, $Si(Z_{11})(Z_{12})$ oder N(Zn) ist,Ring $CY_{11}$ und Ring $CY_{12}$ jeweils unabhängig eine carbocyclische $C_5$-$C_{30}$-Gruppe oder eine heterocyclische $C_2$-$C_{30}$-Gruppe sind,
$Z_1$ bis $Z_3$ und $Z_{11}$ bis $Z_{13}$ jeweils unabhängig gleich sind, wie in Verbindung mit $R_{21}$ in Anspruch 1 definiert,
d1 und d2 jeweils unabhängig eine ganze Zahl von 0 bis 10 sind, und
* und *' jeweils eine Bindungsstelle an M in Formel 1 angeben.

**10.** Organometallische Verbindung nach einem der Ansprüche 1-9, wobei, in Formel 1, $L_2$ eine Gruppe, dargestellt durch Formeln eine von 3-1(1) bis 3-1(66), 3-1(301) und 3-1(308), ist:

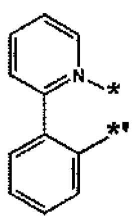

3-1(1)

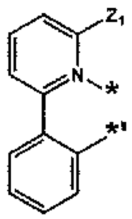

3-1(2)

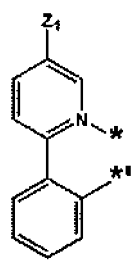

3-1(3)

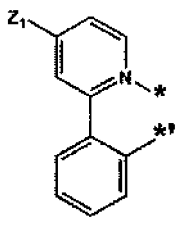

3-1(4)

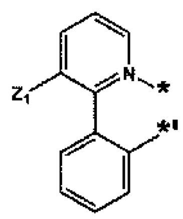

3-1(5)

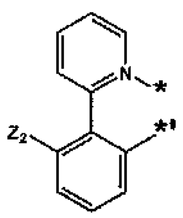

3-1(6)

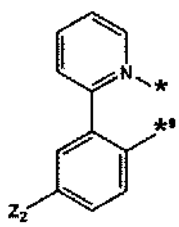

3-1(7)

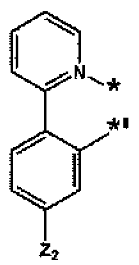

3-1(8)

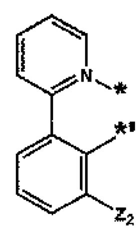

3-1(9)

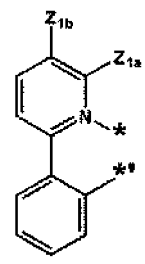

3-1(10)

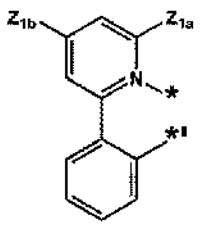
3-1(11)

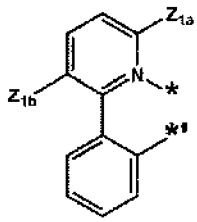
3-1(12)

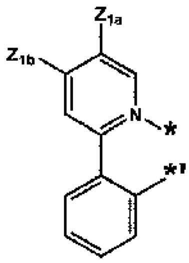
3-1(13)

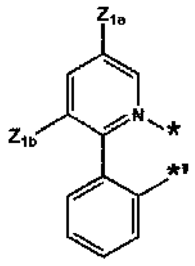
3-1(14)

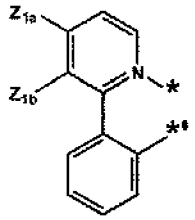
3-1(15)

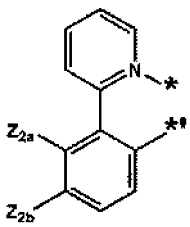
3-1(16)

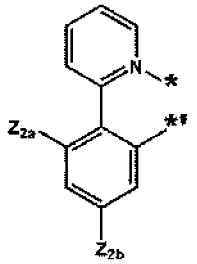
3-1(17)

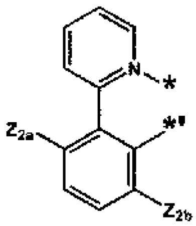
3-1(18)

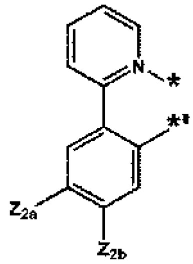
3-1(19)

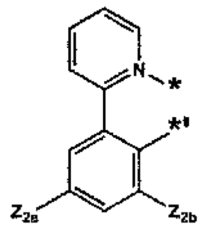
3-1(20)

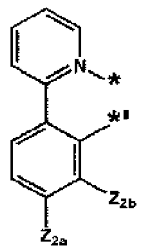
3-1(21)

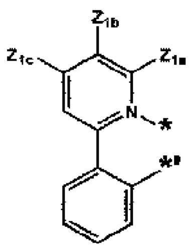
3-1(22)

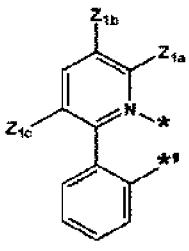
3-1(23)

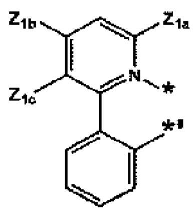
3-1(24)

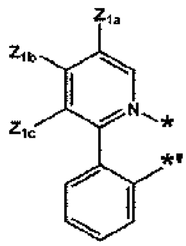
3-1(25)

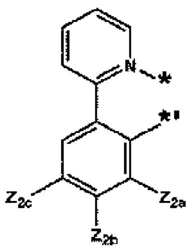
3-1(26)

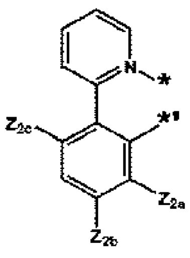
3-1(27)

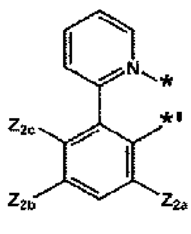
3-1(28)

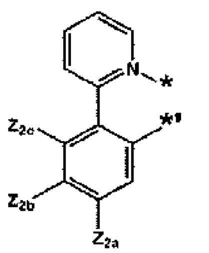
3-1(29)

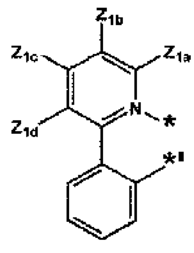
3-1(30)

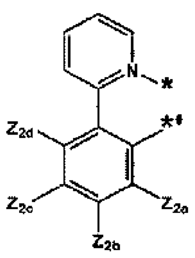
3-1(31)

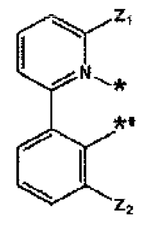
3-1(32)

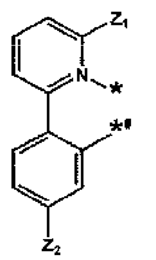
3-1(33)

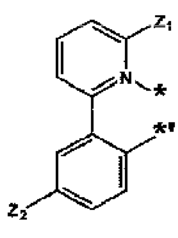
3-1(34)

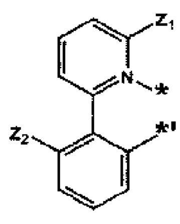
3-1(35)

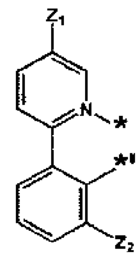
3-1(36)

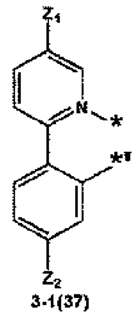
3-1(37)

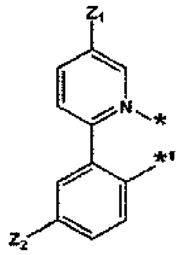
3-1(38)

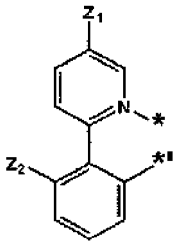
3-1(39)

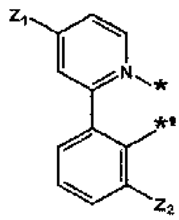
3-1(40)

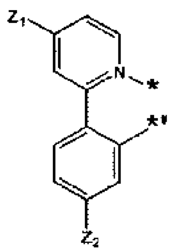
3-1(41)

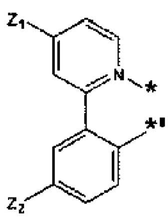
3-1(42)

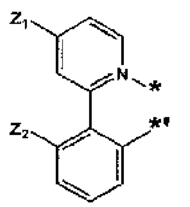
3-1(43)

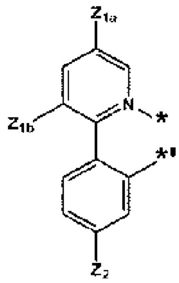
3-1(44)

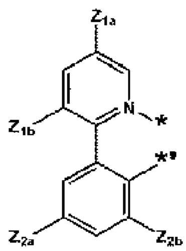
3-1(45)

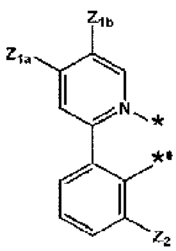
3-1(46)

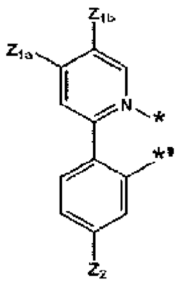
3-1(47)

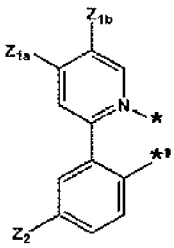
3-1(48)

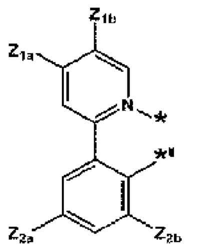
3-1(49)

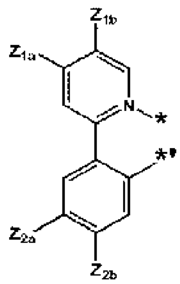
3-1(50)

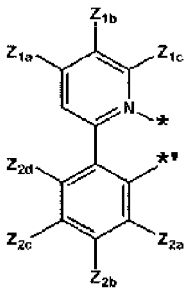
3-1(51)

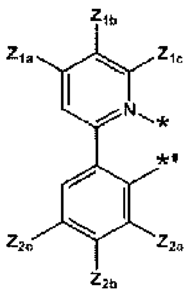
3-1(52)

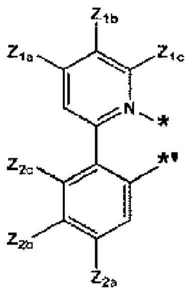
3-1(53)

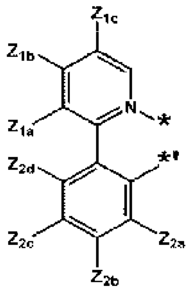
3-1(54)

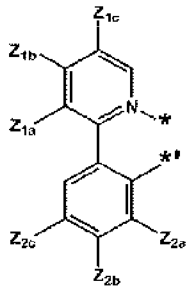
3-1(55)

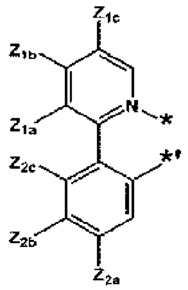
3-1(56)

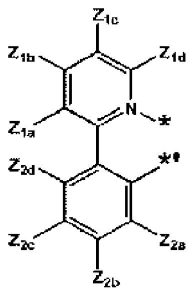
3-1(57)

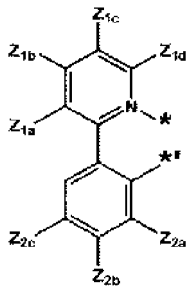
3-1(58)

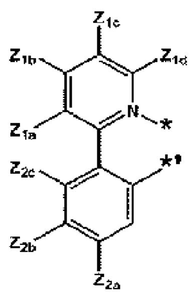
3-1(59)

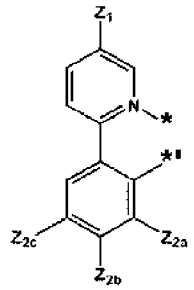
3-1(60)

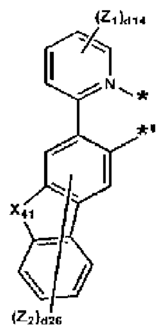
3-1(61)

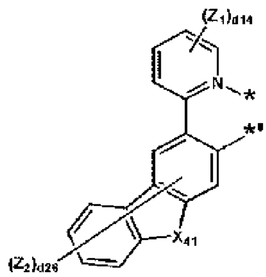
3-1(62)

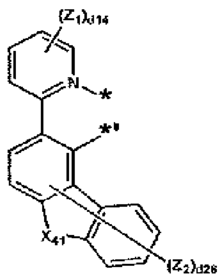
3-1(63)

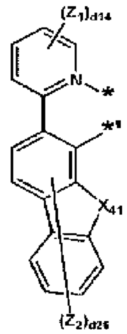
3-1(64)

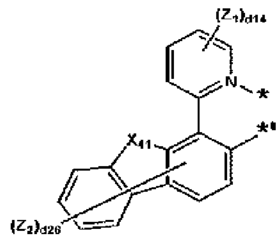
3-1(65)

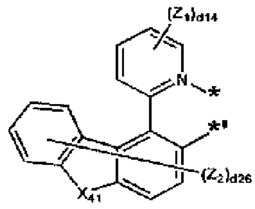
3-1(66)

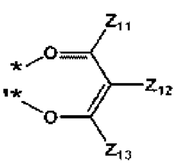
3-1(301)

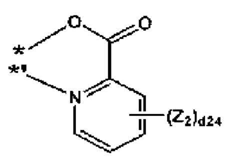
3-1(308)

wobei, in Formeln 3-1(1) bis 3-1(66), 3-1(301) und 3-1(308),

$X_{41}$ O, S, $N(Z_{21})$, $C(Z_{21})(Z_{22})$ oder $Si(Z_{21})(Z_{22})$ ist,
$Z_1$, $Z_2$, $Z_{1a}$, $Z_{1b}$, $Z_{1c}$, $Z_{1d}$, $Z_{2a}$, $Z_{2b}$, $Z_{2c}$, $Z_{2d}$, $Z_{11}$ bis $Z_{13}$, $Z_{21}$ und $Z_{22}$ jeweils unabhängig gleich sind, wie in Verbindung mit $R_{21}$ in Anspruch 1 definiert,
d14 eine ganze Zahl von 0 bis 4 ist,
d26 eine ganze Zahl von 0 bis 6 ist, und
* und *' jeweils eine Bindungsstelle an M in Formel 1 angeben.

**11.** Organometallische Verbindung nach Anspruch 1, wobei die organometallische Verbindung eine von Verbindungen 1 bis 15, 17-218, 220-328, 330-438, 440-453, 455-466, 468 und 469 ist:

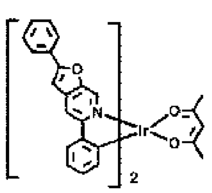
1

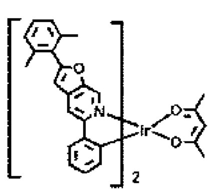
2

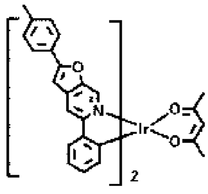
3

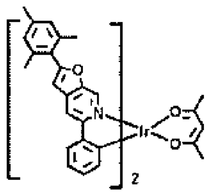
4

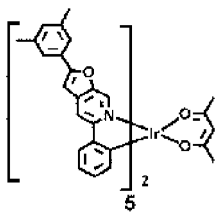
5

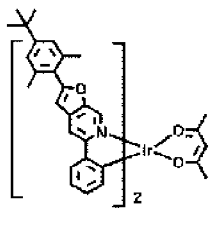
6

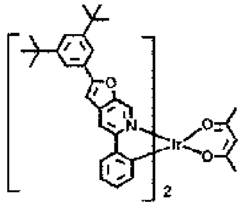
7

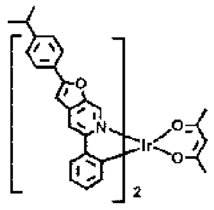
8

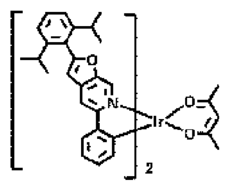
9

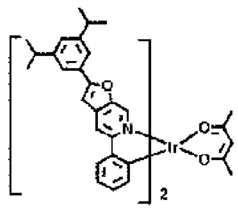
10

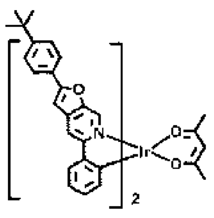
11

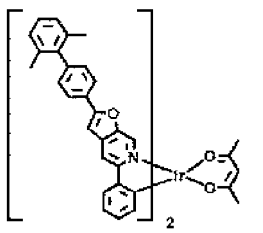
12

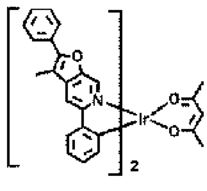
13

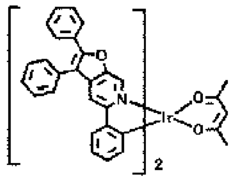
14

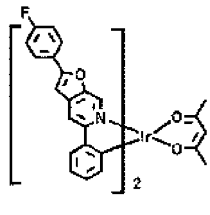
15

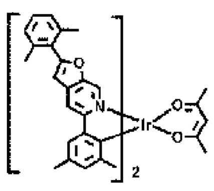
17

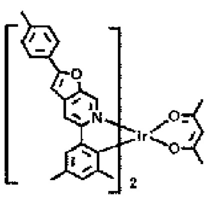
18

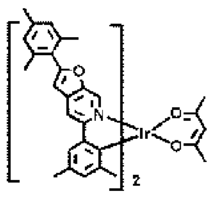
19

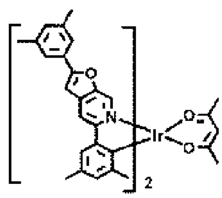
20

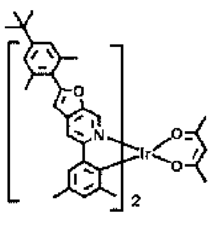
21

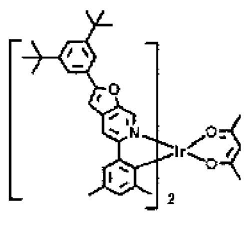
22

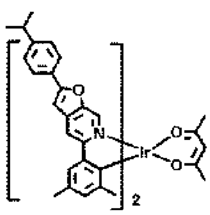
23

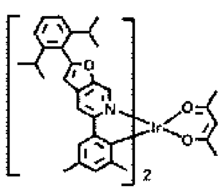
24

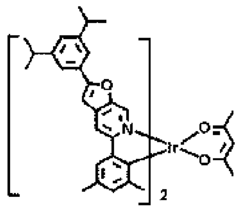
25

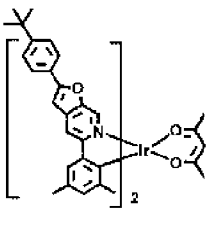 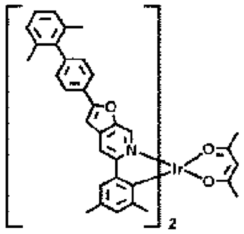 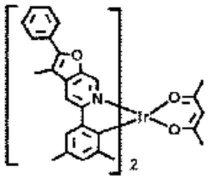 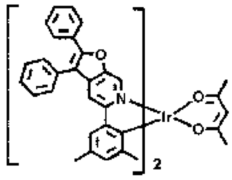 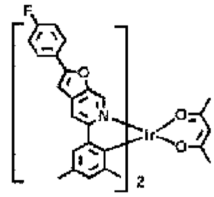

26 27 28 29 30

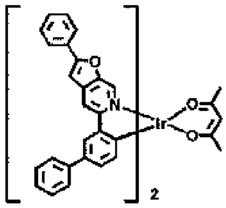 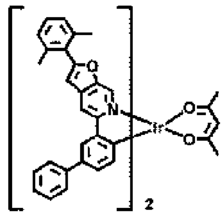 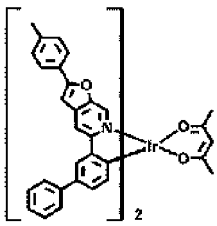 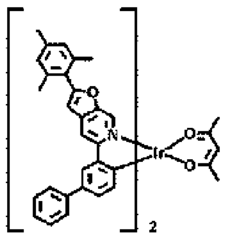 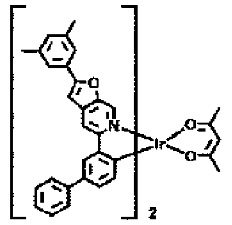

31 32 33 34 35

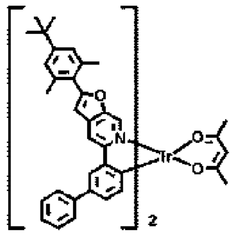 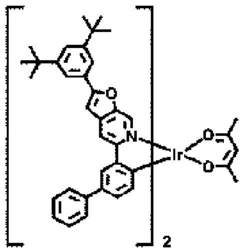 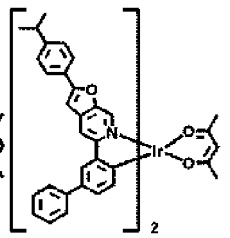 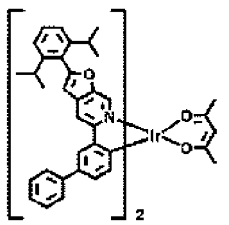 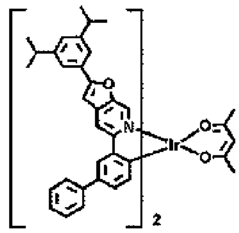

36 37 38 39 40

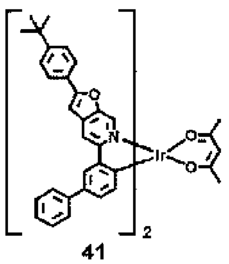 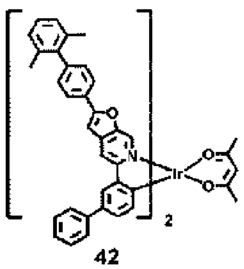 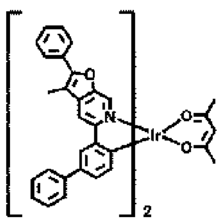 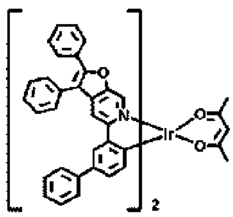 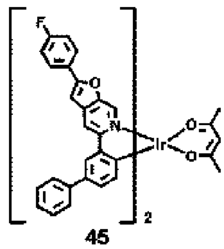

41 42 43 44 45

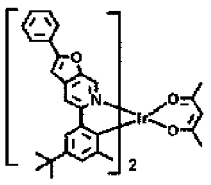 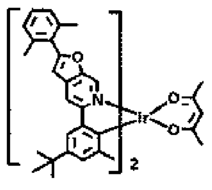 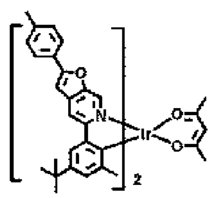 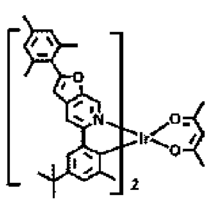 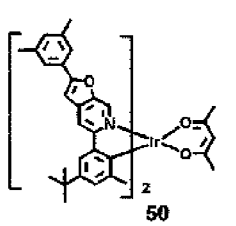

46 47 48 49 50

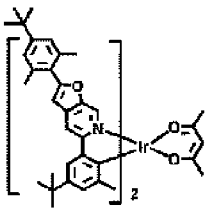
51
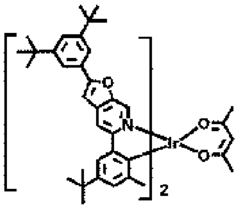
52
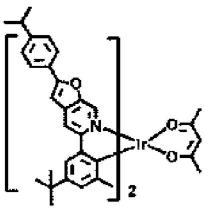
53
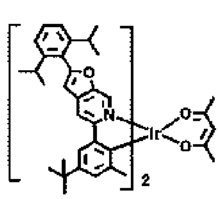
54
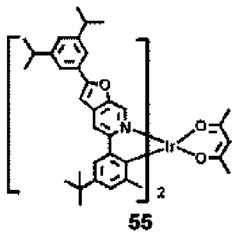
55

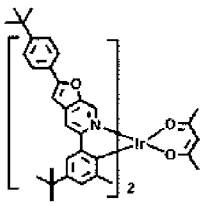
56
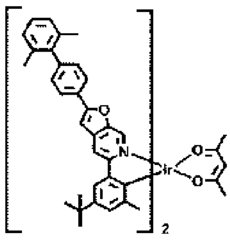
57
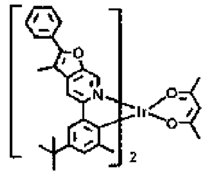
58
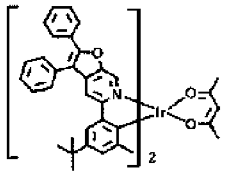
59
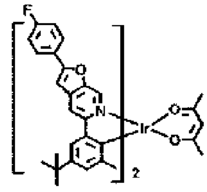
60

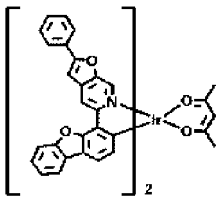
61
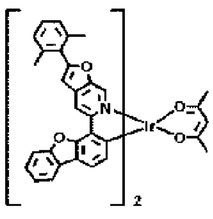
62
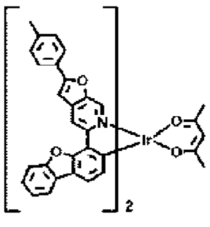
63
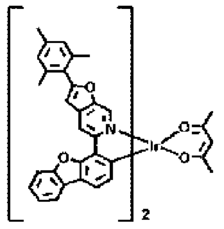
64
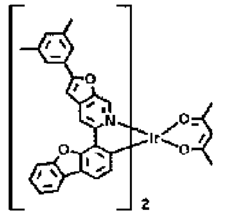
65

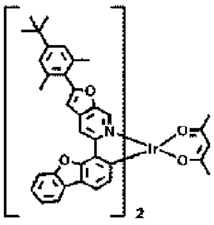
66
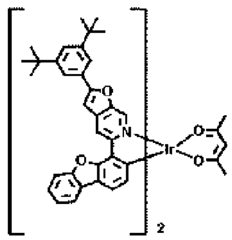
67
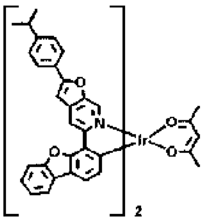
68
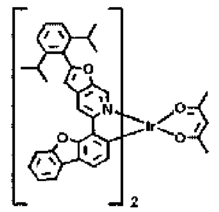
69
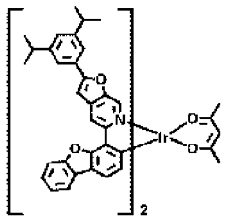
70

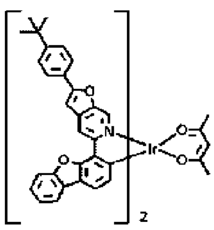
71
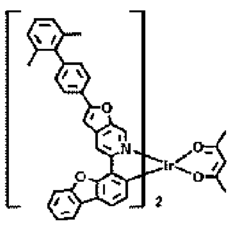
72
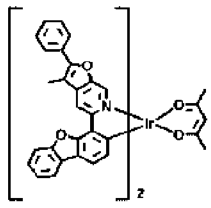
73
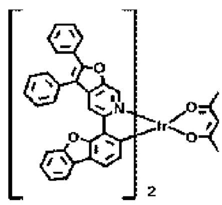
74
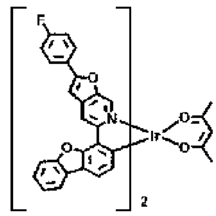
75

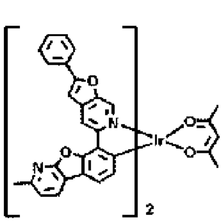
76

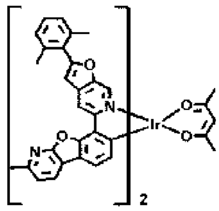
77

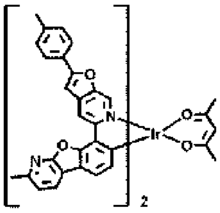
78

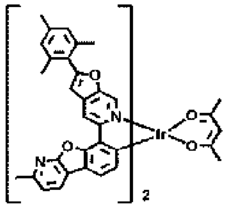
79

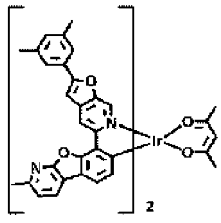
80

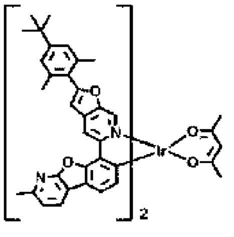
81

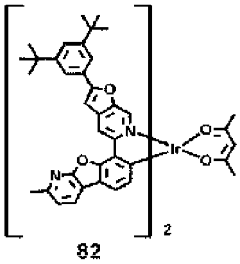
82

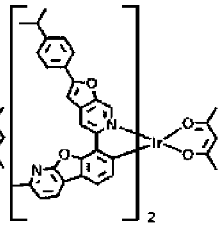
83

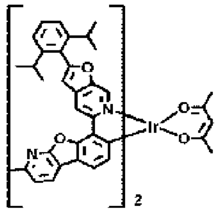
84

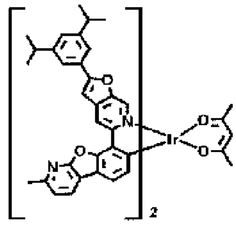
85

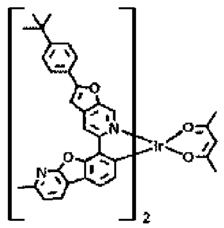
86

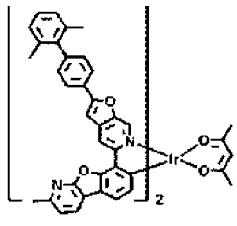
87

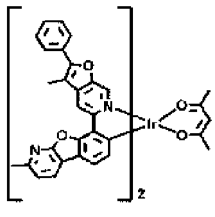
88

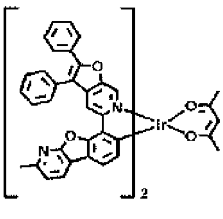
89

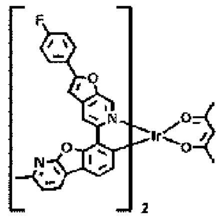
90

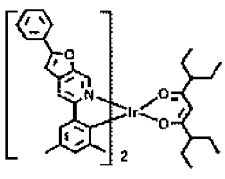
91

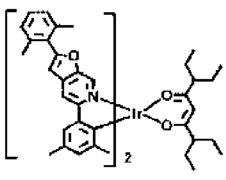
92

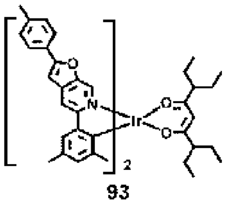
93

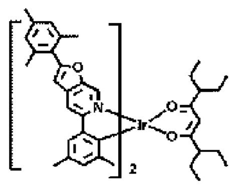
94

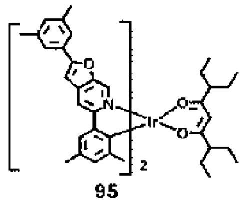
95

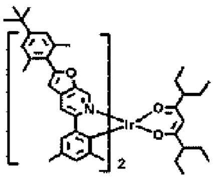
96

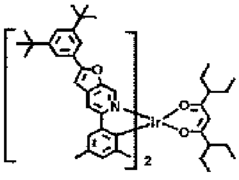
97

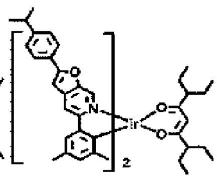
98

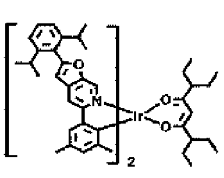
99

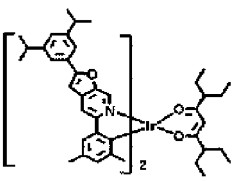
100

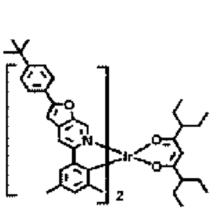
101

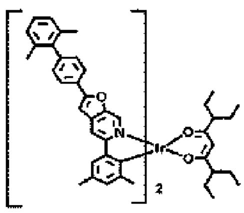
102

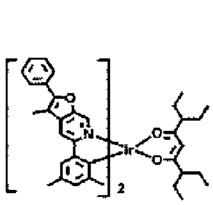
103

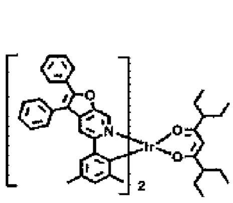
104

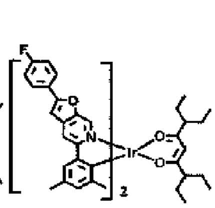
105

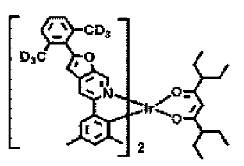
106

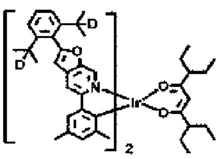
107

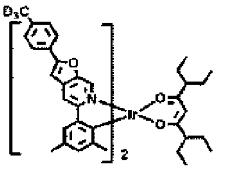
108

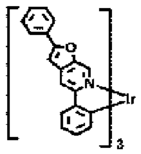
109

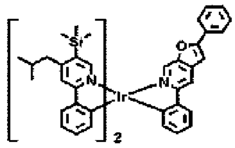
110

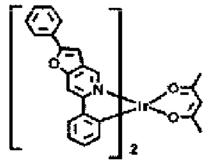
111

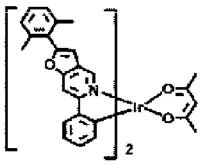
112

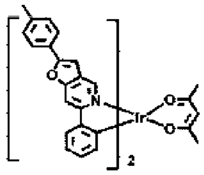
113

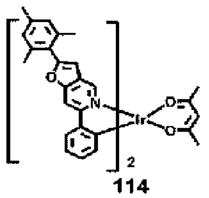
114

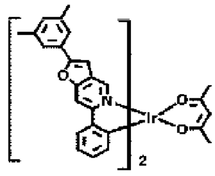
115

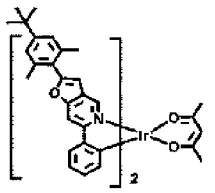
116

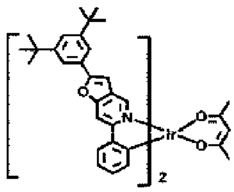
117

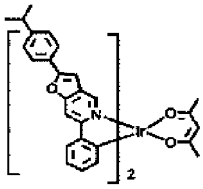
118

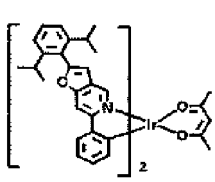
119

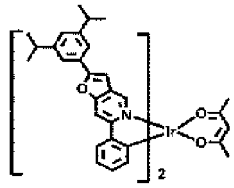
120

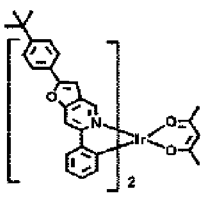
121

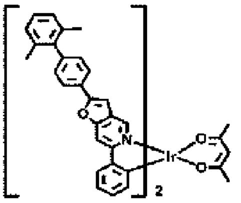
122

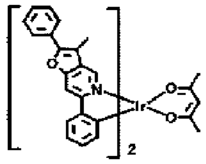
123

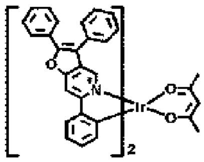
124

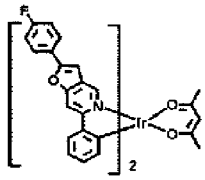
125

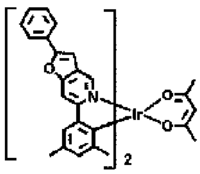
126

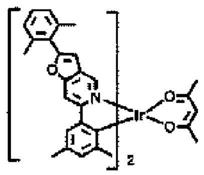
127

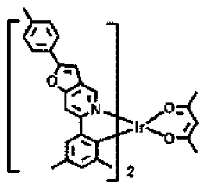
128

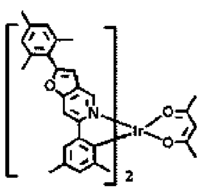
129

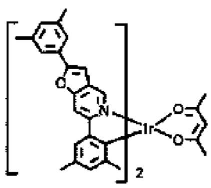
130

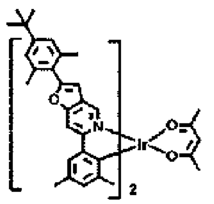
131

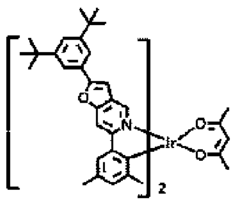
132

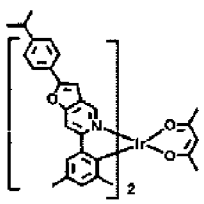
133

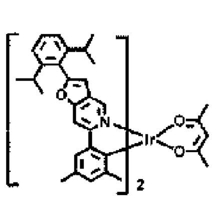
134

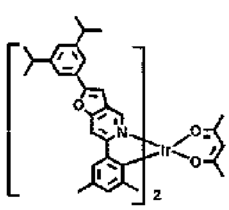
135

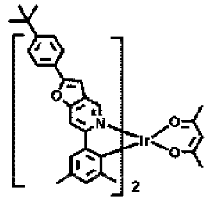
136
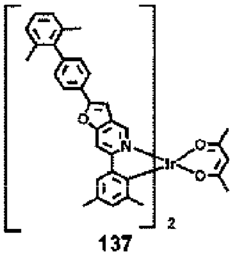
137
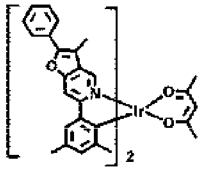
138
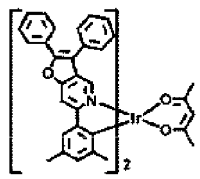
139
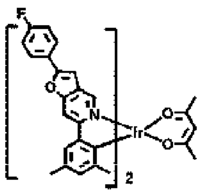
140

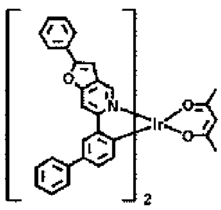
141
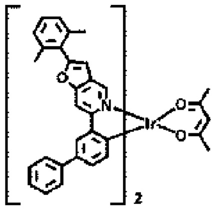
142
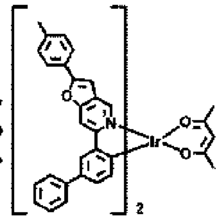
143
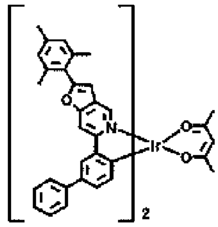
144
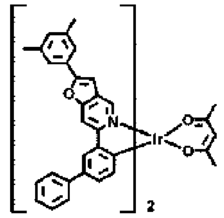
145

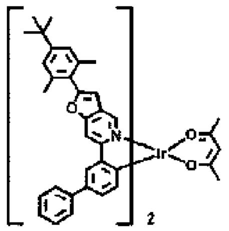
146
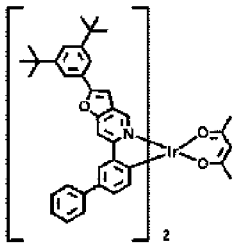
147
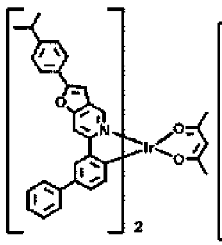
148
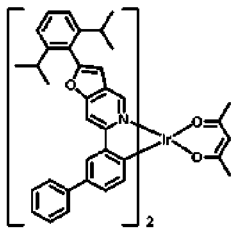
149
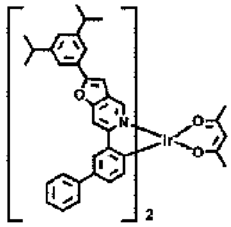
150

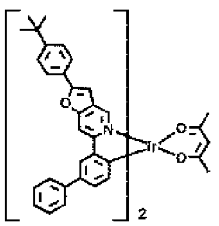
151
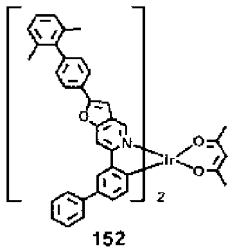
152
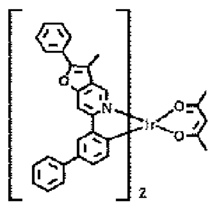
153
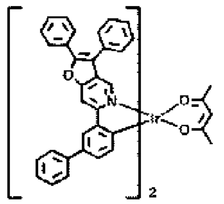
154
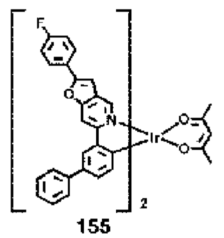
155

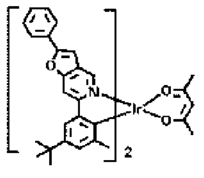
156
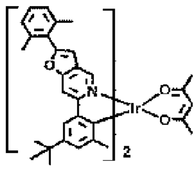
157
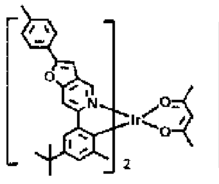
158
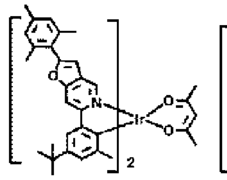
159
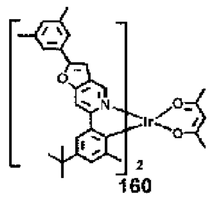
160

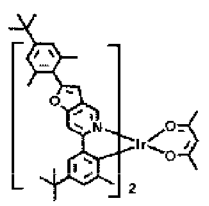
161
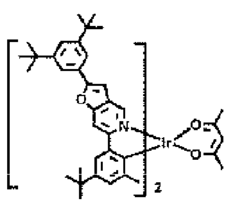
162
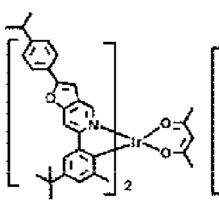
163
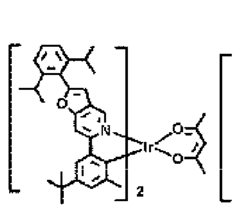
164
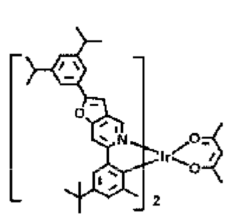
165

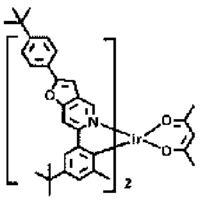
166

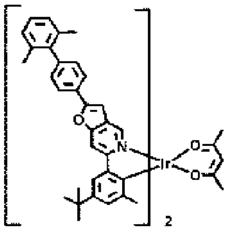
167

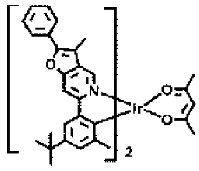
168

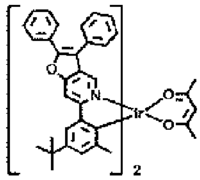
169

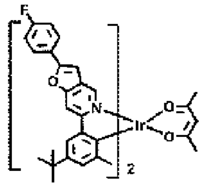
170

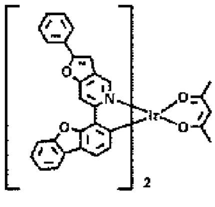
171

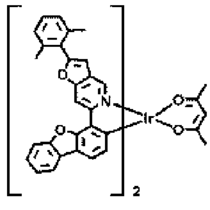
172

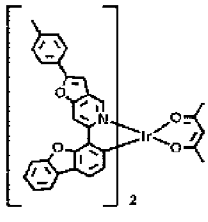
173

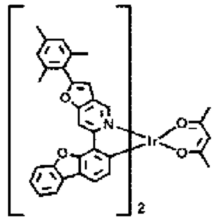
174

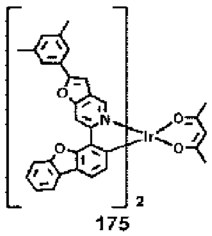
175

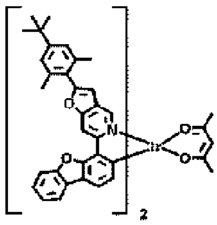
176

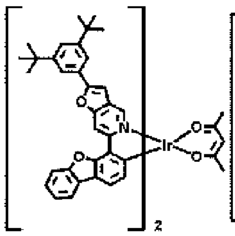
177

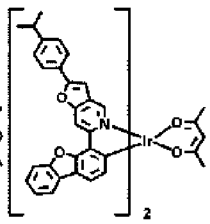
178

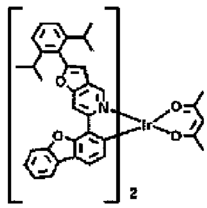
179

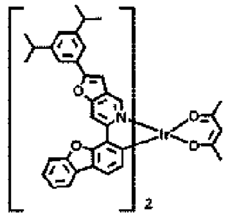
180

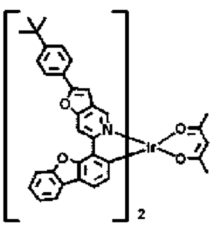
181

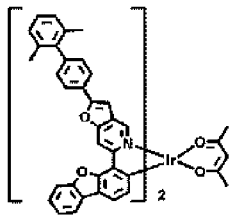
182

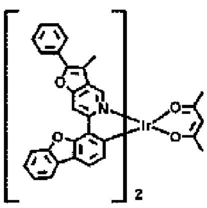
183

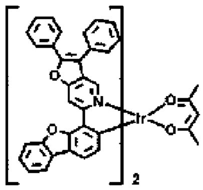
184

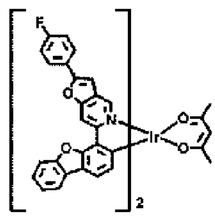
185

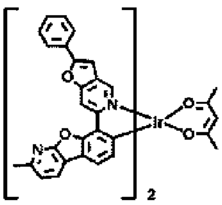
186

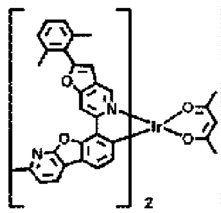
187

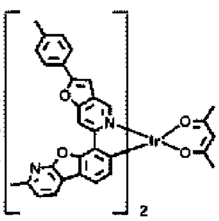
188

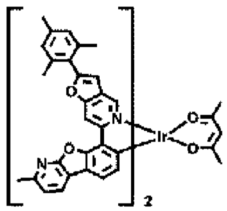
189

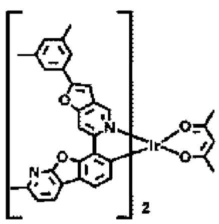
190

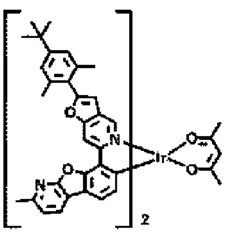
191

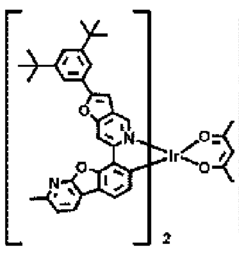
192

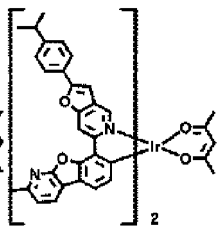
193

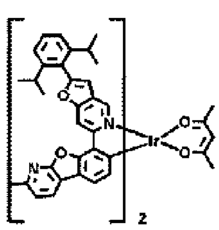
194

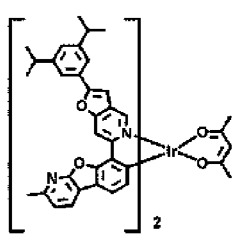
195

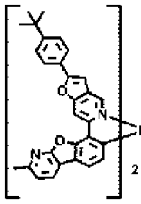
196

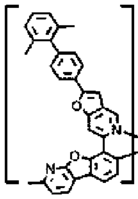
197

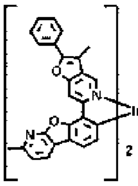
198

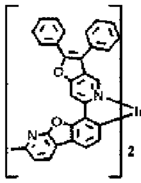
199

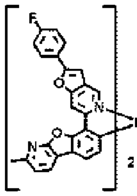
200

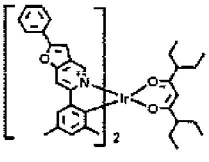
201

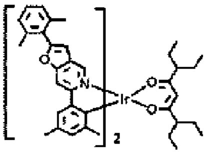
202

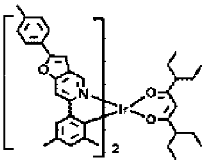
203

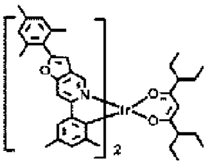
204

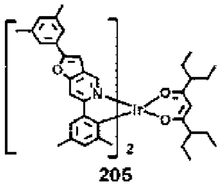
205

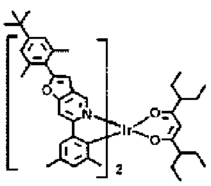
206

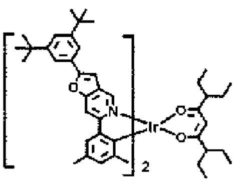
207

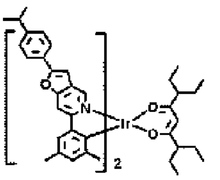
208

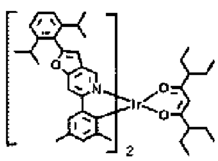
209

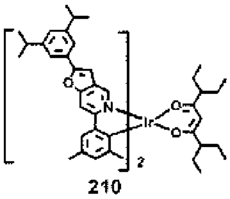
210

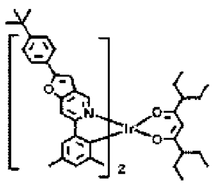
211

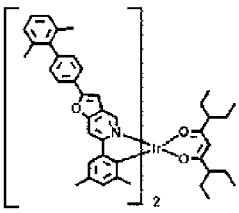
212

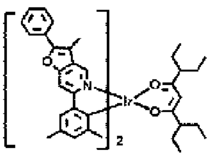
213

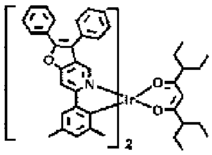
214

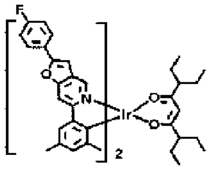
215

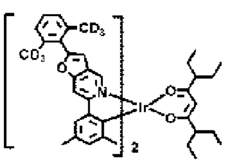
216

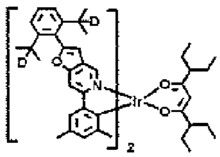
217

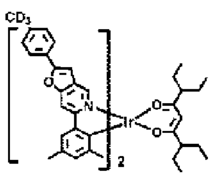
218

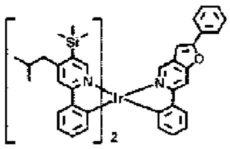
220

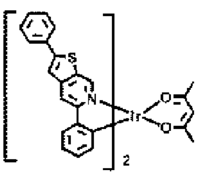
221

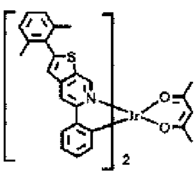
222

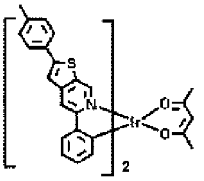
223

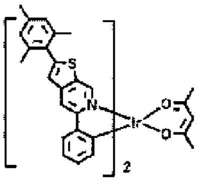
224

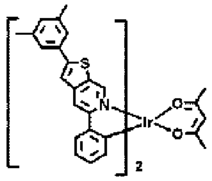
225

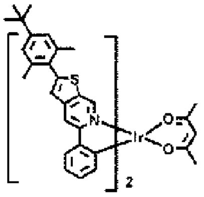
226

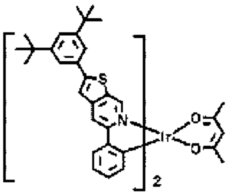
227

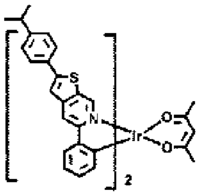
228

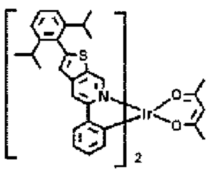
229

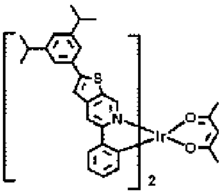
230

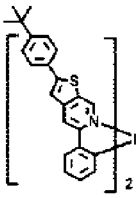
231

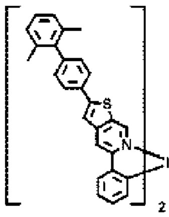
232

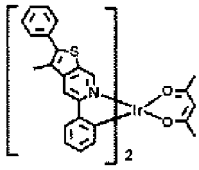
233

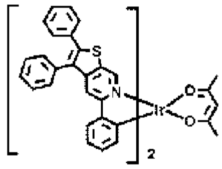
234

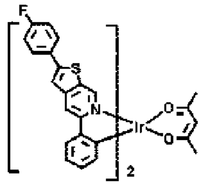
235

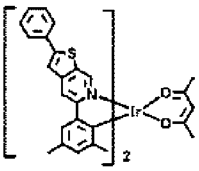
236

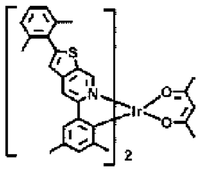
237

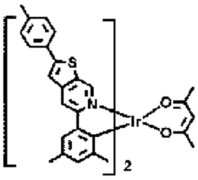
238

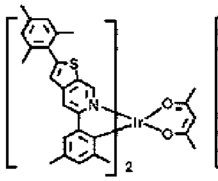
239

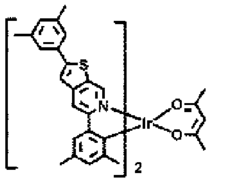
240

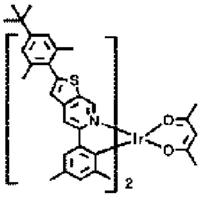
241

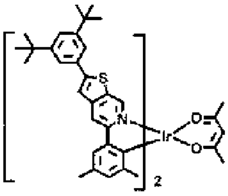
242

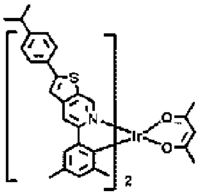
243

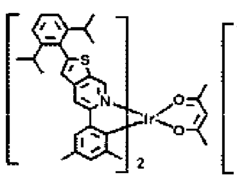
244

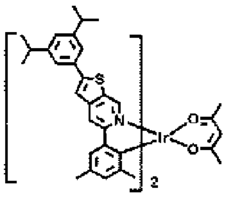
245

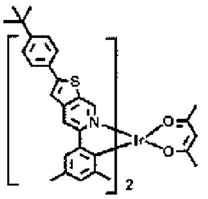
246

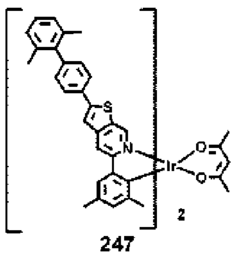
247

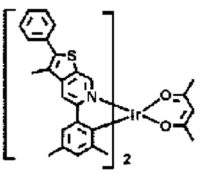
248

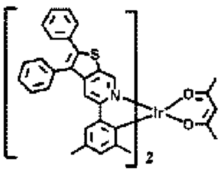
249

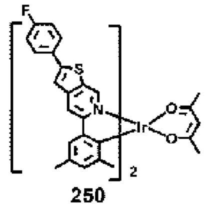
250

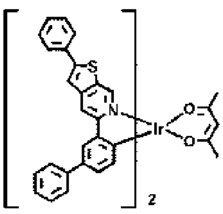
251

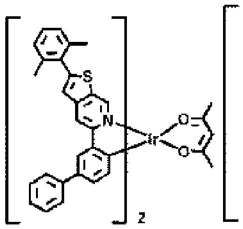
252

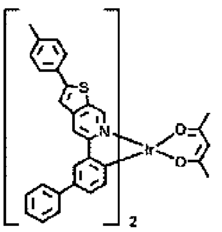
253

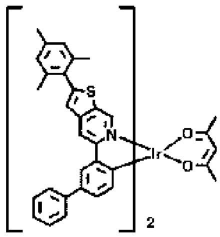
254

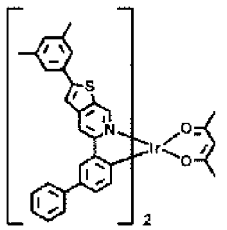
255

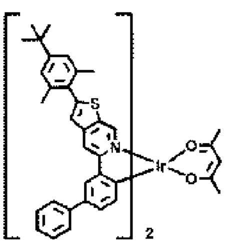
256

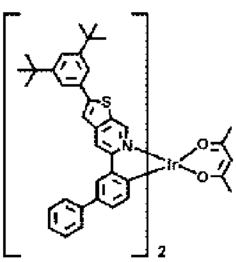
257

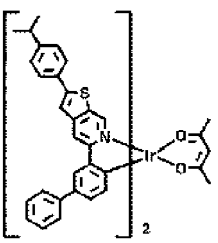
258

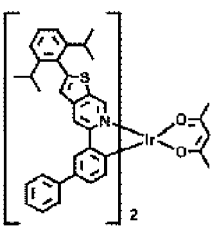
259

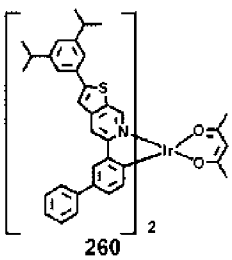
260

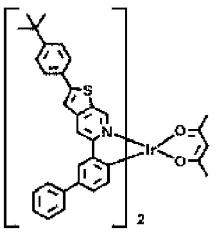
261
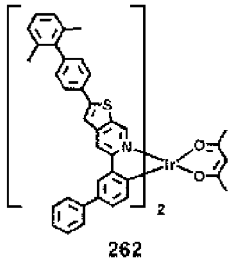
262
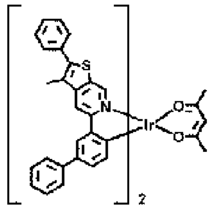
263
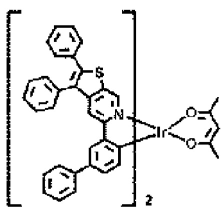
264
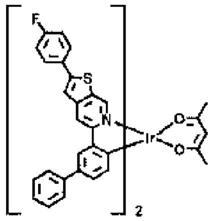
265

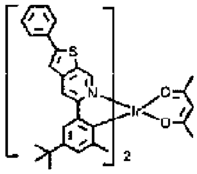
266
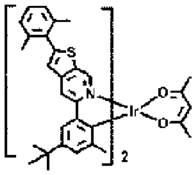
267
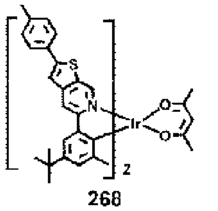
268
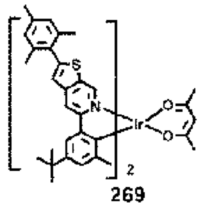
269
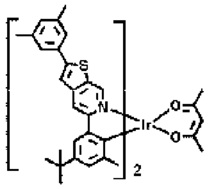
270

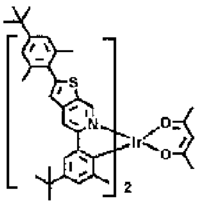
271
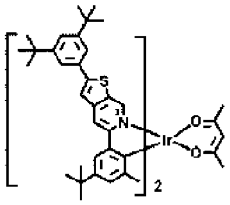
272
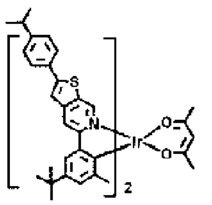
273
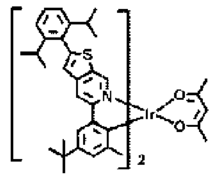
274
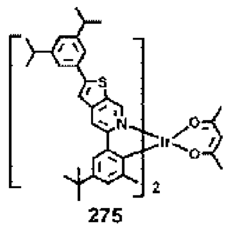
275

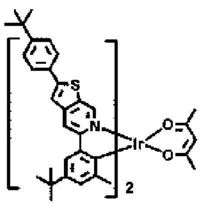
276
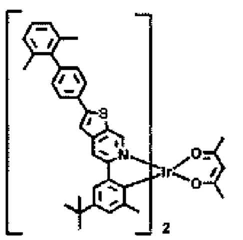
277
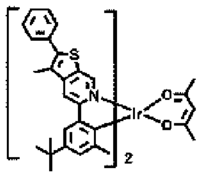
278
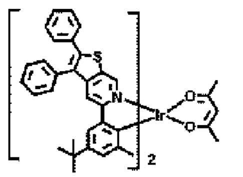
279
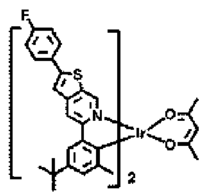
280

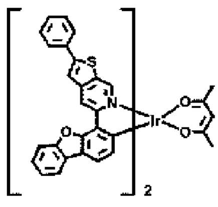
281
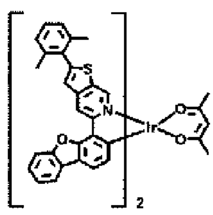
282
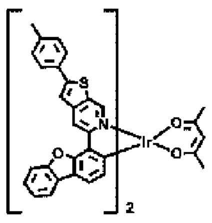
283
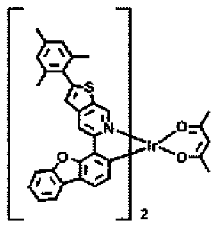
284
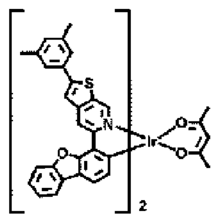
285

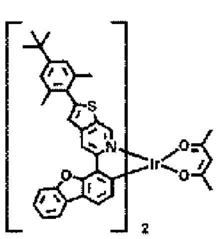
286
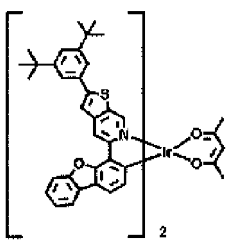
287
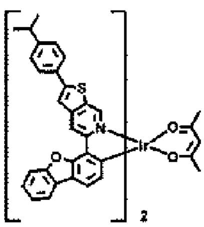
288
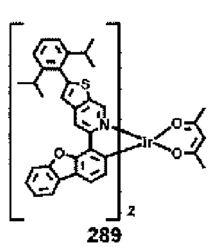
289
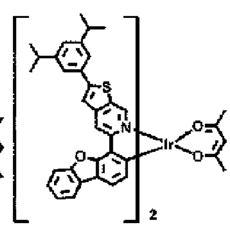
290

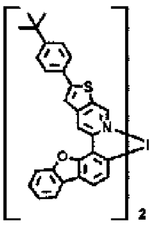
291
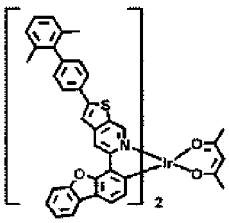
292
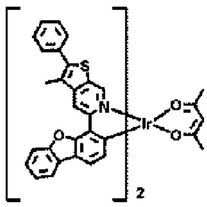
293
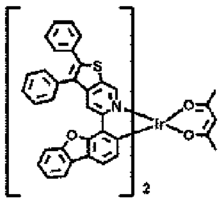
294
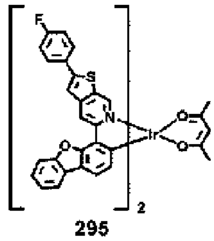
295

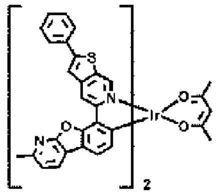
296
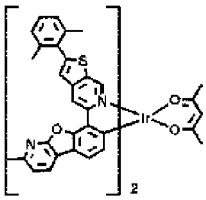
297
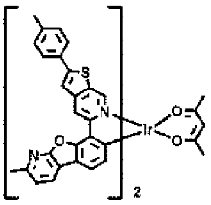
298
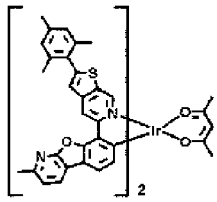
299
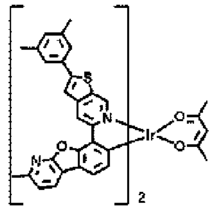
300

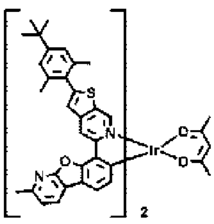
301
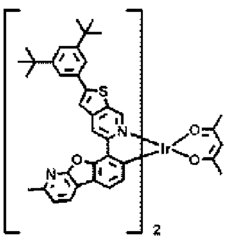
302
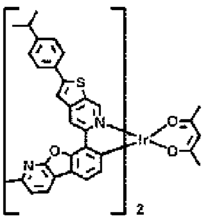
303
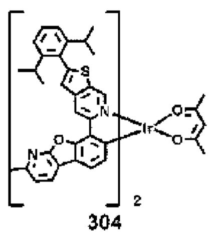
304
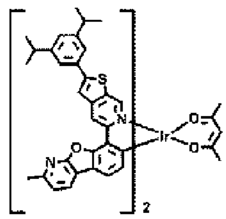
305

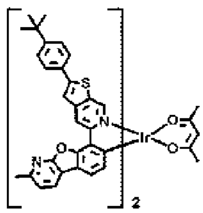
306
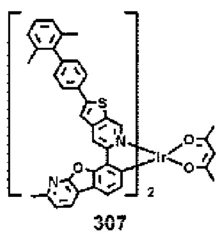
307
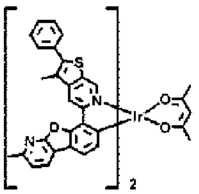
308
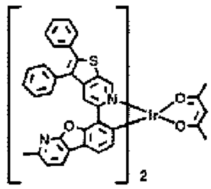
309
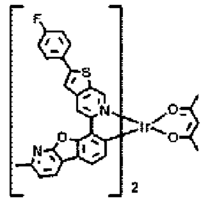
310

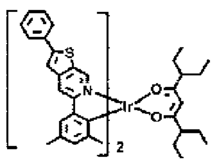
311
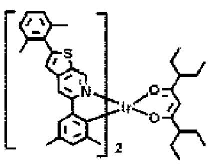
312
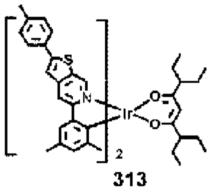
313
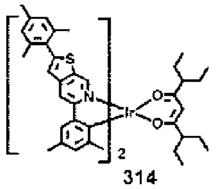
314
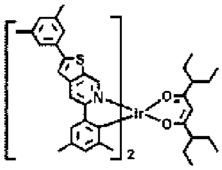
315

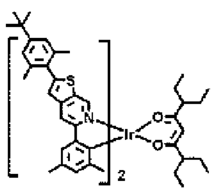
316
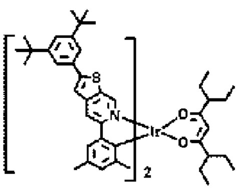
317
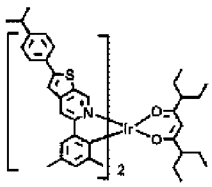
318
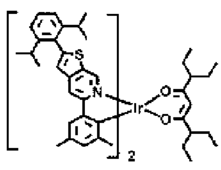
319
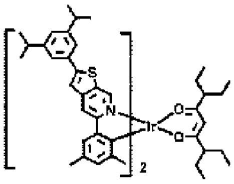
320

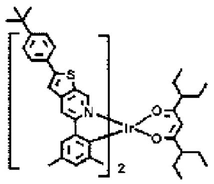
321
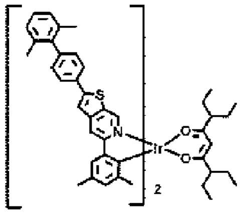
322
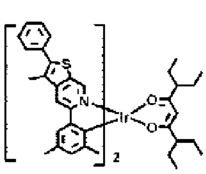
323
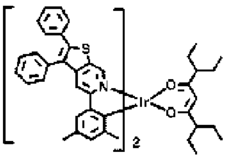
324
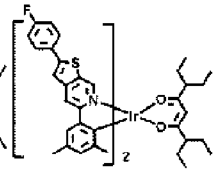
325

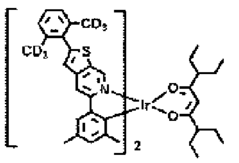
326
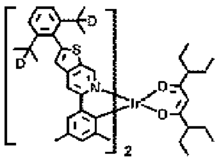
327
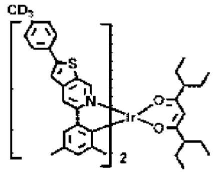
328
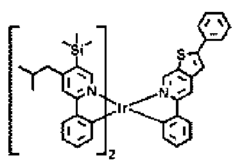
330

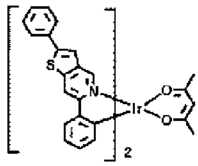
331
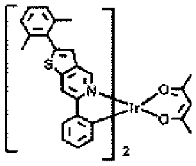
332
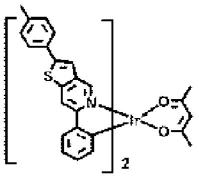
333
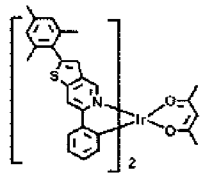
334
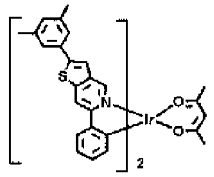
335

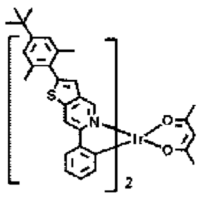
336
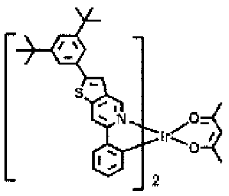
337
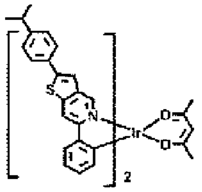
338
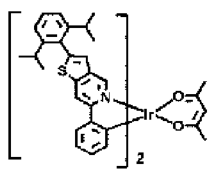
339
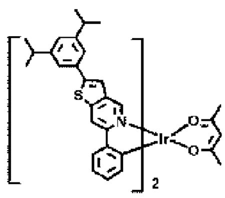
340

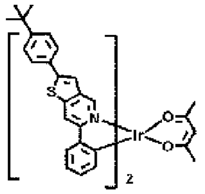
341
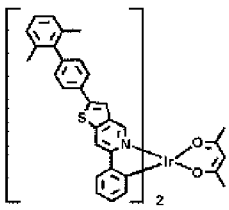
342
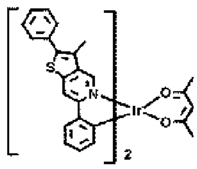
343
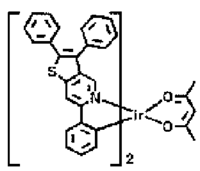
344
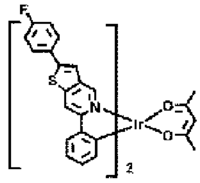
345

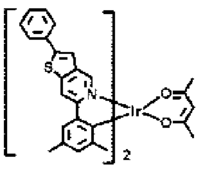
346
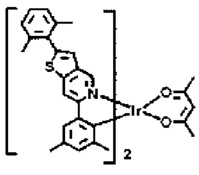
347
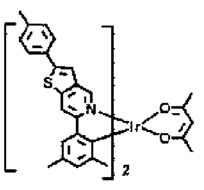
348
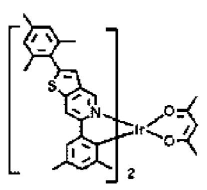
349
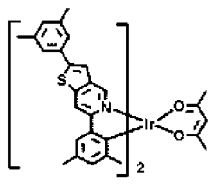
350

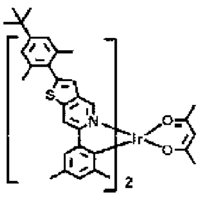
351

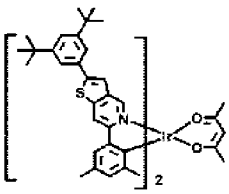
352

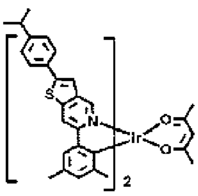
353

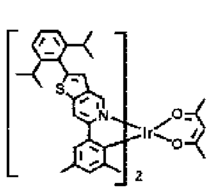
354

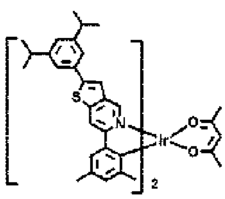
355

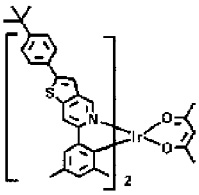
356

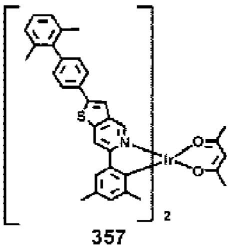
357

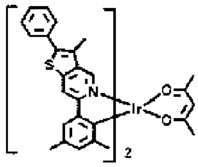
358

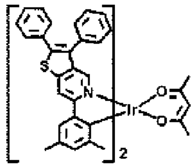
359

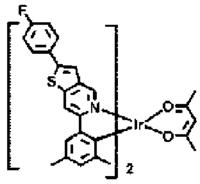
360

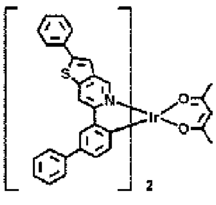
361

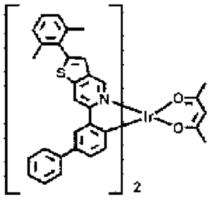
362

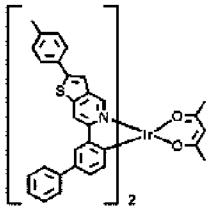
363

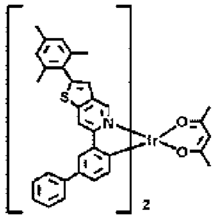
364

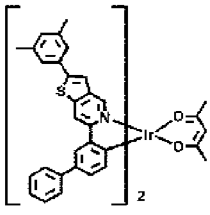
365

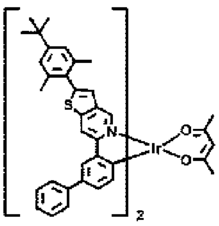
366

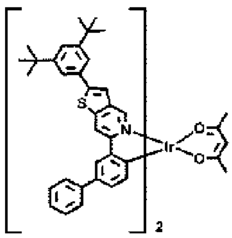
367

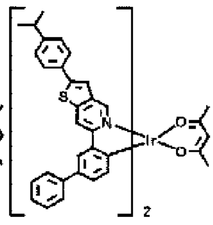
368

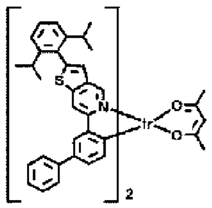
369

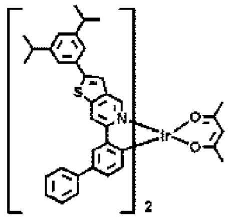
370

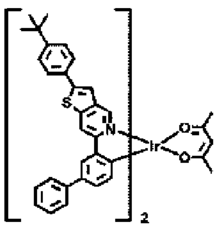
371

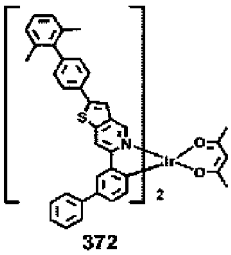
372

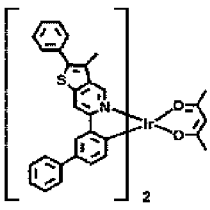
373

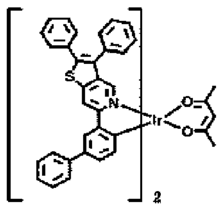
374

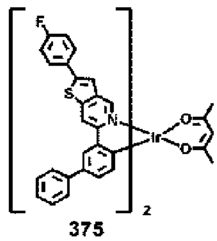
375

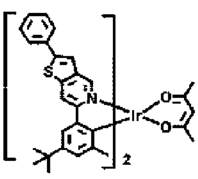
376

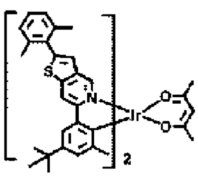
377

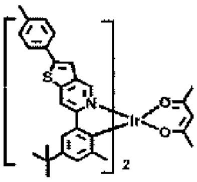
378

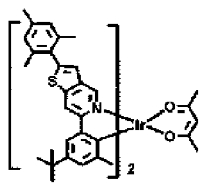
379

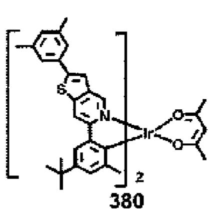
380

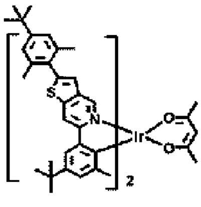
381

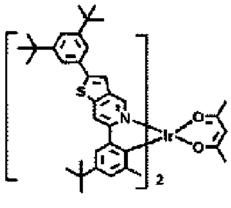
382

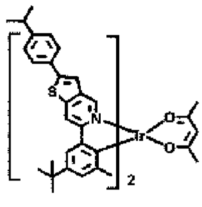
383

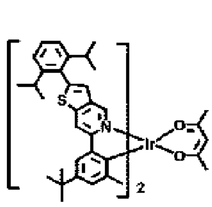
384

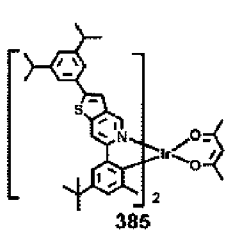
385

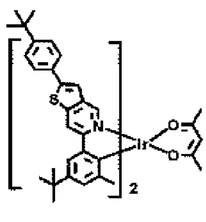
386

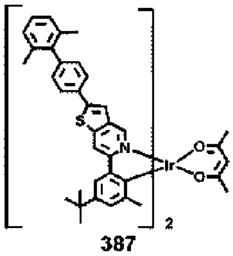
387

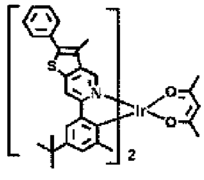
388

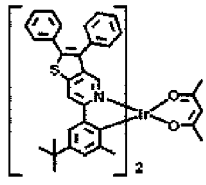
389

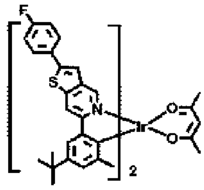
390

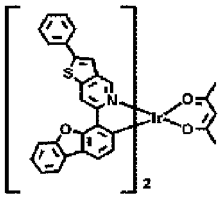
391

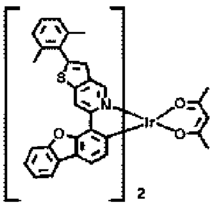
392

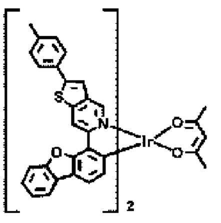
393

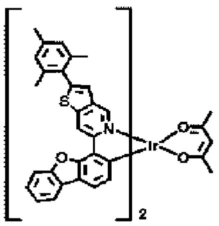
394

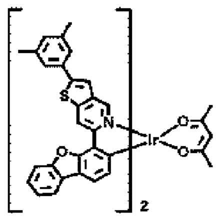
395

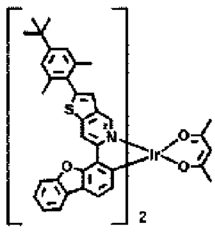
396

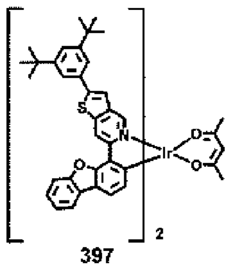
397

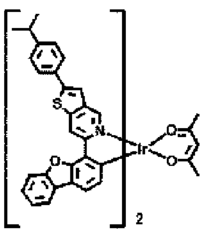
398

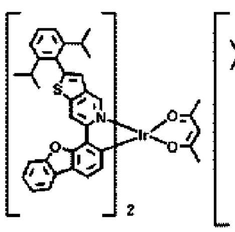
399

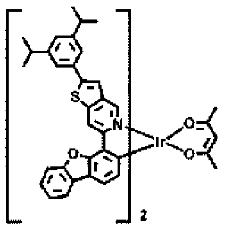
400

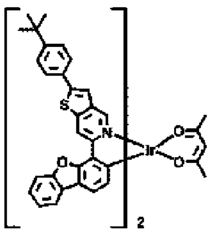
401

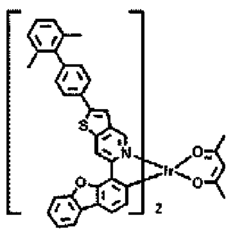
402

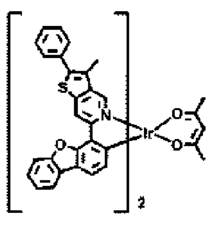
403

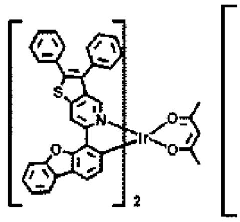
404

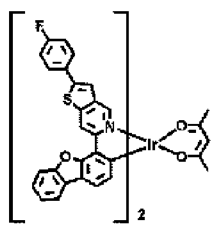
405

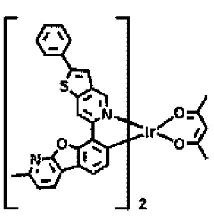
406

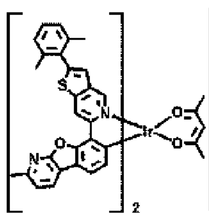
407

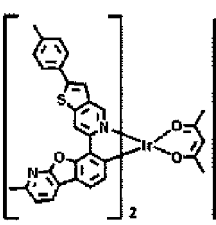
408

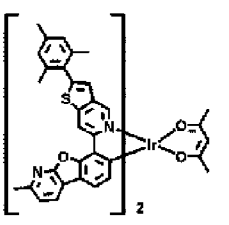
409

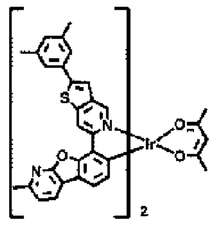
410

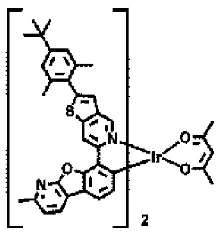
411

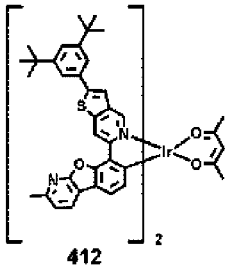
412

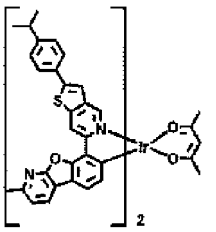
413

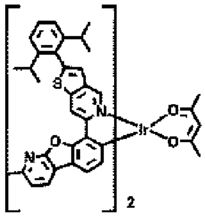
414

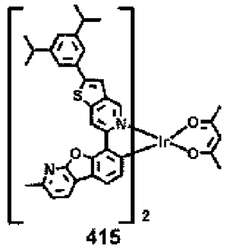
415

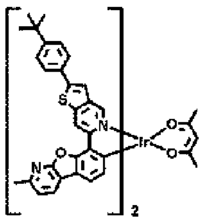
416

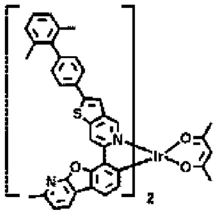
417

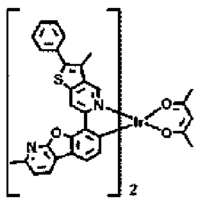
418

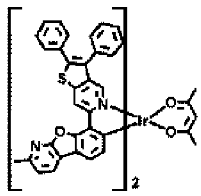
419

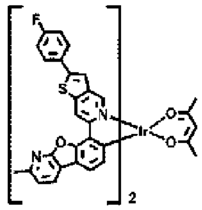
420

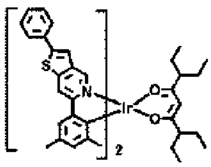
421

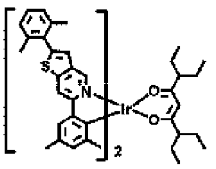
422

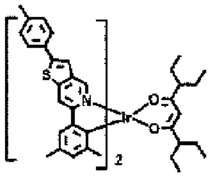
423

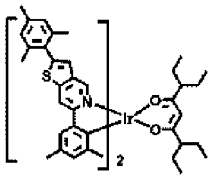
424

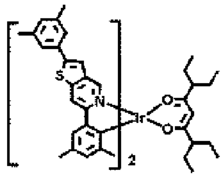
425

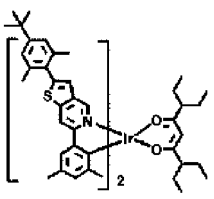
426

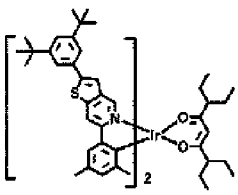
427

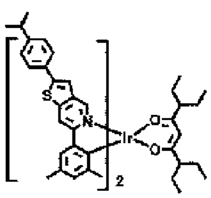
428

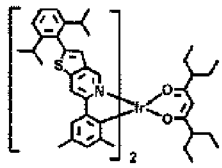
429

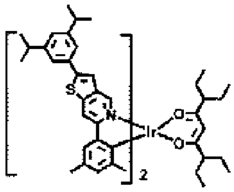
430

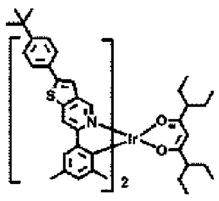
431

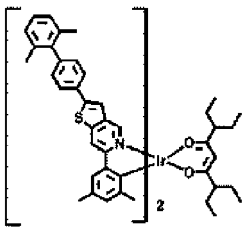
432

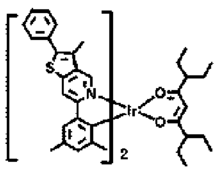
433

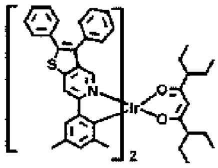
434

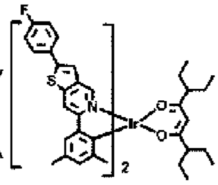
435

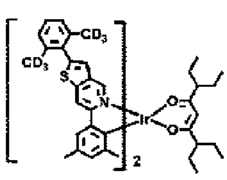
436

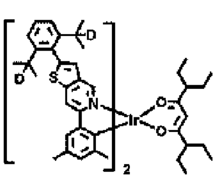
437

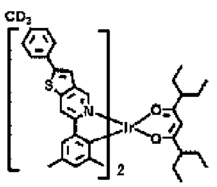
438

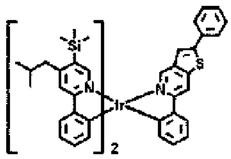
440

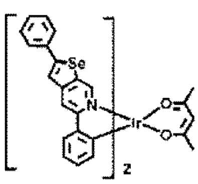
441
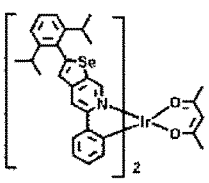
442
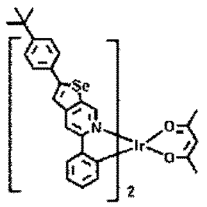
443
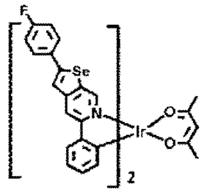
444
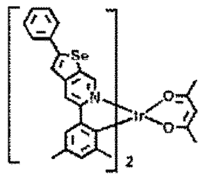
445

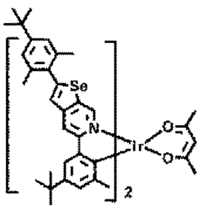
446
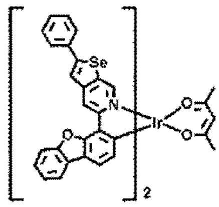
447
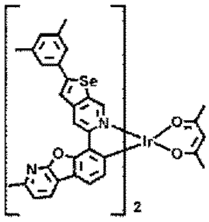
448
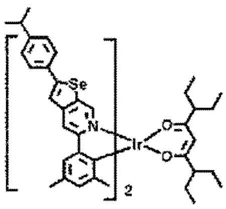
449
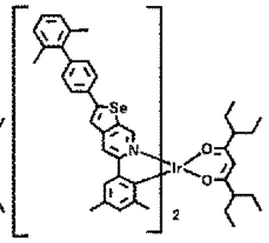
450

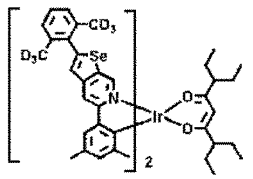
451
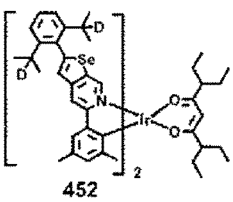
452
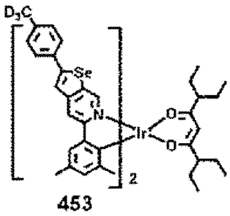
453
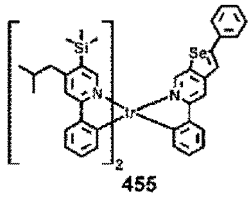
455

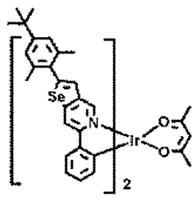
456
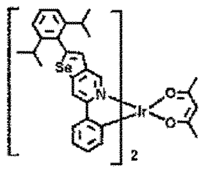
457
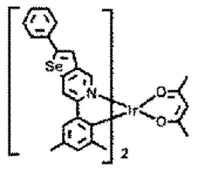
458
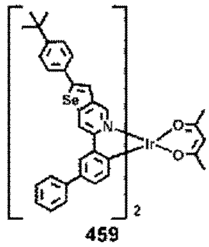
459
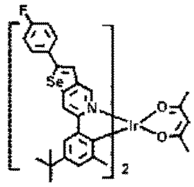
460

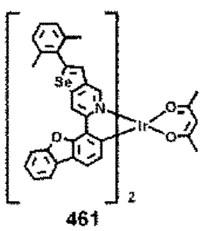
461
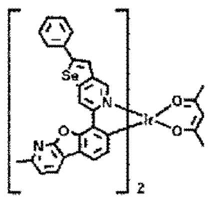
462
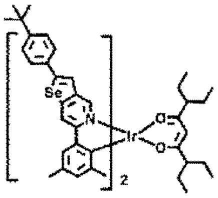
463
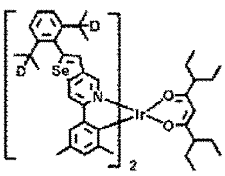
464
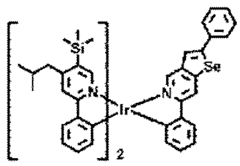
465

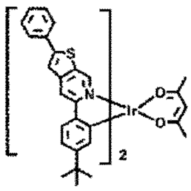
466
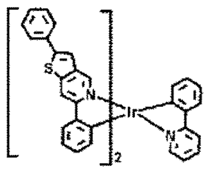
468
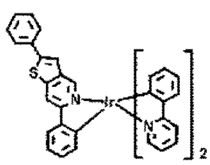
469

**12.** Organische lichtemittierende Vorrichtung, umfassend:

eine erste Elektrode;
eine zweite Elektrode; und

eine organische Schicht, die zwischen der ersten Elektrode und der zweiten Elektrode angeordnet ist und eine Emissionsschicht umfasst,
wobei die organische Schicht mindestens eine organometallische Verbindung nach einem der Ansprüche 1-11 umfasst.

**13.** Organische lichtemittierende Vorrichtung nach Anspruch 12, wobei

die erste Elektrode eine Anode ist,
die zweite Elektrode eine Kathode ist,
die organische Schicht ferner einen Lochtransportbereich zwischen der ersten Elektrode und der Emissionsschicht und einen Elektronentransportbereich zwischen der Emissionsschicht und der zweiten Elektrode umfasst,
der Lochtransportbereich eine Lochinjektionsschicht, eine Lochtransportschicht, eine Elektronenblockierschicht, eine Pufferschicht oder eine beliebige Kombination davon umfasst, und
der Elektronentransportbereich eine Lochblockierschicht, eine Elektronentransportschicht, eine Elektroneninjektionsschicht oder eine beliebige Kombination davon umfasst; und/oder
wobei die Emissionsschicht die organometallische Verbindung umfasst, bevorzugt, wobei die Emissionsschicht ferner einen Wirt umfasst und eine Menge des Wirts größer ist als eine Menge der organometallischen Verbindung.

**14.** Diagnostische Zusammensetzung, umfassend die organometallische Verbindung nach einem der Ansprüche 1-11.

**Revendications**

**1.** Composé organométallique représenté par la Formule 1 :

Formule 1
$M(L_1)_{n1}(L_2)_{n2}$,
avec, dans la Formule 1,
$L_1$ est un ligand représenté par la Formule 2,
n1 est 1, 2, ou 3, avec, lorsque n1 est 2 ou plus, deux ou plusieurs $L_1$ sont identiques ou différents les uns des autres,
$L_2$ est un ligand bidenté représenté par la Formule 3,
n2 est 1, 2, 3, ou 4, avec, lorsque n2 est 2 ou plus, deux ou plusieurs $L_2$ sont identiques ou différents les uns des autres, et
$L_1$ et $L_2$ sont différents l'un de l'autre :

M est Ir ou Os, et la somme de n1 et n2 est 3 ou 4 ; ou M est Pt, et la somme de n1 et n2 est 2,

Formule 2

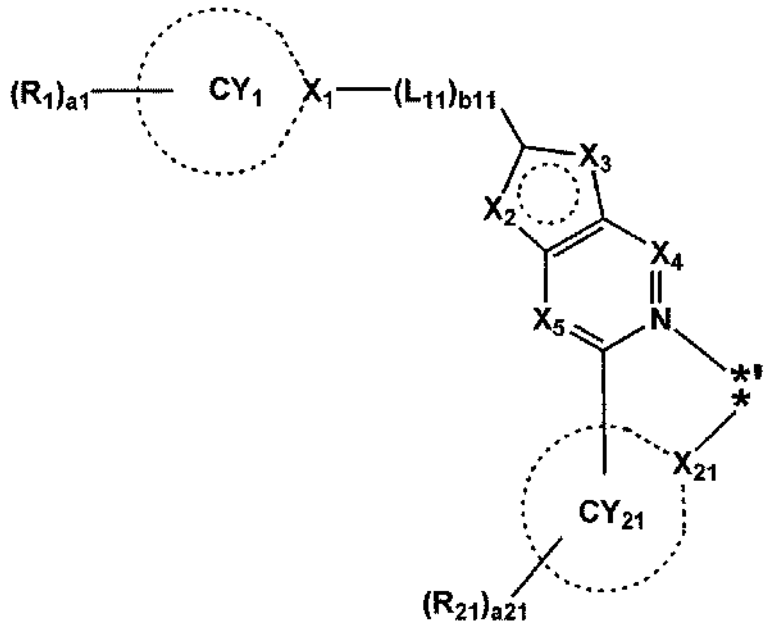

,

avec, dans la Formule 2,

$X_1$ est C, N, Si, ou P,

$X_{21}$ est C ou N,

l'anneau $CY_1$ et l'anneau $CY_{21}$ sont chacun indépendamment un groupe $C_5$-$C_{30}$ carbocyclique ou un groupe $C_2$-$C_{30}$ hétérocyclique,

$X_2$ et $X_3$ sont chacun indépendamment O, S, Se, ou $C(R_2)$, avec $X_2$ ou $X_3$ est O, S, ou Se,

$X_4$ est N ou $C(R_4)$,

$X_5$ est N ou $C(R_5)$,

$R_1$, $R_2$, $R_4$, $R_5$, et $R_{21}$ sont chacun indépendamment l'hydrogène, le deutérium, -F, -Cl, -Br, -I, $-SF_5$, un groupe hydroxyle, un groupe cyano, un groupe nitro, un groupe amino, un groupe amidino, un groupe hydrazine, un groupe hydrazone, un groupe acide carboxylique ou un de ses sels, un groupe acide sulfonique ou un de ses sels, un groupe acide phosphorique ou un de ses sels, un groupe $C_1$-$C_{60}$ alkyle substitué ou non substitué, un groupe $C_2$-$C_{60}$ alcényle substitué ou non substitué, un groupe $C_2$-$C_{60}$ alcynyle substitué ou non substitué, un groupe $C_1$-$C_{60}$ alcoxy substitué ou non substitué, un groupe $C_3$-$C_{10}$ cycloalkyle substitué ou non substitué, un groupe $C_2$-$C_{10}$ hétérocycloalkyle substitué ou non substitué, un groupe $C_3$-$C_{10}$ cycloalcényle substitué ou non substitué, un groupe $C_2$-$C_{10}$ hétérocycloalcényle substitué ou non substitué, un groupe $C_6$-$C_{60}$ aryle substitué ou non substitué, un groupe $C_6$-$C_{60}$ aryloxy substitué ou non substitué, un groupe $C_6$-$C_{60}$ arylthio substitué ou non substitué, un groupe $C_1$-$C_{60}$ hétéroaryle substitué ou non substitué, un groupe polycyclique monovalent non-aromatique condensé substitué ou non substitué, un groupe hétéropolycyclique monovalent non-aromatique condensé substitué ou non substitué, $-N(Q_1)(Q_2)$, $-Si(Q_3)(Q_4)(Q_5)$, $-B(Q_6)(Q_7)$, $-P(=O)(Q_8)(Q_9)$ ou $-P(Q_8)(Q_9)$,

a1 et a21 sont chacun indépendamment un entier de 0 à 20,

l'anneau $CY_1$ et $R_2$ ne sont pas liés l'un à l'autre et $R_1$ et $R_2$ ne sont pas liés l'un à l'autre,

$L_{11}$ est un groupe $C_5$-$C_{30}$ carbocyclique qui est non substitué ou substitué par au moins un $R_{10a}$ ou un groupe $C_2$-$C_{30}$ hétérocyclique qui est non substitué ou substitué par au moins un $R_{10a}$,

b11 est un entier de 0 à 10, avec, lorsque b11 est 0, un groupe représenté par $*\text{-}(L_{11})_{b11}\text{-}*'$ est une liaison simple, et lorsque b11 est 2 ou plus, deux ou plusieurs $L_{11}$ sont identiques ou différents les uns des autres, deux parmi une pluralité de $R_{21}$ voisins sont éventuellement liés pour former un groupe $C_5$-$C_{30}$ carbocyclique qui est non substitué ou substitué par au moins un $R_{10a}$ ou un groupe $C_2$-$C_{30}$ hétérocyclique qui est non substitué ou substitué par au moins un $R_{10a}$,

$R_{10a}$ est identique à celui défini en connexion avec $R_{21}$,

* et *' indiquent chacun un site de liaison à M dans la Formule 1,

un substituant du groupe $C_5$-$C_{30}$ carbocyclique substitué, le groupe $C_2$-$C_{30}$ hétérocyclique substitué, le groupe $C_1$-$C_{60}$ alkyle substitué, le groupe $C_2$-$C_{60}$ alcényle substitué, le groupe $C_2$-$C_{60}$ alcynyle substitué, le groupe $C_1$-$C_{60}$ alcoxy substitué, le groupe $C_3$-$C_{10}$ cycloalkyle substitué, le groupe $C_2$-$C_{10}$ hétérocycloalk-

yle substitué, le groupe $C_3$-$C_{10}$ cycloalcényle substitué, le groupe $C_2$-$C_{10}$ hétérocycloalcényle substitué, le groupe $C_6$-$C_{60}$ aryle substitué, le groupe $C_6$-$C_{60}$ aryloxy substitué, le groupe $C_6$-$C_{60}$ arylthio substitué, le groupe $C_1$-$C_{60}$ hétéroaryle substitué, le groupe polycyclique monovalent non-aromatique condensé substitué, et le groupe hétéropolycyclique monovalent non-aromatique condensé substitué est :

deutérium, -F, -Cl, -Br, -I, $-CD_3$, $-CD_2H$, $-CDH_2$, $-CF_3$, $-CF_2H$, $-CFH_2$, un groupe hydroxyle, un groupe cyano, un groupe nitro, un groupe amino, un groupe amidino, un groupe hydrazine, un groupe hydrazone, un groupe acide carboxylique ou un de ses sels, un groupe acide sulfonique ou un de ses sels, un groupe acide phosphorique ou un de ses sels, un groupe $C_1$-$C_{60}$ alkyle, un groupe $C_2$-$C_{60}$ alcényle, un groupe $C_2$-$C_{60}$ alcynyle, ou un groupe $C_1$-$C_{60}$ alcoxy ;

un groupe $C_1$-$C_{60}$ alkyle, un groupe $C_2$-$C_{60}$ alcényle, un groupe $C_2$-$C_{60}$ alcynyle, ou un groupe $C_1$-$C_{60}$ alcoxy, chacun substitué par le deuterium, -F, -Cl, -Br, -I, $-CD_3$, $-CD_2H$, $-CDH_2$, - $CF_3$, $-CF_2H$, $-CFH_2$, un groupe hydroxyle, un groupe cyano, un groupe nitro, un groupe amino, un groupe amidino, un groupe hydrazine, un groupe hydrazone, un groupe acide carboxylique ou un de ses sels, un groupe acide sulfonique ou un de ses sels, un groupe acide phosphorique ou un de ses sels, un groupe $C_3$-$C_{10}$ cycloalkyle, un groupe $C_2$-$C_{10}$ hétérocycloalkyle, un groupe $C_3$-$C_{10}$ cycloalcényle, un groupe $C_2$-$C_{10}$ hétérocycloalcényle, un groupe $C_6$-$C_{60}$ aryle, un groupe $C_6$-$C_{60}$ aryloxy, un groupe $C_6$-$C_{60}$ arylthio, un groupe $C_1$-$C_{60}$ hétéroaryle, un groupe polycyclique monovalent non-aromatique condensé, un groupe hétéropolycyclique monovalent non-aromatique condensé, $-N(Q_{11})(Q_{12})$, $-Si(Q_{13})(Q_{14})(Q_{15})$, $-B(Q_{16})(Q_{17})$, $-P(=O)(Q_{18})(Q_{19})$, ou une combinaison quelconque de ceux-ci ;

un groupe $C_3$-$C_{10}$ cycloalkyle, un groupe $C_2$-$C_{10}$ hétérocycloalkyle, un groupe $C_3$-$C_{10}$ cycloalcényle, un groupe $C_2$-$C_{10}$ hétérocycloalcényle, un groupe $C_6$-$C_{60}$ aryle, un groupe $C_6$-$C_{60}$ aryloxy, un groupe $C_6$-$C_{60}$ arylthio, un groupe $C_1$-$C_{60}$ hétéroaryle, un groupe polycyclique monovalent non-aromatique condensé, un groupe hétéropolycyclique monovalent non-aromatique condensé ;

un groupe $C_3$-$C_{10}$ cycloalkyle, un groupe $C_2$-$C_{10}$ hétérocycloalkyle, un groupe $C_3$-$C_{10}$ cycloalcényle, un groupe $C_2$-$C_{10}$ hétérocycloalcényle, un groupe $C_6$-$C_{60}$ aryle, un groupe $C_6$-$C_{60}$ aryloxy, un groupe $C_6$-$C_{60}$ arylthio, un groupe $C_1$-$C_{60}$ hétéroaryle, un groupe polycyclique monovalent non-aromatique condensé, ou un groupe hétéropolycyclique monovalent non-aromatique condensé, chacun substitué par le deutérium, -F, -Cl, -Br, -I, $-CD_3$, $-CD_2H$, $-CDH_2$, - $CF_3$, $-CF_2H$, $-CFH_2$, un groupe hydroxyle, un groupe cyano, un groupe nitro, un groupe amino, un groupe amidino, un groupe hydrazine, un groupe hydrazone, un groupe acide carboxylique ou un de ses sels, un groupe acide sulfonique ou un de ses sels, un groupe acide phosphorique ou un de ses sels, un groupe $C_1$-$C_{60}$ alkyle, un groupe $C_2$-$C_{60}$ alcényle, un groupe $C_2$-$C_{60}$ alcynyle, un groupe $C_1$-$C_{60}$ alcoxy, un groupe $C_3$-$C_{10}$ cycloalkyle, un groupe $C_2$-$C_{10}$ hétérocycloalkyle, un groupe $C_3$-$C_{10}$ cycloalcényle, un groupe $C_2$-$C_{10}$ hétérocycloalcényle, un groupe $C_6$-$C_{60}$ aryle, un groupe $C_6$-$C_{60}$ aryloxy, un groupe $C_6$-$C_{60}$ arylthio, un groupe $C_1$-$C_{60}$ hétéroaryle, un groupe polycyclique monovalent non-aromatique condensé, un groupe hétéropolycyclique monovalent non-aromatique condensé, $-N(Q_{21})(Q_{22})$, - $Si(Q_{23})(Q_{24})(Q_{25})$, $-B(Q_{26})(Q_{27})$, $-P(=O)(Q_{28})(Q_{29})$, ou une combinaison quelconque de ceux-ci ;

$-N(Q_{31})(Q_{32})$, $-Si(Q_{33})(Q_{34})(Q_{35})$, $-B(Q_{36})(Q_{37})$, ou $-P(=O)(Q_{38})(Q_{39})$; ou

une combinaison quelconque de ceux-ci, et

$Q_1$ à $Q_9$, $Q_{11}$ à $Q_{19}$, $Q_{21}$ à $Q_{29}$, et $Q_{31}$ à $Q_{39}$ sont chacun indépendamment l'hydrogène, le deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un de ses sels, un groupe acide sulfonique ou un de ses sels, un groupe acide phosphorique ou un de ses sels, un groupe $C_1$-$C_{60}$ alkyle, un groupe $C_2$-$C_{60}$ alcényle, un groupe $C_2$-$C_{60}$ alcynyle, un groupe $C_1$-$C_{60}$ alcoxy, un groupe $C_3$-$C_{10}$ cycloalkyle, un groupe $C_2$-$C_{10}$ hétérocycloalkyle, un groupe $C_3$-$C_{10}$ cycloalcényle, un groupe $C_2$-$C_{10}$ hétérocycloalcényle, un groupe $C_6$-$C_{60}$ aryle, un groupe $C_6$-$C_{60}$ aryle substitué par un groupe $C_1$-$C_{60}$ alkyle, un groupe $C_6$-$C_{60}$ aryle, ou une combinaison quelconque de ceux-ci, un groupe $C_6$-$C_{60}$ aryloxy, un groupe $C_6$-$C_{60}$ arylthio, un groupe $C_1$-$C_{60}$ hétéroaryle, un groupe polycyclique monovalent non-aromatique condensé, ou un groupe hétéropolycyclique monovalent non-aromatique condensé ;

Formule 3

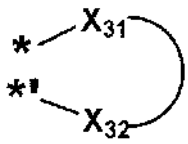

avec dans la Formule 3,
$X_{31}$ et $X_{32}$ sont chacun O ;
$X_{31}$ est O et $X_{32}$ est N ; ou
$X_{31}$ est N et $X_{32}$ est C,

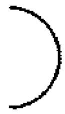

indique un groupe atomique quelconque liant $X_{31}$ et $X_{32}$ l'un à l'autre, et
* et *' indiquent chacun un site de liaison à M dans la Formule 1 ; et le composé organométallique n'étant pas le composé suivant :

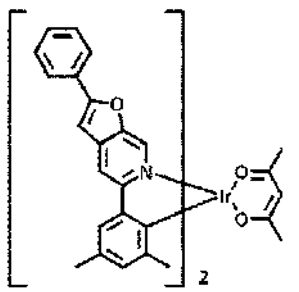

**2.** Composé organométallique de la revendication 1, dans lequel, l'anneau $CY_1$ et l'anneau $CY_{21}$ sont chacun indépendamment un groupe cyclopentène, un groupe cyclohexène, un groupe benzène, un groupe naphtalène, un groupe anthracène, un groupe phénanthrène, un groupe triphénylène, un groupe pyrène, un groupe chrysène, un groupe cyclopentadiène, un groupe 1,2,3,4-tétrahydronaphtalène, un groupe thiophène, un groupe furane, un groupe indole, un groupe benzoborole, un groupe benzophosphole, un groupe indène, un groupe benzosilole, un groupe benzogermole, un groupe benzothiophène, un groupe benzosélénophène, un groupe benzofurane, un groupe carbazole, un groupe dibenzoborole, un groupe dibenzophosphole, un groupe fluorène, un groupe dibenzosilole, un groupe dibenzogermole, un groupe dibenzothiophène, un groupe dibenzosélénophène, un groupe dibenzofurane, un groupe dibenzothiophène 5-oxyde, un groupe 9H-fluorène-9-one, un groupe dibenzothiophène 5,5-dioxyde, un groupe azaindole, un groupe azabenzoborole, un groupe azabenzophosphole, un groupe azaindène, un groupe azabenzosilole, un groupe azabenzogermole, un groupe azabenzothiophène, un groupe azabenzosélénophène, un groupe azabenzofurane, un groupe azacarbazole, un groupe azadibenzoborole, un groupe azadibenzophosphole, un groupe azafluorène, un groupe azadibenzosilole, un groupe azadibenzogermole, un groupe azadibenzothiophène, un groupe azadibenzosélénophène, un groupe azadibenzofurane, un groupe azadibenzothiophène 5-oxyde, un groupe aza-9H-fluorène-9-one, un groupe azadibenzothiophène 5,5-dioxyde, un groupe pyridine, un groupe pyrimidine, un groupe pyrazine, un groupe pyridazine, un groupe triazine, un groupe quinoline, un groupe isoquinoline, un groupe quinoxaline, un groupe quinazoline, un groupe phénanthroline, un groupe pyrrole, un groupe pyrazole, un groupe imidazole, un groupe triazole, un groupe oxazole, un groupe isoxazole, un groupe thiazole, un groupe isothiazole, un groupe oxadiazole, un groupe thiadiazole, un groupe benzopyrazole, un groupe benzimidazole, un groupe benzoxazole, un groupe benzothiazole, un groupe benzoxadiazole, un groupe benzothiadiazole, un groupe 5,6,7,8-tétrahydroisoquinoline, ou un groupe 5,6,7,8-tétrahydroquinoline.

**3.** Composé organométallique des revendications 1 ou 2, dans lequel
$R_1$, $R_2$, $R_4$, $R_5$, $R_{21}$, et $R_{10a}$ sont chacun indépendamment :

hydrogène, deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un de ses sels, un groupe acide sulfonique ou un de ses sels, un groupe acide phosphorique ou un de ses sels, $-SF_5$, un groupe $C_1$-$C_{20}$ alkyle, ou un groupe $C_1$-$C_{20}$ alcoxy ;
un groupe $C_1$-$C_{20}$ alkyle ou un groupe $C_1$-$C_{20}$ alcoxy, chacun substitué par le deutérium, - F, -Cl, -Br, -I, $-CD_3$, $-CD_2H$, $-CDH_2$, $-CF_3$, $-CF_2H$, $-CFH_2$, un groupe hydroxyle, un groupe cyano, un groupe nitro, un groupe amino, un groupe amidino, un groupe hydrazine, un groupe hydrazone, un groupe acide carboxylique ou un de ses sels, un groupe acide sulfonique ou un de ses sels, un groupe acide phosphorique ou un de ses sels, un groupe $C_1$-$C_{10}$ alkyle, un groupe cyclopentyle, un groupe cyclohexyle, un groupe cycloheptyle, un groupe cyclooctyle, un groupe adamantyle, un groupe norboményle, un groupe cyclopentényle, un groupe cyclohexényle, un groupe cycloheptényle, un groupe bicyclo[1.1.1]pentyle, un groupe bicyclo[2.1.1]hexyle, un groupe bicyclo[2.2.1]heptyle, un groupe bicyclo[2.2.2]octyle, un groupe phényle, un groupe biphényle, un groupe terphényle, un groupe naphthyle, un groupe pyridinyle, un groupe pyrimidinyle, ou une combinaison quelconque de ceux-ci ;
un groupe cyclopentyle, un groupe cyclohexyle, un groupe cycloheptyle, un groupe cyclooctyle, un groupe adamantyle, un groupe norboményle, un groupe cyclopentényle, un groupe cyclohexényle, un groupe cycloheptényle, un groupe cyclooctényle, un groupe bicyclo[1.1.1]pentyle, un groupe bicyclo[2.1.1]hexyle, un groupe bicyclo[2.2.1]heptyle, un groupe bicyclo[2.2.2]octyle, un groupe phényle, un groupe biphényle, un groupe terphényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle ou un groupe imidazopyrimidinyle, chacun substitué ou non substitué par le deutérium, -F, -Cl, -Br, -I, $-CD_3$, - $CD_2H$, $-CDH_2$, $-CF_3$, $-CF_2H$, $-CFH_2$, un groupe hydroxyle, un groupe cyano, un groupe nitro, un groupe amino, un groupe amidino, un groupe hydrazine, un groupe hydrazone, un groupe acide carboxylique ou un de ses sels, un groupe acide sulfonique ou un de ses sels, un groupe acide phosphorique ou un de ses sels, un groupe $C_1$-$C_{20}$ alkyle, un groupe $C_1$-$C_{20}$ alcoxy, un groupe cyclopentyle, un groupe cyclohexyle, un groupe cycloheptyle, un groupe cyclooctyle, un groupe adamantyle, un groupe norboményle, un groupe cyclopentényle, un groupe cyclohexényle, un groupe cycloheptényle, un groupe cyclooctényle, un groupe bicyclo[1.1.1]pentyle, un groupe bicyclo[2.1.1]hexyle, un groupe bicyclo[2.2.1]heptyle, un groupe bicyclo[2.2.2]octyle, un groupe phényle, un groupe biphényle, un groupe terphényle, un groupe naphthyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, un groupe imidazopyrimidinyle, $-Si(Q_{33})(Q_{34})(Q_{35})$, ou une combinaison quelconque de ceux-ci ; ou
$-N(Q_1)(Q_2)$, $-Si(Q_3)(Q_4)(Q_5)$, $-B(Q_6)(Q_7)$, $-P(=O)(Q_8)(Q_9)$, ou$-P(Q_8)(Q_9)$, et
$Q_1$ à $Q_9$ et $Q_{33}$ à $Q_{35}$ sont chacun indépendamment :
$-CH_3$, $-CD_3$, $-CD_2H$, $-CDH_2$, $-CH_2CH_3$, $-CH_2CD_3$, $-CH_2CD_2H$, $-CH_2CDH_2$, $-CHDCH_3$, - $CHDCD_2H$, $-CHDCDH_2$, $-CHDCD_3$, $-CD_2CD_3$, $-CD_2CD_2H$, ou $-CD_2CDH_2$ ; ou
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle, ou un groupe naphthyle, chacun substitué ou non substitué par le deutérium, un groupe $C_1$-$C_{10}$ alkyle, un groupe phényle, ou une combinaison quelconque de ceux-ci.

**4.** Composé organométallique de l'une quelconque des revendications 1 à 3, dans lequel

un groupe représenté par

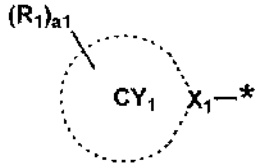

dans la Formule 2 est un groupe $C_3$-$C_{10}$ cycloalcényle, un groupe $C_2$-$C_{10}$ heterocycloalcényle, un groupe $C_6$-$C_{30}$ aryle, un groupe $C_2$-$C_{30}$ heteroaryle, un groupe polycyclique monovalent non-aromatique condensé, ou un groupe hétéropolycyclique monovalent non-aromatique condensé, chacun non substitué ou substitué par $R_1$ dans le nombre de a1,

dans la Formule 2, $R_1$ et $R_2$ sont chacun indépendamment :

hydrogène, deutérium, -F, -Cl, -Br, -I, un groupe cyano ou -$SF_5$ ; ou

un groupe $C_1$-$C_{60}$ alkyle, un groupe $C_1$-$C_{60}$ alcoxy, un groupe $C_3$-$C_{10}$ cycloalkyle, un groupe $C_2$-$C_{10}$ hétérocycloalkyle, un groupe $C_3$-$C_{10}$ cycloalcényle, ou un groupe $C_2$-$C_{10}$ hétérocycloalcényle, chacun non substitué ou substitué par le deutérium, -F, -Cl, -Br, -I, -$CD_3$, - $CD_2H$, -$CDH_2$, -$CF_3$, -$CF_2H$, -$CFH_2$, un groupe cyano, un groupe $C_1$-$C_{20}$ alkyle, un groupe cyclopentyle, un groupe cyclohexyle, un groupe cycloheptyle, un groupe cyclooctyle, un groupe adamantyle, un groupe norbornyle, un groupe norboményle, un groupe cyclopentényle, un groupe cyclohexényle, un groupe cycloheptényle, un groupe phényle, un groupe biphényle, un groupe naphtyle, un groupe pyridinyle, un groupe pyrimidinyle, ou une combinaison quelconque de ceux-ci, et

a1 est un entier de 0 à 10.

**5.** Composé organométallique de l'une quelconque des revendications 1 à 4, dans lequel

un groupe représenté par

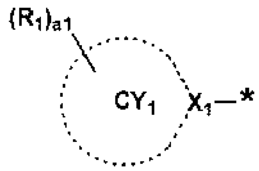

dans la Formule 2 est un groupe phényle, un groupe biphényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, ou un groupe imidazopyrimidinyle, chacun non substitué ou substitué par $R_1$ dans le nombre de a1,

dans la Formule 2, $R_1$ et $R_2$ sont chacun indépendamment :

hydrogène, deutérium, -F, -Cl, -Br, -I, un groupe cyano, ou -$SF_5$ ; ou

un groupe méthyle, un groupe éthyle, un groupe propyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe n-hexyle, un groupe isohexyle, un groupe sec-hexyle, un groupe tert-hexyle, un groupe n-heptyle, un groupe isoheptyle, un groupe sec-heptyle, un groupe tert-heptyle, un groupe n-octyle, un groupe isooctyle, un groupe sec-octyle, un groupe tert-octyle, un groupe n-nonanyle, un groupe isononanyle, un groupe sec-nonanyle, un groupe tert-nonanyle, un groupe n-décanyle, un groupe isodécanyle, un groupe sec-décanyle, un groupe tert-décanyle, un groupe $C_1$-$C_{10}$ alcoxy, un groupe cyclopentyle,

un groupe cyclohexyle, un groupe cycloheptyle, un groupe cycloctyle, un groupe adamantyle, un groupe norbornyle, un groupe norbornényle, un groupe cyclopentényle, un groupe cyclohexényle, ou un groupe cycloheptényle, chacun non substitué ou substitué par le deutérium, -F, -Cl, -Br, -I, $-CD_3$, $-CD_2H$, $-CDH_2$, $-CF_3$, $-CF_2H$, $-CFH_2$, un groupe cyano, un groupe $C_1$- $C_{20}$ alkyle, un groupe cyclopentyle, un groupe cyclohexyle, un groupe cycloheptyle, un groupe cyclooctyle, un groupe adamantyle, un groupe norbornyle, un groupe norbornényle, un groupe cyclopentényle, un groupe cyclohexényle, un groupe cycloheptényle, un groupe phényle, un groupe biphényle, un groupe naphthyle, un groupe pyridinyle, un groupe pyrimidinyle, ou une combinaison quelconque de ceux-ci, et

a1 est un entier de 0 à 5.

**6.** Composé organométallique de l'une quelconque des revendications 1 à 5, dans lequel un groupe représenté par

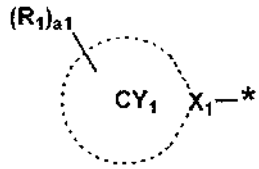

est un groupe représenté par une des Formules 10-13(1) à 10-13(18) et 10-13 :

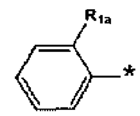
10-13(1)

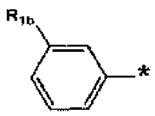
10-13(2)

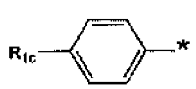
10-13(3)

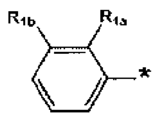
10-13(4)

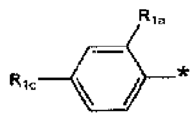
10-13(5)

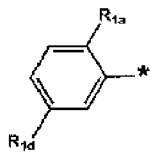
10-13(6)

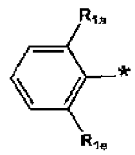
10-13(7)

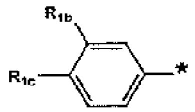
10-13(8)

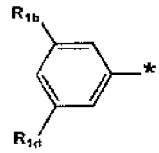
10-13(9)

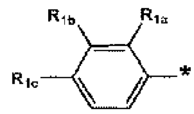
10-13(10)

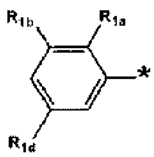
10-13(11)

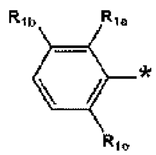
10-13(12)

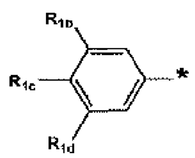
10-13(13)

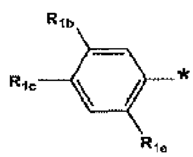
10-13(14)

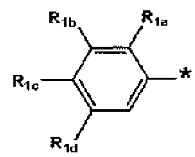
10-13(15)

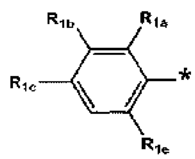
10-13(16)

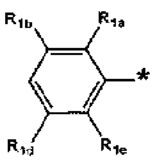
10-13(17)

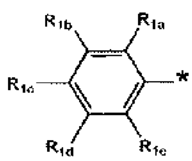
10-13(18)

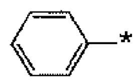
10-13 ,

avec, dans les Formules 10-13(1) à 10-13(18) et 10-13, $R_{1a}$ à $R_{1e}$ sont chacun indépendamment les mêmes que ceux décrits en connexion avec $R_1$ dans la revendication 1, $R_{1a}$ à $R_{1e}$ chacun n'étant pas l'hydrogène, et * indique un site de liaison à un atome voisin.

**7.** Composé organométallique de l'une quelconque des revendications 1 à 6, dans lequel un groupe représenté par

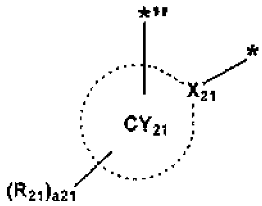

dans la Formule 2 est un groupe représenté par une des Formules un de CY21-1 à CY21-25 :

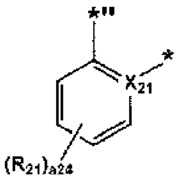
CY21-1

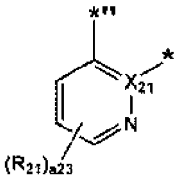
CY21-2

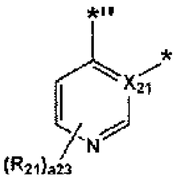
CY21-3

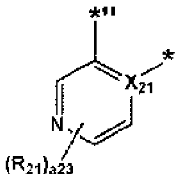
CY21-4

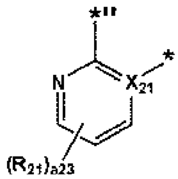
CY21-5

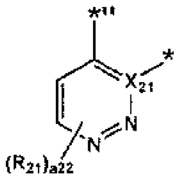
CY21-6

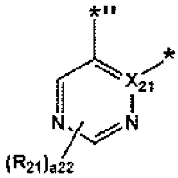
CY21-7

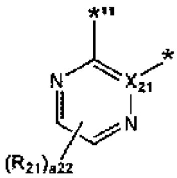
CY21-8

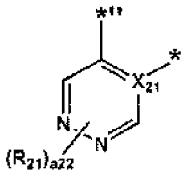
CY21-9

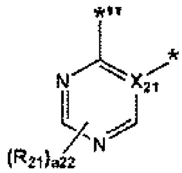
CY21-10

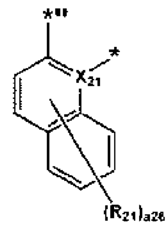
CY21-11

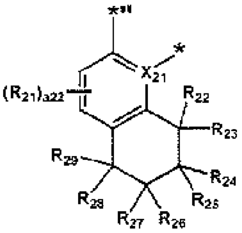
CY21-12

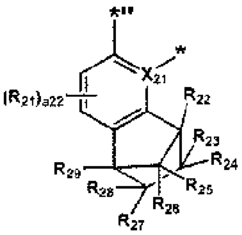
CY21-13

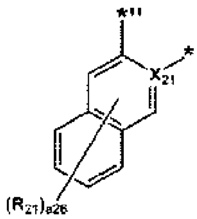
CY21-14

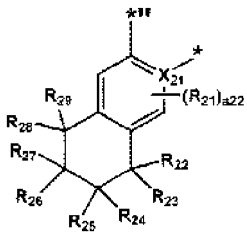
CY21-15

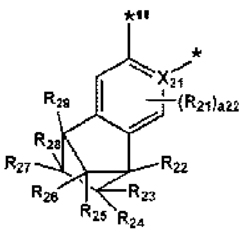
CY21-16

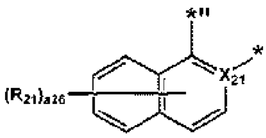
CY21-17

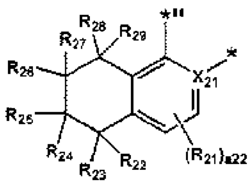
CY21-18

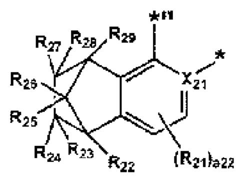
CY21-19

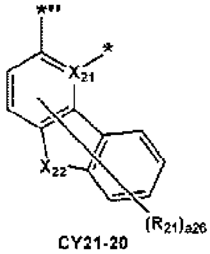
CY21-20

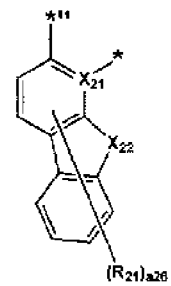
CY21-21

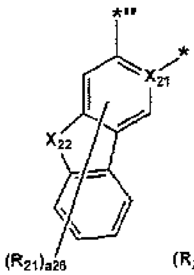
CY21-22

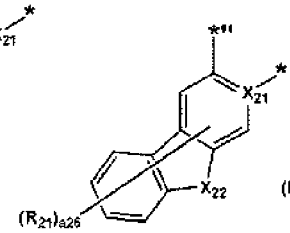
CY21-23

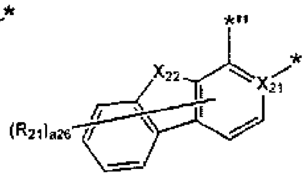
CY21-24

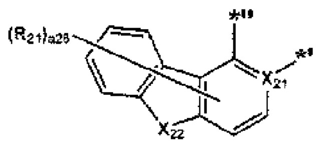

CY21-25

avec, dans les Formules CY21-1 to CY21-25,

$X_{21}$ et $R_{21}$ sont chacun indépendamment les mêmes que ceux décrits dans la revendication 1,
$X_{22}$ est $C(R_{22})(R_{23})$, $N(R_{22})$, O, S, ou $Si(R_{22})(R_{23})$,
$R_{22}$ à $R_{29}$ sont chacun indépendamment les mêmes que ceux définis en connexion avec $R_{21}$ dans la revendication 1,
a26 est un entier de 0 à 6,
a24 est un entier de 0 à 4,
a23 est un entier de 0 à 3,
a22 est un entier de 0 à 2,
*" indique un site de liaison avec un atome de carbone d'un anneau à 6 chaînons voisin dans la Formule 2, et
* indique un site de liaison à M dans la Formule 1 ; de préférence dans lequel
un groupe représenté par

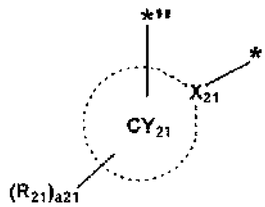

dans la Formule 2 est un groupe représenté par une des Formules CY21(1) à CY21(56) ou un groupe représenté par une des Formules CY21-20 à CY21-26 :

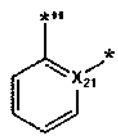
CY21(1)

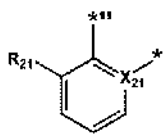
CY21(2)

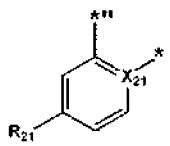
CY21(3)

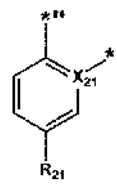
CY21(4)

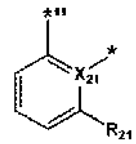
CY21(5)

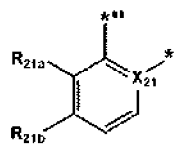
CY21(6)

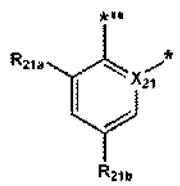
CY21(7)

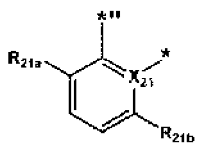
CY21(8)

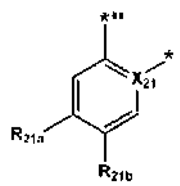
CY21(9)

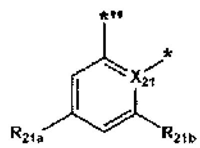
CY21(10)

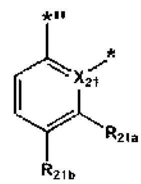
CY21(11)

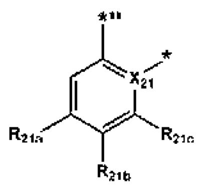
CY21(12)

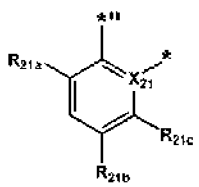
CY21(13)

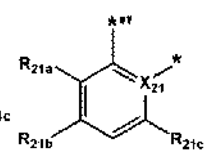
CY21(14)

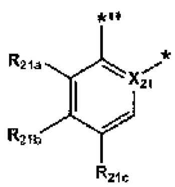
CY21(15)

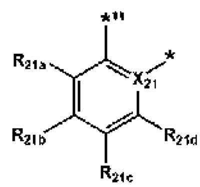
CY21(16)

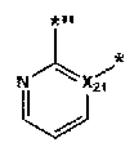
CY21(17)

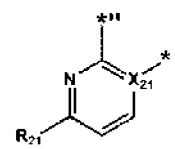
CY21(18)

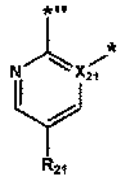
CY21(19)

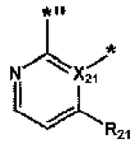
CY21(20)

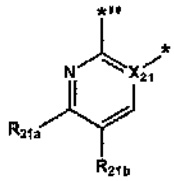
CY21(21)

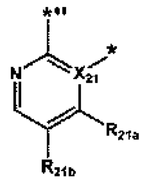
CY21(22)

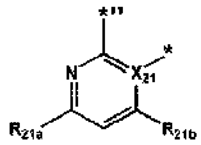
CY21(23)

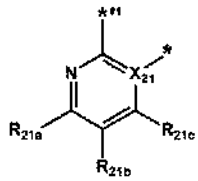
CY21(24)

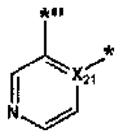
CY21(25)

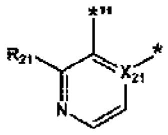
CY21(26)

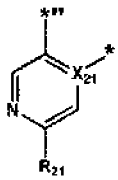
CY21(27)

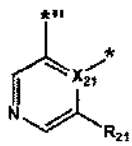
CY21(28)

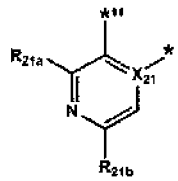
CY21(29)

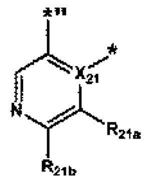
CY21(30)

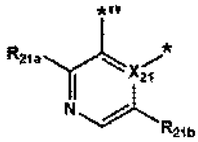
CY21(31)

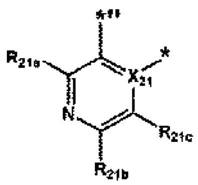
CY21(32)

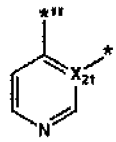
CY21(33)

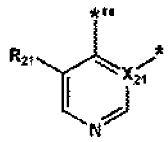
CY21(34)

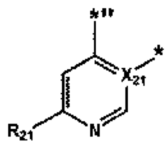
CY21(35)

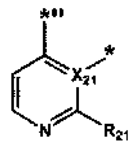
CY21(36)

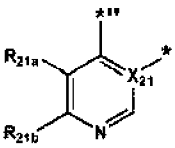
CY21(37)

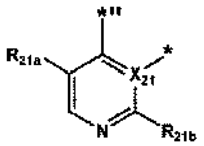
CY21(38)

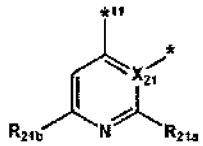
CY21(39)

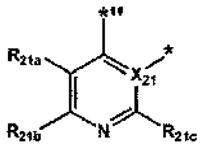
CY21(40)

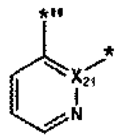
CY21(41)

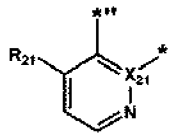
CY21(42)

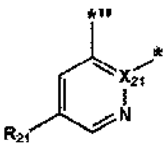
CY21(43)

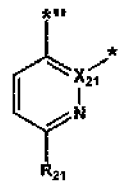
CY21(44)

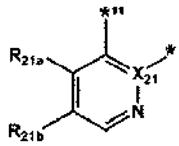
CY21(45)

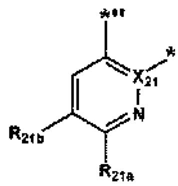
CY21(46)

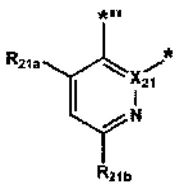
CY21(47)

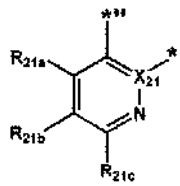
CY21(48)

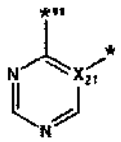
CY21(49)

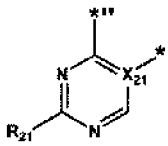
CY21(50)

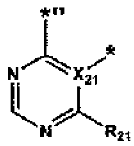
CY21(51)

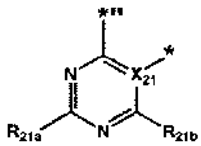
CY21(52)

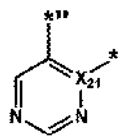
CY21(53)

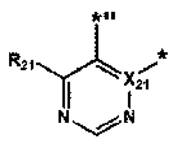
CY21(54)

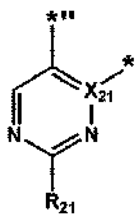
CY21(55)

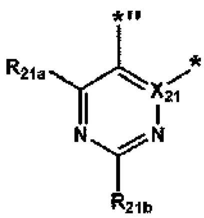
CY21(56)

,

avec, dans les Formules CY21(1) à CY21(56),

$X_{21}$ et $R_{21}$ sont chacun indépendamment les mêmes que ceux décrits dans la revendication 1,
$R_{21a}$ à $R_{21d}$ sont chacun indépendamment les mêmes que ceux définis en connexion avec $R_{21}$ dans la revendication 1, chaque $R_{21}$ et $R_{21a}$ à $R_{21d}$ n'étant pas l'hydrogène,
*'' indique un site de liaison avec un atome de carbone d'un anneau à 6 chaînons voisin dans la Formule 2, et
* indique un site de liaison à M dans la Formule 1.

**8.** Composé organométallique de l'une quelconque des revendications 1 à 7, dans lequel $L_1$ est un ligand représenté par la Formule 2A ou 2B :

Formule 2A

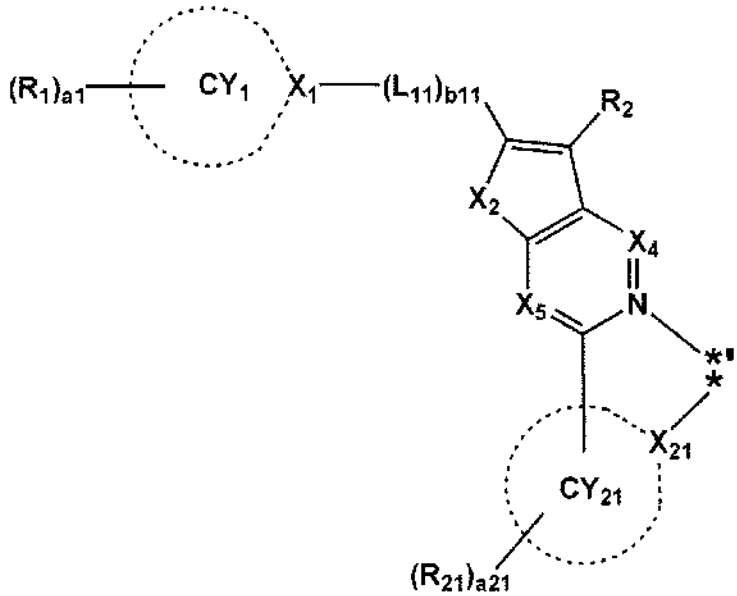

Formule 2B

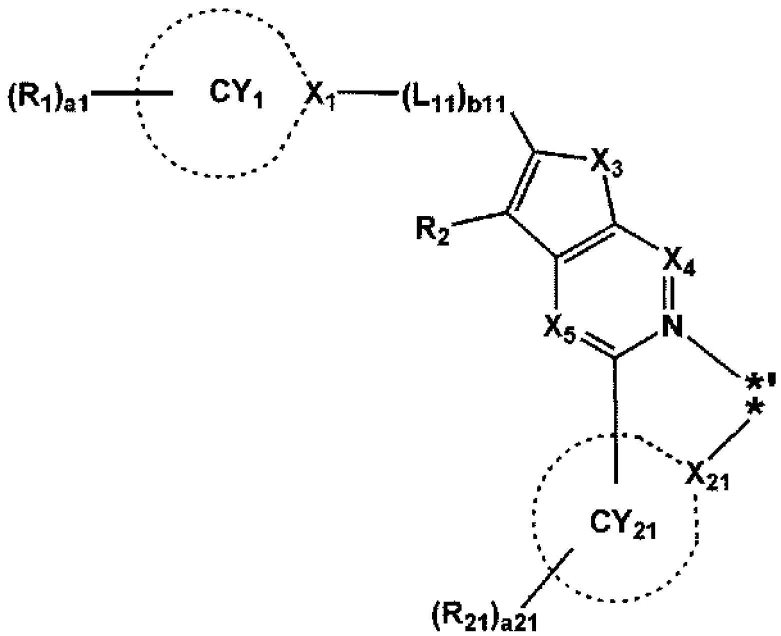

,

avec, dans les Formules 2A et 2B, $X_1$, $X_{21}$, l'anneau $CY_1$, l'anneau $CY_{21}$, $X_4$, $X_5$, $R_1$, $R_2$, $R_{21}$, a1,a21, $L_{11}$, b11, *, et *' sont chacun indépendamment les mêmes que ceux décrits dans la revendication 1, chaque $X_2$ and $X_3$ étant chacun indépendamment O, S, ou Se.

9. Composé organométallique de l'une quelconque des revendications 1 à 8, dans lequel dans la Formule 1, $L_2$ est un groupe représenté par une des Formules 3A à 3D :

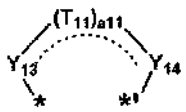

3A

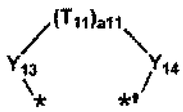

3B

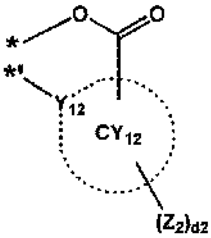

3C

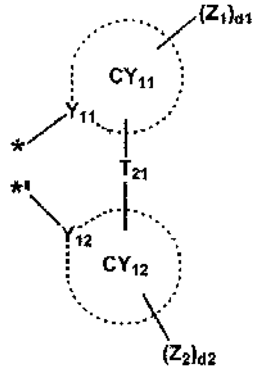

3D

,

avec, dans les Formules 3A à 3D,

$Y_{13}$ est O, N, ou $N(Z_1)$,
$Y_{14}$ est O, N, ou $N(Z_3)$,
$T_{11}$ est une liaison simple, une double liaison, *-$C(Z_{11})(Z_{12})$-*', *-$C(Z_{11})$=$C(Z_{12})$-*', *=$C(Z_{11})$-*', -*$C(Z_{11})$=*', *-$C(Z_{11})$-$C(Z_{12})$=$C(Z_{13})$-*', *-$C(Z_{11})$=$C(Z_{12})$-$C(Z_{13})$=*', *-$N(Z_{11})$-*', ou un groupe $C_5$-$C_{30}$ carbocyclique non substitué ou substitué par au moins un $Z_{11}$,
a11 est un entier de 1 à 10,
$Y_{11}$ et $Y_{12}$ sont chacun indépendamment C ou N,
$T_{21}$ est une liaison simple, une double liaison, O, S, $C(Z_{11})(Z_{12})$, $Si(Z_{11})(Z_{12})$, or $N(Z_{11})$,
l'anneau $CY_{11}$ et l'anneau $CY_{12}$ sont chacun indépendamment un groupe $C_5$-$C_{30}$ carbocyclique ou un groupe $C_2$-$C_{30}$ hétérocyclique,
$Z_1$ à $Z_3$ et $Z_{11}$ à $Z_{13}$ sont chacun indépendamment les mêmes que ceux définis en connexion avec $R_{21}$ dans la revendication 1,
d1 et d2 sont chacun indépendamment un entier de 0 à 10, et
* et *' indiquent chacun un site de liaison à M dans la Formule 1.

10. Composé organométallique de l'une quelconque des revendications 1 à 9, dans lequel, dans la Formule 1, $L_2$ est un groupe représenté par les Formules un de 3-1(1) à 3-1(66), 3-1(301) et 3-1(308) :

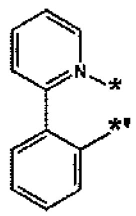

3-1(1)

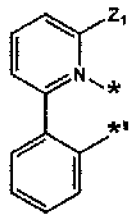

3-1(2)

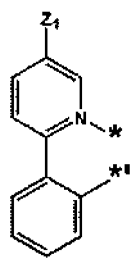

3-1(3)

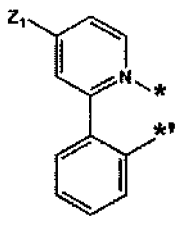

3-1(4)

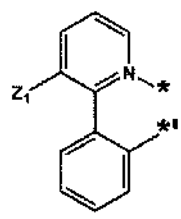

3-1(5)

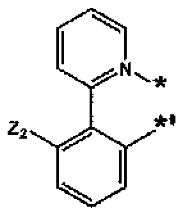
3-1(6)

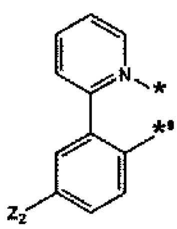
3-1(7)

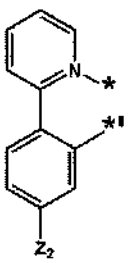
3-1(8)

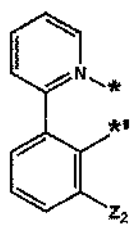
3-1(9)

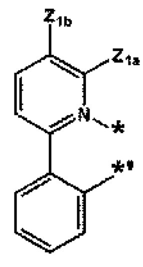
3-1(10)

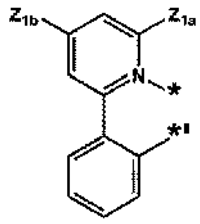
3-1(11)

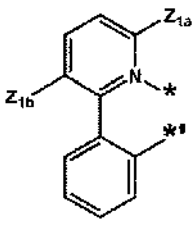
3-1(12)

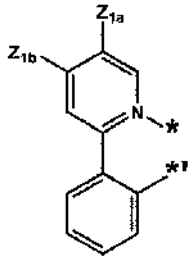
3-1(13)

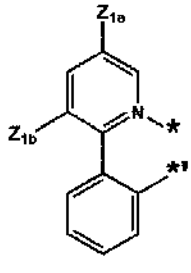
3-1(14)

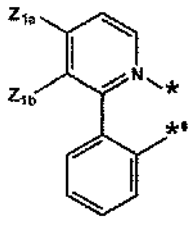
3-1(15)

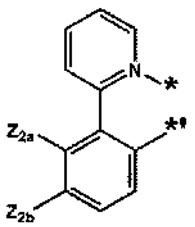
3-1(16)

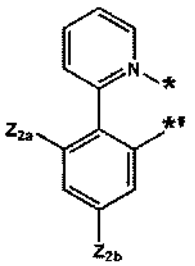
3-1(17)

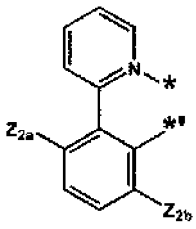
3-1(18)

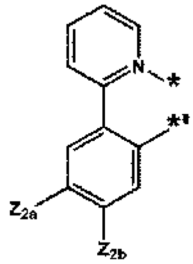
3-1(19)

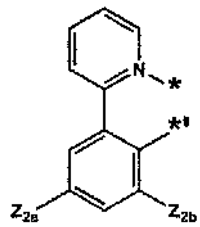
3-1(20)

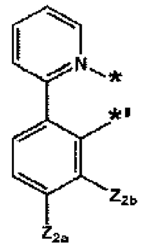
3-1(21)

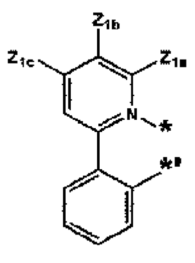
3-1(22)

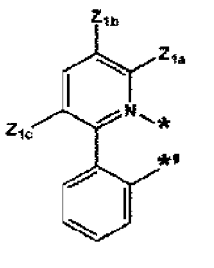
3-1(23)

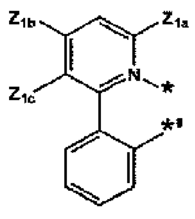
3-1(24)

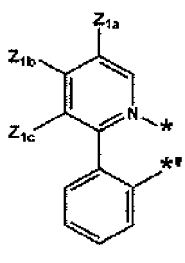
3-1(25)

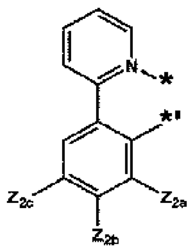
3-1(26)

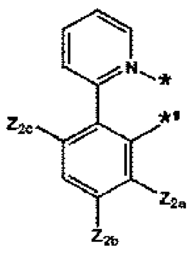
3-1(27)

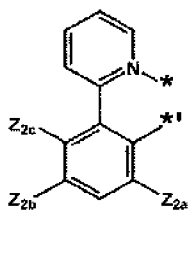
3-1(28)

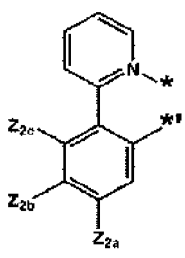
3-1(29)

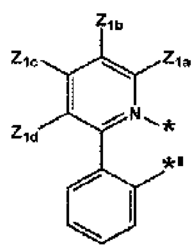
3-1(30)

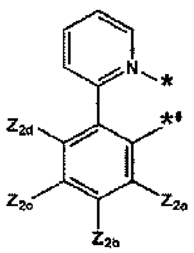
3-1(31)

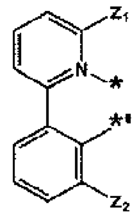
3-1(32)

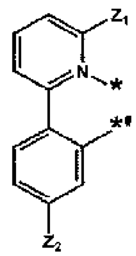
3-1(33)

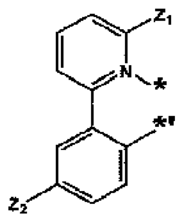
3-1(34)

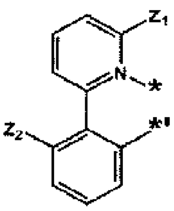
3-1(35)

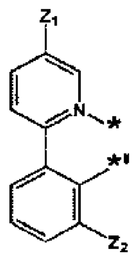
3-1(36)

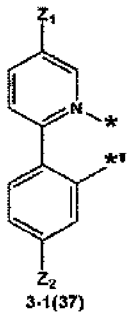
3-1(37)

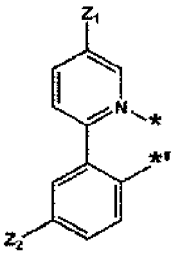
3-1(38)

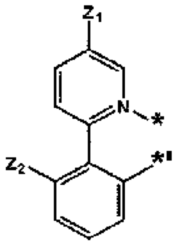
3-1(39)

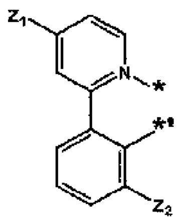
3-1(40)

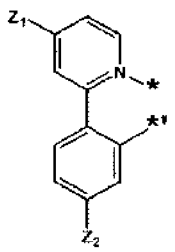
3-1(41)

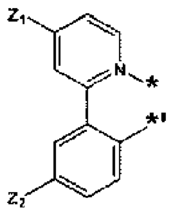
3-1(42)

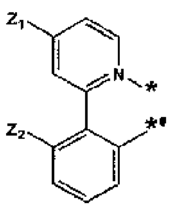
3-1(43)

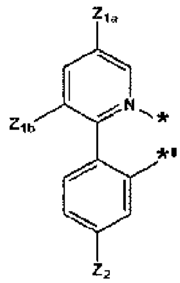
3-1(44)

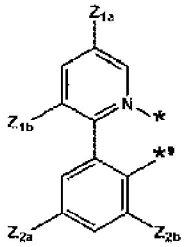
3-1(45)

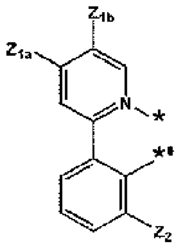
3-1(46)

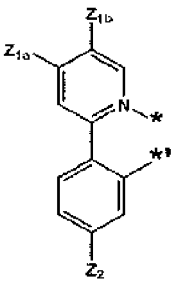
3-1(47)

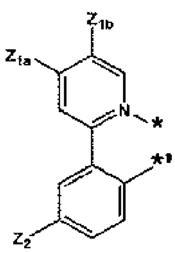
3-1(48)

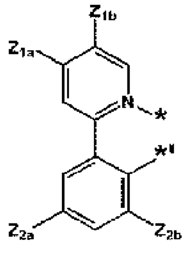
3-1(49)

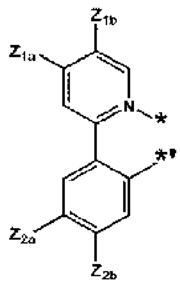
3-1(50)

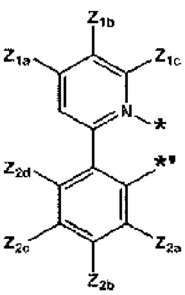
3-1(51)

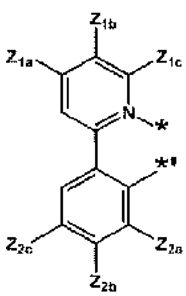
3-1(52)

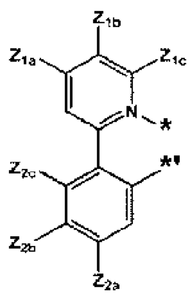
3-1(53)

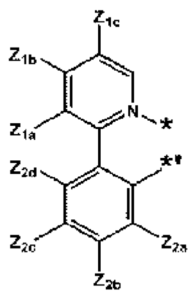
3-1(54)

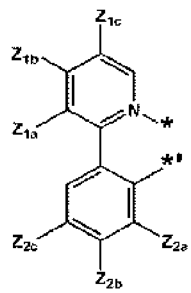
3-1(55)

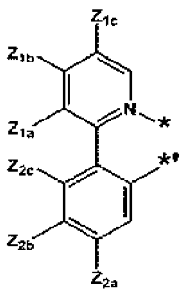
3-1(56)

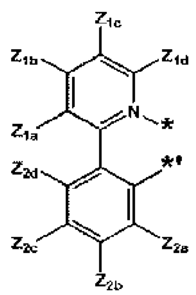
3-1(57)

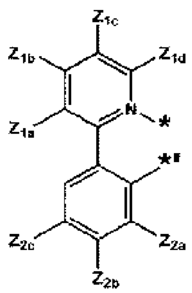
3-1(58)

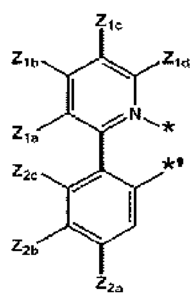
3-1(59)

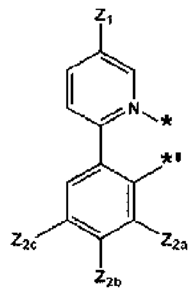
3-1(60)

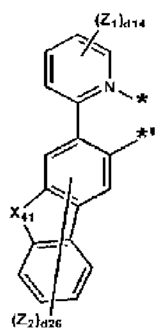
3-1(61)

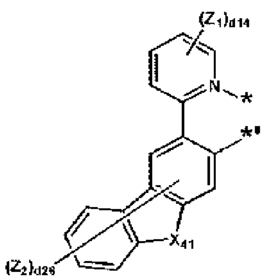
3-1(62)

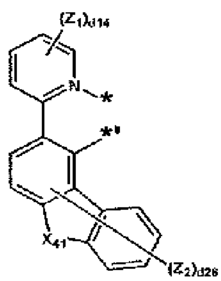
3-1(63)

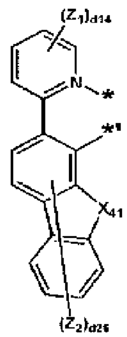
3-1(64)

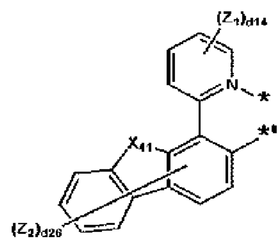
3-1(65)

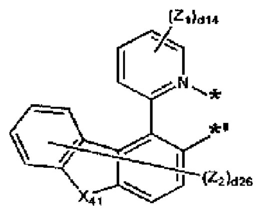
3-1(66)

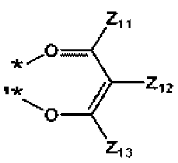
3-1(301)

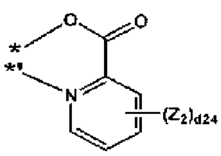
3-1(308)

avec, dans les Formules 3-1(1) à 3-1(66), 3-1(301) et 3-1(308),

$X_{41}$ est O, S, $N(Z_{21})$, $C(Z_{21})(Z_{22})$, ou $Si(Z_{21})(Z_{22})$,
$Z_1$, $Z_2$, $Z_{1a}$, $Z_{1b}$, $Z_{1c}$, $Z_{1d}$, $Z_{2a}$, $Z_{2b}$, $Z_{2c}$, $Z_{2d}$, $Z_{11}$ à $Z_{13}$, $Z_{21}$ et $Z_{22}$ sont chacun indépendamment les mêmes que ceux définis en connexion avec $R_{21}$ dans la revendication 1,
d14 est un entier de 0 à 4,
d26 est un entier de 0 à 6, et
* et *' indiquent chacun un site de liaison à M dans la Formule 1.

**11.** Composé organométallique de la revendication 1, dans lequel le composé organométallique est un des composés 1 à 15, 17-218, 220-328, 330-438, 440-453, 455-466, 468 et 469 :

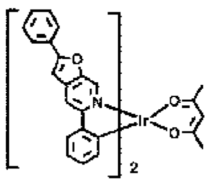
1

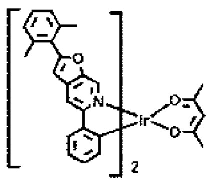
2

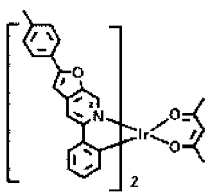
3

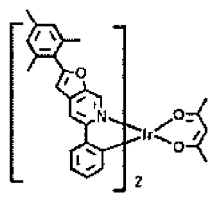
4

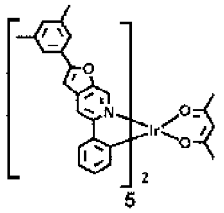
5

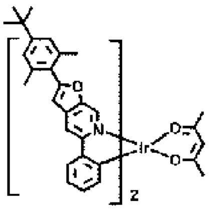
6

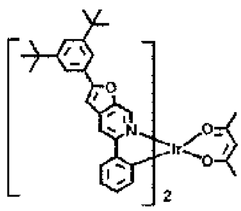
7

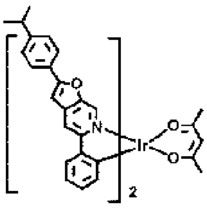
8

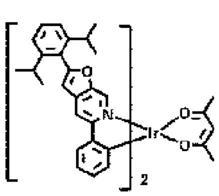
9

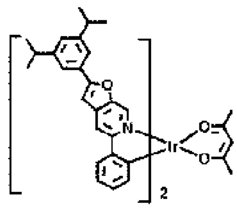
10

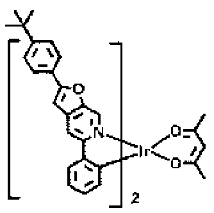
11

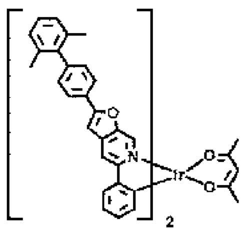
12

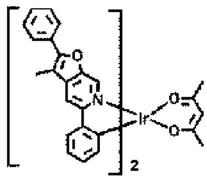
13

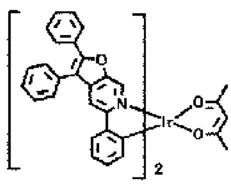
14

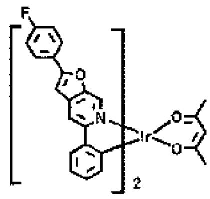
15

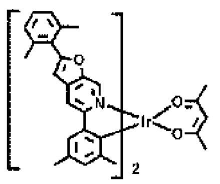
17

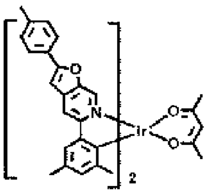
18

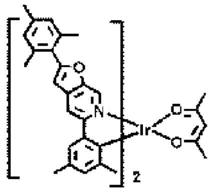
19

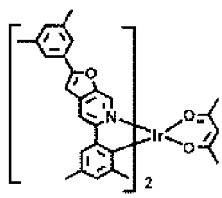
20

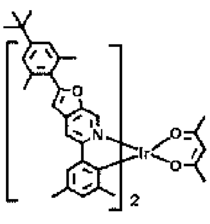
21

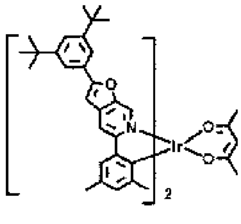
22

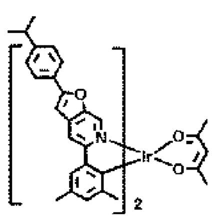
23

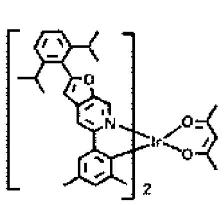
24

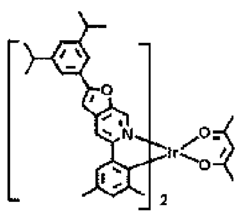
25

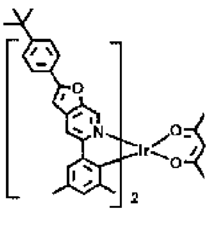

26

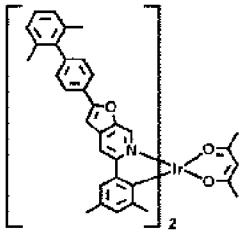

27

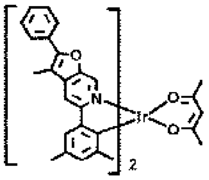

28

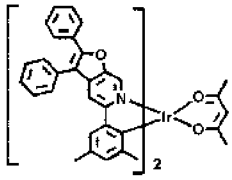

29

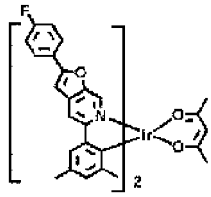

30

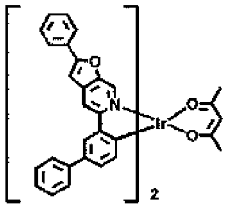

31

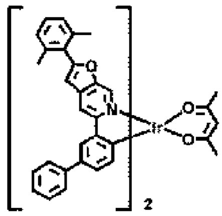

32

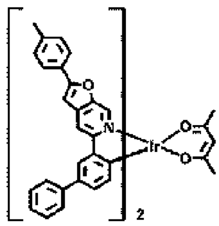

33

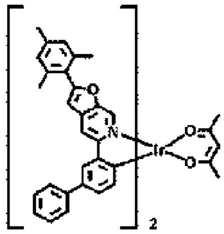

34

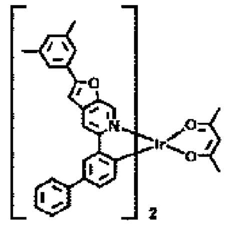

35

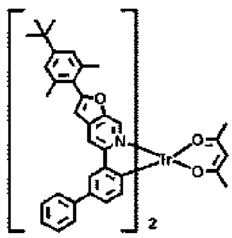

36

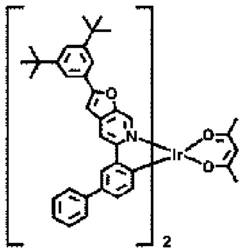

37

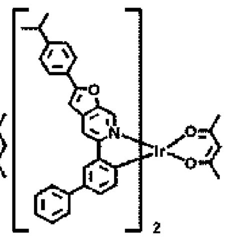

38

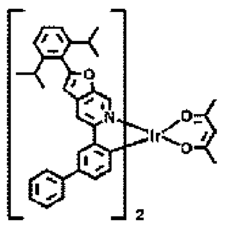

39

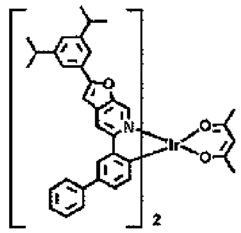

40

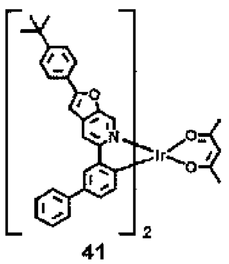

41

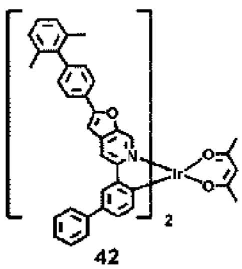

42

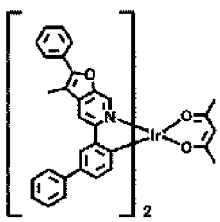

43

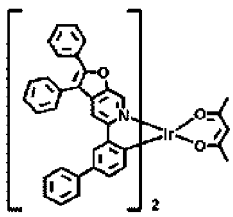

44

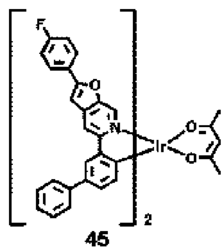

45

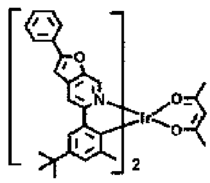

46

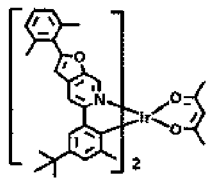

47

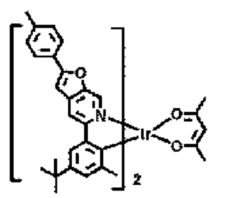

48

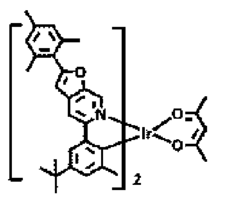

49

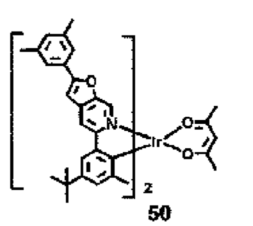

50

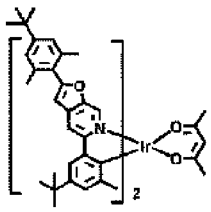
51
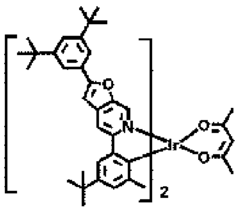
52
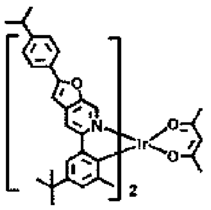
53
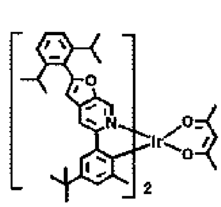
54
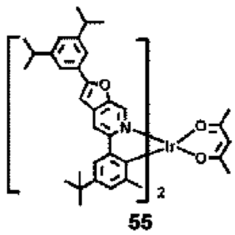
55

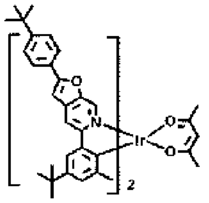
56
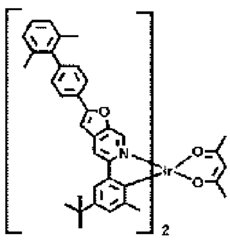
57
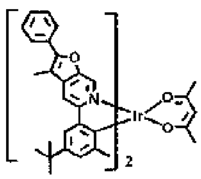
58
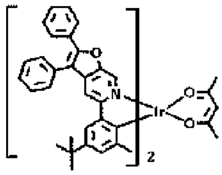
59
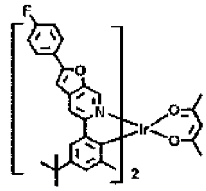
60

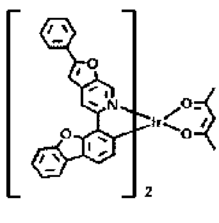
61
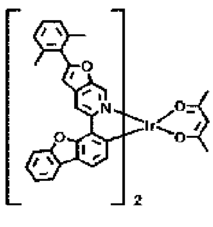
62
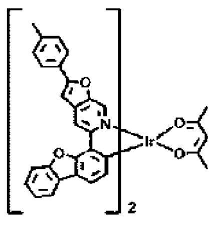
63
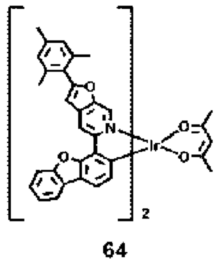
64
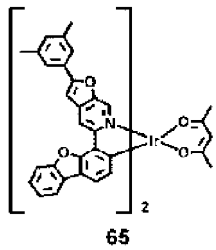
65

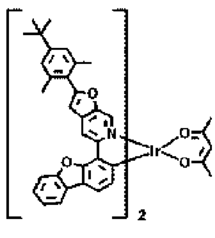
66
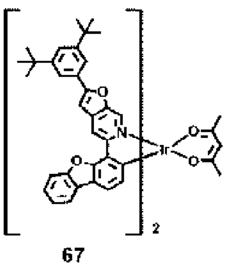
67
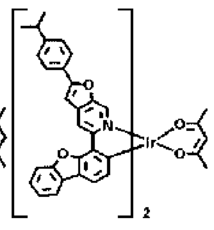
68
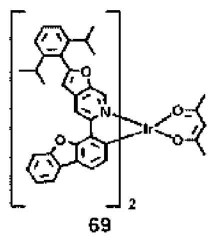
69
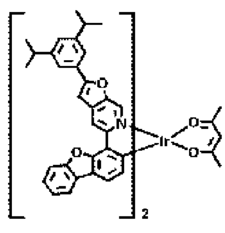
70

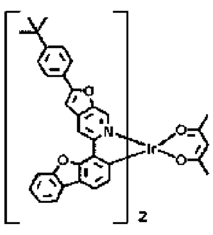
71
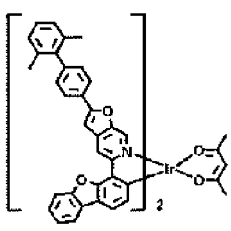
72
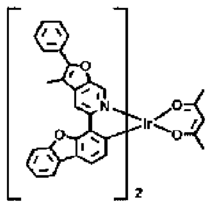
73
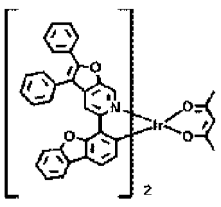
74
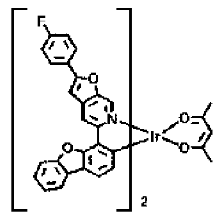
75

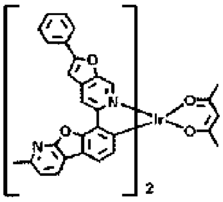
76

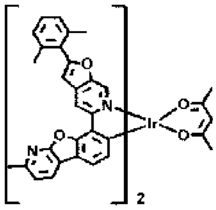
77

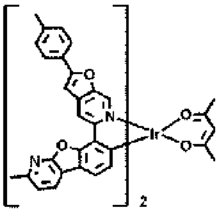
78

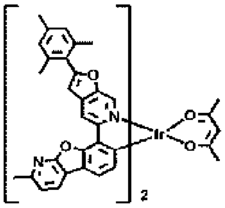
79

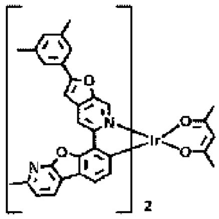
80

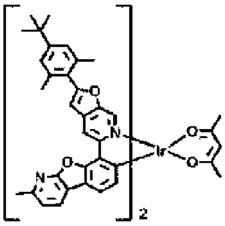
81

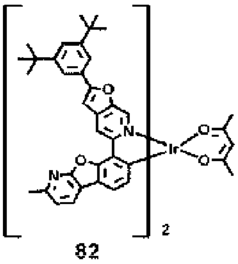
82

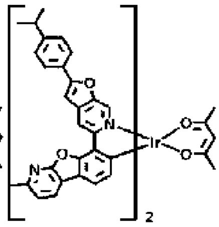
83

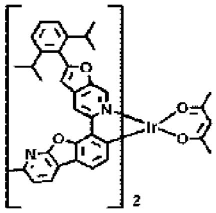
84

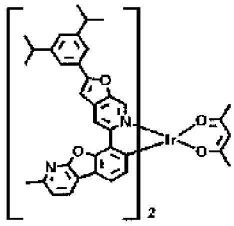
85

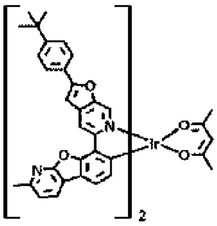
86

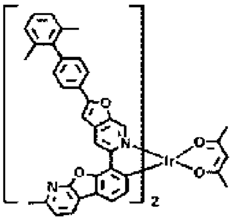
87

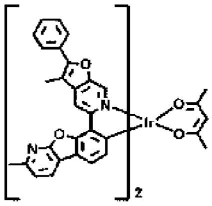
88

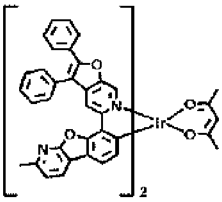
89

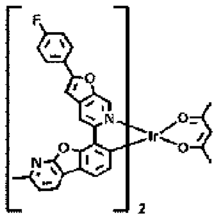
90

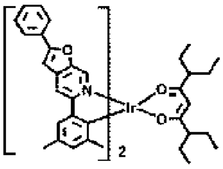
91

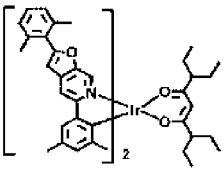
92

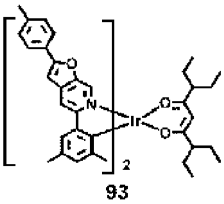
93

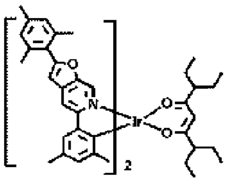
94

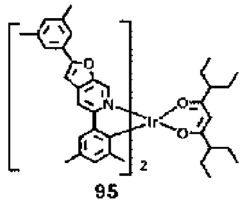
95

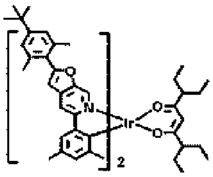
96

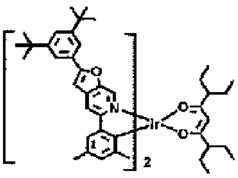
97

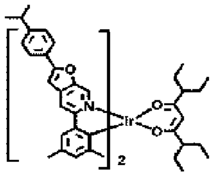
98

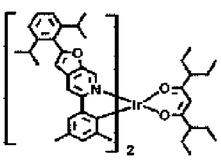
99

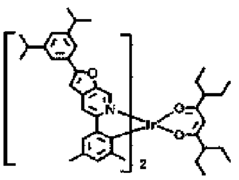
100

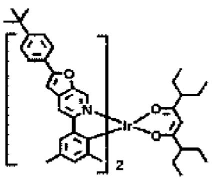
101

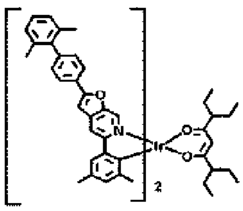
102

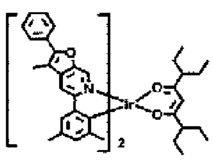
103

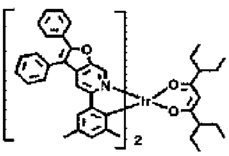
104

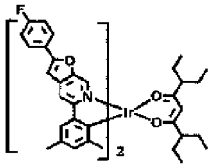
105

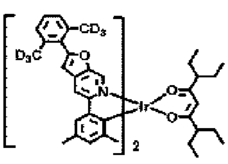
106

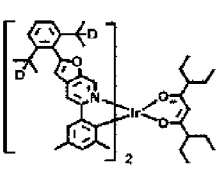
107

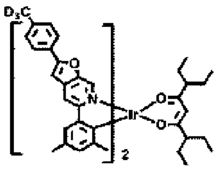
108

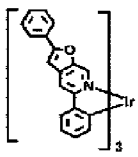
109

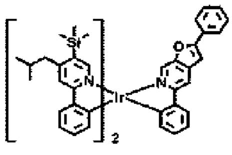
110

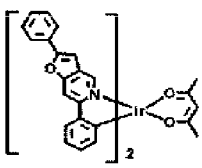
111

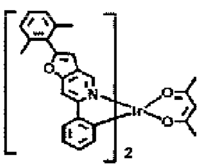
112

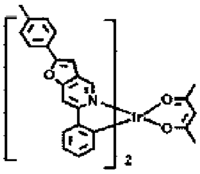
113

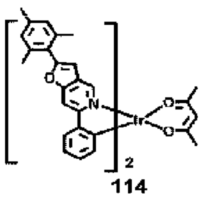
114

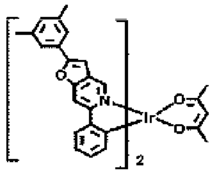
115

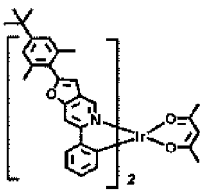
116

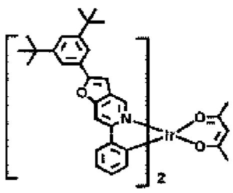
117

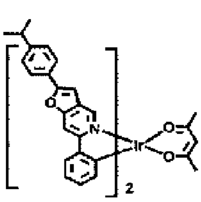
118

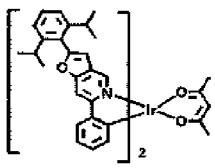
119

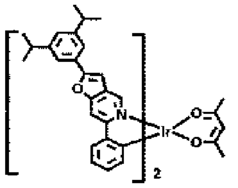
120

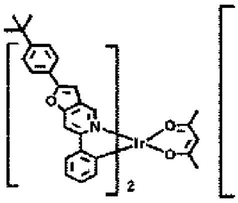
121

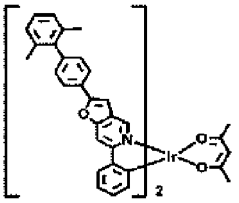
122

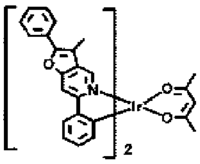
123

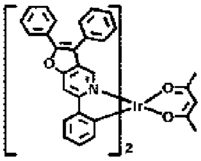
124

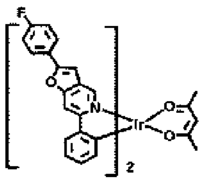
125

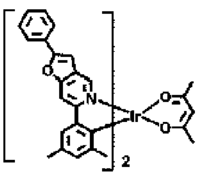
126

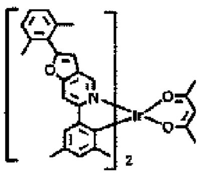
127

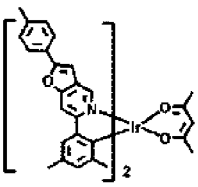
128

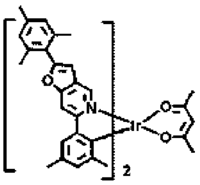
129

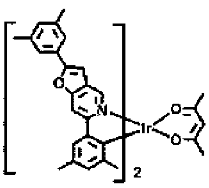
130

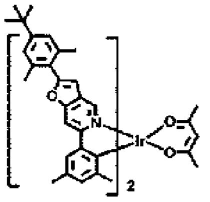
131

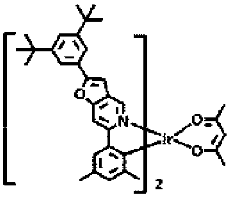
132

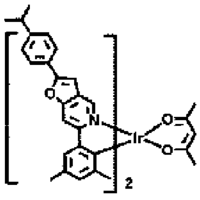
133

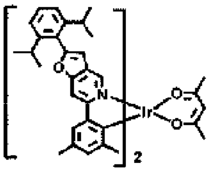
134

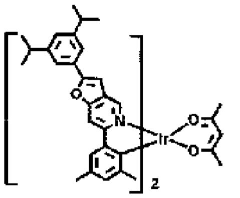
135

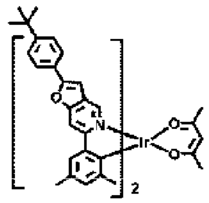 136 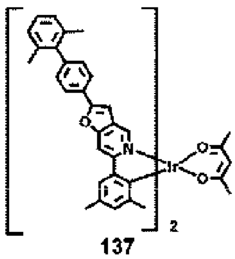 137 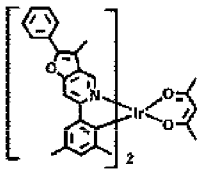 138 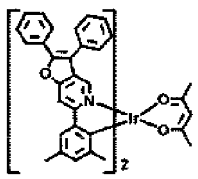 139 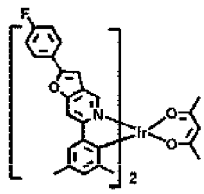 140

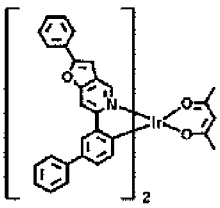 141 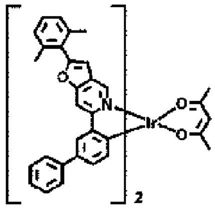 142 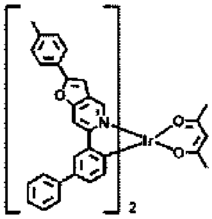 143 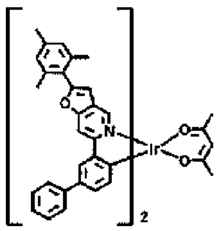 144 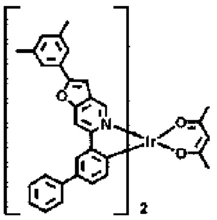 145

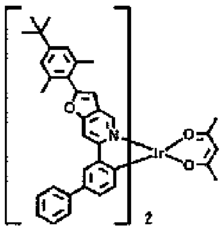 146 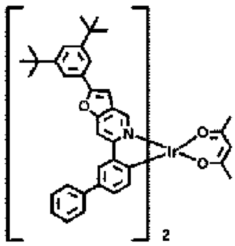 147 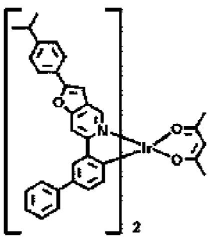 148 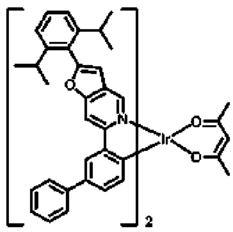 149 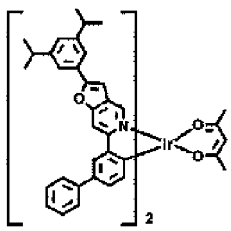 150

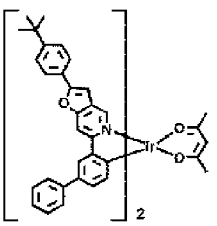 151 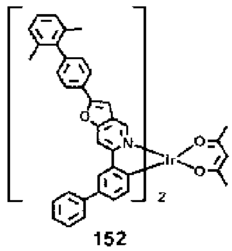 152 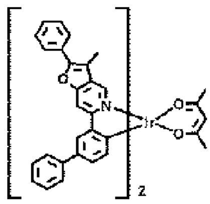 153 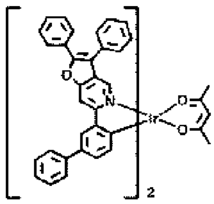 154 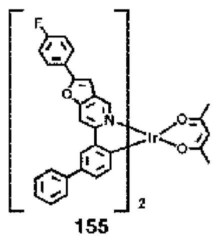 155

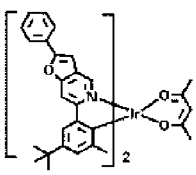 156 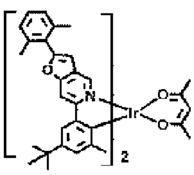 157 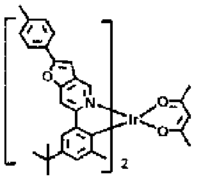 158 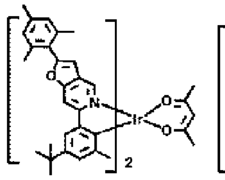 159 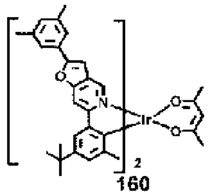 160

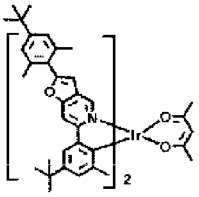 161 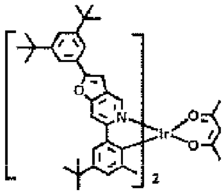 162 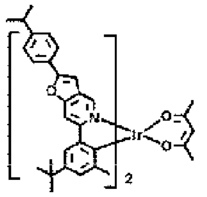 163 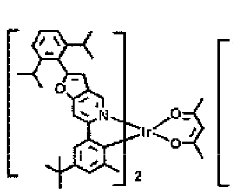 164 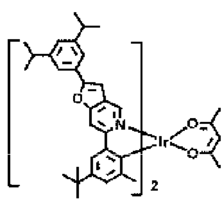 165

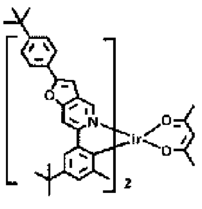
166

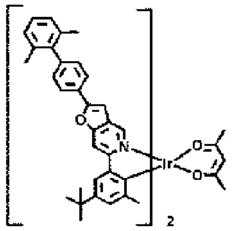
167

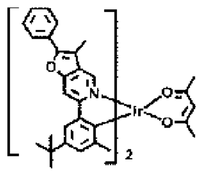
168

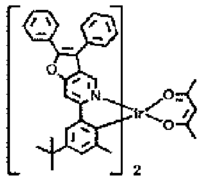
169

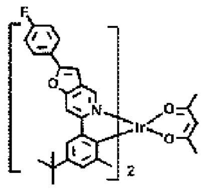
170

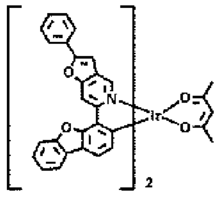
171

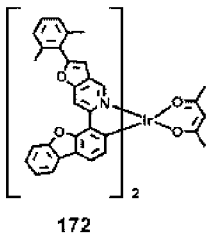
172

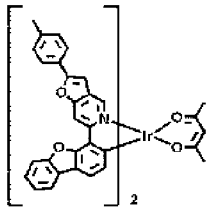
173

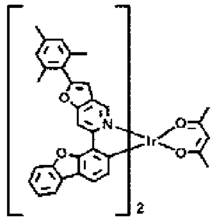
174

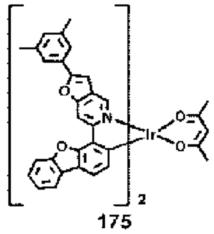
175

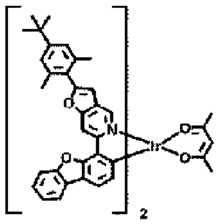
176

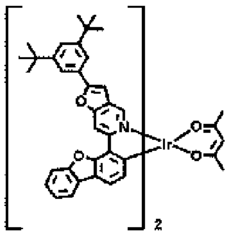
177

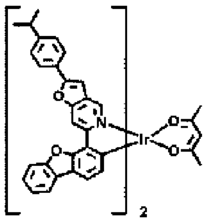
178

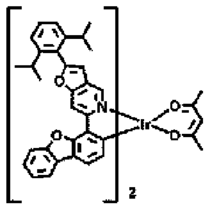
179

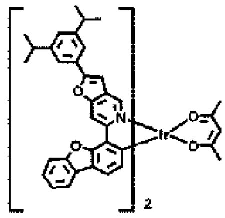
180

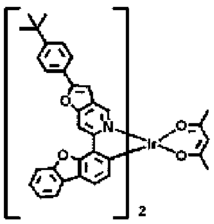
181

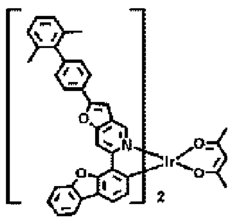
182

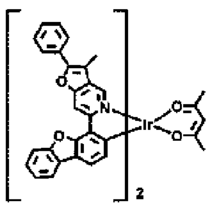
183

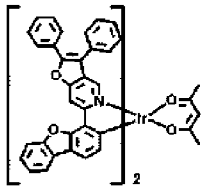
184

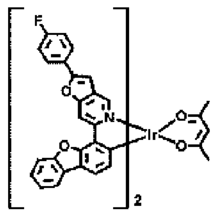
185

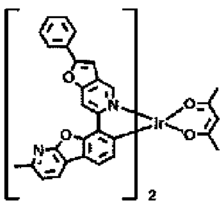
186

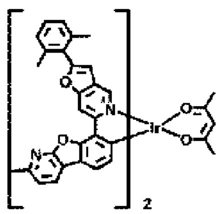
187

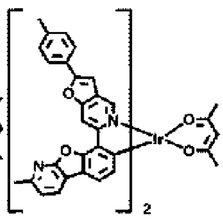
188

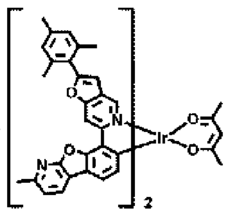
189

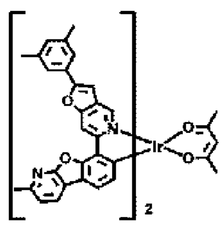
190

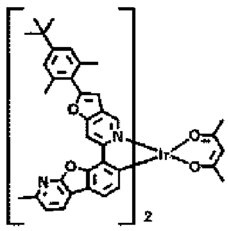
191

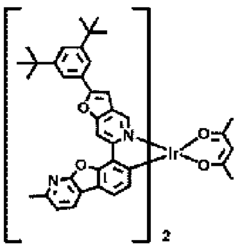
192

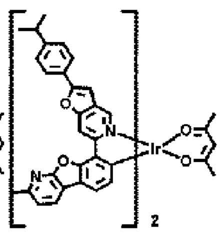
193

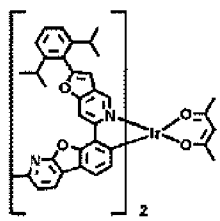
194

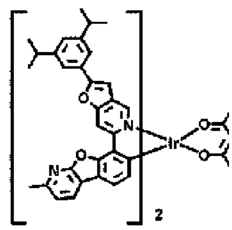
195

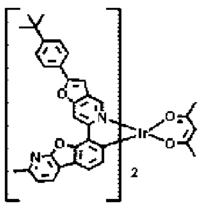
196

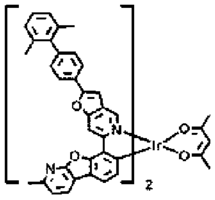
197

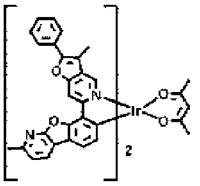
198

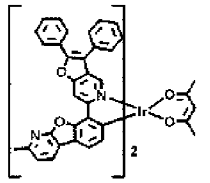
199

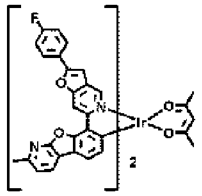
200

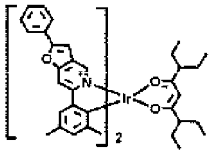
201

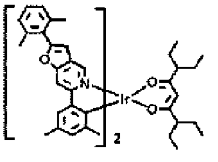
202

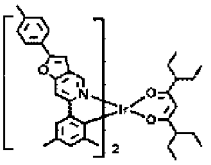
203

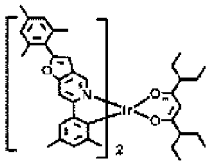
204

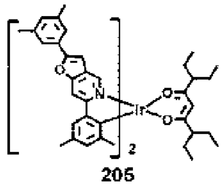
205

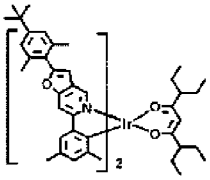
206

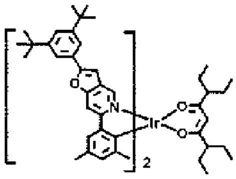
207

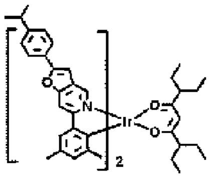
208

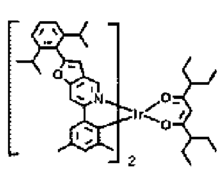
209

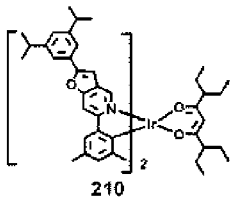
210

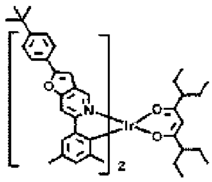
211

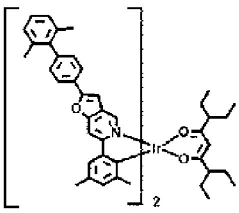
212

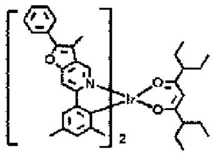
213

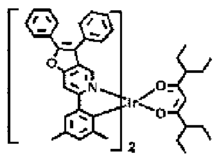
214

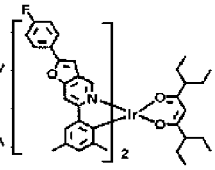
215

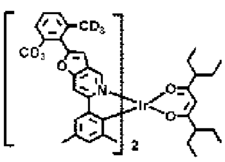
216

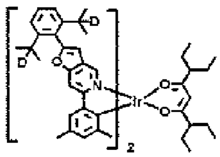
217

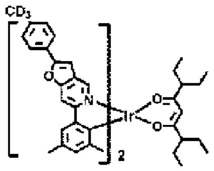
218

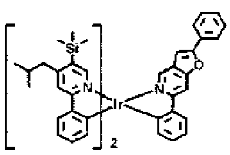
220

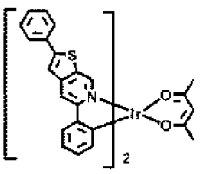
221

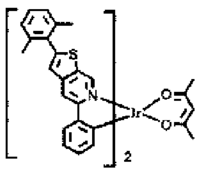
222

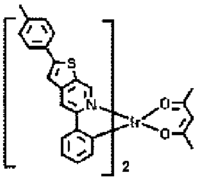
223

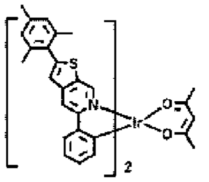
224

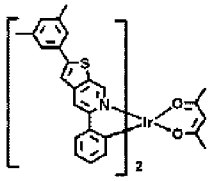
225

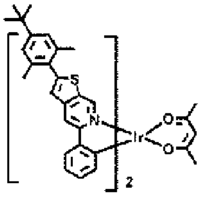
226

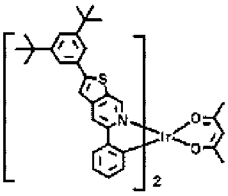
227

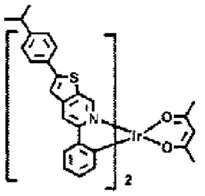
228

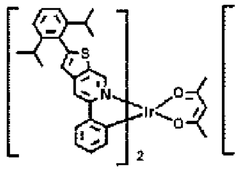
229

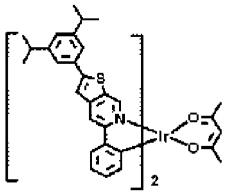
230

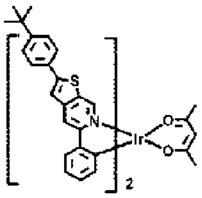
231

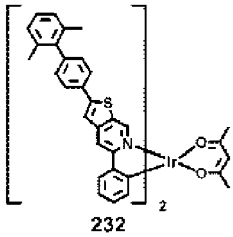
232

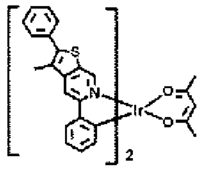
233

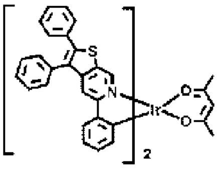
234

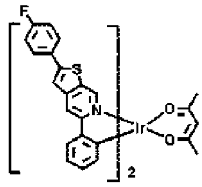
235

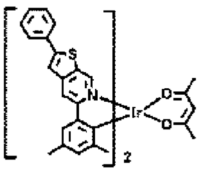
236

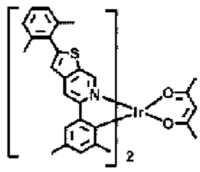
237

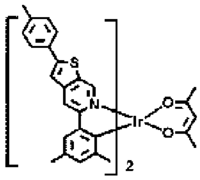
238

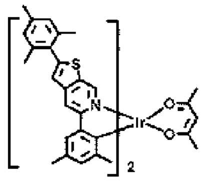
239

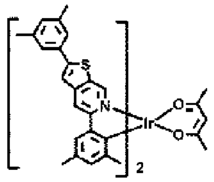
240

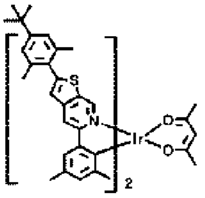
241

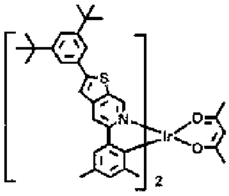
242

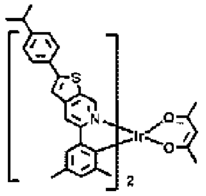
243

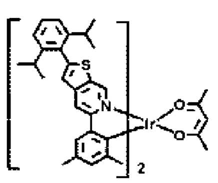
244

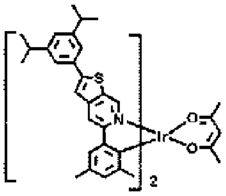
245

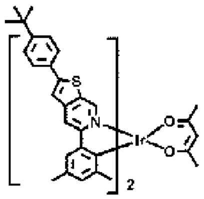
246

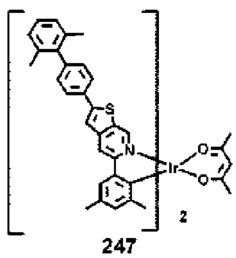
247

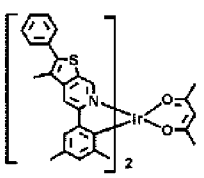
248

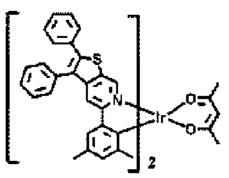
249

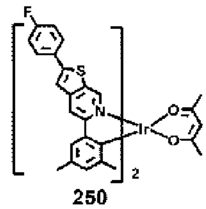
250

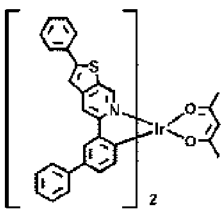
251

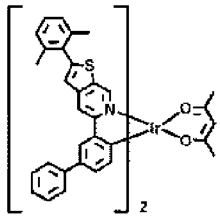
252

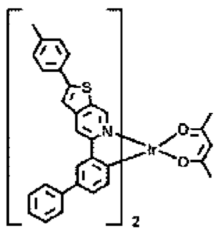
253

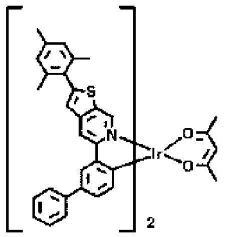
254

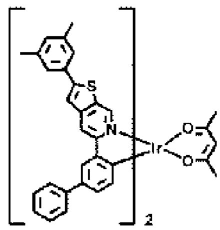
255

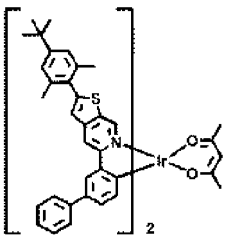
256

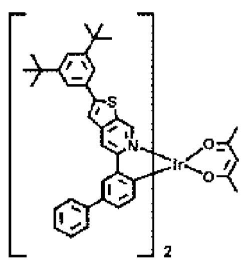
257

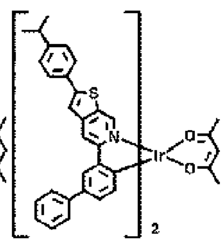
258

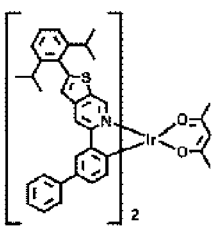
259

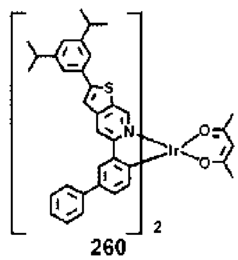
260

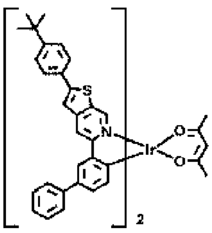
261
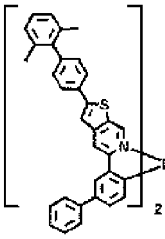
262
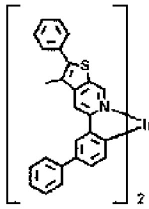
263
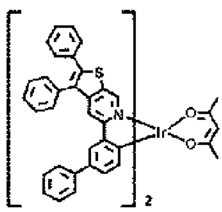
264
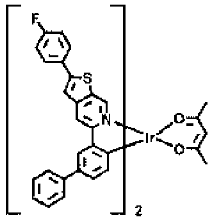
265

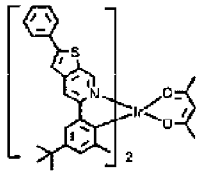
266
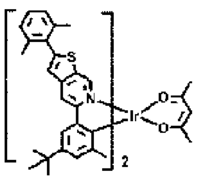
267
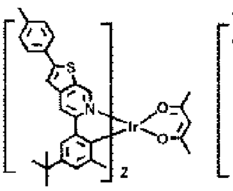
268
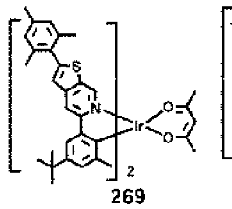
269
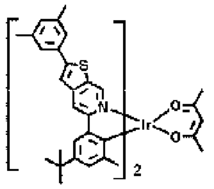
270

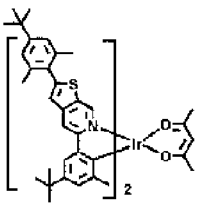
271
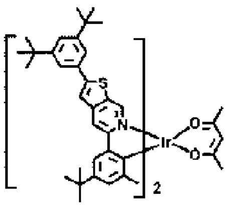
272
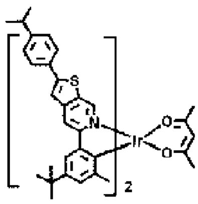
273
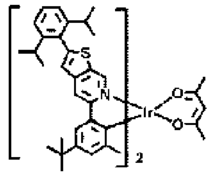
274
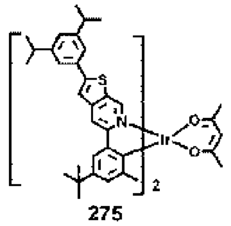
275

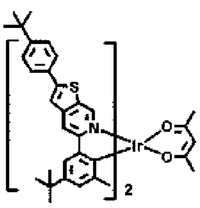
276
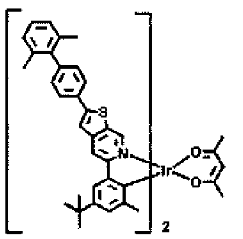
277
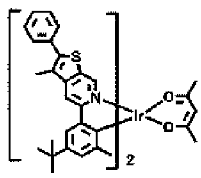
278
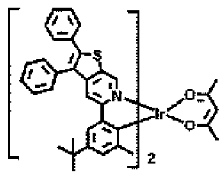
279
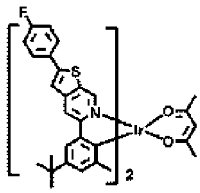
280

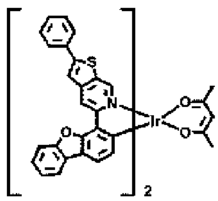
281
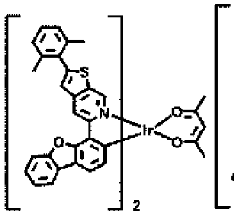
282
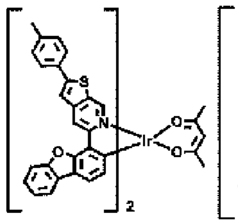
283
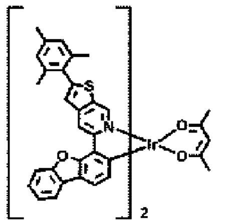
284
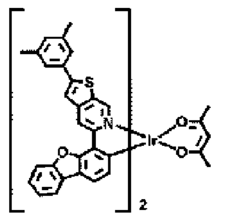
285

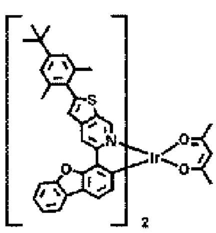
286
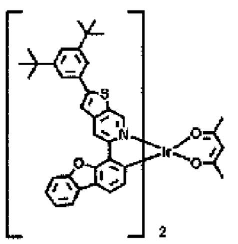
287
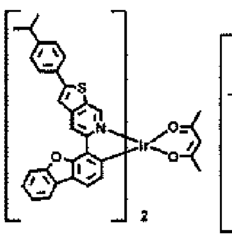
288
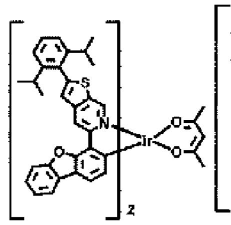
289
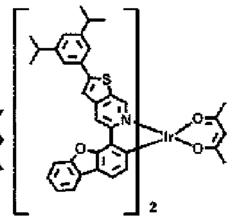
290

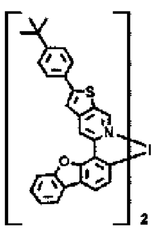
291
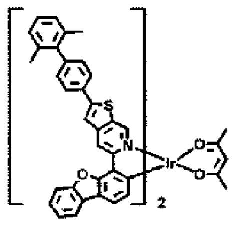
292
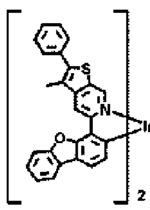
293
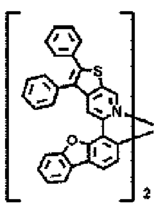
294
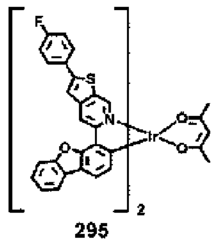
295

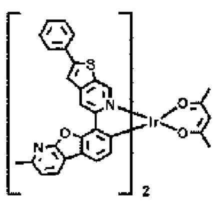
296
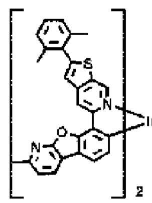
297
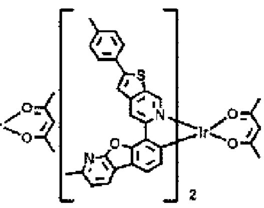
298
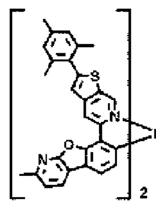
299
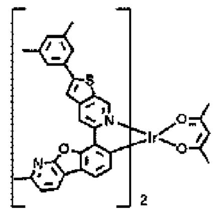
300

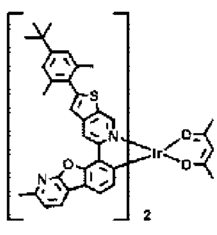
301
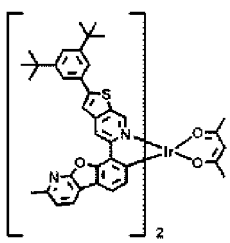
302
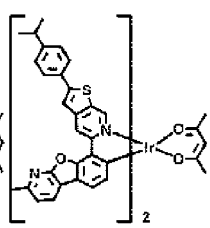
303
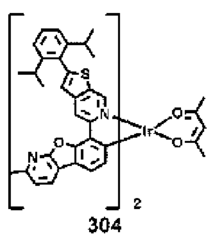
304
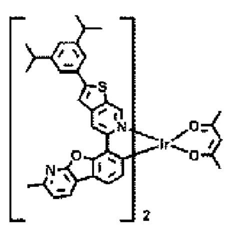
305

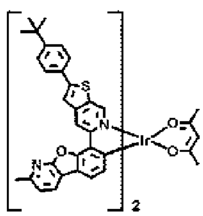
306
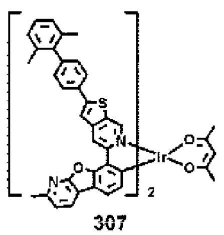
307
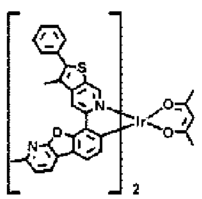
308
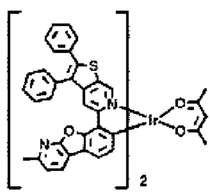
309
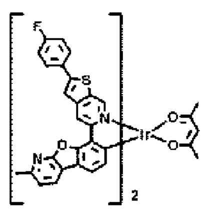
310

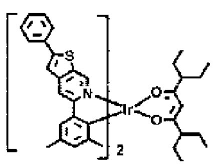
311
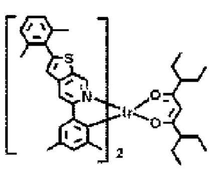
312
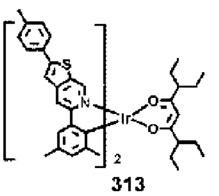
313
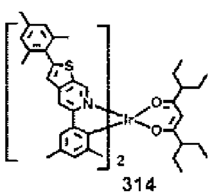
314
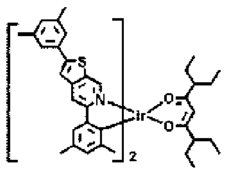
315

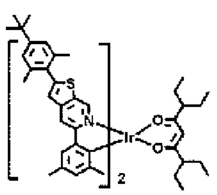
316
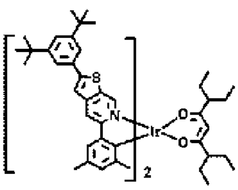
317
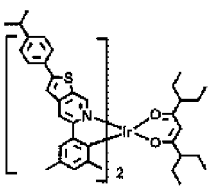
318
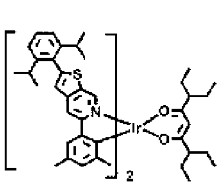
319
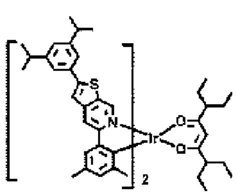
320

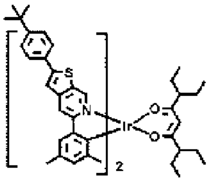
321
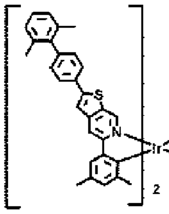
322
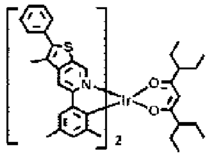
323
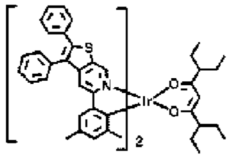
324
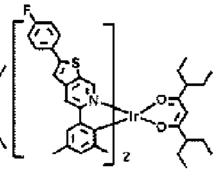
325

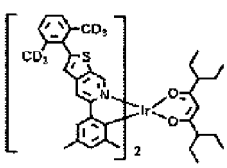
326
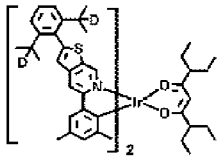
327
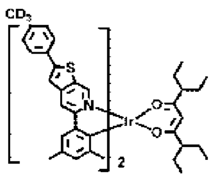
328
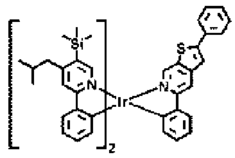
330

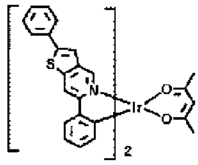
331
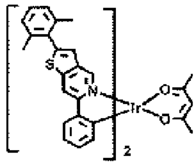
332
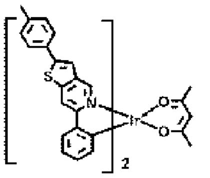
333
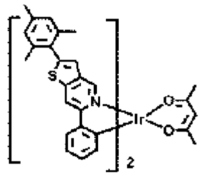
334
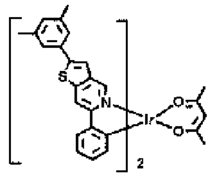
335

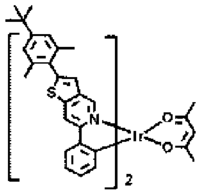
336
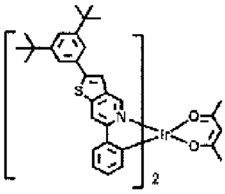
337
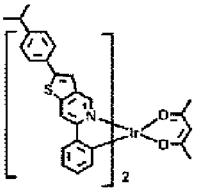
338
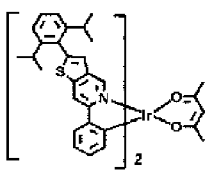
339
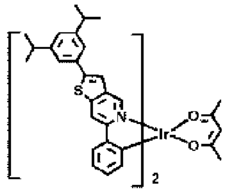
340

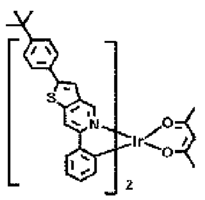
341
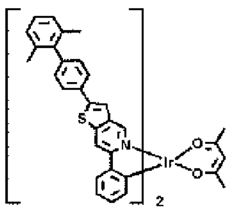
342
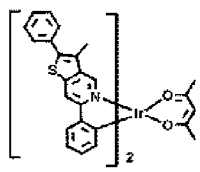
343
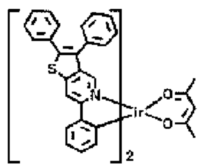
344
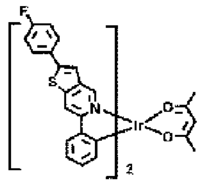
345

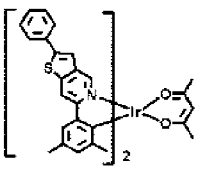
346
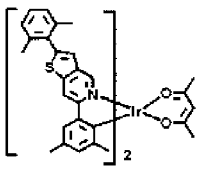
347
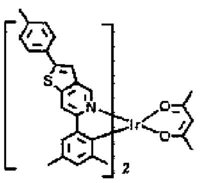
348
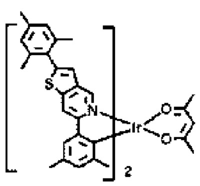
349
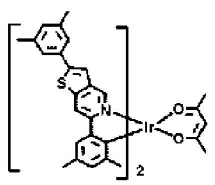
350

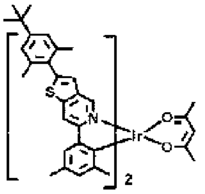
351
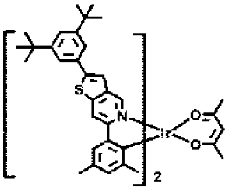
352
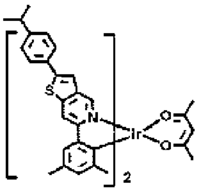
353
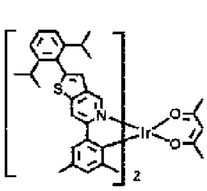
354
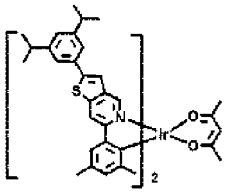
355

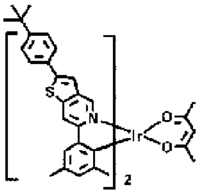
356
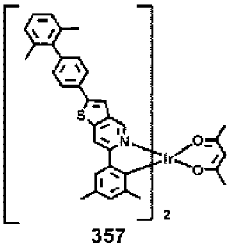
357
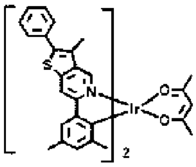
358
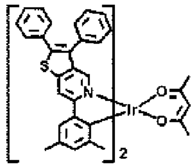
359
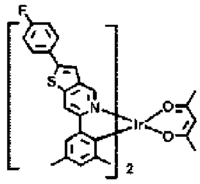
360

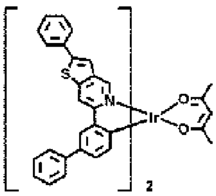
361
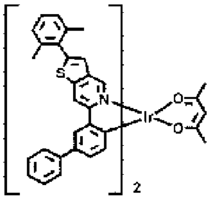
362
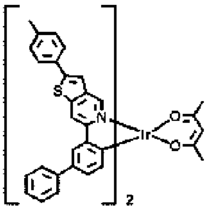
363
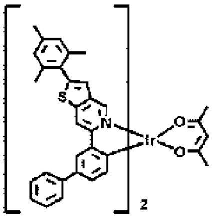
364
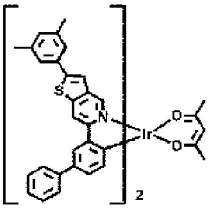
365

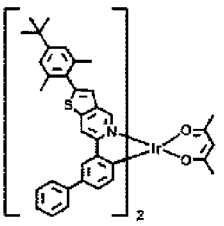
366
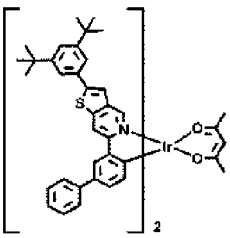
367
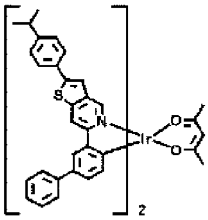
368
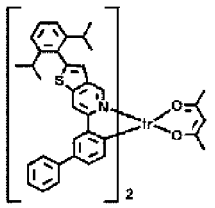
369
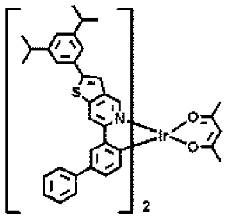
370

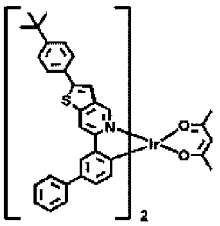
371
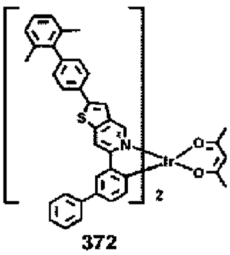
372
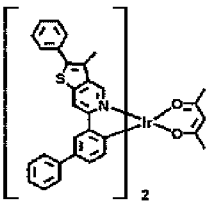
373
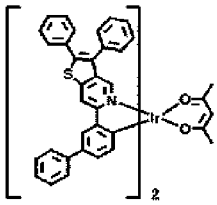
374
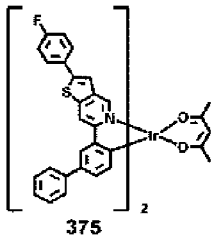
375

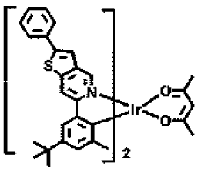
376
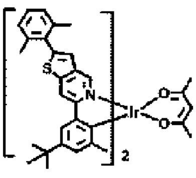
377
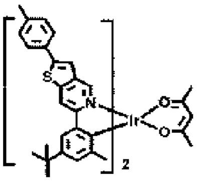
378
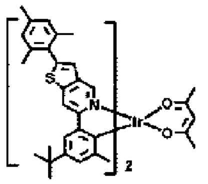
379
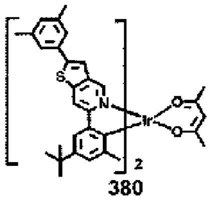
380

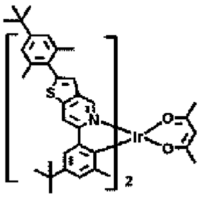
381

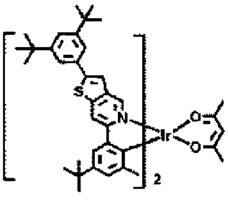
382

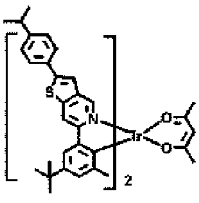
383

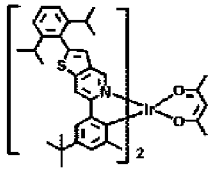
384

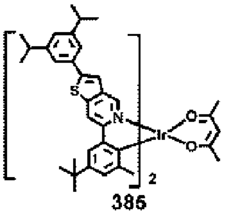
385

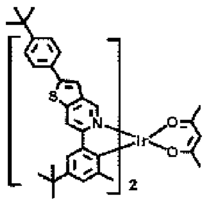
386

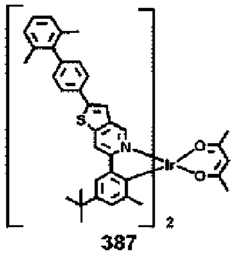
387

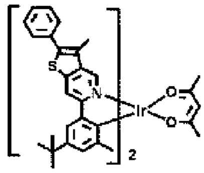
388

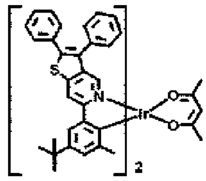
389

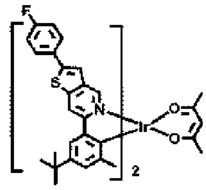
390

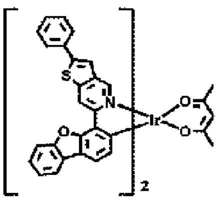
391

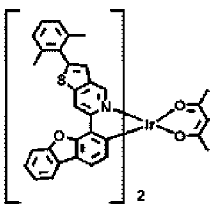
392

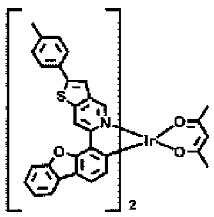
393

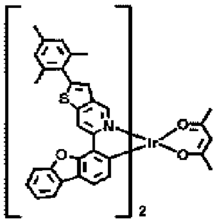
394

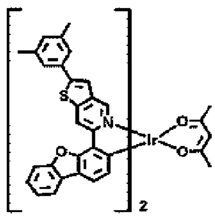
395

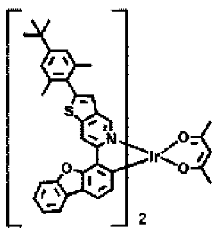
396

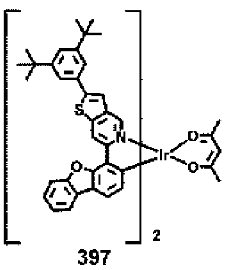
397

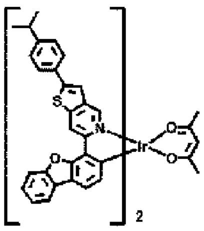
398

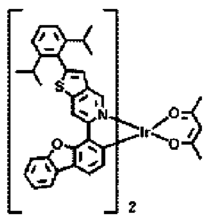
399

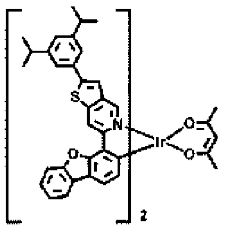
400

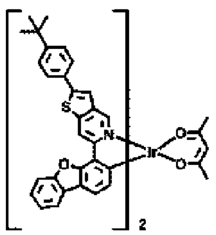
401

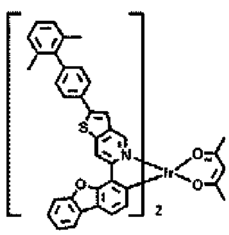
402

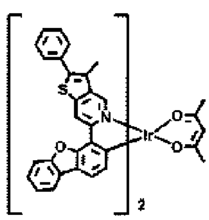
403

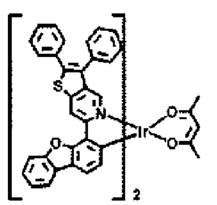
404

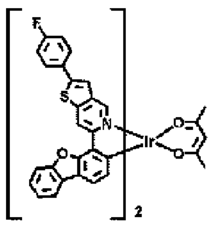
405

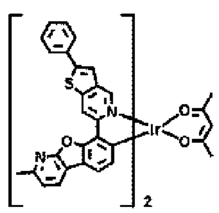
406

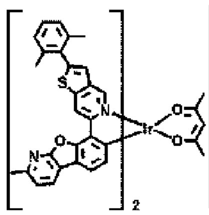
407

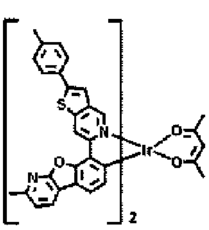
408

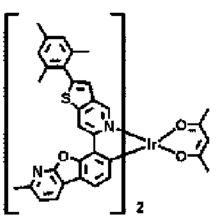
409

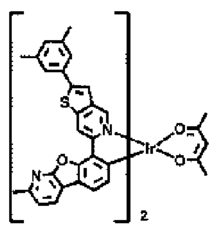
410

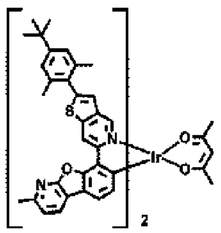
411
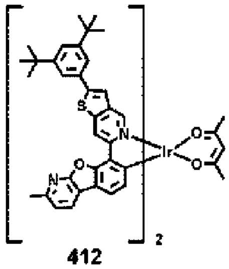
412
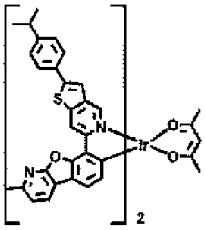
413
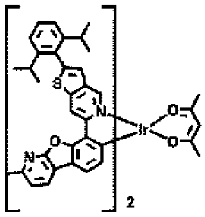
414
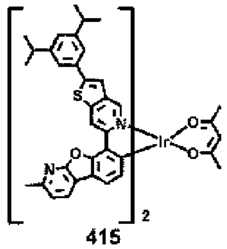
415

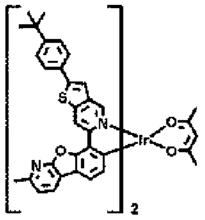
416
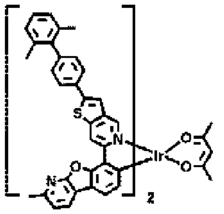
417
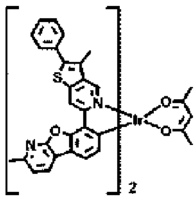
418
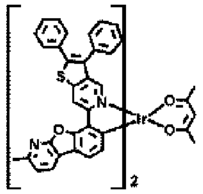
419
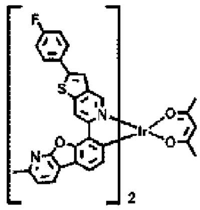
420

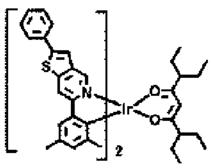
421
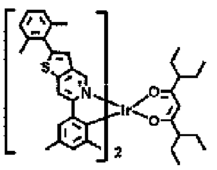
422
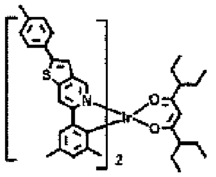
423
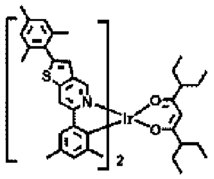
424
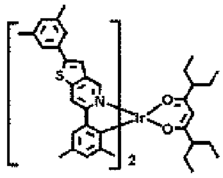
425

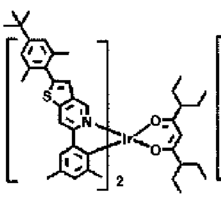
426
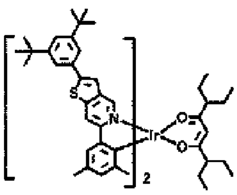
427
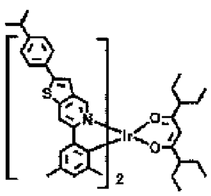
428
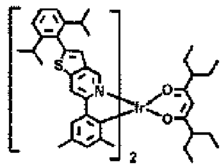
429
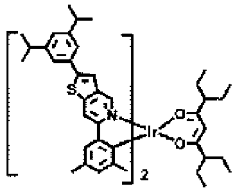
430

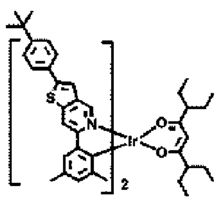
431
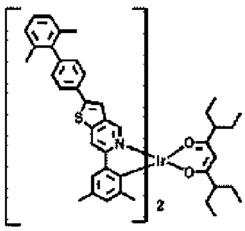
432
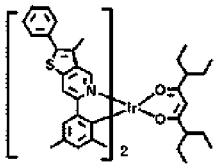
433
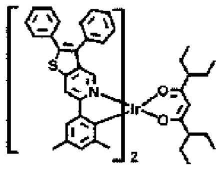
434
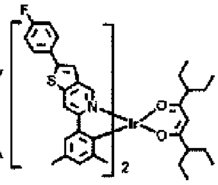
435

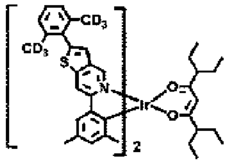
436
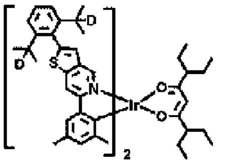
437
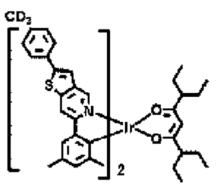
438
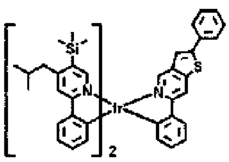
440

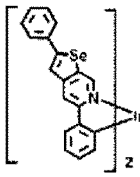
441

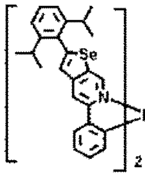
442

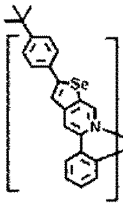
443

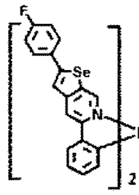
444

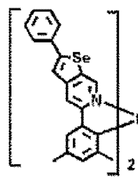
445

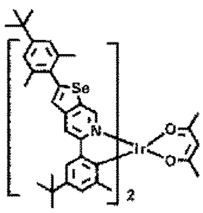
446

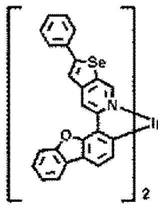
447

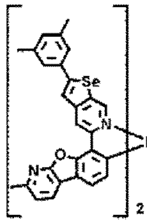
448

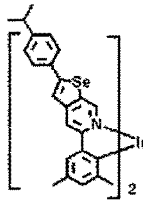
449

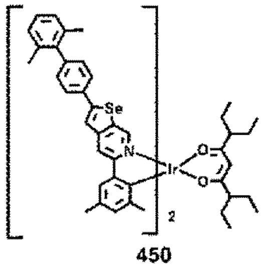
450

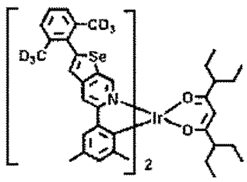
451

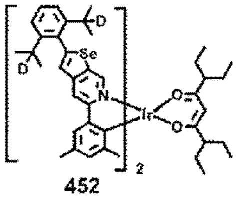
452

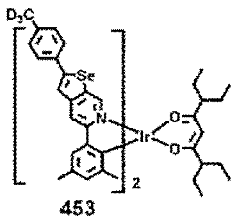
453

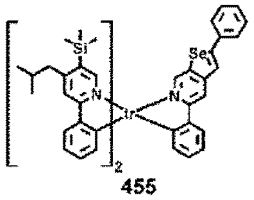
455

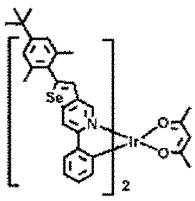
456

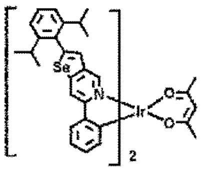
457

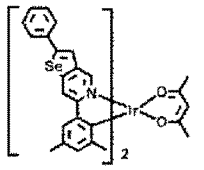
458

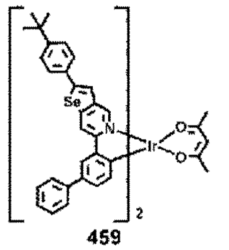
459

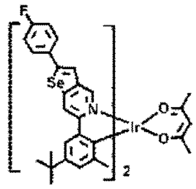
460

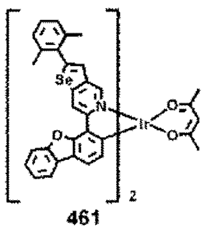
461

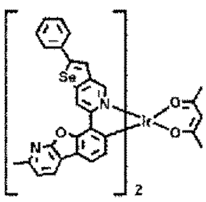
462

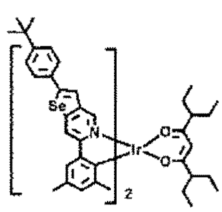
463

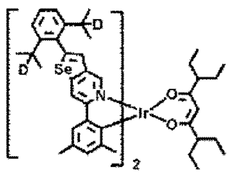
464

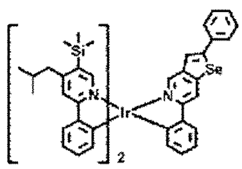
465

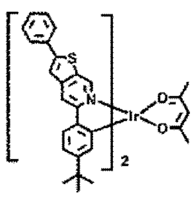
466

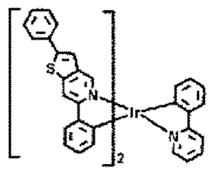
468

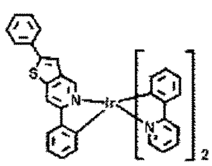
469

**12.** Dispositif organique émetteur de lumière comprenant :

une première électrode ;
une seconde électrode ; et
une couche organique disposée entre la première électrode et la seconde électrode et comprenant une couche d'émission,
la couche organique comprenant au moins un composé organométallique de l'une quelconque des revendications 1 à 11.

**13.** Dispositif organique émetteur de lumière de la revendication 12, dans lequel

la première électrode est une anode,
la seconde électrode est une cathode,
la couche organique comprend en outre une région de transport de trous entre la première électrode et la couche d'émission et une région de transport d'électrons entre la couche d'émission et la seconde électrode,
la région de transport de trous comprend une couche d'injection de trous, une couche de transport de trous, une couche de blocage d'électrons, une couche tampon ou une combinaison quelconque de celles-ci, et
la région de transport d'électrons comprend une couche de blocage de trous, une couche de transport d'électrons, une couche d'injection d'électrons, ou une combinaison quelconque de celles-ci ; et/ou
dans lequel la couche d'émission comprend le composé organométallique, de préférence dans lequel la couche d'émission comprend un hôte, et une quantité de l'hôte est plus grande qu'une quantité du composé organométallique.

**14.** Composition diagnostique comprenant le composé organométallique de l'une quelconque des revendications 1 à 11.

# FIGURE

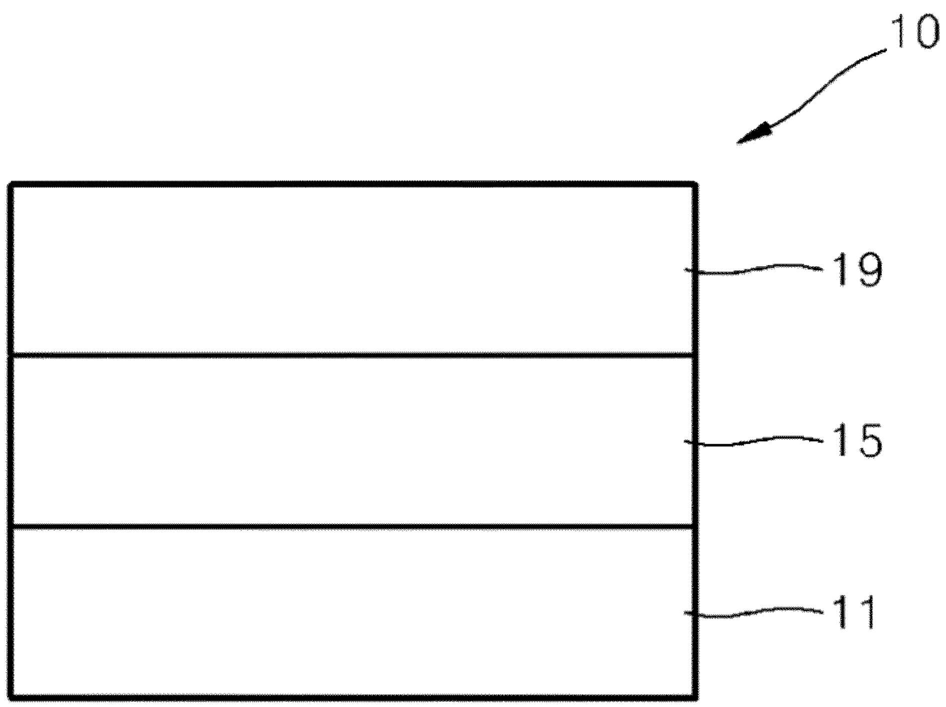

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3637489 A
- US 2016164007 A
- EP 3560940 A


### Non-patent literature cited in the description


- **T. LI.** Rational design of phosphorescent iridium(III) complexes for emission color tunability and their applications in OLEDs. *Coordination Chemistry Reviews,* 01 November 2018, vol. 374, 55-92